(19) Europäisches Patentamt
European Patent Office
Office européen des brevets

(11) EP 1 764 075 A1

(12) # EUROPEAN PATENT APPLICATION
published in accordance with Art. 158(3) EPC

(43) Date of publication:
**21.03.2007 Bulletin 2007/12**

(21) Application number: **05755860.3**

(22) Date of filing: **01.07.2005**

(51) Int Cl.:
***A61K 6/00*** *(2006.01)*

(86) International application number:
**PCT/JP2005/012185**

(87) International publication number:
**WO 2006/004030 (12.01.2006 Gazette 2006/02)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.07.2004 JP 2004196468**

(71) Applicant: **Sankyo Company, Limited**
**Tokyo 103-8426 (JP)**

(72) Inventors:
- **TERASAKA, Naoki,**
**SANKYO COMPANY, LIMITED**
**Shinagawa-ku, Tokyo 1408710 (JP)**
- **HIROSHIMA, Ayano,**
**SANKYO COMPANY, LIMITED**
**Shinagawa-ku, Tokyo 1408710 (JP)**

(74) Representative: **Gibson, Christian John Robert**
**Marks & Clerk**
**90 Long Acre**
**London WC2E 9RA (GB)**

(54) **TISSUE FACTOR PRODUCTION INHIBITOR**

(57) A medicament which has an activity of inhibiting production of tissue factor and comprises an LXR ligand as an active ingredient; and a medicament for treatment and/or prophylaxis of vascular restenosis following angioplasty, endarterectomy, percutaneous transluminal coronary angioplasty (PTCA) or stent implantation, or treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor or cancer metastasis, which comprises an LXR ligand as an active ingredient.

EP 1 764 075 A1

## Description

[Technical field]

The present invention relates to an inhibitor of production of tissue factor comprising a liver X receptor ligand.

[Background of the invention]

The number of cases of atherosclerosis is continuing to increase accompanying the Westernization of the diet and the growing size of the elderly population. Atherosclerosis is the primary cause of diseases such as ischemic heart disease (e.g. myocardial infarction and unstable angina pectoris), ischemic cerebral disease (e.g. cerebral infarction and cerebral hemorrhage) and peripheral circulatory insufficiency. In addition, examples of risk factors responsible for atherosclerosis include hyperlipemia (and particularly hypercholesterolemia), hypertension, and sugar metabolism disorders based on insulin resistance. Hyperlipemia not only has an activity of damaging vascular endothelial cells, but also supplies cholesterol which is deposited on vessel walls, thus making it important to control Hyperlipemia.

In the case of ischemic heart disease, and particularly acute coronary syndrome and unstable angina pectoris, tissue factor, which is an initiator of blood coagulation, has been determined to increase in plaque, thereby enhancing thrombus formation. Thrombotic disease is not only directly related to cause of death, but also has a poor prognosis and imposes numerous personal and social burdens in terms of restriction of activity in life and so forth. Thus, the importance of anti-coagulation therapy is believed to increase even more in the future as a method for treating these thromboses. In ischemic heart disease, intravascular surgery, namely percutaneous transluminal coronary angioplasty (PTCA), has been widely performed by using a catheter method to dilate the affected portions of occluded or constricted coronary arteries. However, since restenosis following PTCA has problems in terms of prognosis, there is still a need for the development of an effective method for treating or preventing the ischemic heart disease. If it were possible to inhibit the production of tissue factor, then it would be possible to decrease thrombus formation, which in turn would be expected to enable fundamental treatment of ischemic diseases. However, a medicament having an activity of inhibiting the production of tissue factor has not yet been provided.

On the other hand, since ATP binding cassette transporter Al (ABCA1) has activity of removing cholesterol deposited on vessel walls, an increase in the levels thereof is thought to prevent or ameliorate the progression of arteriosclerosis. Since an agonist against liver X receptor (abbreviated as LXR in this specification) has activity of increasing the expression of ABCA1, it is expected to be a novel antiarteriosclerotic medicament. However, the activity of LXR on the production of tissue factor has not been known.

[Patent document 1] International Patent Publication No. WO02/062302
[Patent document 2] International Patent Publication No. WO03/039480
[Patent document 3] International Patent Publication No. WO03/090746
[Patent document 4] International Patent Publication No. WO02/46141
[Patent document 5] International Patent Publication No. WO03/103651
[Patent document 6] International Patent Publication No. WO03/084544
[Patent document 7] International Patent Publication No. WO02/046181
[Patent document 8] International Patent Publication No. WO02/046172
[Patent document 9] International Patent Publication No. WO02/024632
[Patent document 10] International Patent Publication No. WO2004/009091
[Patent document 11] International Patent Publication No. WO03/031408
[Patent document 12] International Patent Publication No. WO03/045382
[Patent document 13] International Patent Publication No. WO03/053352
[Patent document 14] International Patent Publication No. WO2004/011448
[Patent document 15] International Patent Publication No. WO03/099769
[Patent document 16] International Patent Publication No. WO03/099775
[Patent document 17] International Patent Publication No. WO03/059874
[Patent document 18] International Patent Publication No. WO03/082192
[Patent document 19] International Patent Publication No. WO03/082802
[Patent document 20] International Patent Publication No. WO03/082205
[Patent document 21] International Patent Publication No. WO01/60818
[Patent document 22] International Patent Publication No. WO00/54759
[Patent document 23] International Patent Publication No. WO03/063796
[Patent document 24] International Patent Publication No. WO03/063576
[Patent document 25] International Patent Publication No. WO03/059884

[Patent document 26] International Patent Publication No. WO01/41704
[Patent document 27] International Patent Publication No. WO03/090869
[Patent document 28] International Patent Publication No. WO2004/024161
[Patent document 29] International Patent Publication No. WO2004/024162
[Patent document 30] International Patent Publication No. WO2004/026816
[Patent document 31] International Patent Publication No. WO03/090732
[Patent document 32] International Patent Publication No. W02004/043939
[Patent document 33] International Patent Publication No. WO2004/072041
[Patent document 34] International Patent Publication No. WO2004/072042
[Patent document 35] International Patent Publication No. WO02004/072046
[Patent document 36] International Patent Publication No. W02004/076418
[Patent document 37] International Patent Publication No. W02004/103376
[Patent document 38] International Patent Publication No. WO2005/005416
[Patent document 39] International Patent Publication No. W02005/005417
[Patent document 40] International Patent Publication No. WO2005/016277
[Patent document 41] International Patent Publication No. WO2005/023188
[Patent document 42] International Patent Publication No. WO2005/023196
[Patent document 43] International Patent Publication No. WO2005/023247
[Patent document 44] U.S. Patent Publication No. US2004/0152681
[Patent document 45] International Patent Publication No. WO03/106435
[Patent document 46] International Patent Publication No. WO2005/023782

[Disclosure of the invention]

An object of the present invention is to provide a medicament which has an activity of decreasing thrombus formation, and is useful for the treatment and/or prophylaxis of vascular restenosis following angioplasty, endarterectomy, percutaneous transluminal coronary angioplasty (PTCA) or stent implantation, or treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor or cancer metastasis. In addition, an object of the present invention is to provide a medicament having an activity of inhibiting production of tissue factor as a specific means thereof.

As a result of conducting extensive research to accomplish the aforementioned objects, the inventors of the present invention found that an LXR ligand such as an agonist or antagonist of LXR, has an activity of inhibiting the production of tissue factor and is able to decrease thrombus formation in the living bodies of warm-blooded animals, and that said LXR ligand is useful as an active ingredient of a medicament for the treatment and/or prophylaxis of vascular restenosis following angioplasty, endarterectomy, percutaneous transluminal coronary angioplasty or stent implantation, or treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor or cancer metastasis. The present invention was completed on the basis of these findings.

Namely, the present invention provides a medicament which has an activity of inhibiting production of tissue factor, and comprises an LXR ligand as an active ingredient. The medicament mentioned above has an activity which decreases thrombus formation in the living bodies of warm-blooded animals (including a human). In addition, the present invention provides a medicament for the treatment and/or prophylaxis of vascular restenosis following angioplasty, endarterectomy, percutaneous transluminal coronary angioplasty or stent implantation, or treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor or cancer metastasis, comprising an LXR ligand as an active ingredient. LXR agonists or LXR antagonists, for example, can be used as an LXR ligand, and an LXR agonist can be used preferably.

In a different aspect, the present invention provides a method for inhibiting the production of tissue factor in the living bodies of warm-blooded animals (including a human), comprising a step of administering an effective amount of an LXR ligand to a warm-blooded animal; a method for decreasing thrombus formation in the bodies of warm-blooded

animals (including a human), comprising a step of administering an effective amount of an LXR ligand to a warm-blooded animal; or a method for treatment and/or prophylaxis of vascular restenosis following angioplasty, endarterectomy, percutaneous transluminal coronary angioplasty or stent implantation, or a method for treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor or cancer metastasis, wherein the method decreases thrombus formation in the bodies of warm-blooded animals (including a human), comprising a step of administering an effective amount of an LXR ligand to a warm-blooded animal; and, the use of an LXR ligand for production of the aforementioned medicament.

[Effect of the invention]

A medicament of the present invention has an activity of inhibiting production of tissue factor, and has an activity of decreasing thrombus formation in the living bodies of warm-blooded animals. Thus, a medicament of the present invention is useful for the treatment and/or prophylaxis of vascular restenosis following angioplasty, endarterectomy, percutaneous transluminal coronary angioplasty (PTCA) or stent implantation, or treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor or cancer metastasis.

[Best mode for carrying out the invention]

In the present specification, the term "LXR ligand" means a substance having the property of binding to LXR as a ligand. This term is to be interpreted in the broadest sense, and includes LXR agonists or LXR antagonists, and should not be interpreted in a limiting manner in any meaning thereof. Any arbitrary substance can be used as an LXR ligand, examples of which include organic low molecular compounds, organic polymer compounds, inorganic compounds, and bio-related compounds such as proteins, nucleic acids, lipids, steroids and sugars. Organic low molecular compounds can preferably be used. LXR can be easily acquired by a person skilled in the art in the art in the form of a purified protein by a method described in, for example, International Patent Publication WO 03/106435, and whether or not a substance binds to LXR as a ligand can be easily confirmed by a person skilled in the art by a method described in, for example, International Patent Publication WO 03/106435, or a method similar thereto. More specifically, the activity of a compound as an LXR ligand can also be measured using the co-transfection assay described in the examples of the present specification. LXR agonists or LXR antagonists are preferable as LXR ligands, and LXR agonists are particularly preferable. Since an LXR ligand has an activity of inhibiting production of tissue factor regardless of the structure thereof, any substance can be used as an active ingredient of a medicament of the present invention provided it is known to be an LXR ligand or can be demonstrated to be an LXR ligand.

More specifically, the following compound or a pharmacologically acceptable salt or ester thereof described in International Patent Publication WO 03/106435, can be used as an LXR ligand:

a compound represented by the general formula (Ia)

[Chemical formula 1]

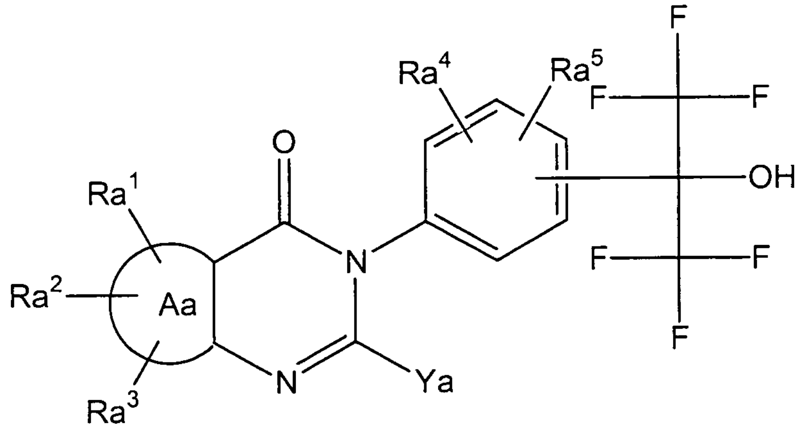

(Ia)

or a pharmacologically acceptable salt or ester thereof;

wherein $Ra^1$, $Ra^2$ and $Ra^3$ may be the same or different, and each represents a hydrogen atom, a hydroxyl group, a fluorine atom, a chlorine atom, a methyl group, an ethyl group, a trifluoromethyl group, a methoxy group, an ethoxy group or an acetylamino group, or $Ra^1$ and $Ra^2$ together represent a methylenedioxy group;
$Ra^4$ and $Ra^5$ may be the same or different, and each represents a hydrogen atom, a chlorine atom, a methyl group or a methoxy group;
Ya represents a benzyl group, a substituted benzyl group (said substituent is one group selected from the group consisting of a $C_1$-$C_6$ alkyl group, a $C_1$-$C_6$ alkoxy group and a halogeno group), a thienylmethyl group, a substituted thienylmethyl group (said substituent is one group selected from the group consisting of a $C_1$-$C_6$ alkyl group, a $C_1$-$C_6$ alkoxy group and a halogeno group), a pyridylmethyl group or a substituted pyridylmethyl group (said substituent is one group selected from the group consisting of a $C_1$-$C_6$ alkyl group, a $C_1$-$C_6$ alkoxy group and a halogeno group); and
Aa represents a phenyl group.

The "$C_1$-$C_6$ alkyl group" in the definition of the respective substituents of the general formula (Ia) is a straight or branched alkyl group having 1 to 6 carbon atoms and can include a methyl group, an ethyl group, a propyl group, an isopropyl group, a butyl group, an isobutyl group, a s-butyl group, a t-butyl group, a pentyl group, an isopentyl group, a neopentyl group, a t-pentyl group, a 1-methylbutyl group, a hexyl group, a 1-methylpentyl group, a 2-methylpentyl group, a 3-methylpentyl group, a 1-ethylbutyl group or a 2-ethylbutyl group, and is preferably a $C_1$-$C_4$ alkyl group such as a methyl group, an ethyl group, a propyl group, an isopropyl group, a butyl group, an isobutyl group, a s-butyl group and a t-butyl group, more preferably a methyl group, an ethyl group, a propyl group or an isopropyl group, and most preferably a methyl group or an ethyl group.

The "$C_1$-$C_6$ alkoxy group" in the definition of the respective substituents of the general formula (Ia) is a hydroxyl group substituted by the above $C_1$-$C_6$ alkyl group and can include a methoxy group, an ethoxy group, a 1-propoxy group, a 2-propoxy group, a 1-butoxy group, a 2-butoxy group, a 2-methyl-1-propoxy group, a 2-methyl-2-propoxy group, a 1-pentyloxy group, a 2-pentyloxy group, a 3-pentyloxy group, a 2-methyl-2-butoxy group, a 3-methyl-2-butoxy group, a 1-hexyloxy group, a 2-hexyloxy group, a 3-hexyloxy group, a 2-methyl-1-pentyloxy group, a 3-methyl-1-pentyloxy group, a 2-ethyl-1-butoxy group, a 2,2-dimethyl-1-butoxy group or a 2,3-dimethyl-1-butoxy group, and is preferably a $C_1$-$C_4$ alkoxy group, more preferably a methoxy group or an ethoxy group, and most preferably a methoxy group.

The "halogeno group" in the definition of the respective substituents of the general formula (Ia) can include a fluoro group, a chloro group, a bromo group or an iodo group, and is preferably a fluoro group, a chloro group or a bromo group, more preferably a fluoro group or a chloro group, and most preferably a fluoro group.

A compound represented by the general formula (Ia) can be prepared by a method described in International Patent Pubication W02003/106435.

The following compound or a pharmacologically acceptable salt or ester thereof published in International Patent Publication WO2005/023782 can be used as an LXR ligand:

a compound represented by the general formula (Ib)

[Chemical formula 2]

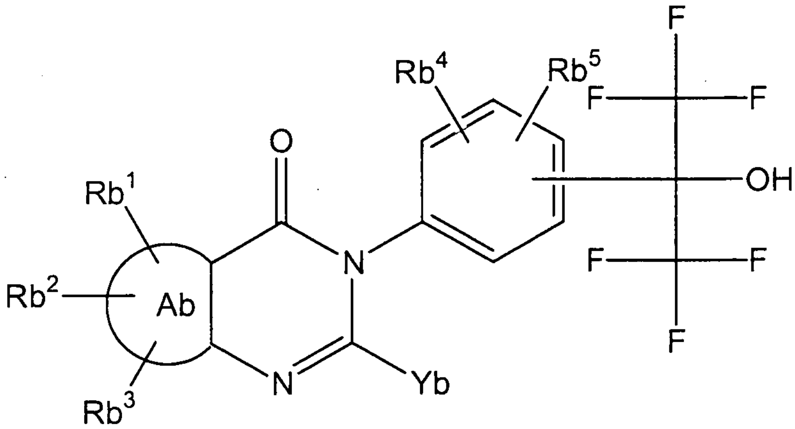

(Ib)

or a pharmacologically acceptable salt or ester thereof;

wherein Ab represents a phenyl group;
$Rb^1$ represents a 5- to 7-membered heterocyclyl group or a group represented by the formula: $-O-Rb^{1a}$ [wherein $Rb^{1a}$ represents a substituted $C_1$-$C_6$ alkyl group (said substituent(s) are the same or different and are 1 or 2 group(s) selected from the group consisting of a hydroxyl group, a hydroxymethoxy group, a hydroxyethoxy group, an amino group, a methylamino group and an ethylamino group)];
$Rb^2$ represents a hydrogen atom, a methyl group, a hydroxyl group, a methoxy group, an amino group, a fluoro group or a chloro group;
$Rb^3$ represents a hydrogen atom;
$Rb^4$ and $Rb^5$ are the same or different and each represents a hydrogen atom, a methyl group, an ethyl group, a methoxy group, a fluoro group or a chloro group; and
Yb represents a benzyl group, a substituted benzyl group (said substituent is one group selected from the group consisting of $C_1$-$C_6$ alkyl group, $C_1$-$C_6$ alkoxy group and a halogeno group), a thienylmethyl group, a substituted thienylmethyl group (said substituent is one group selected from the group consisting of $C_1$-$C_6$ alkyl group, $C_1$-$C_6$ alkoxy group and a halogeno group), a pyridylmethyl group or a substituted pyridylmethyl group (the substituent is one group selected from the group consisting of $C_1$-$C_6$ alkyl group, $C_1$-$C_6$ alkoxy group and a halogeno group).

The "$C_1$-$C_6$ alkyl group" in the definition of the respective substituents of the general formula (Ib) is a straight or branched alkyl group having 1 to 6 carbon atoms and can include a methyl group, an ethyl group, a 1-propyl group, a 2-propyl group, a 1-butyl group, a 2-butyl group, a-2-methyl-1-propyl group, a 2-methyl-2-propyl group, a 1-pentyl group, a 2-pentyl group, a 3-pentyl group, a 2-methyl-2-butyl group, a 3-methyl-2-butyl group, a 1-hexyl group, a 2-hexyl group, a 3-hexyl group, a 2-methyl-1-pentyl group, a 3-methyl-1-pentyl group, a 2-ethyl-1-butyl group, a 2,2-dimethyl-1-butyl group or a 2,3-dimethyl-1-butyl group, and is preferably a $C_1$-$C_4$ alkyl group, more preferably a methyl group or an ethyl group, and most preferably a methyl group.

The "5- to 7-membered heterocyclyl group" in the definition of the respective substituents of the general formula (Ib) is a 5- to 7-membered heterocyclic group containing 1 to 4 atoms selected from the group consisting of a nitrogen atom, an oxygen atom and a sulfur atom and can include an unsaturated heterocyclic group such as a furyl group, a thienyl group, a pyrrolyl group, a pyrazolyl group, an imidazolyl group, an oxazolyl group, an isoxazolyl group, a thiazolyl group, an isothiazolyl group, a 1,2,3-oxadiazolyl group, a 1,2,3-thiadiazolyl group, a triazolyl group, a tetrazolyl group, a pyranyl group, a pyridyl group, a pyridazinyl group, a pyrimidinyl group, a pyradinyl group and an azepinyl group, or a group in which the above unsaturated heterocyclic group is partially or completely reduced such as a pyrrolidinyl group, pyrrolinyl group, an imidazolidinyl group, an imidazolinyl group, a pyrazolidinyl group, a pyrazolinyl group, a piperidyl group, a piperazinyl group, a morpholinyl group, a thiomorpholinyl group and a perhydroazepinyl group, and is preferably an imidazolyl group, a triazolyl group, a pyridyl group or a morpholinyl group.

The "$C_1$-$C_6$ alkoxy group" in the definition of the respective substituents of the general formula (Ib) is a hydroxyl group substituted by the above $C_1$-$C_6$ alkyl group and can include a methoxy group, an ethoxy group, a 1-propoxy group, a 2-propoxy group, a 1-butoxy group, a 2-butoxy group, a 2-methyl-1-propoxy group, a 2-methyl-2-propoxy group, a 1-pentyloxy group, a 2-pentyloxy group, a 3-pentyloxy group, a 2-methyl-2-butoxy group, a 3-methyl-2-butoxy group, a 1-hexyloxy group, a 2-hexyloxy group, a 3-hexyloxy group, a 2-methyl-1-pentyloxy group, a 3-methyl-1-pentyloxy group, a 2-ethyl-1-butoxy group, a 2,2-dimethyl-1-butoxy group or a 2,3-dimethyl-1-butoxy group, and is preferably a $C_1$-$C_4$ alkoxy group, more preferably a methoxy group or an ethoxy group, and most preferably a methoxy group.

The "halogeno group" in the definition of the respective substituents of the general formula (Ib) can include a fluoro group, a chloro group, a bromo group or an iodo group, and is preferably a fluoro group, a chloro group or a bromo group, more preferably a fluoro group or a chloro group, and most preferably a fluoro group.

A compound represented by the general formula (Ib) can be prepared by a method described in International Patent Publication WO2005/023782.

Further, the following compound or a pharmacologically acceptable salt or ester thereof disclosed in PCT/JP2005/009142 specification and Japanese Patent Application No. 2005-146390 specification can be also used as an LXR ligand:

a compound represented by the general formula (Ic)

[Chemical formula 3]

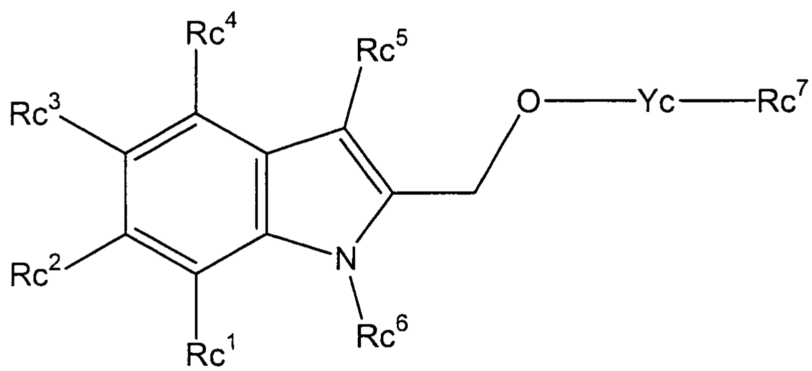

(Ic)

or a pharmacologically acceptable salt or ester thereof;

wherein $Rc^1$, $Rc^2$, $Rc^3$ and $Rc^4$ are the same or different and each represents a hydrogen atom, a $C_1$-$C_3$ alkyl group, a fluoromethyl group, a chloromethyl group, a difluoromethyl group, a trifluoromethyl group, a pentafluoroethyl group, a methoxy group, an ethoxy group, a fluoromethoxy group, a chloromethoxy group, a difluoromethoxy group, a trifluoromethoxy group, a pentafluoroethoxy group, a methanesulfonyl group, an ethanesulfonyl group, a fluoro group, a chloro group or a bromo group;

$Rc^5$ represents a hydrogen atom;

$Rc^6$ represents a group having the formula: $-CORc^8$ [wherein $Rc^8$ represents a $C_3$-$C_6$ alkoxy group or a halogeno $C_3$-$C_5$ alkoxy group (said halogeno $C_3$-$C_5$ alkoxy group represents a $C_3$-$C_5$ alkoxy group substituted with 1 to 5 fluoro or chloro groups)];

$Rc^7$ represents a group having the formula: - $N(Rc^{10})ZcRc^{11}$ [wherein $Rc^{10}$ represents a methyl group, an ethyl group or a cyclopropyl group, $Rc^{11}$ represents a $C_1$-$C_4$ alkyl group, a substituted $C_1$-$C_4$ alkyl group (said substituent is one group selected from Substituent group $\alpha$c) , a cyclopropyl- ($C_1$-$C_2$ alkyl) group, a $C_3$-$C_4$ cycloalkyl group or a $C_2$-$C_4$ alkenyl group, and Zc represents a group having the formula: -CO-, -CS- or $-SO_2-$];

Yc represents a phenyl group, a substituted phenyl group (said substituent is one group selected from Substituent group ($\beta$c), a pyridyl group or a substituted pyridyl group (said substituent is one group selected from Substituent group $\beta$c);

Substituent group $\alpha$c represents the group consisting of a hydroxyl group, a methoxy group, an ethoxy group, a fluoromethoxy group, a chloromethoxy group, a difluoromethoxy group, a trifluoromethoxy group, a pentafluoroethoxy group, a benzyloxy group, a phenyloxy group, an amino group, a methylamino group, an ethylamino group, a dimethylamino group, a diethylamino group, a dimethylaminocarbonyl group, a diethylaminocarbonyl group, a fluoro group and a chloro group; and

Substituent group $\beta$c represents the group consisting of a $C_1$-$C_4$ alkyl group, a halogeno $C_1$-$C_4$ alkyl group (said halogeno $C_1$-$C_4$ alkyl group represents a $C_1$-$C_4$ alkyl group substituted with 1 to 5 fluoro, chloro or bromo groups), a $C_1$-$C_4$ alkoxy group, a ($C_1$-$C_4$ alkoxy)carbonyl group, a cyano group, a nitro group, a fluoro group, a chloro group and a bromo group.

The "$C_1$-$C_3$ alkyl group" in the definition of the respective substituents of the general formula (Ic) is a straight or branched alkyl group having 1 to 3 carbon atoms and can include a methyl group, an ethyl group, a 1-propyl group

or a 2-propyl group, and is preferably a methyl group or an ethyl group.

The "$C_3$-$C_6$ alkoxy group" in the definition of the respective substituents of the general formula (Ic) is a hydroxyl group substituted by a $C_3$-$C_6$ alkyl group and can include a 1-propoxy group, a 2-propoxy group, a 1-butoxy group, a 2-butoxy group, a 2-methyl-1-propoxy group, a 2-methyl-2-propoxy group, a 1-pentyloxy group, a 2-pentyloxy group, a 3-pentyloxy group, a 2-methyl-2-butoxy group, a 3-methyl-2-butoxy group, a 1-hexyloxy group, a 2-hexyloxy group, a 3-hexyloxy group, a 2-methyl-1-pentyloxy group, a 3-methyl-1-pentyloxy group, a 2-ethyl-1-butoxy group, a 2,2-dimethyl-1-butoxy group or a 2,3-dimethyl-1-butoxy group, and is preferably a 2-propoxy group or a 2-methyl-2-propoxy group.

The "halogeno $C_3$-$C_5$ alkoxy group" in the definition of the respective substituents of the general formula (Ic) is a $C_3$-$C_5$ alkoxy group substituted with 1 to 5 fluoro or chloro groups and can include a 3-fluoropropoxy group, a 3-chloropropoxy group, a 4-fluorobutoxy group or a 5-fluoropentyloxy group.

The "$C_1$-$C_4$ alkyl group" in the definition of the respective substituents of the general formula (Ic) is a straight or branched alkyl group having 1 to 4 carbon atoms and can include a methyl group, an ethyl group, a 1-propyl group, a 2-propyl group, a 1-butyl group, a 2-butyl group, a 2-methyl-1-propyl group or a 2-methyl-2-propyl group, and is preferably a methyl group or an ethyl group, and most preferably a methyl group.

The "cyclopropyl-($C_1$-$C_2$ alkyl) group" in the definition of the respective substituents of the general formula (Ic) can include a cyclopropylmethyl group or a cyclopropylethyl group, and is preferably a cyclopropylmethyl group.

The "$C_3$-$C_4$ cycloalkyl group" in the definition of the respective substituents of the general formula (Ic) can include a cyclopropyl group or a cyclobutyl group, and is preferably a cyclopropyl group.

The "$C_2$-$C_4$ alkenyl group" in the definition of the respective substituents of the general formula (Ic) is an alkenyl group having 1 or 2 carbon-carbon double bonds and 2 to 4 carbon atoms and can include a vinyl group, a 2-propenyl group, a 2-butenyl group, a 1,3-butadien-1-yl group or a 2-methyl-2-propenyl group, and is preferably a vinyl group, a 2-propenyl group or a 2-butenyl group.

The "halogeno $C_1$-$C_4$ alkyl group" in the definition of the respective substituents of the general formula (Ic) is the above $C_1$-$C_4$ alkyl group substituted with 1 to 5 fluoro, chloro or bromo groups and can include a fluoromethyl group, a difluoromethyl group, a dichloromethyl group, a dibromomethyl group, a trifluoromethyl group, a trichloromethyl group, a 2-fluoroethyl group, a 2-bromoethyl group, a 2-chloroethyl group, a 2-iodoethyl group, a 2,2-difluoroethyl group, a 2,2,2-trifluoroethyl group, a trichloroethyl group, a pentafluoroethyl group, a 3-fluoropropyl group, a 3-chloropropyl group or a 4-fluorobutyl group, and is preferably a fluoromethyl group, a chloromethyl group, a difluoromethyl group, a trifluoromethyl group or a pentafluoroethyl group, and most preferably a trifluoromethyl group.

The "$C_1$-$C_4$ alkoxy group" in the definition of the respective substituents of the general formula (Ic) is a hydroxyl group substituted by the above $C_1$-$C_4$ alkyl group and can include a methoxy group, an ethoxy group, a 1-propoxy group, a 2-propoxy group, a 1-butoxy group, a 2-butoxy group, a 2-methyl-1-propoxy group or a 2-methyl-2-propoxy group, and is preferably a methoxy group or an ethoxy group, and most preferably a methoxy group.

The "($C_1$-$C_4$ alkoxy)carbonyl group" in the definition of the respective substituents of the general formula (Ic) is a carbonyl group (-CO-) substituted by the above $C_1$-$C_4$ alkoxy group and can include a methoxycarbonyl group, an ethoxycarbonyl group, a 1-propoxycarbonyl group, a 2-propoxycarbonyl group, a 1-butoxycarbonyl group, a 2-butoxycarbonyl group, a 2-methyl-1-propoxycarbonyl group or a 2-methyl-2-propoxycarbonyl group, and is preferably a methoxycarbonyl group or an ethoxycarbonyl group, and most preferably a methoxycarbonyl group.

A compound represented by the general formula (Ic) can be prepared according to the following Method Ac or Method Bc described in PCT/JP2005/009142 specification.

[Chemical formula 4]

Method A

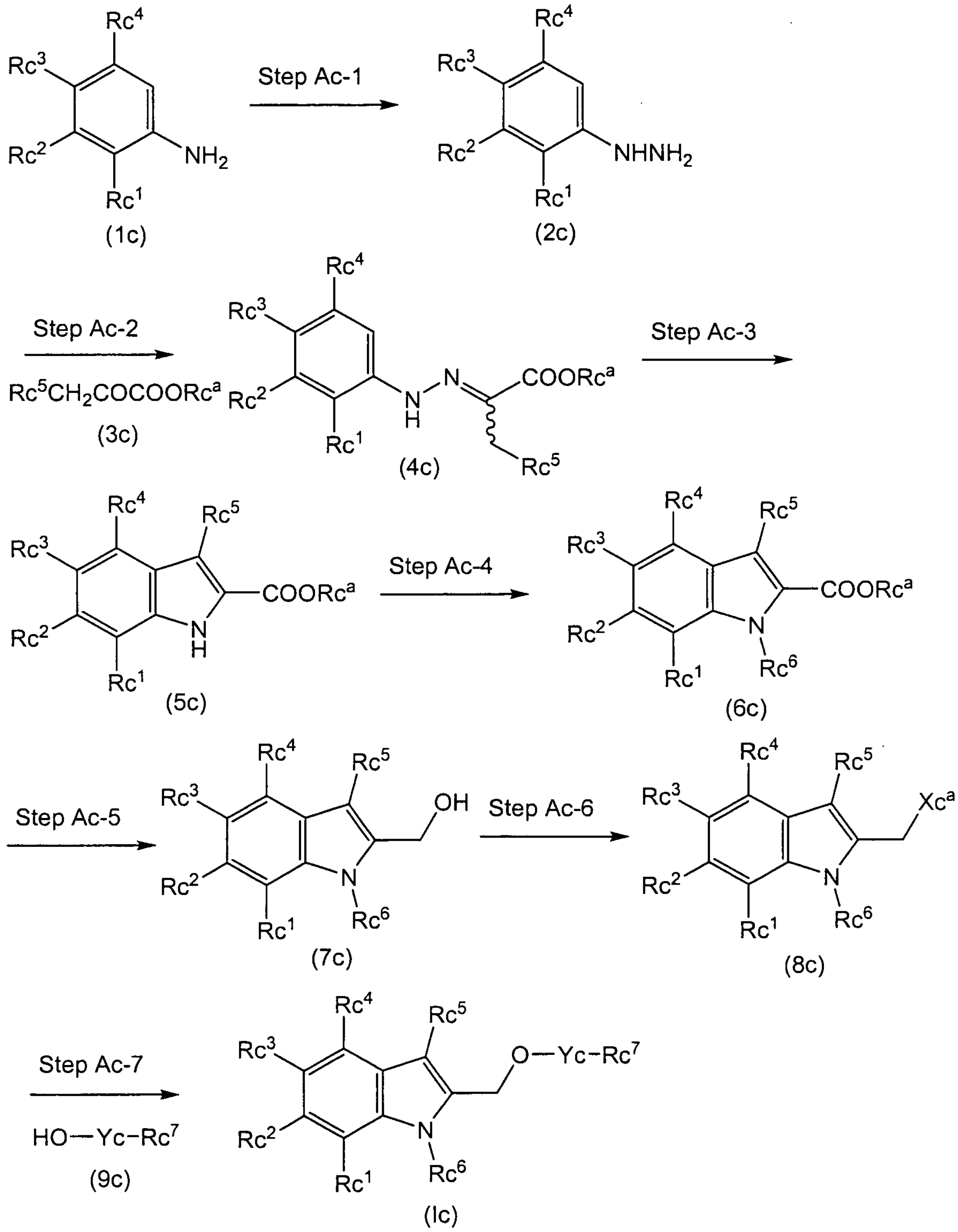


[Chemical formula 5]

Method Bc

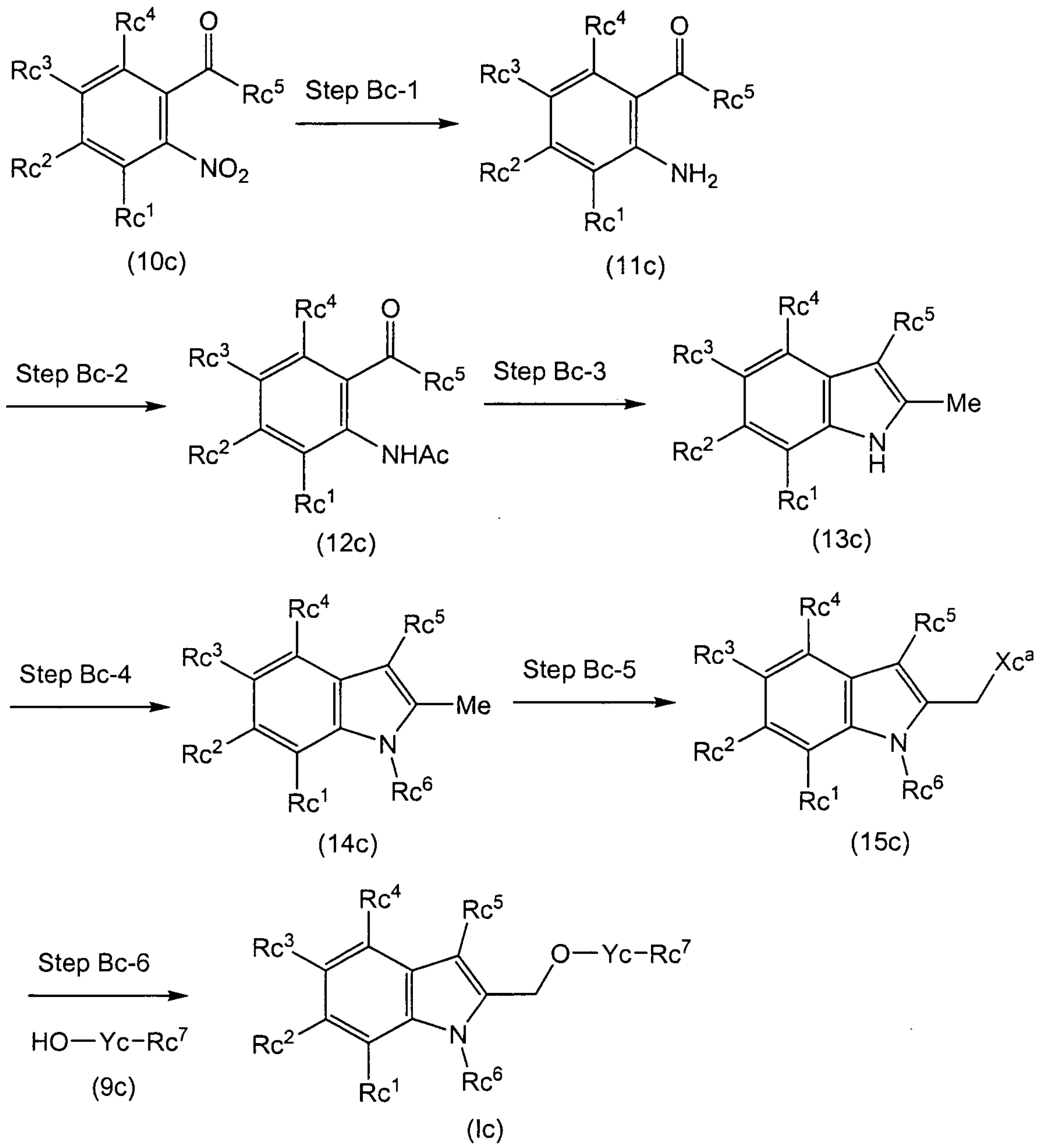


In the structural formulae of the compounds of the above Method Ac or Method Bc, $Rc^1$, $Rc^2$, $Rc^3$, $Rc^4$, $Rc^5$, $Rc^6$, $Rc^7$ and Yc have the same meanings as defined above; $Rc^a$ represents a $C_1$-$C_6$ alkyl group; and $Xc^a$ represents a chloro group, a bromo group or an iodo group.

In the reactions of the following Method Ac or Method Bc, in cases where the compound becoming the reaction substrate has a group which inhibits the desired reaction such as an amino group, a hydroxyl group or a carboxyl group, introduction of a protective group to those groups may be appropriately carried out, if necessary, and further, removal of the introduced protective group may be appropriately carried out, if necessary, (for example, T. H. Greene, P. G. Wuts, Protective Groups in Organic Synthesis. Third Edition. 1999, John Wiley & Sons. Inc., etc.).

(Method Ac)

Method Ac is a method to prepare the compound (Ic).

(Step Ac-1)

Step Ac-1 is a step to prepare the compound (2c) by reducing a diazonium salt, which is obtained by reacting

the compound (1c), which is publicly known or easily obtained from publicly known compounds, with a nitrite in the presence of an acid, with a reducing agent.

The nitrite used can be, for example, an alkali metal nitrite, and is preferably sodium nitrite.

The acid used can be, for example, an inorganic acid, and is preferably hydrochloric acid.

The reducing agent employed can be, for example, a metal chloride, and is preferably tin chloride (II).

The solvent used is preferably an organic acid, water or a mixture thereof, more preferably water.

The reaction temperature is preferably from -30 to 30˚C.

The reaction time is preferably from 30 minutes to 3 hours.

(Step Ac-2)

Step Ac-2 can be

(Step Ac-2a): a step to prepare the compound (4c) by reacting the compound (2c) obtained in Step Ac-1 with the compound (3c) which is publicly known or easily obtained from publicly known compounds.

Step Ac-2 can also be carried out as the following steps: (Step Ac-2b): a step of reacting the compound (2c) obtained in Step Ac-1 with a compound having the formula:

$Rc^5CH_2COCOOH$ which is publicly known or easily obtained from publicly known compounds; and

(Step Ac-2c): a step of preparing the compound (4c) by subsequently reacting the compound obtained in Step Ac-2b with a compound having the formula: $Rc^aOH$ in the presence of an acid.

In Step Ac-2a and Step Ac-2b, the solvent used is preferably an aromatic hydrocarbon, more preferably benzene or toluene.

The reaction temperature is preferably from 50 to 100˚C.

The reaction time is preferably from 30 minutes to 3 hours.

In Step Ac-2c, the acid used can be, for example, an inorganic acid, and is preferably hydrochloric acid or sulfuric acid.

The solvent used is preferably an alcohol having the formula: $Rc^aOH$ .

The reaction temperature is preferably from 50 to 100˚C.

The reaction time is preferably from 30 minutes to 3 hours.

(Step Ac-3)

Step Ac-3 is a step to prepare the compound (5c) by reacting the compound (4c) obtained in Step Ac-2 in the presence of an acid.

The acid used can be, for example, an inorganic acid, and is preferably sulfuric acid or polyphosphoric acid.

The solvent used is preferably an aliphatic hydrocarbon, an aromatic hydrocarbon or a mixture of these, and more preferably toluene or xylene.

The reaction temperature is preferably from 70 to 150˚C.

The reaction time is preferably from 12 hours to 48 hours.

(Step Ac-4)

Step Ac-4 is a step to prepare the compound (6c) by reacting the compound (5c) obtained in Step Ac-3 with a compound having the formula: $(Rc^8CO_2)O$ or $Rc^8COCl$ in the presence of a base.

The base used can be, for example, an alkali metal hydroxide, an organic amine or a mixture of these, and is preferably triethylamine, 4-(N,N-dimethylamino)pyridine or a mixture of these.

The solvent used is preferably a halogenated hydrocarbon or an ether, more preferably methylene chloride.

The reaction temperature is preferably from 0 to 50˚C.

The reaction time is preferably from 30 minutes to 15 hours.

(Step Ac-5)

Step Ac-5 is a step to prepare the compound (7c) by reducing the compound (6c) obtained in Step Ac-4.

The reducing agent used can be, for example, a hydrogenated organic aluminium compound, and is preferably di(isobutyl)aluminium hydride.

The solvent used is preferably an aromatic hydrocarbon or an ether, more preferably toluene, diethyl ether or tetrahydrofuran.

The reaction temperature is preferably from -70 to 30˚C.

The reaction time is preferably from 30 minutes to 3 hours.

(Step Ac-6)

Step Ac-6 is a step to prepare the compound (8c) by reacting the compound (7c) obtained in Step Ac-5 with a halogenating reagent.
The halogenating reagent used can be, for example, a combination of carbon tetrachloride or carbon tetrabromide and triphenylphosphine, and is preferably the combination of carbon tetrabromide and triphenylphosphine.
The solvent used is preferably an ether, more preferably tetrahydrofuran.
The reaction temperature is preferably from -20 to 40˚C.
The reaction time is preferably from 10 minutes to 3 hours.

(Step Ac-7)

Step Ac-7 is a step to prepare the compound (Ic) by reacting the compound (8c) obtained in Step Ac-6 with the compound (9c), which is publicly known or easily obtained from publicly known compounds, in the presence of a base.
The base used can be, for example, an alkali metal carbonate or an alkali metal hydrogencarbonate, and is preferably sodium carbonate, potassium carbonate or cesium carbonate.
The solvent used is preferably an amide, more preferably dimethylformamide or dimethylacetamide.
The reaction temperature is preferably from 20 to 60˚C.
The reaction time is preferably from 30 minutes to 15 hours.

(Method Bc)

Method Bc is a method to prepare the compound (Ic).

(Step Bc-1)

Step Bc-1 is a step to prepare the compound (11c) by subjecting the compound (10c), which is publicly known or easily obtained from publicly known compounds, to a hydrogenation reaction in the presence of a catalyst.
The catalyst used is preferably palladium-carbon or palladium hydroxide-carbon.
The present step is usually carried out under a hydrogen atmosphere of from atmospheric pressure to 10,000 hPa, preferably from atmospheric pressure to 5,000 hPa.
The solvent used is preferably an alcohol, more preferably methanol or ethanol.
The reaction temperature is preferably from 20 to 50˚C.
The reaction time is preferably from 30 minutes to 3 hours.

(Step Bc-2)

Step Bc-2 is a step to prepare the compound (12c) by acetylating the compound (11c) obtained in Step Bc-1.
The acetylating reagent used can be, for example, acetic anhydride or acetyl chloride, and is preferably acetic anhydride.
In the present step, a base may be appropriately used, if necessary. The base used is preferably pyridine.
The solvent used is preferably an alcohol, more preferably methanol or alcohol.
The reaction temperature is preferably from 20 to 70˚C.
The reaction time is preferably from 30 minutes to 3 hours.

(Step Bc-3)

Step Bc-3 is a step to prepare the compound (13c) by treating the compound (12c) obtained in Step Bc-2 with a metal reagent.
The metal reagent used is preferably a combination of titanium trichloride and zinc or a combination of titanium tetrachloride and zinc.
The solvent used is preferably an ether, more preferably tetrahydrofuran.
The reaction temperature is preferably from 50 to 80˚C.
The reaction time is preferably from 30 minutes to 3 hours.

(Step Bc-4)

Step Bc-4 is a step to prepare the compound (14c) using the compound (13c) obtained in Step Bc-3.

Step Bc-4 can be carried out according to a method similar to Step Ac-4.

(Step Bc-5)

Step Bc-5 is a step to prepare the compound (15c) by reacting the compound (14c) obtained in Step Bc-4 with a halogenating reagent.

The halogenating reagent used can be, for example, an N-halogeno succinimide, and is preferably N-bromosuccinimide.

In the present step, an organic peroxide may be appropriately used, if necessary. The organic peroxide used is preferably benzoyl peroxide.

The reaction of the present step is preferably carried out under light-shielding conditions.

The solvent used is preferably a halogenated hydrocarbon, more preferably carbon tetrachloride.

The reaction temperature is preferably from 20 to 90˚C.

The reaction time is preferably from 30 minutes to 3 hours.

(Step Bc-6)

Step Bc-6 is a step to prepare the compound (Ic) by reacting the compound (15c) obtained in Step Bc-5 with the compound (9c), which is publicly known or easily obtained from publicly known compounds, in the presence of a base.

Step Bc-6 can be carried out according to a method similar to Step Ac-7.

Further, the following compound or a pharmacologically acceptable salt or ester thereof disclosed in PCT/JP2005/011928 specification and Japanese Patent Application No. 2005-189264 specification can be also used as an LXR ligand:

a compound represented by the formula (1d)

[Chemical formula 6]

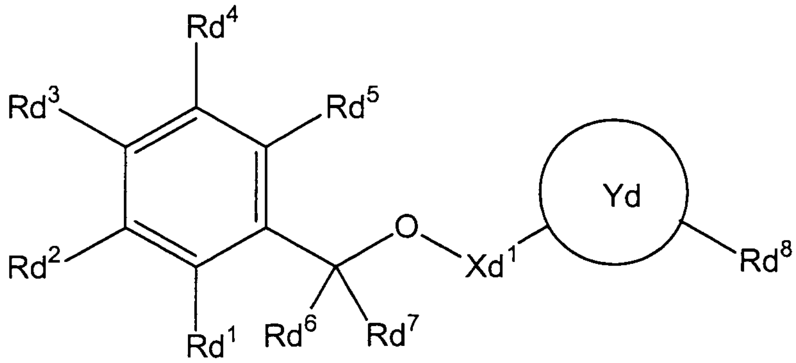

(Id)

or a pharmacologically acceptable salt or ester thereof;

wherein $Rd^1$ represents a group having the formula: - $CORd^9$ [wherein $Rd^9$ represents a $C_1$-$C_6$ alkoxy group or a halogeno $C_1$-$C_4$ alkoxy group (said halogeno $C_1$-$C_4$ alkoxy group represents a $C_1$-$C_4$ alkoxy group substituted with 1 to 5 halogeno groups)];

$Rd^2$ represents a hydrogen atom, a trifluoromethyl group, a 2,2,2-trifluoroethyl group, a pentafluoroethyl group, a hydroxyl group, a fluoro group or a chloro group;

$Rd^3$ represents a $C_1$-$C_4$ alkyl group, a halogeno $C_1$-$C_4$ alkyl group (said halogeno $C_1$-$C_4$ alkyl group represents a $C_1$-$C_4$ alkyl group substituted with 1 to 5 halogeno groups), a $C_3$-$C_5$ cycloalkyl group, a $C_2$-$C_4$ alkenyl group, a $C_1$-$C_4$ alkoxy group, a fluoro group or a chloro group;

$Rd^4$ and $Rd^5$ represent a hydrogen atom;

$Rd^6$ and $Rd^7$ represent a hydrogen atom;

$Rd^8$ represents a group having the formula: - $N(Rd^{10})ZdRd^{11}$ [wherein $Rd^{10}$ represents a methyl group, an ethyl group, a 1-propyl group or a 2-propyl group, $Rd^{11}$ represents a $C_1$-$C_4$ alkyl group, a substituted $C_1$-$C_4$ alkyl group (said substituent is one group selected from Substituent group αd), a ($C_3$-$C_4$ cycloalkyl)methyl group, a $C_3$-$C_4$ cycloalkyl group or a vinyl group, and Zd represents a group having the formula: -CO-, -CS- or $-SO_2-$];

$Xd^1$ represents a single bond;

Yd represents a phenyl group, a substituted phenyl group (said substituent is one group selected from Substituent group βd) or a pyridyl group;
Substituent group αd represents the group consisting of a methoxy group, a methylthio group, a methylamino group and a dimethylamino group; and
Substituent group βd represents the group consisting of a methoxy group, a methylamino group, a dimethylamino group, a fluoro group and a chloro group.

The "$C_1$-$C_6$ alkoxy group" in the definition of the respective substituents of the general formula (Id) is a hydroxyl group substituted by a $C_1$-$C_6$ alkyl group similar to that described above and can include a methoxy group, an ethoxy group, a 1-propoxy group, a 2-propoxy group, a 1-butoxy group, a 2-butoxy group, a 2-methyl-1-propoxy group, a 2-methyl-2-propoxy group, a 1-pentyloxy group, a 2-pentyloxy group, a 3-pentyloxy group, a 2-methyl-2-butoxy group, a 3-methyl-2-butoxy group, a 2-methyl-2-butoxy group, a 1-hexyloxy group, a 2-hexyloxy group, a 3-hexyloxy group, a 2-methyl-1-pentyloxy group, a 3-methyl-3-pentyloxy group, a 2-ethyl-1-butoxy group or a 2,3-dimethyl-1-butoxy group, and is preferably a $C_2$-$C_6$ alkoxy group, more preferably a $C_3$-$C_6$ alkoxy group, further preferably a $C_3$-$C_5$ alkoxy group (particularly a 2-propoxy group, a 2-methyl-2-propoxy group or a 2-methyl-2-butoxy group), and most preferably a 2-methyl-2-propoxy group.

The "halogeno $C_1$-$C_4$ alkoxy group" in the definition of the respective substituents of the general formula (Id) is a $C_1$-$C_4$ alkoxy group substituted with 1 to 5 halogeno groups similar to those described above and can include a fluoromethoxy group, a difluoromethoxy group, a dichloromethoxy group, a dibromomethoxy group, a trifluoromethoxy group, a trichloromethoxy group, a 2-fluoroethoxy group, a 2-bromoethoxy group, a 2-chloroethoxy group, a 2-iodoethoxy group, a 2,2-difluoroethoxy group, a 2,2,2-trifluoroethoxy group, a 2,2,2-trichloroethoxy group, a pentafluoroethoxy group, a 3,3,3-trifluoro-1-propoxy group, a 1,1,1-trifluoro-2-propoxy group, a 1,1,1-trichloro-2-propoxy group, a 4,4,4-trifluoro-1-butoxy group, a 4,4,4-trifluoro-2-butoxy group, a 2-trifluoromethyl-1-propoxy group or a 2-trifluoromethyl-2-propoxy group, and is preferably a halogeno $C_3$-$C_4$ alkoxy group (said halogeno $C_3$-$C_4$ alkoxy group represents a $C_3$-$C_4$ alkoxy group substituted with 1 to 5 halogeno groups), and more preferably a 1,1,1-trifluoro-2-propoxy group or a 2-trifluoromethyl-2-propoxy group.

The "$C_1$-$C_4$ alkyl group" in the definition of the respective substituents of the general formula (Id) is a straight or branched alkyl group having 1 to 4 carbon atoms and can include a methyl group, an ethyl group, a 1-propyl group, a 2-propyl group, a 1-butyl group, a 2-butyl group, a 2-methyl-1-propyl group or a 2-methyl-2-propyl group, and is preferably a $C_1$-$C_3$ alkyl group (particularly a methyl group, an ethyl group or a propyl group), more preferably a methyl group or an ethyl group, and most preferably a methyl group.

The "halogeno $C_1$-$C_4$ alkyl group" in the definition of the respective substituents of the general formula (Id) is the above $C_1$-$C_4$ alkyl group substituted with 1 to 5 halogeno groups similar to those described above and can include a fluoromethyl group, a difluoromethyl group, a dichloromethyl group, a dibromomethyl group, a trifluoromethyl group, a trichloromethyl group, a 2-fluoroethyl group, a 2-bromoethyl group, a 2-chloroethyl group, a 2-iodoethyl group, a 2,2-difluoroethyl group, a 2,2,2-trifluoroethyl group, a trichloroethyl group, a pentafluoroethyl group, a 3-fluoropropyl group, a 3-chloropropyl group, a 3,3,3-trifluoropropyl group, a 4-fluorobutyl group or a 4,4,4-trifluorobutyl group, and is preferably a halogeno $C_1$-$C_4$ alkyl group (said halogeno $C_1$-$C_4$ alkyl group represents a $C_1$-$C_4$ alkyl group substituted with 1 to 5 fluoro, chloro or bromo groups), more preferably a trifluoromethyl group, a 2,2,2-trifluoroethyl group or a pentafluoroethyl group, and most preferably a trifluoromethyl group.

The "$C_3$-$C_5$ cycloalkyl group" in the definition of the respective substituents of the general formula (Id) is a cyclic alkyl group having 3 to 5 carbon atoms and can include a cyclopropyl group, a cyclobutyl group or a cyclopentyl group, and is preferably a $C_3$-$C_4$ cycloalkyl group, and most preferably a cyclopropyl group.

The "$C_2$-$C_4$ alkenyl group" in the definition of the respective substituents of the general formula (Id) is an alkenyl group having 1 or 2 carbon-carbon double bonds and 2 to 4 carbon atoms and can include a vinyl group, a 2-propenyl group, a 2-butenyl group, a 1,3-butadien-1-yl group or a 2-methyl-2-propenyl group, and is preferably a $C_2$-$C_3$ alkenyl group, and most preferably a vinyl group.

The "$C_1$-$C_4$ alkoxy group" in the definition of the respective substituents of the general formula (Id) is a hydroxyl group substituted by one $C_1$-$C_4$ alkyl group described above and can include a methoxy group, an ethoxy group, a 1-propoxy group, a 2-propoxy group, a 1-butoxy group, a 2-butoxy group, a 2-methyl-1-propoxy group or a 2-methyl-2-propoxy group, and is preferably a $C_1$-$C_3$ alkoxy group (particularly a methoxy group, an ethoxy group or a propoxy group), and is more preferably a methoxy group or an ethoxy group, and most preferably a methoxy group.

The "$C_3$-$C_4$ cycloalkyl group" in the definition of the respective substituents of the general formula (Id) and the $C_3$-$C_4$ cycloalkyl moiety in the respective substituents is a cyclic alkyl group having 3 or 4 carbon atoms and can include a cyclopropyl group or a cyclobutyl group, and is most preferably a cyclopropyl group.

A compound represented by the general formula (Id) can be prepared according to the following Method Ad or Method Bd described in PCT/JP2005/011928 specification and Japanese Patent Application No. 2005-189264 specification.

[Chemical formula 7]

Method Ad

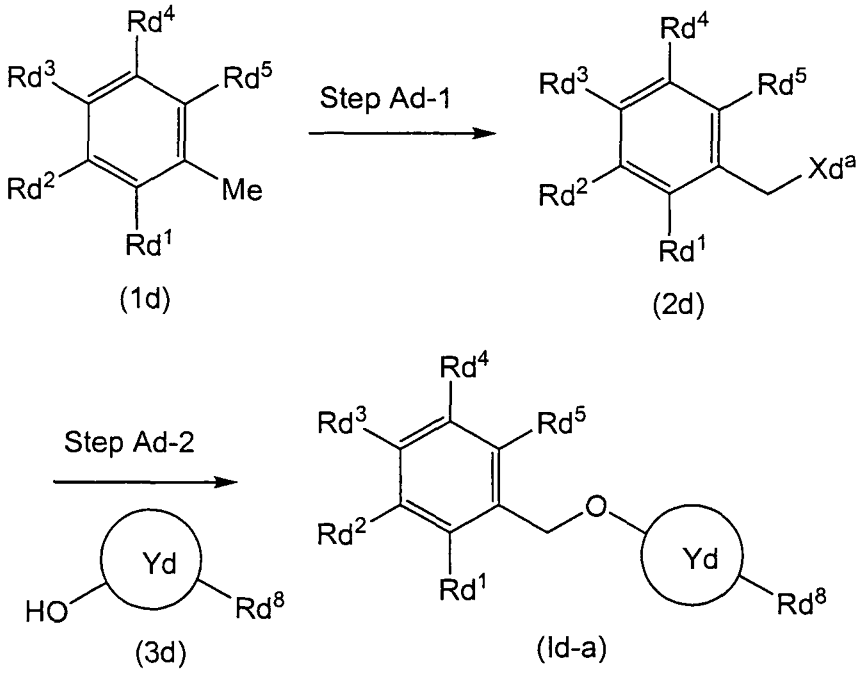


(1d) (2d) (3d) (Id-a)

[Chemical formula 8]

Method Bd

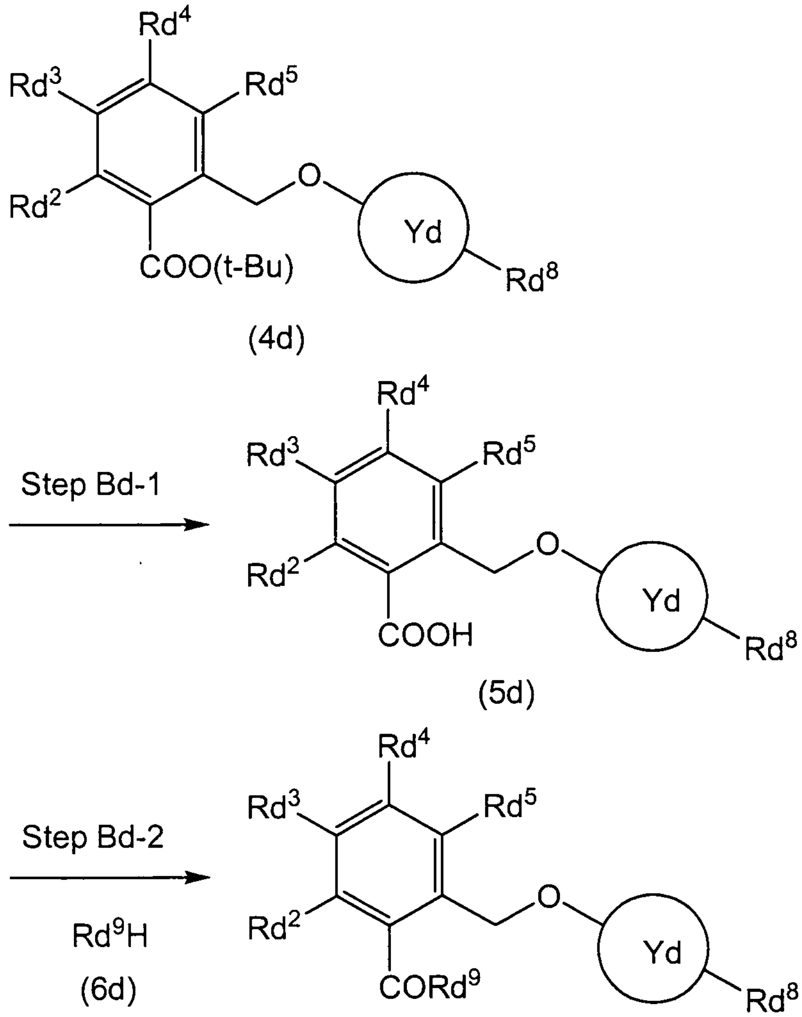


(4d)

(5d)

(Id-b)

In the structural formulae of the compounds of the above Method Ad or Method Bd, $Rd^1$, $Rd^2$, $Rd^3$, $Rd^4$, $Rd^5$, $Rd^6$, $Rd^7$, $Rd^8$, $Rd^9$ and Yd have the same meanings as defined above and $Rd^a$ represents a hydrogen atom or a $C_1$-$C_6$ alkyl group.

In the reactions of the following Method Ad or Method Bd, in cases where the compound becoming a reaction substrate has a group which inhibits the desired reaction such as an amino group, a hydroxyl group or a carboxyl group, introduction of a protective group to those groups may be appropriately carried out, if necessary, and removal of the introduced protective group may be appropriately carried out, if necessary (for example, T. H. Greene, P. G. Wuts, Protective Groups in Organic Synthesis. Third Edition, 1999, John Wiley & Sons, Inc. and the like).

(Method Ad)

Method Ad is a method to prepare the compound (Id-a) in which $Rd^6$ and $Rd^7$ are hydrogen atoms and $Xd^1$ is a single bond in the general formula (Id).

(Step Ad-1)

Step Ad-1 is a step to prepare the compound (2d) by halogenating the compound (1d), which is publicly known

or easily obtained from publicly known compounds, with a halogenating agent.

The halogenating agent used is, for example, an N-halogenosuccinimide, and is preferably N-bromosuccinimide. Step Ad-1 can be carried out, if necessary, in the presence of a radical reaction initiator such as azoisobutyronitrile (preferably azoisobutyronitrile).

The solvent used is preferably an aromatic hydrocarbon or a halogenated hydrocarbon, more preferably benzene or carbon tetrachloride.

The reaction temperature is preferably from 50 to 150˚C.

The reaction time is preferably from 1 hour to 9 hours.

(Step Ad-2)

Step Ad-2 is a step to prepare the compound (Id-a) by reacting the compound (2d) obtained in Step Ad-1 with the compound (3d) in the presence of a base. The compound (3d) is publicly known or easily obtained from publicly known compounds.

The base used is, for example, an alkali metal carbonate, an alkali metal hydrogencarbonate or an alkali metal hydride, and is preferably potassium carbonate or cesium carbonate.

The solvent used is preferably an amide, more preferably dimethylformamide.

The reaction temperature is preferably from 0 to 50˚C.

The reaction time is preferably from 1 hour to 24 hours.

(Method Bd)

Method Bd is a method to prepare the compound (Id-b) in which $Rd^1$ is $-CORd^9$, $Rd^6$ and $Rd^7$ are hydrogen atoms and $Xd^1$ is a single bond in the general formula (Id).

(Step Bd-1)

Step Bd-1 is a step to prepare the compound (5d) by treating the compound (4d) obtained in the Process Ad or the like with an acid.

The acid used is, for example, trifluoroacetic acid or hydrochloric acid, and is preferably trifluoroacetic acid.

The solvent used is preferably a halogenated hydrocarbon, more preferably methylene chloride.

The reaction temperature is preferably from 20 to 60˚C.

The reaction time is preferably from 3 hours to 24 hours.

(Step Bd-2)

Step Bd-2 is a step to prepare the compound (Id-b) by reacting the compound (5d) obtained in Step Bd-1 with the compound (6d), which is publicly known or easily obtained from publicly known compounds, in the presence of a condensing agent.

The condensing agent used can be a condensing agent described in R. C. Larock. Comprehensive Organic Transformations. Second Edition, 1999, John Wiley & Sons, Inc. or the like. The condensing agent used can be, for example,

(i) a combination of a phosphoric acid ester such as diethylphosphoryl cyanide and diphenylphosphoryl azide and the base described below;
(ii) a carbodiimide such as 1,3-dicyclohexylcarbodiimide, 1,3-diisopropylcarbodiimide and 1-ethyl-3-(3-dimethylaminopropyl)carbodiimide (WSC); a combination of the above carbodiimide and the following base; and a combination of the above carbodiimide and an N-hydroxy compound such as N-hydroxysuccinimide, 1-hydroxybenzotriazole and N-hydroxy-5-norbornene-2,3-dicarboxiimide;
(iii) a combination of a disulfide such as 2,2'-dipyridyl disulfide and 2,2'-dibenzothiazolyl disulfide and a phosphine such as triphenylphosphine and tributylphosphine;
(iv) a combination of a 2-halogeno-1-lower alkylpyridinium halide such as 2-chloro-1-methylpyridinium iodide and 2-bromo-1-ethylpyridinium chloride and the base described below;
(v) an imidazole such as 1,1'-oxazolyldiimidazole and N,N'-carbonyldiimidazole; or
(vi) a combination of a sulfonyl chloride such as p-toluenesulfonyl chloride, 2,4,6-trimethylsulfonyl chloride and 2,4,6-triisopropylsulfonyl chloride and the base described below,
and is preferably the combination of a carbodiimide and a base, the combination of a 2-halogeno-1-lower alkylpyridinium halide and a base or the combination of a sulfonyl chloride and a base, more preferably the combination

of 1-ethyl-3-(3-dimethylaminopropyl)carbodiimide and a base, the combination of 2-chloro-1-methylpyridinium iodide and a base or the combination of 2,4,6-triisopropylsulfonyl chloride and a base.

The base used in combination with the above condensing agent is, for example, triethylamine, diisopropylethylamine, pyridine, 4-(N,N-dimethylamino)pyridine or a mixture of these, and is preferably triethylamine, 4-(N,N-dimethylamino)pyridine or a mixture of these.

The solvent used is preferably a halogenated hydrocarbon, more preferably methylene chloride.

The reaction temperature is preferably from 20 to 60°C.

The reaction time is preferably from 3 hours to 24 hours.

Further, Step Bd-2 can be also carried out by converting the compound (5d) to the acid chloride with oxalyl chloride, thionyl chloride or the like in a solvent (methylene chloride or the like), followed by reacting the acid chloride with the compound (6d) or an alkali metal alkoxide in the presence of a base (for example, triethylamine or the like).

Further, the following compound or a pharmacologically acceptable salt or ester thereof disclosed in Japanese Patent Application No. 2005-110908 specification can be also used as an LXR ligand:

a compound represented by the general formula (Ie)

[Chemical formula 9]

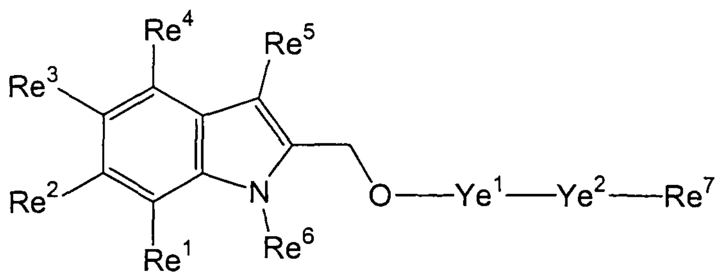

(Ie)

or a pharmacologically acceptable salt or ester thereof;

wherein $Re^1$, $Re^2$, $Re^3$ and $Re^4$ are the same or different and each represents a hydrogen atom, a $C_1$-$C_3$ alkyl group, a trifluoromethyl group, a 2,2,2-trifluoroethyl group, a pentafluoroethyl group, a hydroxyl group, a methoxy group, an ethoxy group, a fluoro group, a chloro group or a bromo group;

$Re^5$ represents a hydrogen atom;

$Re^6$ represents a group having the formula: $-CORe^8$ [wherein $Re^8$ represents a $C_1$-$C_6$ alkoxy group or a halogeno $C_1$-$C_4$ alkoxy group (said halogeno $C_1$-$C_4$ alkoxy group represents a $C_1$-$C_4$ alkoxy group substituted with 1 to 5 fluoro or chloro groups)];

$Re^7$ represents a group having the formula: $-Xe^2Re^{10}$ [wherein $Re^{10}$ represents a group having the formula: $-CORe^{11}$ (wherein $Re^{11}$ represents a hydroxyl group, a methoxy group or an ethoxy group) or a group having the formula: $-SO_2Re^{12}$ (wherein $Re^{12}$ represents a methyl group or an ethyl group), and $Xe^2$ represents a single bond, a methylene group or a substituted methylene group (said substituents are two fluoro groups, or two substituents may together form an ethylene group)];

$Ye^1$ represents a phenyl group;

$Ye^2$ represents a phenyl group, a substituted phenyl group (said substituent(s) are the same or different and are one or two group(s) selected from Substituent group $\alpha$e), a thienyl group, a thiazolyl group, a pyridyl group or a substituted thienyl group, a substituted thiazolyl group or a substituted pyridyl group (said substituent(s) are the same or different and are one or two group(s) selected from Substituent group $\alpha$e); and

Substituent group $\alpha$e represents the group consisting of a $C_1$-$C_4$ alkyl group, a trifluoromethyl group, a 2,2,2-trifluoroethyl group, a pentafluoroethyl group, a $C_2$-$C_4$ alkenyl group, a $C_2$-$C_4$ alkynyl group, a $C_3$-$C_4$ cycloalkyl group, a hydroxyl group, a methoxy group, an ethoxy group, a methanesulfonyl group, an ethanesulfonyl group, an amino group, a methylamino group, an ethylamino group, a dimethylamino group, a diethylamino group, a formyl group, a methylcarbonyl group, an ethylcarbonyl group, a nitro group, a fluoro group and a chloro group.

The "$C_1$-$C_3$ alkyl group" in the definition of the respective substituents of the general formula (Ie) is a straight

or branched alkyl group having 1 to 3 carbon atoms and can include a methyl group, an ethyl group, a 1-propyl group or a 2-propyl group, and is preferably a methyl group or an ethyl group, and most preferably a methyl group.

The "$C_1$-$C_6$ alkoxy group" in the definition of the respective substituents of the general formula (Ie) is a hydroxyl group substituted by $C_1$-$C_6$ alkyl group similar to that described above and can include a methoxy group, an ethoxy group, a 1-propoxy group, a 2-propoxy group, a 1-butoxy group, a 2-butoxy group, a 2-methyl-1-propoxy group, a 2-methyl-2-propoxy group, a 1-pentyloxy group, a 2-pentyloxy group, a 3-pentyloxy group, a 2-methyl-2-butoxy group, a 3-methyl-2-butoxy group, a 2-methyl-2-butoxy group, a 1-hexyloxy group, a 2-hexyloxy group, a 3-hexyloxy group, a 2-methyl-1-pentyloxy group, a 3-methyl-3-pentyloxy group, a 2-ethyl-1-butoxy group or a 2,3-dimethyl-1-butoxy group, and is preferably a $C_2$-$C_6$ alkoxy group, more preferably a $C_3$-$C_6$ alkoxy group, further preferably a $C_3$-$C_5$ alkoxy group (particularly a 2-propoxy group, a 2-methyl-2-propoxy group or a 2-methyl-2-butoxy group), and most preferably a 2-methyl-2-propoxy group.

The "halogeno $C_1$-$C_4$ alkoxy group" in the definition of the respective substituents of the general formula (Ie) is a $C_1$-$C_4$ alkoxy group similar to that described above substituted with 1 to 5 fluoro or chloro groups and can include a fluoromethoxy group, a difluoromethoxy group, a dichloromethoxy group, a dibromomethoxy group, a trifluoromethoxy group, a trichloromethoxy group, a 2-fluoroethoxy group, a 2-bromoethoxy group, a 2-chloroethoxy group, a 2-iodoethoxy group, a 2,2-difluoroethoxy group, a 2,2,2-trifluoroethoxy group, a 2,2,2-trichloroethoxy group, a pentafluoroethoxy group, a 3,3,3-trifluoro-1-propoxy group, a 1,1,1-trifluoro-2-propoxy group, a 1,1,1-trichloro-2-propoxy group, a 4,4,4-trifluoro-1-butoxy group, a 4,4,4-trifluoro-2-butoxy group, a 2-trifluoromethyl-1-propoxy group or a 2-trifluoromethyl-2-propoxy group, and is preferably a 2,2,2-trichloroethoxy group, a 1,1,1-trifluoro-2-propoxy group or a 2-trifluoromethyl-2-propoxy group.

The "$C_1$-$C_4$ alkyl group" in the definition of the respective substituents of the general formula (Ie) is a straight or branched alkyl group having 1 to 4 carbon atoms and can include a methyl group, an ethyl group, a 1-propyl group, a 2-propyl group, a 1-butyl group, a 2-butyl group, a 2-methyl-1-propyl group or a 2-methyl-2-propyl group, and is preferably a $C_1$-$C_3$ alkyl group, more preferably a methyl group or an ethyl group, and most preferably a methyl group.

The "$C_2$-$C_4$ alkenyl group" in the definition of the respective substituents of the general formula (Ie) is a straight or branched alkenyl group having 2 to 4 carbon atoms (which may have one or more carbon-carbon double bonds) and can include a vinyl group, a 2-propenyl group (allyl group) or a 2-butenyl group, and is preferably a vinyl group or a 2-propenyl group.

The "$C_2$-$C_4$ alkynyl group" in the definition of the respective substituents of the general formula (Ie) is a straight or branched alkynyl group having 2 to 4 carbon atoms (which may have one or more carbon-carbon triple bonds) and can include an ethynyl group, a 2-propynyl group or a 2-butynyl group, and is preferably an ethynyl group or a 2-propynyl group.

The "$C_3$-$C_4$ cycloalkyl group" in the definition of the respective substituents of the general formula (Ie) is a cyclic alkyl group having 3 or 4 carbon atoms and can include a cyclopropyl group or a cyclobutyl group, and is most preferably a cyclopropyl group.

A compound represented by the general formula (Ie) can be prepared according to the above described Method Ac or Method Bc described in PCT/JP2005/009142 specification, provided that in Step Ac-7 or Step Bc-6, the compound (9e) having the formula: HO-$Ye^1$-$Ye^2$-$Re^7$ is used instead of the compound (9c) .

The compound (9e) can be also prepared according to the following Method Ae or the like.

[Chemical formula 10]

Method Ae

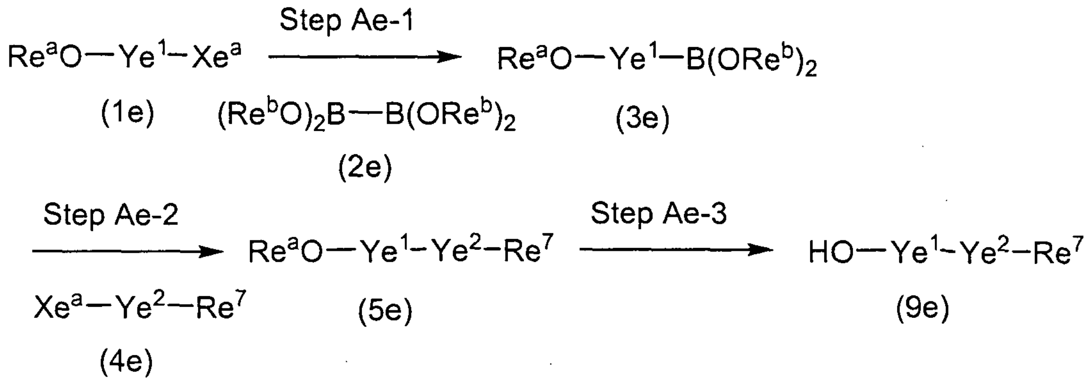


In the structural formulae of the compounds of the above Method Ae, $Re^7$, $Ye^1$ and $Ye^2$ have the same meanings as defined above, $Re^a$ represents a protective group of a hydroxyl group, preferably a silyl group substituted by three groups selected from the group consisting of a $C_1$-$C_6$ alkyl group and a phenyl group (particularly a tert-butyldimethylsilyl group, a tert-butyldiphenylsilyl group or a triisopropylsilyl group), a tetrahydrofuranyl group, a tetrahydropyranyl group, a methoxymethyl group or an allyl group, $Re^b$ represents a hydrogen atom or a $C_1$-$C_6$ alkyl group, two $Re^b$s together may form an ethylene group or a trimethylene group (said ethylene group or trimethylene group may be substituted with 1 to 4 methyl groups) and $Xe^a$ represents a chloro group, a bromo group, an iodo group or a trifluoromethanesulfonyloxy group.

(Method Ae)

Method Ae is a method to prepare the compound (9e).

(Step Ae-1)

Step Ae-1 is a step to prepare the compound (3e) by reacting the compound (1e), which is publicly known or easily obtained from publicly known compounds, with the compound (2e), which is publicly known or easily obtained from publicly known compounds, in the presence of a palladium reagent and a base.

The palladium catalyst used is preferably [1,1'-bis(diphenylphosphino)ferrocene]dichloro palladium (II)-dichloromethane adduct. Further, a phosphorus ligand such as triphenylphosphine, tri-o-tolylphosphine, tris[2-(diphenylphosphino)ethyl]phosphine or 1,2-bis(diphenylphosphino)ethane can be appropriately used, if necessary.

The base used is, for example, an alkali metal salt of acetic acid, and is preferably potassium acetate.

The solvent used is preferably an ether, a sulfoxide or a mixture of these, more preferably tetrahydrofuran, dioxane, dimethyl sulfoxide or a mixture of these, further preferably dimethyl sulfoxide or dioxane.

The reaction temperature is preferably from 50 to 150°C.

The reaction time is preferably from 2 hours to 12 hours.

(Step Ae-2)

Step Ae-2 is a step to prepare the compound (5e) by reacting the compound (3e) obtained in Step Ae-1 with the compound (4e), which is publicly known or easily obtained from publicly known compounds, in the presence of a palladium catalyst and a base.

The palladium catalyst used is preferably tetrakis(triphenylphosphine)palladium (0), palladium acetate (II) or tris(dibenzylideneacetone)dipalladium (0).

In Step Ae-2, a phosphorus ligand may be appropriately used, if necessary. The phosphorus ligand used is preferably triphenylphosphine, tri-o-tolylphosphine or 1,3-bis(diphenylphosphino)propane.

The base used is preferably an alkali metal carbonate, more preferably sodium carbonate or potassium carbonate.

The solvent used is preferably a hydrocarbon, an alcohol, an amide, water or a mixture of these, more preferably

toluene, ethanol, dimethylacetamide, water or a mixture of these, and most preferably a mixture of toluene and ethanol or a mixture of dimethylacetamide and water.

The reaction temperature is preferably from 50 to 150°C.

The reaction time is preferably from 3 hours to 24 hours.

(Step Ae-3)

Step Ae-3 is a step to prepare the compound (9e) by carrying out removal of the $Re^a$ group in the compound (5e) obtained in Step Ae-2.

Step Ae-3 can be carried out according to a usually used method (for example, a method described in T. H. Greene, P. G. Wuts, Protective Groups in Organic Synthesis. Third Edition, 1999, John Wiley & Sons, Inc. or the like) depending on the kind of the $Re^a$ group.

Further, the following compound or a pharmacologically acceptable salt or ester thereof disclosed in Japanese Patent Application No. 2004-311821 specification and Japanese Patent Application No. 2005-187686 specification can be also used as an LXR ligand:

(1) a compound represented by the general formula (If)

[Chemical formula 11]

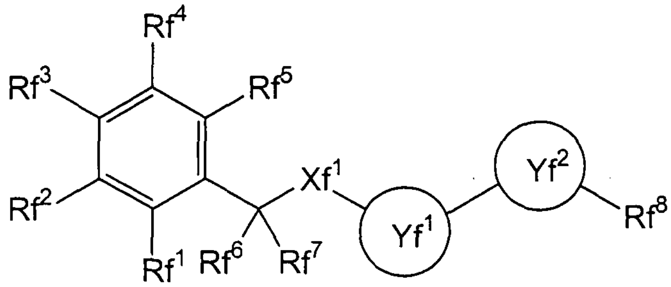

(If)

or a pharmacologically acceptable salt or ester thereof;

wherein $Rf^1$ represents a group having the formula - $CORf^9$ [wherein $Rf^9$ represents a $C_1$-$C_{10}$ alkyl group, a $C_1$-$C_{10}$ alkoxy group, a halogeno $C_1$-$C_{10}$ alkoxy group (wherein said halogeno $C_1$-$C_{10}$ alkoxy group represents a $C_1$-$C_{10}$ alkoxy group substituted with 1 to 7 halogeno groups), a phenyl-($C_1$-$C_{10}$ alkoxy) group, a $C_1$-$C_{10}$ alkylamino group or a di($C_1$-$C_{10}$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom)];

$Rf^2$ represents a hydrogen atom, a halogeno $C_1$-$C_4$ alkyl group (wherein said halogeno $C_1$-$C_4$ alkyl group represents a $C_1$-$C_4$ alkyl group substituted with 1 to 5 halogeno groups), a hydroxyl group, a $C_1$-$C_4$ alkoxy group, an amino group, a $C_1$-$C_4$ alkylamino group, a di ($C_1$-$C_4$ alkyl)amino group (wherein said alkyl groups may be the same or different) or a halogeno group;

$Rf^3$ represents a hydrogen atom, a $C_1$-$C_6$ alkyl group, a halogeno $C_1$-$C_6$ alkyl group (wherein said halogeno $C_1$-$C_6$ alkyl group represents a $C_1$-$C_6$ alkyl group substituted with 1 to 7 halogeno groups), a ($C_1$-$C_4$ alkoxy) - ($C_1$-$C_4$ alkyl) group, a ($C_1$-$C_4$ alkylthio)-($C_1$-$C_4$ alkyl) group, a ($C_1$-$C_4$ alkylsulfinyl) - ($C_1$-$C_4$ alkyl) group, a ($C_1$-$C_4$ alkylsulfonyl)-($C_1$-$C_4$ alkyl) group, a ($C_1$-$C_4$ alkylamino) - ($C_1$-$C_4$ alkyl) group, a [di ($C_1$-$C_4$ alkyl)amino]-($C_1$-$C_4$ alkyl) group (wherein said alkyl groups may be the same or different), a $C_3$-$C_6$ cycloalkyl group, a $C_2$-$C_6$ alkenyl group, a $C_2$-$C_6$ alkynyl group, a hydroxyl group, a $C_1$-$C_6$ alkoxy group, a halogeno $C_1$-$C_6$ alkoxy group (wherein said halogeno $C_1$-$C_6$ alkoxy group represents a $C_1$-$C_6$ alkoxy group substituted with 1 to 7 halogeno groups) , a $C_1$-$C_6$ alkylthio group, a $C_1$-$C_6$ alkylsulfinyl group, a $C_1$-$C_6$ alkylsulfonyl group, an amino group, a $C_1$-$C_6$ alkylamino group, a di ($C_1$-$C_6$ alkyl)amino group (wherein said alkyl groups may be the same or different, and two of said alkyl groups

may, together with the nitrogen atom of said amino group, form a 5- to 7-membered saturated heterocyclyl group containing 1 to 3 atoms selected from the group consisting of a nitrogen atom, an oxygen atom and a sulfur atom), a ($C_1$-$C_6$ alkoxy)carbonyl group, a cyano group, a nitro group or a halogeno group;

$Rf^4$ and $Rf^5$ may be the same or different and each represents a hydrogen atom, a $C_1$-$C_4$ alkyl group, a halogeno $C_1$-$C_4$ alkyl group (wherein said halogeno $C_1$-$C_4$ alkyl group represents a $C_1$-$C_4$ alkyl group substituted with 1 to 5 halogeno groups), a $C_3$-$C_6$ cycloalkyl group, a hydroxyl group, a $C_1$-$C_4$ alkoxy group, a halogeno $C_1$-$C_4$ alkoxy group (wherein said halogeno $C_1$-$C_4$ alkoxy group represents a $C_1$-$C_4$ alkoxy group substituted with 1 to 5 halogeno groups) or a halogeno group;

$Rf^6$ and $Rf^7$ may be the same or different and each represents a hydrogen atom or a $C_1$-$C_3$ alkyl group;

$Rf^8$ represents a group having the formula -$Xf^2Rf^{10}$ [wherein $Rf^{10}$ represents a group having the formula -$CORf^{11}$ [wherein $Rf^{11}$ represents a hydroxyl group, a $C_1$-$C_6$ alkoxy group, a ($C_3$-$C_8$ cycloalkyl) - ($C_1$-$C_6$ alkyl)oxy group, a $C_3$-$C_8$ cycloalkyloxy group, an amino group, a $C_1$-$C_6$ alkylamino group, a [($C_3$-$C_8$ cycloalkyl)-($C_1$-$C_6$ alkyl)]amino group, a $C_3$-$C_8$ cycloalkylamino group, a di ($C_1$-$C_6$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom) , a di [($C_3$-$C_8$ cycloalkyl) - ($C_1$-$C_6$ alkyl)]amino group, a di ($C_3$-$C_8$ cycloalkyl) amino group, a N-[($C_3$-$C_8$ cycloalkyl) - ($C_1$-$C_6$ alkyl)]-N-($C_1$-$C_6$ alkyl) amino group, a N- ($C_3$-$C_8$ cycloalkyl) -N- ($C_1$-$C_6$ alkyl) amino group, a N- [($C_3$-$C_8$ cycloalkyl) - ($C_1$-$C_6$ alkyl)]-N-($C_3$-$C_8$ cycloalkyl)amino group, a hydroxylamino group or a hydroxy($C_1$-$C_6$ alkyl) amino group,

a group having the formula -$SO_2Rf^{12}$ [wherein $Rf^{12}$ represents a $C_1$-$C_6$ alkyl group, a ($C_3$-$C_8$ cycloalkyl)-($C_1$-$C_6$ alkyl) group, a $C_3$-$C_8$ cycloalkyl group, an amino group, a $C_1$-$C_6$ alkylamino group, a [($C_3$-$C_8$ cycloalkyl)-($C_1$-$C_6$ alkyl)]amino group, a $C_3$-$C_8$ cycloalkylamino group, a di($C_1$-$C_6$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a di[($C_3$-$C_8$ cycloalkyl)-($C_1$-$C_6$ alkyl)] amino group, a di ($C_3$-$C_8$ cycloalkyl) amino group, a N- [($C_3$-$C_8$ cycloalkyl)-($C_1$-$C_6$ alkyl)]-N-($C_1$-$C_6$ alkyl) amino group, a N-($C_3$-$C_8$ cycloalkyl)-N-($C_1$-$C_6$ alkyl) amino group or a N-[($C_3$-$C_8$ cycloalkyl)-($C_1$-$C_6$ alkyl) -N-($C_3$-$C_8$ cycloalkyl) amino group],

a group having the formula -N ($Rf^{13}$) $CORf^{14}$ [wherein $Rf^{13}$ represents a hydrogen atom, a $C_1$-$C_6$ alkyl group, a ($C_3$-$C_8$ cycloalkyl) - ($C_1$-$C_6$ alkyl) group or a $C_3$-$C_8$ cycloalkyl group, and $Rf^{14}$ represents a hydrogen atom, a $C_1$-$C_6$ alkyl group, a ($C_3$-$C_8$ cycloalkyl) - ($C_1$-$C_6$ alkyl) group or a $C_3$-$C_8$ cycloalkyl group],

a group having the formula -N($Rf^{13}$)$SO_2Rf^{15}$ [wherein $Rf^{13}$ has the same meaning as defined above, and $Rf^{15}$ represents a $C_1$-$C_6$ alkyl group, a ($C_3$-$C_8$ cycloalkyl)-($C_1$-$C_6$ alkyl) group or a $C_3$-$C_8$ cycloalkyl group], or a tetrazol-5-yl group, and

$Xf^2$ represents a single bond, a $C_1$-$C_4$ alkylene group or a substituted $C_1$-$C_4$ alkylene group (wherein said substituent (s) may be the same or different, and are 1 or 2 group(s) selected from Substituent group γf, or two of said substituents may together form an ethylene group or a trimethylene group)];

$Xf^1$ is a group having the formula -NH-, -$NRf^{16}$-(wherein $Rf^{16}$ represents a $C_1$-$C_4$ alkyl group), -O-, -S-, - SO- or -$SO_2$-;

$Yf^1$ is a phenyl group, a substituted phenyl group (wherein said substituent(s) may be the same or different, and are 1 to 3 group(s) selected from Substituent group αf), a 5- to 6-membered aromatic heterocyclyl group or a substituted 5- to 6-membered aromatic heterocyclyl group (wherein said substituent(s) may be the same or different, and are 1 to 3 group(s) selected from Substituent group αf) ;

$Yf^2$ represents a 6- to 10-membered aryl group, a substituted 6- to 10-membered aryl group (wherein said substituent (s) may be the same or different, and are 1 to 3 group(s) selected from Substituent group βf), a 9- to 10-membered unsaturated cyclic hydrocarbon group (provided that $Yf^1$ is bound to a benzene ring moiety in said unsaturated cyclic hydrocarbon group), a substituted 9- to 10-membered unsaturated cyclic hydrocarbon group (provided that $Yf^1$ is bound to a benzene ring moiety in said unsaturated cyclic hydrocarbon group, and said substituent(s) may be the same or different, and are 1 to 3 group(s) selected from Substituent group βf) , a 5- to 10-membered aromatic heterocyclyl group, a substituted 5- to 10-membered aromatic heterocyclyl group (wherein said substituent(s) may be the same or different, and are 1 to 3 group(s) selected from Substituent group (βf), a 9- to 10-membered unsaturated heterocyclyl group (provided that $Yf^1$ is bound to an aromatic ring moiety in said unsaturated heterocyclyl group) or a substituted 9- to 10-membered unsaturated heterocyclyl group (provided that $Yf^1$ is bound to an aromatic ring moiety in said unsaturated heterocyclyl group, and said substituent(s) may be the same or different and are 1 to 3 group(s) selected from Substituent group βf);

Substituent group αf represents the group consisting of a $C_1$-$C_4$ alkyl group, a halogeno $C_1$-$C_4$ alkyl group (wherein said halogeno $C_1$-$C_4$ alkyl group represents a $C_1$-$C_4$ alkyl group substituted with 1 to 5 halogeno groups), a hydroxyl group, a $C_1$-$C_4$ alkoxy group and a halogeno group;

Substituent group βf represents the group consisting of a $C_1$-$C_6$ alkyl group, a hydroxy ($C_1$-$C_6$ alkyl) group, a carboxy ($C_1$-$C_6$ alkyl) group, a ($C_1$-$C_6$ alkoxy) carbonyl- ($C_1$-$C_6$ alkyl) group, a halogeno $C_1$-$C_6$ alkyl group (wherein said

halogeno $C_1$-$C_6$ alkyl group represents a $C_1$-$C_6$ alkyl group substituted with 1 to 7 halogeno groups), a ($C_3$-$C_8$ cycloalkyl) - ($C_1$-$C_6$ alkyl) group, a $C_2$-$C_7$ alkenyl group, a $C_2$-$C_7$ alkynyl group, a $C_3$-$C_8$ cycloalkyl group, a hydroxyl group, a $C_1$-$C_6$ alkoxy group, a halogeno $C_1$-$C_6$ alkoxy group (wherein said halogeno $C_1$-$C_6$ alkoxy group represents a $C_1$-$C_6$ alkoxy group substituted with 1 to 7 halogeno groups), a $C_1$-$C_6$ alkylthio group, a $C_1$-$C_6$ alkylsulfinyl group, a $C_1$-$C_6$ alkylsulfonyl group, an amino group, a $C_1$-$C_6$ alkylamino group, a $C_3$-$C_8$ cycloalkylamino group, a di($C_1$-$C_6$ alkyl)amino group (wherein said alkyl groups may be the same or different and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a di($C_3$-$C_8$ cycloalkyl) amino group, a N-($C_3$-$C_8$ cycloalkyl) -N- ($C_1$-$C_6$ alkyl)amino group, a formylamino group, a ($C_1$-$C_6$ alkyl) carbonylamino group, a ($C_3$-$C_8$ cycloalkyl) carbonylamino group, a N-[($C_1$-$C_6$ alkyl)carbonyl]-N-($C_1$-$C_6$ alkyl) amino group, a N-[($C_3$-$C_8$ cycloalkyl) carbonyl] -N- ($C_1$-$C_6$ alkyl) amino group, a $C_1$-$C_6$ alkylsulfonylamino group, a N-($C_1$-$C_6$ alkylsulfonyl) -N- ($C_1$-$C_6$ alkyl) amino group, a N- ($C_1$-$C_6$ alkylsulfonyl)-N-($C_3$-$C_8$ cycloalkyl) amino group, a formyl group, a ($C_1$-$C_6$ alkyl)carbonyl group, a carboxyl group, a ($C_1$-$C_6$ alkoxy)carbonyl group, a carbamoyl group, a ($C_1$-$C_6$ alkylamino)carbonyl group, a ($C_3$-$C_8$ cycloalkylamino)carbonyl group, a di($C_1$-$C_6$ alkyl)aminocarbonyl group (wherein said alkyl groups may be the same or different and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a N-($C_3$-$C_8$ cycloalkyl)-N-($C_1$-$C_6$ alkyl)aminocarbonyl group, a cyano group, a nitro group and a halogeno group; and

Substituent group $\gamma$f represents the group consisting of a $C_1$-$C_6$ alkyl group, a hydroxy($C_1$-$C_6$ alkyl) group, a ($C_1$-$C_6$ alkoxy)-($C_1$-$C_6$ alkyl) group, a mercapto ($C_1$-$C_6$ alkyl) group, a ($C_1$-$C_6$ alkylthio) - ($C_1$-$C_6$ alkyl) group, a ($C_1$-$C_6$ alkylsulfinyl) - ($C_1$-$C_6$ alkyl) group, a ($C_1$-$C_6$ alkylsulfonyl)-($C_1$-$C_6$ alkyl) group, an amino ($C_1$-$C_6$ alkyl) group, a ($C_1$-$C_6$ alkylamino) - ($C_1$-$C_6$ alkyl) group, a ($C_3$-$C_8$ cycloalkylamino) - ($C_1$-$C_6$ alkyl) group, a di ($C_1$-$C_6$ alkyl) amino- ($C_1$-$C_6$ alkyl) group (wherein said alkyl groups may be the same or different, and two of said alkyl groups of the di($C_1$-$C_6$ alkyl)amino moiety may, together with the nitrogen atom of said amino group, form a 5- to 7-membered saturated heterocyclyl group containing 1 to 3 atoms selected from the group consisting of a nitrogen atom, an oxygen atom and a sulfur atom), a di ($C_3$-$C_8$ cycloalkyl) amino- ($C_1$-$C_6$ alkyl) group, a [N-($C_3$-$C_8$ cycloalkyl) -N- ($C_1$-$C_6$ alkyl) amino] - ($C_1$-$C_6$ alkyl) group, a hydroxyl group, a $C_1$-$C_6$ alkoxy group, a $C_3$-$C_8$ cycloalkyloxy group, a mercapto group, a $C_1$-$C_6$ alkylthio group, a $C_3$-$C_8$ cycloalkylthio group, a $C_1$-$C_6$ alkylsulfinyl group, a $C_3$-$C_8$ cycloalkylsulfinyl group, a $C_1$-$C_6$ alkylsulfonyl group, a $C_3$-$C_8$ cycloalkylsulfonyl group, an amino group, a $C_1$-$C_6$ alkylamino group, a $C_3$-$C_8$ cycloalkylamino group, a di($C_1$-$C_6$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a di($C_3$-$C_8$ cycloalkyl)amino group, a N-($C_3$-$C_8$ cycloalkyl)-N-($C_1$-$C_6$ alkyl) amino group, and a halogeno group.

In the compounds represented by the general formula (If) described in the above (1), preferable compounds are described below, for example:

(2) a compound according to (1),

wherein $Rf^1$ is a group having the formula $-CORf^{9a}$ [wherein $Rf^{9a}$ represents a $C_1$-$C_6$ alkyl group, a $C_1$-$C_8$ alkoxy group, a halogeno $C_1$-$C_6$ alkoxy group (wherein said halogeno $C_1$-$C_6$ alkoxy group represents a $C_1$-$C_6$ alkoxy group substituted with 1 to 7 halogeno groups), a $C_1$-$C_6$ alkylamino group or a di($C_1$-$C_6$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom)];

$Rf^2$ is a hydrogen atom, a trifluoromethyl group, a 2,2,2-trifluoroethyl group, a pentafluoroethyl group, a hydroxyl group, a fluoro group or a chloro group;

$Rf^3$ is a hydrogen atom, a $C_1$-$C_4$ alkyl group, a halogeno $C_1$-$C_4$ alkyl group (wherein said halogeno $C_1$-$C_4$ alkyl group represents a $C_1$-$C_4$ alkyl group substituted with 1 to 5 halogeno groups) , a $C_3$-$C_5$ cycloalkyl group, a $C_2$-$C_4$ alkenyl group, a $C_2$-$C_4$ alkynyl group, a hydroxyl group, a $C_1$-$C_4$ alkoxy group, a halogeno $C_1$-$C_4$ alkoxy group (wherein said halogeno $C_1$-$C_4$ alkoxy group represents a $C_1$-$C_4$ alkoxy group substituted with 1 to 5 halogeno groups), a $C_1$-$C_4$ alkylthio group, a $C_1$-$C_4$ alkylsulfinyl group, a $C_1$-$C_4$ alkylsulfonyl group, an amino group, a $C_1$-$C_4$ alkylamino group, di($C_1$-$C_4$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a fluoro group, a chloro group or a bromo group;

$Rf^4$ and $Rf^5$ may be the same or different, and each is a hydrogen atom, a methyl group, an ethyl group, a trifluoromethyl group, a methoxy group, a fluoro group, a chloro group or a bromo group;

$Rf^6$ and $Rf^7$ may be the same or different, and each is a hydrogen atom or a methyl group;

$Rf^8$ represents a group having the formula $-Xf^{2a}Rf^{10a}$ [wherein $Rf^{10a}$ represents a group having the formula $-CORf^{11a}$

[wherein $Rf^{11a}$ represents a hydroxyl group, a $C_1$-$C_4$ alkoxy group, a ($C_3$-$C_6$ cycloakyl) - ($C_1$-$C_4$ alkyl)oxy group, a $C_3$-$C_6$ cycloalkyloxy group, an amino group, a $C_1$-$C_4$ alkylamino group, a [($C_3$-$C_6$ cycloalkyl)-($C_1$-$C_4$ alkyl)]amino group, a $C_3$-$C_6$ cycloalkylamino group, a di($C_1$-$C_4$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a hydroxylamino group or a hydroxy ($C_1$-$C_4$ alkyl) amino group],

a group having the formula $-SO_2Rf^{12a}$ [wherein $Rf^{12a}$ represents a $C_1$-$C_4$ alkyl group, a ($C_3$-$C_6$ cycloalkyl) - ($C_1$-$C_4$ alkyl) group, a $C_3$-$C_6$ cycloalkyl group, an amino group, a $C_1$-$C_4$ alkylamino group, a [($C_3$-$C_6$ cycloalkyl) - ($C_1$-$C_4$ alkyl)]amino group, a $C_3$-$C_6$ cycloalkylamino group or a di($C_1$-$C_4$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom)],

a group having the formula $-N(Rf^{13a})CORf^{14a}$ [wherein $Rf^{13a}$ represents a hydrogen atom, a $C_1$-$C_4$ alkyl group, a ($C_3$-$C_5$ cycloalkyl)-($C_1$-$C_2$ alkyl) group or a $C_3$-$C_5$ cycloalkyl group, and $Rf^{14a}$ represents a hydrogen atom, a $C_1$-$C_4$ alkyl group, a ($C_3$-$C_5$ cycloalkyl)-($C_1$-$C_2$ alkyl) group or a $C_3$-$C_5$ cycloalkyl group],

a group having the formula $-N(Rf^{13a}) SO_2Rf^{15a}$ [wherein $Rf^{13a}$ has the same meaning as defined above, and $Rf^{15a}$ represents a $C_1$-$C_4$ alkyl group, a ($C_3$-$C_5$ cycloalkyl)-($C_1$-$C_2$ alkyl) group or a $C_3$-$C_5$ cycloalkyl group], or a tetrazol-5-yl group, and

$Xf^{2a}$ represents a single bond, a $C_1$-$C_2$ alkylene group or a substituted $C_1$-$C_2$ alkylene group (wherein said substituent (s) may be the same or different, and are 1 or 2 group(s) selected from Substituent group γf1, or two of said substituents may together form an ethylene group or a trimethylene group)];

$Xf^1$ is a group having the formula -NH-, -O- or -S-;

$Yf^1$ is a phenyl group, a substituted phenyl group (wherein said substituent(s) may be the same or different, and are 1 or 2 group(s) selected from Substituent group αf1), a 5- to 6-membered aromatic heterocyclyl group (wherein said heterocyclyl group represents a pyrrolyl group, a furyl group, a thienyl group, an imidazolyl group, an oxazolyl group, a thiazolyl group, a pyridyl group or a pyridazinyl group), or a substituted 5- to 6-membered aromatic heterocyclyl group (wherein said heterocyclyl group is a pyrrolyl group, a furyl group, a thienyl group, an imidazolyl group, an oxazolyl group, a thiazolyl group, a pyridyl group or a pyridazinyl group, and said substituent(s) may be the same or different, and are 1 or 2 group(s) selected from Substituent group αf1);

$Yf^2$ is a phenyl group, a substituted phenyl group (wherein said substituent(s) may be the same or different, and are 1 to 3 group(s) selected from Substituent group βf1), an indanyl group or a tetrahydronaphthyl group (provided that $Yf^1$ is bound to a benzene ring moiety in said indanyl group or said tetrahydronaphthyl group), a substituted indanyl group or a substituted tetrahydronaphthyl group (provided that $Yf^1$ is bound to a benzene ring moiety in said indanyl group or said tetrahydronaphthyl group, and said substituent(s) may be the same or different, and are 1 to 3 group(s) selected from Substituent group βf1), a 5- to 6-membered aromatic heterocyclyl group (wherein said heterocyclyl group represents a pyrrolyl group, a furyl group, a thienyl group, an imidazolyl group, an oxazolyl group, a thiazolyl group, a pyridyl group or a pyrimidinyl group), a substituted 5- to 6-membered aromatic heterocyclyl group (said heterocyclyl group represents a pyrrolyl group, a furyl group, a thienyl group, an imidazolyl group, an oxazolyl group, a thiazolyl group, a pyridyl group or a pyrimidinyl group, and said substituent(s) may be the same or different, and are 1 to 3 group(s) selected from Substituent group βf1), a 9- to 10-membered unsaturated heterocyclyl group (provided that $Yf^1$ is bound to an aromatic ring moiety in said unsaturated heterocyclyl group, and said unsaturated heterocyclyl group represents an indolinyl group, a dihydrobenzofuryl group, a dihydrobenzothienyl group, a tetrahydroquinolyl group or a cromanyl group) or a substituted 9- to 10-membered unsaturated heterocyclyl group (provided that $Yf^1$ is bound to an aromatic ring moiety in said unsaturated heterocyclyl group, said unsaturated heterocyclyl group represents an indolinyl group, a dihydrobenzofuryl group, a dihydrobenzothienyl group, a tetrahydroquinolyl group or a cromanyl group, and said substituent(s) may be the same or different and are 1 to 3 group(s) selected from Substituent group βf1);

Substituent group αf1 is the group consisting of a methyl group, an ethyl group, a trifluoromethyl group, a methoxy group, an ethoxy group, a fluoro group and a chloro group;

Substituent group βf1 is the group consisting of a $C_1$-$C_6$ alkyl group, a hydroxy ($C_1$-$C_6$ alkyl) group, a carboxy ($C_1$-$C_4$ alkyl) group, a ($C_1$-$C_4$ alkoxy) carbonyl- ($C_1$-$C_4$ alkyl) group, a halogeno $C_1$-$C_4$ alkyl group (wherein said halogeno $C_1$-$C_4$ alkyl group represents a $C_1$-$C_4$ alkyl group substituted with 1 to 5 halogeno groups), a ($C_3$-$C_6$ cycloalkyl) - ($C_1$-$C_4$ alkyl) group, a $C_2$-$C_5$ alkenyl group, a $C_2$-$C_5$ alkynyl group, a $C_3$-$C_6$ cycloalkyl group, a hydroxyl group, a $C_1$-$C_4$ alkoxy group, a halogeno $C_1$-$C_4$ alkoxy group (wherein said halogeno $C_1$-$C_4$ alkoxy group represents a $C_1$-$C_4$ alkoxy group substituted with 1 to 5 halogeno groups), a $C_1$-$C_4$ alkylthio group, a $C_1$-$C_4$ alkylsulfinyl group, a $C_1$-$C_4$ alkylsulfonyl group, an amino group, a $C_1$-$C_4$ alkylamino group, a $C_3$-$C_6$ cycloalkylamino group, a di ($C_1$-$C_4$ alkyl) amino group (wherein said alkyl groups may be the same or different and two of said alkyl groups may, together with the nitrogen atom of said amino group, form a 5- to 7-membered saturated heterocyclyl group containing 1 to

3 atoms selected from the group consisting of the nitrogen atom, an oxygen atom and a sulfur atom), a formylamino group, a ($C_1$-$C_4$ alkyl)carbonylamino group, a ($C_3$-$C_6$ cycloalkyl)carbonylamino group, a N-[($C_1$-$C_4$ alkyl)carbonyl]-N-($C_1$-$C_4$ alkyl) amino group, a N- [($C_3$-$C_6$ cycloalkyl)carbonyl]-N-($C_1$-$C_4$ alkyl) amino group, a $C_1$-$C_4$ alkylsulfonylamino group, a N-($C_1$-$C_4$ alkylsulfonyl)-N-($C_1$-$C_4$ alkyl)amino group, a formyl group, a ($C_1$-$C_4$ alkyl)carbonyl group, a carboxyl group, a ($C_1$-$C_4$ alkoxy)carbonyl group, a carbamoyl group, a ($C_1$-$C_4$ alkylamino)carbonyl group, a di($C_1$-$C_4$ alkyl)aminocarbonyl group (wherein said alkyl groups may be the same or different and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a cyano group, a nitro group, a fluoro group, a chloro group and a bromo group; and

Substituent group γf1 is the group consisting of a methyl group, an ethyl group, a hydroxymethyl group, a hydroxyethyl group, a methoxymethyl group, a methoxyethyl group, a methylthiomethyl group, a methylthioethyl group, an aminomethyl group, an aminoethyl group, a methylaminomethyl group, an ethylaminomethyl group, a methylaminoethyl group, a cyclopropylaminomethyl group, a cyclopropylaminoethyl group, a dimethylaminomethyl group, a dimethylaminoethyl group, a (N-methyl-N-ethylamino)methyl group, a dicyclopropylaminomethyl group, a hydroxyl group, a methoxy group, an ethoxy group, a cyclopropyloxy group, a methylthio group, an ethylthio group, a cyclopropylthio group, an amino group, a methylamino group, an ethylamino group, a cyclopropylamino group, a cyclobutylamino group, a dimethylamino group, a diethylamino group, a dicyclopropylamino group, a N-cyclopropyl-N-methylamino group, a fluoro group and a chloro group,

(3) a compound according to (1),

wherein $Rf^1$ is a group having the formula $-CORf^{9b}$ [wherein $Rf^{9b}$ represents a $C_1$-$C_6$ alkoxy group or a halogeno $C_1$-$C_4$ alkoxy group (wherein said halogeno $C_1$-$C_4$ alkoxy group represents a $C_1$-$C_4$ alkoxy group substituted with 1 to 5 halogeno groups)];

$Rf^2$ is a hydrogen atom or a hydroxyl group;

$Rf^3$ is a hydrogen atom, a $C_1$-$C_4$ alkyl group, a halogeno $C_1$-$C_4$ alkyl group (wherein said halogeno $C_1$-$C_4$ alkyl group represents a $C_1$-$C_4$ alkyl group substituted with 1 to 5 halogeno groups) , a $C_3$-$C_5$ cycloalkyl group, a $C_2$-$C_4$ alkenyl group, a $C_1$-$C_4$ alkoxy group, a fluoro group or a chloro group;

$Rf^4$ and $Rf^5$ may be the same or different, and each is a hydrogen atom, a methyl group, an ethyl group, a trifluoromethyl group, a methoxy group, a fluoro group, a chloro group or a bromo group;

$Rf^6$ and $Rf^7$ may be the same or different, and each is a hydrogen atom or a methyl group;

$Rf^8$ is a group having the formula $-Xf^{2b}Rf^{10b}$ [wherein $Rf^{10b}$ represents a group having the formula $-CORf^{11b}$ [wherein $Rf^{11b}$ represents a hydroxyl group, a $C_1$-$C_4$ alkoxy group, a ($C_3$-$C_5$ cycloalkyl) - ($C_1$-$C_2$ alkyl)oxy group, a $C_3$-$C_5$ cycloalkyloxy group, an amino group, a methylamino group, an ethylamino group, a dimethylamino group, a diethylamino group, a methylethylamino group or a hydroxylamino group], a group having the formula $-SO_2Rf^{12b}$ [wherein $Rf^{12b}$ represents a $C_1$-$C_4$ alkyl group, a ($C_3$-$C_5$ cycloalkyl) - ($C_1$-$C_2$ alkyl) group or a $C_3$-$C_5$ cycloalkyl group], or a tetrazol-5-yl group, and

$Xf^{2b}$ represents a single bond, a methylene group, an ethylene group or a substituted methylene group or a substituted ethylene group (wherein said substituent(s) may be the same or different, and are 1 or 2 group(s) selected from Substituent group γf2, or two of said substituents may together form an ethylene group or a trimethylene group)];

$Xf^1$ is a group having the formula -NH-, -O- or -S-;

$Yf^1$ is a phenyl group (wherein the substitution positions at which $Xf^1$ and $Yf^2$ bind to said phenyl group are the 1 and 3 positions or the 1 and 4 positions), a substituted phenyl group (wherein said substituent is one group selected from Substituent group αf2, and the substitution positions at which $Xf^1$ and $Yf^2$ bind to said phenyl group are the 1 and 3 positions or the 1 and 4 positions), a thienyl group (wherein the substitution positions at which $Xf^1$ and $Yf^2$ bind to said thienyl group are the 2 and 5 positions), a substituted thienyl group (wherein said substituent is one group selected from Substituent group αf2, and the substitution positions at which $Xf^1$ and $Yf^2$ bind to said thienyl group are the 2 and 5 positions), a pyridyl group (wherein the substitution positions at which $Xf^1$ and $Yf^2$ bind to said pyridyl group are the 2 and 5 positions or the 3 and 6 positions) or a substituted pyridyl group (wherein said substituent is one group selected from Substituent group αf2, and the substitution positions at which $Xf^1$ and $Yf^2$ bind to said pyridyl group are the 2 and 5 positions or the 3 and 6 positions) ;

$Yf^2$ is a phenyl group (wherein the substitution positions at which $Yf^1$ and $Rf^8$ bind to said phenyl group are the 1 and 3 positions or the 1 and 4 positions), a substituted phenyl group (wherein said substituent(s) may be the same or different, and are 1 or 2 group(s) selected from Substituent group βf2, and the substitution positions at which $Yf^1$ and $Rf^8$ bind to said phenyl group are the 1 and 3 positions or the 1 and 4 positions), a thienyl group (wherein the substitution positions at which $Yf^1$ and $Rf^8$ bind to said thienyl group are the 2 and 5 positions), a substituted thienyl group (wherein said substituent(s) may be the same or different, and are 1 or 2 group(s) selected from Substituent group βf2, and the substitution positions at which $Yf^1$ and $Rf^8$ bind to said thienyl group are the 2 and 5 positions), a thiazolyl group (wherein the substitution positions at which $Yf^1$ and $Rf^8$ bind to said thiazolyl group are the 2 and 5 positions), a substituted thiazolyl group (wherein said substituent(s) may be the same or different, and are 1 or 2

group(s) selected from Substituent group βf2, and the substitution positions at which $Yf^1$ and $Rf^8$ bind to said thiazolyl group are the 2 and 5 positions), a pyridyl group (wherein the substitution positions at which $Yf^1$ and $Rf^8$ bind to said pyridyl group are the 2 and 5 positions) or a substituted pyridyl group (wherein said substituent(s) may be the same or different, and are 1 or 2 group(s) selected from Substituent group βf2, and the substitution positions at which $Yf^1$ and $Rf^8$ bind to said pyridyl group are the 2 and 5 positions);

Substituent group αf2 is the group consisting of a methyl group, a fluoro group and a chloro group;

Substituent group βf2 is the group consisting of a $C_1$-$C_4$ alkyl group, a hydroxymethyl group, a 1-hydroxyethyl group, a trifluoromethyl group, a 2,2,2-trifluoroethyl group, a pentafluoroethyl group, a $C_2$-$C_4$ alkenyl group, a $C_2$-$C_4$ alkynyl group, a $C_3$-$C_4$ cycloalkyl group, a hydroxyl group, a methoxy group, an ethoxy group, a methanesulfonyl group, an ethanesulfonyl group, an amino group, a methylamino group, an ethylamino group, a dimethylamino group, a diethylamino group, a formyl group, a methylcarbonyl group, an ethylcarbonyl group, a cyano group, a nitro group, a fluoro group and a chloro group; and

Substituent group γf2 is the group consisting of a methyl group, an ethyl group, a hydroxymethyl group, a methoxymethyl group, an aminomethyl group, a methylaminomethyl group, a dimethylaminomethyl group, a (N-methyl-N-ethylamino)methyl group, a methoxy group, a methylamino group, a dimethylamino group, a fluoro group and a chloro group,

(4) a compound according to (1),

wherein $Rf^1$ is a group having the formula $-CORf^{9c}$ (wherein $Rf^{9c}$ represents a $C_3$-$C_5$ alkoxy group);

$Rf^2$ is a hydroxyl group;

$Rf^3$ is a methyl group, an ethyl group, a 2-propyl group, a 2-methyl-2-propyl group, a trifluoromethyl group, a 2,2,2-trifluoroethyl group, a cyclopropyl group or a vinyl group;

$Rf^4$ and $Rf^5$ are a hydrogen atom;

$Rf^6$ and $Rf^7$ are a hydrogen atom;

$Rf^8$ is a group having the formula $-Xf^{2c}Rf^{10c}$ [wherein $Rf^{10c}$ represents a group having the formula $-CORf^{11c}$ (wherein $Rf^{11c}$ represents a hydroxyl group or a methoxy group), or

a group having the formula $-SO_2Rf^{12c}$ (wherein $Rf^{12c}$ represents a methyl group), and

$Xf^{2c}$ represents a single bond, a methylene group or a substituted methylene group (wherein said substituent represents a hydroxymethyl group, or two substituents may together form an ethylene group)];

$Xf^1$ is a group having the formula -O-;

$Yf^1$ is a phenyl group (wherein the substitution positions at which $Xf^1$ and $Yf^2$ bind to said phenyl group are the 1 and 4 positions);

$Yf^2$ is a phenyl group (wherein the substitution positions at which $Yf^1$ and $Rf^8$ bind to said phenyl group are the 1 and 4 positions), a substituted phenyl group (wherein said substituent is one group selected from Substituent group βf3, and the substitution positions at which $Yf^1$ and $Rf^8$ bind to said phenyl group are the 1 and 4 positions) or a substituted phenyl group (wherein said substituent is one group selected from Substituent group βf3, and the substitution positions at which $Yf^1$, $Rf^8$ and the group selected from Substituent group βf3 bind to said phenyl group are the 1, 3 and 2 positions, respectively); and

Substituent group βf3 is the group consisting of a methyl group, an ethyl group, a 2-propyl group, a hydroxymethyl group, a trifluoromethyl group, a cyclopropyl group, a methoxy group, a methanesulfonyl group, an amino group, a methylamino group, a dimethylamino group, a methylcarbonyl group, an ethylcarbonyl group, a cyano group, a nitro group, a fluoro group and a chloro group,

(5) a compound according to (1),

wherein $Rf^1$ is a group having the formula $-CORf^{9d}$ (wherein $Rf^{9d}$ represents a 2-methyl-2-propoxy group);

$Rf^2$ is a hydroxyl group;

$Rf^3$ is a trifluoromethyl group;

$Rf^4$ and $Rf^5$ are a hydrogen atom;

$Rf^6$ and $Rf^7$ are a hydrogen atom;

$Rf^8$ is a group having the formula $-Xf^{2d}Rf^{10d}$ [wherein $Rf^{10d}$ represents a group having the formula $-CORf^{11d}$ (wherein $Rf^{11d}$ represents a hydroxyl group), and

$Xf^{2d}$ is a methylene group or a substituted methylene group (wherein two of said substituents together form an ethylene group)];

$Xf^1$ is a group having the formula -O-;

$Yf^1$ is a phenyl group (wherein the substitution positions at which $Xf^1$ and $Yf^2$ bind to said phenyl group are the 1 and 4 positions); and,

$Yf^2$ is a phenyl group (wherein the substitution positions at which $Yf^1$ and $Rf^8$ bind to said phenyl group are the 1 and 4 positions), a substituted phenyl group (wherein said substituent is one group selected from Substituent group βf3, and the substitution positions at which $Yf^1$ and $Rf^8$ bind to said phenyl group are the 1 and 4 positions) or a substituted phenyl group (wherein said substituent is one group selected from Substituent group βf3, and the sub-

stitution positions at which $Yf^1$, $Rf^8$ and the group selected from Substituent group βf3 bind to said phenyl group are the 1, 3 and 2 positions, respectively), or

(6) a compound according to (1) which is selected from the group consisting of

(4'-{ [2- (tert-Butoxycarbonyl) -3-hydroxy-4-(trifluoromethyl)benzyl]oxy-1,1'-biphenyl-4-yl)acetic acid,
1-(4'-{[2-(tert-Butoxycarbonyl)-3-hydroxy-4-(trifluoromethyl)benzyl]oxy}-1,1'-biphenyl-4-yl)cyclopropanecarboxylic acid,
2-(4'-{[2-(tert-Butoxycarbonyl)-3-hydroxy-4-(trifluoromethyl)benzyl]oxy}-1,1'-biphenyl-4-yl)-3-hydroxypropanoic acid,
(4'-{[2-(tert-Butoxycarbonyl)-3-hydroxy-4-(trifluoromethyl)benzyl]oxy}-2-methyl-1,1'-biphenyl-3-yl)acetic acid,
(4'-{[2-(tert-Butoxycarbonyl)-3-hydroxy-4-(trifluoromethyl)benzyl]oxy}-2-methyl-1,1'-biphenyl-4-yl)acetic acid,
(4'-{[2-(tert-Butoxycarbonyl)-3-hydroxy-4-(trifluoromethyl)benzyl]oxy}-2-chloro-1,1'-biphenyl-4-yl)acetic acid,
(4'-{[2-(tert-Butoxycarbonyl)-3-hydroxy-4-(trifluoromethyl)benzyl]oxy}-3-fluoro-1,1'-biphenyl-4-yl)acetic acid,
(4'-{[2-(tert-Butoxycarbonyl)-3-hydroxy-4-(trifluoromethyl) benzyl] oxy}-3-chloro-1,1'-biphenyl-4-yl)acetic acid,
1-(4'-{[2-(tert-Butoxycarbonyl)-3-hydroxy-4-(trifluoromethyl)benzyl]oxy}-3-fluoro-1,1'-biphenyl-4-yl)cyclopropanecarboxylic acid,
(4'-{[2-(tert-Butoxycarbonyl)-3-hydroxy-4-(trifluoromethyl)benzyl]oxy}-3-methoxy-1,1'-biphenyl-4-yl)acetic acid,
(4'-{[2-(tert-Butoxycarbonyl)-3-hydroxy-4-(trifluoromethyl)benzyl]oxy}-2-trifluoromethyl-1,1'-biphenyl-4-yl)acetic acid,
tert-Butyl 6-[({2'-ethyl-4'-[(methoxycarbonyl)methyl]-1,1.'-biphenyl-4-yl}oxy)methyl]-2-hydroxy-3-(trifluoromethyl) benzoate,
(4'-{[2-(tert-Butoxycarbonyl)-3-hydroxy-4-(trifluoromethyl)benzyl]oxy}-2-ethyl-1,1'-biphenyl-4-yl)acetic acid,
(4'-{[2-(tert-Butoxycarbonyl)-3-hydroxy-4-(trifluoromethyl)benzyl]oxy}-2-nitro-1,1'-biphenyl-4-yl)acetic acid,
(2-Amino-4'-{[2-(tert-butoxycarbonyl)-3-hydroxy-4-(trifluoromethyl)benzyl]oxy}-1,1'-biphenyl-4-yl)acetic acid,
(4'-{[2-(tert-Butoxycarbonyl)-3-hydroxy-4-(trifluoromethyl)benzyl]oxy}-2-formyl-1,1'-biphenyl-4-yl)acetic acid,
(4'-{[2-(tert-Butoxycarbonyl)-3-hydroxy-4-(trifluoromethyl)benzyl]oxy}-2-(hydroxymethyl)-1,1'-biphenyl-4-yl)acetic acid, and
(4'-{[2-(tert-Butoxycarbonyl)-3-hydroxy-4-(trifluoromethyl) benzyl] oxy}-2-cyano-1,1'-biphenyl-4-yl)acetic acid.

The "$C_1$-$C_{10}$ alkyl group" in $Rf^9$ of the general formula (If) is a straight or branched alkyl group having 1 to 10 carbon atoms and can include a methyl group, an ethyl group, a 1-propyl group, a 2-propyl group, a 1-butyl group, a 2-butyl group, a 2-methyl-1-propyl group, a 2-methyl-2-propyl group, a 1-pentyl group, a 2-pentyl group, a 3-pentyl group, a 2-methyl-2-butyl group, a 3-methyl-2-butyl group, a 1,1-dimethyl-1-propyl group, a 1-hexyl group, a 2-hexyl group, a 3-hexyl group, a 2-methyl-1-pentyl group, a 3-methyl-3-pentyl group, a 2-ethyl-1-butyl group, a 2,3-dimethyl-1-butyl group, a 3-heptyl group, a 4-heptyl group, a 3-methyl-3-hexyl group, a 3-ethyl-3-pentyl group, a 3-octyl group, a 4-octyl group, a 3-ethyl-3-hexyl group, a 4-nonyl group, a 5-nonyl group, a 4-ethyl-4-heptyl group, a 4-decyl group, a 5-decyl group or a 4-(1-propyl)-4-heptyl group, and is preferably a $C_1$-$C_6$ alkyl group, more preferably a $C_2$-$C_6$ alkyl group, and further preferably a $C_3$-$C_5$ alkyl group.

The "$C_1$-$C_{10}$ alkoxy group" in $Rf^9$ of the general formula (If) is a hydroxyl group substituted by the above $C_1$-$C_{10}$ alkyl group and can include a methoxy group, an ethoxy group, a 1-propoxy group, a 2-propoxy group, a 1-butoxy group, a 2-butoxy group, a 2-methyl-1-propoxy group, a 2-methyl-2-propoxy group, a 1-pentyloxy group, a 2-pentyloxy group, a 3-pentyloxy group, a 2-methyl-2-butoxy group, a 3-methyl-2-butoxy group, a 2-methyl-2-butoxy group, a 1-hexyloxy group, a 2-hexyloxy group, a 3-hexyloxy group, a 2-methyl-1-pentyloxy group, a 3-methyl-3-pentyloxy group, a 2-ethyl-1-butoxy group, a 2,3-dimethyl-1-butoxy group, a 1-heptyloxy group, a 3-heptyloxy group, a 4-heptyloxy group, a 3-methyl-3-hexyloxy group, a 3-ethyl-3-pentyloxy group, a 3-octyloxy group, a 4-octyloxy group, a 3-ethyl-3-hexyloxy group, a 4-nonyloxy group, a 5-nonyloxy group, a 4-ethyl-4-heptyloxy group, a 4-decyloxy group, a 5-decyloxy group or a 4-(1-propyl)-4-heptyloxy group, and is preferably a $C_1$-$C_8$ alkoxy group, more preferably a $C_1$-$C_6$ alkoxy group, further preferably a $C_2$-$C_6$ alkoxy group, still more preferably a $C_3$-$C_6$ alkoxy group, particularly preferably a $C_3$-$C_5$ alkoxy group (particularly a 2-propoxy group, a 2-methyl-2-propoxy group or a 2-methyl-2-butoxy group), and most preferably a 2-methyl-2-propoxy group.

The "$C_1$-$C_4$ alkyl group" in $Rf^4$, $Rf^5$, $Rf^{16}$ and Substituent group αf or the like of the general formula (If) is a straight or branched alkyl group having 1 to 4 carbon atoms and can include a methyl group, an ethyl group, a 1-propyl group, a 2-propyl group, a 1-butyl group, a 2-butyl group, a 2-methyl-1-propyl group or a 2-methyl-2-propyl group, and is preferably a $C_1$-$C_3$ alkyl group, more preferably a methyl group or an ethyl group, and most preferably a methyl group.

The "$C_1$-$C_4$ alkoxy group" in $Rf^2$, $Rf^4$, $Rf^5$ and Substituent group αf of the general formula (If) is a hydroxyl group substituted by one $C_1$-$C_4$ alkyl group described above and can include a methoxy group, an ethoxy group, a 1-propoxy group, a 2-propoxy group, a 1-butoxy group, a 2-butoxy group or a 2-methyl-2-propoxy group, and is preferably a $C_1$-$C_3$ alkoxy group, more preferably a methoxy group or an ethoxy group, and most preferably a methoxy group.

The "halogeno $C_1$-$C_{10}$ alkoxy group" in $Rf^9$ of the general formula (If) is the above $C_1$-$C_{10}$ alkoxy group sub-

stituted with 1 to 7 halogeno groups described below and can include a fluoromethoxy group, a difluoromethoxy group, a dichloromethoxy group, a dibromomethoxy group, a trifluoromethoxy group, a trichloromethoxy group, a 2-fluoroethoxy group, a 2-bromoethoxy group, a 2-chloroethoxy group, a 2-iodoethoxy group, a 2,2-difluoroethoxy group, a 2,2,2-trifluoroethoxy group, a 2,2,2-trichloroethoxy group, a pentafluoroethoxy group, a 3,3,3-trifluoro-1-propoxy group, a 1,1,1-trifluoro-2-propoxy group, a 1,1,1-trichloro-2-propoxy group, a 4,4,4-trifluoro-1-butoxy group, a 4,4,4-trifluoro-2-butoxy group, a 2-trifluoromethyl-1-propoxy group, a 2-trifluoromethyl-2-propoxy group, a 5,5,5-trifluoro-1-pentyloxy group, a 5,5,5-trifluoro-2-pentyloxy group, a 1,1,1-trifluoro-3-pentyloxy group, a 4,4,4-trifluoro-2-methyl-2-butoxy group, a 4,4,4-trifluoro-3-methyl-2-butoxy group, a 4,4,4-trifluoro-2-methyl-2-butoxy group, a 6,6,6-trifluoro-1-hexyloxy group, a 6,6,6-trifluoro-2-hexyloxy group, a 6,6,6-trifluoro-3-hexyloxy group, a 5,5,5-trifluoro-2-methyl-1-pentyloxy group, a 1,1,1-trifluoro-3-methyl-3-pentyloxy group, a 6,6,6-trifluoro-2-ethyl-1-butoxy group, a 6,6,6-trifluoro-2,3-dimethyl-1-butoxy group, a 7,7,7-trifluoro-1-heptyloxy group, a 7,7,7-trifluoro-3-heptyloxy group, a 1,1,1-trifluoro-4-heptyloxy group, a 6,6,6-trifluoro-3-methyl-3-hexyloxy group, a 1,1,1-trifluoro-3-ethyl-3-pentyloxy group, a 8,8,8-trifluoro-3-octyloxy group, a 8,8,8-trifluoro-4-octyloxy group, a 6,6,6-trifluoro-3-ethyl-3-hexyloxy group, a 9,9,9-trifluoro-4-nonyloxy group, a 9,9,9-trifluoro-5-nonyloxy group, a 1,1,1-trifluoro-4-ethyl-4-heptyloxy group, a 9,9,9-trifluoro-4-decyloxy group, a 9,9,9-trifluoro-5-decyloxy group or a 1,1,1-trifluoro-4-(1-propyl)-4-heptyloxy group, and is preferably a halogeno $C_1$-$C_6$ alkoxy group (said halogeno $C_1$-$C_6$ alkoxy group represents a $C_1$-$C_6$ alkoxy group substituted with 1 to 7 halogeno groups), more preferably a halogeno $C_1$-$C_4$ alkoxy group (said halogeno $C_1$-$C_4$ alkoxy group represents a $C_1$-$C_4$ alkoxy group substituted with 1 to 5 halogeno groups), further preferably a halogeno $C_3$-$C_4$ alkoxy group (said halogeno $C_3$-$C_4$ alkoxy group represents a $C_3$-$C_4$ alkoxy group substituted with 1 to 5 halogeno groups), and most preferably a 1,1,1-trifluoro-2-propoxy group or a 2-trifluoromethyl-2-propoxy group.

The "phenyl-($C_1$-$C_{10}$ alkoxy) group" in $Rf^9$ of the general formula (If) is the above $C_1$-$C_{10}$ alkoxy group substituted by one phenyl group and can include a phenylmethoxy group, a phenylethoxy group, a 3-phenyl-1-propoxy group, a 1-phenyl-2-propoxy group, a 4-phenyl-1-butoxy group, a 1-phenyl-2-butoxy group, a 3-phenyl-2-methyl-1-propoxy group, a 1-phenyl-2-methyl-2-propoxy group, a 5-phenyl-1-pentyloxy group, a 5-phenyl-2-pentyloxy group, a 1-phenyl-3-pentyloxy group, a 4-phenyl-2-methyl-2-butoxy group, a 4-phenyl-3-methyl-2-butoxy group, a 4-phenyl-2-methyl-2-butoxy group, a 6-phenyl-1-hexyloxy group, a 6-phenyl-2-hexyloxy group, a 6-phenyl-3-hexyloxy group, a 5-phenyl-2-methyl-1-pentyloxy group, a 1-phenyl-3-methyl-3-pentyloxy group, a 4-phenyl-2-ethyl-1-butoxy group, a 4-phenyl-2,3-dimethyl-1-butoxy group, a 7-phenyl-1-heptyloxy group, a 7-phenyl-3-heptyloxy group, a 1-phenyl-4-heptyloxy group, a 6-phenyl-3-methyl-3-hexyloxy group, a 1-phenyl-3-ethyl-3-pentyloxy group, a 8-phenyl-3-octyloxy group, a 8-phenyl-4-octyloxygroup, a 6-phenyl-3-ethyl-3-hexyloxy group, a 9-phenyl-4-nonyloxy group, a 1-phenyl-5-nonyloxy group, a 1-phenyl-4-ethyl-4-heptyloxy group, a 9-phenyl-4-decyloxy group, a 1-phenyl-5-decyloxy group or a 1-phenyl-4-(1-propyl)-4-heptyloxy group, and is preferably a phenyl-($C_1$-$C_6$ alkoxy) group, more preferably a phenyl-($C_1$-$C_4$ alkoxy) group, further preferably a phenyl-($C_1$-$C_3$ alkoxy) group, and most preferably a phenylmethoxy group or a 1-phenylethoxy group.

The "$C_1$-$C_{10}$ alkylamino group" in $Rf^9$ of the general formula (If) is an amino group substituted by one $C_1$-$C_{10}$ alkyl group described above and can include a methylamino group, an ethylamino group, a 1-propylamino group, a 2-propylamino group, a 1-butylamino group, a 2-butylamino group, a 2-methyl-1-propylamino group, a 2-methyl-2-propylamino group, a 1-pentylamino group, a 2-pentylamino group, a 3-pentylamino group, a 2-methyl-2-butylamino group, a 3-methyl-2-butylamino group, a 2-methyl-2-butylamino group, a 1-hexylamino group, a 2-hexylamino group, a 3-hexylamino group, a 2-methyl-1-pentylamino group, a 3-methyl-3-pentylamino group, a 2-ethyl-1-butylamino group, a 2,3-dimethyl-1-butylamino group, a 1-heptylamino group, a 3-heptylamino group, a 4-heptylamino group, a 3-methyl-3-hexylamino group, a 3-ethyl-3-pentylamino group, a 3-octylamino group, a 4-octylamino group, a 3-ethyl-3-hexylamino group, a 4-nonylamino group, a 5-nonylamino group, a 4-ethyl-4-heptylamino group, a 4-decylamino group, a 5-decylamino group or a 4-(1-propyl)-4-heptylamino group, and is preferably a $C_1$-$C_6$ alkylamino group, and is more preferably a $C_2$-$C_6$ alkylamino group, further preferably a $C_3$-$C_6$ alkylamino group, still more preferably a $C_3$-$C_5$ alkylamino group (particularly a 2-propylamino group, a 2-methyl-2-propylamino group or a 2-methyl-2-butylamino group), and most preferably a 2-methyl-2-propylamino group.

The "di($C_1$-$C_{10}$ alkyl)amino group" in $Rf^9$ of the general formula (If) is an amino group substituted by two same or different $C_1$-$C_{10}$ alkyl groups described above and can include a dimethylamino group, a methylethylamino group, a methylpropylamino group [for example, a N-(1-propyl)-N-methylamino group or the like], a methylbutylamino group [for example, a N-(1-butyl)-N-methylamino group, a N-methyl-N-(2-methyl-2-propyl)amino group or the like], a N-methyl-N-(2-methyl-2-butyl)amino group, a N-methyl-N-(3-methyl-3-pentyl)amino group, a N-methyl-N-(3-ethyl-3-pentyl)amino group, a N-methyl-N-(3-ethyl-3-hexyl)amino group, a N-methyl-N-(4-ethyl-4-heptyl)amino group, a N-methyl-N-[4-(1-propyl)-4-heptyl]amino group, a diethylamino group, an ethylpropylamino group [for example, a N-(1-propyl)-N-ethylamino group or the like], a N-ethyl-N-(2-methyl-2-propyl)amino group, a N-ethyl-N-(2-methyl-2-butyl)amino group, a N-ethyl-N-(3-methyl-3-pentyl)amino group, a N-ethyl-N-(3-ethyl-3-pentyl)amino group, a dipropylamino group [for example, a di(1-propyl)amino group, a di(2-propyl)amino group or the like], a N-(1-propyl)-N-(2-methyl-2-propyl)amino group, a dibutylamino group [for example, a di(1-butyl)amino group, a di(2-butyl)amino group or the like], a di(2-methyl-1-propyl) amino group, a di(2-methyl-2-propyl)amino group, a N-(1-butyl)-N-(2-methyl-2-propyl)amino group, a dipentylamino

group [for example, a di(1-pentyl)amino group, a di(2-pentyl)amino group, a di(3-pentyl)amino group or the like], a di(2-methyl-1-butyl)amino group, a di(2-ethyl-1-propyl)amino group, a N-(1-pentyl)-N-(2-methyl-2-propyl)amino group, a dihexylamino group [for example, a di(1-hexyl)amino group, a di(2-hexyl)amino group, a di(3-hexyl)amino group or the like], a di(2-methyl-1-pentyl)amino group, a di(3-methyl-1-pentyl)amino group, a di(4-methyl-1-pentyl)amino group, a di (2-methyl-2-pentyl)amino group, a di(3-methyl-2-pentyl)amino group, a di(4-methyl-2-pentyl)amino group, a di(2,2-dimethyl-1-butyl)amino group, a di(3,3-dimethyl-1-butyl)amino group, a di(2,3-dimethyl-1-butyl)amino group, a di(2-ethyl-1-butyl)amino group, a N-(1-hexyl)-N-(2-methyl-2-propyl)amino group, a diheptylamino group [for example, a di(1-heptyl) amino group, a di(2-heptyl)amino group or the like], a di(3-ethyl-3-pentyl)amino group, a dioctylamino group [for example, a di(1-octyl)amino group, a di(2-octyl)amino group, a di(4-octyl)amino group or the like], a di(3-ethyl-3-hexyl)amino group, a dinonylamino group [for example, a di(5-nonyl)amino group or the like], a di(4-ethyl-4-heptyl)amino group, a didecylamino group [for example, a di(5-decyl)amino group or the like] or a di[4-(1-propyl)-4-heptyl]amino group, and is preferably a di($C_1$-$C_6$ alkyl)amino group, more preferably a di($C_2$-$C_6$ alkyl)amino group or a N-($C_1$-$C_4$ alkyl)-N-($C_2$-$C_6$ alkyl)amino group, further preferably a di($C_3$-$C_6$ alkyl)amino group or a N-($C_1$-$C_4$ alkyl)-N-($C_3$-$C_6$ alkyl)amino group, still more preferably a di($C_3$-$C_5$ alkyl)amino group or a N-($C_1$-$C_4$ alkyl)-N-($C_3$-$C_5$ alkyl)amino group, most preferably a N-methyl-N-(2-methyl-2-propyl)amino group, a N-ethyl-N-(2-methyl-2-propyl)amino group, a N-(1-propyl)-N-(2-methyl-2-propyl)amino group, a N-(1-butyl)-N-(2-methyl-2-propyl)amino group or a di(2-methyl-2-propyl)amino group. Further, in the "di($C_1$-$C_{10}$ alkyl)amino group", said two alkyl groups may, together with the nitrogen atom of the amino group, form a 5- to 7-membered saturated heterocyclyl group containing 1 to 3 atoms selected from the group consisting of a nitrogen atom, an oxygen atom and a sulfur atom, and the 5- to 7-membered saturated heterocyclyl group can be, for example, a pyrrolidinyl group, a piperidyl group, a piperazinyl group, a morpholinyl group, a thiomorpholinyl group or a perhydroazepinyl group, and is preferably a 5- or 6-membered saturated heterocyclyl group containing 1 or 2 atoms selected from the group consisting of a nitrogen atom, an oxygen atom and a sulfur atom, more preferably a pyrrolidinyl group, a piperidyl group, a morpholinyl group or a thiomorpholinyl group, and further preferably a piperidyl group or a morpholinyl group.

The "halogeno $C_1$-$C_4$ alkyl group" in $Rf^2$, $Rf^4$, $Rf^5$ and Substituent group $\alpha$f of the general formula (If) is the above $C_1$-$C_4$ alkyl group substituted with 1 to 5 halogeno groups described below and can include a fluoromethyl group, a difluoromethyl group, a dichloromethyl group, a dibromomethyl group, a trifluoromethyl group, a trichloromethyl group, a 2-fluoroethyl group, a 2-bromoethyl group, a 2-chloroethyl group, a 2-iodoethyl group, a 2,2-difluoroethyl group, a 2,2,2-trifluoroethyl group, a 2,2,2-trichloroethyl group, a pentafluoroethyl group, a 3-fluoropropyl group, a 3-chloropropyl group, a 3,3,3-trifluoropropyl group, a 4-fluorobutyl group or a 4,4,4-trifluorobutyl group, and is preferably a halogeno $C_1$-$C_2$ alkyl group (said halogeno $C_1$-$C_2$ alkyl group represents a $C_1$-$C_2$ alkyl group substituted with 1 to 5 halogeno groups), more preferably a trifluoromethyl group, a 2,2,2-trifluoroethyl group or a pentafluoroethyl group, and most preferably a trifluoromethyl group.

The "$C_1$-$C_4$ alkylamino group" in $Rf^2$ of the general formula (If) is an amino group substituted by one $C_1$-$C_4$ alkyl group described above and can include a methylamino group, an ethylamino group, a propylamino group (for example, a 1-propylamino group and a 2-propylamino group), a 1-butylamino group, a 2-butylamino group, a 2-methyl-1-propylamino group or a 2-methyl-2-propylamino group, and is preferably a $C_1$-$C_3$ alkylamino group, more preferably a methylamino group or an ethylamino group, and most preferably a methylamino group.

The "di ($C_1$-$C_4$ alkyl)amino group" in $Rf^2$ of the general formula (If) is an amino group substituted by two same or different $C_1$-$C_4$ alkyl groups described above and can include a dimethylamino group, a methylethylamino group, a methylpropylamino group [for example, a N-(1-propyl)-N-methylamino group or the like], a methylbutylamino group [for example, a N-(1-butyl)-N-methylamino group or the like], a diethylamino group, an ethylpropylamino group [for example, a N-(1-propyl)-N-ethylamino group or the like], a dipropylamino group [for example, a di(1-propyl)amino group, a di(2-propyl)amino group or the like], a di(1-butyl)amino group, a di(2-butyl)amino group, a di(2-methyl-1-propyl)amino group or a di(2-methyl-2-propyl)amino group, and is preferably a di($C_1$-$C_3$ alkyl) amino group (said alkyl groups are the same or different), more preferably a dimethylamino group, a methylethylamino group, a methylpropylamino group, a diethylamino group, an ethylpropylamino group or a dipropylamino group, further preferably a dimethylamino group or a diethylamino group, and most preferably a dimethylamino group.

The "halogeno group" in $Rf^2$, $Rf^3$, $Rf^4$, $Rf^5$, Substituent group $\alpha$f, Substituent group $\beta$f and Substituent group $\gamma$f of the general formula (If) can include a fluoro group, a chloro group, a bromo group or an iodo group, and is preferably a fluoro group, a chloro group or a bromo group, more preferably a fluoro group or a chloro group, and most preferably a fluoro group.

The "$C_1$-$C_6$ alkyl group" in $Rf^3$, $Rf^{12}$, $Rf^{13}$, $Rf^{14}$, $Rf^{15}$, Substituent group $\beta$f and Substituent group $\gamma$f of the general formula (If) is a straight or branched alkyl group having 1 to 6 carbon atoms and can include a methyl group, an ethyl group, a 1-propyl group, a 2-propyl group, a 1-butyl group, a 2-butyl group, a 2-methyl-1-propyl group, a 2-methyl-2-propyl group, a 1-pentyl group, a 2-pentyl group, a 3-pentyl group, a 2-methyl-2-butyl group, a 3-methyl-2-butyl group, a 1-hexyl group, a 2-hexyl group, a 3-hexyl group, a 2-methyl-1-pentyl group, a 3-methyl-1-pentyl group, a 2-ethyl-1-butyl group, a 2,2-dimethyl-1-butyl group or a 2,3-dimethyl-1-butyl group, and is preferably a $C_1$-$C_4$ alkyl group,

more preferably a $C_1$-$C_3$ alkyl group (particularly a methyl group, an ethyl group or a propyl group), further preferably a methyl group or an ethyl group, and most preferably a methyl group.

The "halogeno $C_1$-$C_6$ alkyl group" in $Rf^3$ and Substituent group βf of the general formula (If) is the above $C_1$-$C_6$ alkyl group substituted by 1 to 7 halogeno groups described above and can include a fluoromethyl group, a difluoromethyl group, a dichloromethyl group, a dibromomethyl group, a trifluoromethyl group, a trichloromethyl group, a 2-fluoroethyl group, a 2-bromoethyl group, a 2-chloroethyl group, a 2-iodoethyl group, a 2,2-difluoroethyl group, a 2,2,2-trifluoroethyl group, a trichloroethyl group, a pentafluoroethyl group, a 3-fluoropropyl group, a 3-chloropropyl group, a 3,3,3-trifluoropropyl group, a 4-fluorobutyl group, a 4,4,4-trifluorobutyl group, a 5-fluoropentyl group, a 5,5,5-trifluoropentyl group, a 6-fluorohexyl group or a 6,6,6-trifluorohexyl group, and is preferably a halogeno $C_1$-$C_4$ alkyl group (said halogeno $C_1$-$C_4$ alkyl group represents a $C_1$-$C_4$ alkyl group substituted with 1 to 5 halogeno groups), more preferably a halogeno $C_1$-$C_4$ alkyl group (said halogeno $C_1$-$C_4$ alkyl group represents a $C_1$-$C_4$ alkyl group substituted with 1 to 5 fluoro, chloro or bromo groups), still more preferably a trifluoromethyl group, a 2,2,2-trifluoroethyl group or a pentafluoroethyl group, particularly preferably a trifluoromethyl group or a 2,2,2-trifluoroethyl group, and most preferably a trifluoromethyl group.

The "($C_1$-$C_4$ alkoxy)-($C_1$-$C_4$ alkyl) group" in $Rf^3$ of the general formula (If) is the above $C_1$-$C_4$ alkyl group substituted by one $C_1$-$C_4$ alkoxy group described above and can include a methoxymethyl group, an ethoxymethyl group, a (1-propoxy)methyl group, a (2-propoxy)methyl group, a (1-butoxy)methyl group, a (2-butoxy)methyl group, a (2-methyl-2-propoxy)methyl group, a methoxyethyl group, an ethoxyethyl group, a (1-propoxy)ethyl group, a (2-propoxy)ethyl group, a (1-butoxy)ethyl group, a (2-butoxy)ethyl group, a (2-methyl-2-propoxy)ethyl group, a methoxy(1-propyl) group, an ethoxy(1-propyl) group, a (1-propoxy)-(1-propyl) group, a (1-butoxy)-(1-propyl) group, a methoxy(1-butyl) group, an ethoxy(1-butyl) group, a (1-propoxy)-(1-butyl) group or a (1-butoxy)-(1-butyl) group, and is preferably a ($C_1$-$C_2$ alkoxy) - ($C_1$-$C_2$ alkyl) group, more preferably a methoxymethyl group or an ethoxymethyl group, and most preferably a methoxymethyl group.

The $C_1$-$C_4$ alkylthio moiety of the "($C_1$-$C_4$ alkylthio)-($C_1$-$C_4$ alkyl) group" in $Rf^3$ of the general formula (If) is a mercapto group substituted by one $C_1$-$C_4$ alkyl group described above and can include a methylthio group, an ethylthio group, a 1-propylthio group, a 2-propylthio group, a 1-butylthio group, a 2-butylthio group or a 2-methyl-2-propylthio group, and is preferably a $C_1$-$C_3$ alkylthio group, more preferably a methylthio group or an ethylthio group, and most preferably a methylthio group.

The " ($C_1$-$C_4$ alkylthio)-($C_1$-$C_4$ alkyl) group" in $Rf^3$ of the general formula (If) is the above $C_1$-$C_4$ alkyl group substituted by one $C_1$-$C_4$ alkylthio group described above and can include a methylthiomethyl group, an ethylthiomethyl group, a (1-propylthio)methyl group, a (2-propylthio)methyl group, a (1-butylthio)methyl group, a (2-butylthio)methyl group, a (2-methyl-2-propylthio)methyl group, a methylthioethyl group, an ethylthioethyl group, a (1-propylthio)ethyl group, a (2-propylthio)ethyl group, a (1-butylthio)ethyl group, a (2-butylthio)ethyl group, a (2-methyl-2-propylthio)ethyl group, a methylthio(1-propyl) group, an ethylthio(1-propyl) group, a (1-propylthio)-(1-propyl) group, a (1-butylthio)-(1-propyl) group, a methylthio(1-butyl) group, an ethylthio(1-butyl) group, a (1-propylthio)-(1-butyl) group or a (1-butylthio)-(1-butyl) group, and is preferably a ($C_1$-$C_2$ alkylthio) - ($C_1$-$C_2$ alkyl) group, more preferably a methylthiomethyl group or an ethylthiomethyl group, and most preferably a methylthiomethyl group.

The $C_1$-$C_4$ alkylsulfinyl moiety of the " ($C_1$-$C_4$ alkylsulfinyl)-($C_1$-$C_4$ alkyl) group" in $Rf^3$ of the general formula (If) is a sulfinyl group (-SO-) substituted by one $C_1$-$C_4$ alkyl group described above and can include a methylsulfinyl group, an ethylsulfinyl group, a 1-propylsulfinyl group, a 2-propylsulfinyl group, a 1-butylsulfinyl group, a 2-butylsulfinyl group or a 2-methyl-2-propylsulfinyl group, and is preferably a $C_1$-$C_3$ alkylsulfinyl group, more preferably a methylsulfinyl group or an ethylsulfinyl group, and most preferably a methylsulfinyl group.

The "($C_1$-$C_4$ alkylsulfinyl) - ($C_1$-$C_4$ alkyl) group" in $Rf^3$ of the general formula (If) is the above $C_1$-$C_4$ alkyl group substituted by one $C_1$-$C_4$ alkylsulfinyl group described above and can include a methylsulfinylmethyl group, an ethylsulfinylmethyl group, a (1-propylsulfinyl)methyl group, a (2-propylsulfinyl)methyl group, a (1-butylsulfinyl) methyl group, a (2-butylsulfinyl)methyl group or a (2-methyl-2-propylsulfinyl)methyl group, a methylsulfinylethyl group, an ethylsulfinylethyl group, a (1-propylsulfinyl)ethyl group, a (2-propylsulfinyl)ethyl group, a (1-butylsulfinyl)ethyl group, a (2-butylsulfinyl)ethyl group, a (2-methyl-2-propylsulfinyl)ethyl group, a methylsulfinyl(1-propyl) group, an ethylsulfinyl(1-propyl) group, a (1-propylsulfinyl)-(1-propyl) group, a (1-butylsulfinyl)-(1-propyl) group, a methylsulfinyl(1-butyl) group, an ethylsulfinyl(1-butyl) group, a (1-propylsulfinyl)-(1-butyl) group or a (1-butylsulfinyl)-(1-butyl) group, and is preferably a ($C_1$-$C_2$ alkylsulfinyl) - ($C_1$-$C_2$ alkyl) group, more preferably a methylsulfinylmethyl group or an ethylsulfinylmethyl group, and most preferably a methylsulfinylmethyl group.

The $C_1$-$C_4$ alkylsulfonyl moiety of the "($C_1$-$C_4$ alkylsulfonyl)-($C_1$-$C_4$ alkyl) group" in $Rf^3$ of the general formula (If) is a sulfonyl group (-$SO_2$-) substituted by one $C_1$-$C_4$ alkyl group described above and can include a methanesulfonyl group, an ethanesulfonyl group, a 1-propanesulfonyl group, a 2-propanesulfonyl group, a 1-butanesulfonyl group, a 2-butanesulfonyl group or a 2-methyl-2-propanesulfonyl group, and is preferably a $C_1$-$C_3$ alkylsulfonyl group, more preferably a methanesulfonyl group or an ethanesulfonyl group, and most preferably a methanesulfonyl group.

The "($C_1$-$C_4$ alkylsulfonyl) - ($C_1$-$C_4$ alkyl) group" in $Rf^3$ of the general formula (If) is the above $C_1$-$C_4$ alkyl group substituted by one $C_1$-$C_4$ alkylsulfonyl group described above and can include a methanesulfonylmethyl group, an

ethanesulfonylmethyl group, a (1-propanesulfonyl)methyl group, a (2-propanesulfonyl)methyl group, a (1-butanesulfonyl) methyl group, a (2-butanesulfonyl)methyl group, a (2-methyl-2-propanesulfonyl)methyl group, a methanesulfonylethyl group, an ethanesulfonylethyl group, a (1-propanesulfonyl)ethyl group, a (2-propanesulfonyl)ethyl group, a (1-butanesulfonyl)ethyl group, a (2-butanesulfonyl)ethyl group, a (2-methyl-2-propanesulfonyl)ethyl group, a methanesulfony(1-propyl) group, an ethanesulfonyl(1-propyl) group, a (1-propanesulfonyl)-(1-propyl) group, a (1-butanesulfonyl)-(1-propyl) group, a methanesulfonyl(1-butyl) group, an ethanesulfonyl(1-butyl) group, a (1-propanesulfonyl)-(1-butyl) group or a (1-butanesulfonyl)-(1-butyl) group, and is preferably a ($C_1$-$C_2$ alkylsulfonyl)-($C_1$-$C_2$ alkyl) group, more preferably a methanesulfonylmethyl group or an ethanesulfonylmethyl group, and most preferably a methanesulfonylmethyl group.

The "($C_1$-$C_4$ alkylamino) - ($C_1$-$C_4$ alkyl) group" in $Rf^3$ of the general formula (If) is the above $C_1$-$C_4$ alkyl group substituted by one $C_1$-$C_4$ alkylamino group described above and can include a methylaminomethyl group, an ethylaminomethyl group, a (1-propylamino)methyl group, a (2-propylamino)methyl group, a (1-butylamino)methyl group, a (2-butylamino)methyl group, a (2-methyl-2-propylamino)methyl group, a methylaminoethyl group, an ethylaminoethyl group, a (1-propylamino)ethyl group, a (2-propylamino)ethyl group, a (1-butylamino)ethyl group, a (2-butylamino)ethyl group, a (2-methyl-2-propylamino)ethyl group, a methylamino(1-propyl) group, an ethylamino(1-propyl) group, a (1-propylamino)-(1-propyl) group, a (1-butylamino)-(1-propyl) group, a methylamino(1-butyl) group, an ethylamino(1-butyl) group, a (1-propylamino)-(1-butyl) group or a (1-butylamino)-(1-butyl) group, and is preferably a ($C_1$-$C_2$ alkylamino)-($C_1$-$C_2$ alkyl) group, more preferably a methylaminomethyl group or an ethylaminomethyl group, and most preferably a methylaminomethyl group.

The di ($C_1$-$C_4$ alkylamino) - ($C_1$-$C_4$ alkyl) group" in $Rf^3$ of the general formula (If) is the above $C_1$-$C_4$ alkyl group substituted by two same or different $C_1$-$C_4$ alkylamino groups described above and can include a dimethylaminomethyl group, a methylethylaminomethyl group, a methylpropylaminomethyl group [for example, a [N-(1-propyl)-N-methylamino] methyl group or the like], a methylbutylaminomethyl group [for example, a [N-(1-butyl)-N-methylamino]methyl group or the like], a diethylaminomethyl group, an ethylpropylaminomethyl group [for example, a [N-(1-propyl)-N-ethylamino] methyl group or the like], a dipropylaminomethyl group [for example, a di(1-propyl)aminomethyl group, a di(2-propyl) aminomethyl group or the like], a dibutylaminomethyl group [for example, a di(1-butyl)aminomethyl group, a di(2-butyl) aminomethyl group], a di (2-methyl-1-propyl)aminomethyl group, a di(2-methyl-2-propyl)aminomethyl group, a dimethylaminoethyl group [for example, a 2-dimethylaminoethyl group or the like], a methylethylaminoethyl group [for example, a 2-(N-methyl-N-ethylamino)ethyl group or the like], a methylpropylaminoethyl group [for example, a 2-[N-methyl-N-(1-propyl)amino]ethyl group or the like], a methylbutylaminoethyl group [for example, a 2-[N-methyl-N-(1-butyl)amino]ethyl group or the like], a diethylaminoethyl group (for example, a 2-diethylaminoethyl group or the like), an ethylpropylaminoethyl group [for example, a 2-[N-(1-propyl)-N-ethylamino]ethyl group or the like], a dipropylaminoethyl group [for example, a 2-[di(1-propyl)amino]ethyl group or the like], a dibutylaminoethyl group [for example, a 2-di(1-butyl)aminoethyl group or the like], a di (2-methyl-1-propyl)aminoethyl group [for example, a 2-di(2-methyl-1-propyl)aminoethyl group or the like], a di (2-methyl-2-propyl)aminoethyl group [for example, a 2-di(2-methyl-2-propyl)aminoethyl group or the like], a dimethylaminopropyl group [for example, a 3-dimethylamino-1-propyl group or the like], a methylethylaminopropyl group [for example, a 3-(N-methyl-N-ethylamino)-1-propyl group or the like], a diethylaminopropyl group [for example, a 3-diethylamino-1-propyl group or the like], a dipropylaminopropyl group [for example, a 3-di(1-propyl)amino-1-propyl group or the like], a dibutylaminopropyl group [for example, a 3-di(1-butyl)amino-1-propyl group or the like], a dimethylaminobutyl group [for example, a 4-dimethylamino-1-butyl group or the like], a methylethylaminobutyl group [for example, a 4-(N-methyl-N-ethylamino)-1-butyl group or the like], a diethylaminobutyl group [for example, a 4-diethylamino-1-butyl group or the like], a dipropylaminobutyl group [for example, a 4-di(1-propyl)amino-1-butyl group or the like] or a dibutylaminobutyl group [for example, a 4-di(1-butyl)amino-1-butyl group or the like], and is preferably a di($C_1$-$C_2$ alkylamino)-($C_1$-$C_2$ alkyl) group, more preferably a dimethylaminomethyl group or a diethylaminomethyl group, and most preferably a dimethylaminomethyl group.

The "$C_3$-$C_6$ cycloalkyl group" in $Rf^3$, $Rf^4$ and $Rf^5$ of the general formula (If) is a cyclic alkyl group having 3 to 6 carbon atoms and can include a cyclopropyl group, a cyclobutyl group, a cyclopentyl group or a cyclohexyl group, and is preferably a $C_3$-$C_5$ cycloalkyl group, more preferably a $C_3$-$C_4$ cycloalkyl group, and most preferably a cyclopropyl group.

The "$C_2$-$C_6$ alkenyl group" in $Rf^3$ of the general formula (If) is an alkenyl group having 1 or 2 carbon-carbon double bonds and 2 to 6 carbon atoms and can include a vinyl group, a 2-propenyl group, a 2-butenyl group, a 1,3-butadien-1-yl group, a 2-methyl-2-propenyl group, a 2-pentenyl group, a 2-methyl-2-butenyl group or a 2-hexenyl group, and is preferably a $C_2$-$C_4$ alkenyl group, more preferably a $C_2$-$C_3$ alkenyl group, and most preferably a vinyl group.

The "$C_2$-$C_6$ alkynyl group" in $Rf^3$ of the general formula (If) is an alkynyl group having 1 or 2 carbon-carbon triple bonds and 2 to 6 carbon atoms and can include an ethynyl group, a 1-propynyl group, a 1-butynyl group, a 1,3-butadiyn-1-yl group, a 1-pentynyl group or a 1-hexynyl group, and is preferably a $C_2$-$C_4$ alkynyl group, more preferably a $C_2$-$C_3$ alkynyl group, and most preferably an ethynyl group.

The "$C_1$-$C_6$ alkoxy group" in $Rf^3$, $Rf^{11}$, Substituent group βf and Substituent group γf of the general formula (If) is a hydroxyl group substituted by one $C_1$-$C_6$ alkyl group described above and can include a methoxy group, an ethoxy group, a 1-propoxy group, a 2-propoxy group, a 1-butoxy group, a 2-butoxy group, a 2-methyl-1-propoxy group, a 2-methyl-2-propoxy group, a 1-pentyloxy group, a 2-pentyloxy group, a 3-pentyloxy group, a 2-methyl-2-butoxy group, a 3-methyl-2-butoxy group, a 1-hexyloxy group, a 2-hexyloxy group, a 3-hexyloxy group, a 2-methyl-1-pentyloxy group, a 3-methyl-1-pentyloxy group, a 2-ethyl-1-butoxy group, a 2,2-dimethyl-1-butoxy group or a 2,3-dimethyl-1-butoxy group, and is preferably a $C_1$-$C_4$ alkoxy group, more preferably a $C_1$-$C_3$ alkoxy group (particularly a methoxy group, an ethoxy group or a propoxy group), further preferably a methoxy group or an ethoxy group, and most preferably a methoxy group.

The "halogeno $C_1$-$C_6$ alkoxy group" in $Rf^3$ and Substituent group βf of the general formula (If) is the above $C_1$-$C_6$ alkyl group substituted with 1 to 7 halogeno groups described above and can include a fluoromethoxy group, a difluoromethoxy group, a dichloromethoxy group, a dibromomethoxy group, a trifluoromethoxy group, a trichloromethoxy group, a 2-fluoroethoxy group, a 2-bromoethoxy group, a 2-chloroethoxy group, a 2-iodoethoxy group, a 2,2-difluoroethoxy group, a 2,2,2-trifluoroethoxy group, a 2,2,2-trichloroethoxy group, a pentafluoroethoxy group, a 3,3,3-trifluoro-1-propoxy group, a 1,1,1-trifluoro-2-propoxy group, a 1,1,1-trichloro-2-propoxy group, a 4,4,4-trifluoro-l-butoxy group, a 4,4,4-trifluoro-2-butoxy group, a 2-trifluoromethyl-l-propoxy group, a 2-trifluoromethyl-2-propoxy group, a 5,5,5-trifluoro-1-pentyloxy group, a 5,5,5-trifluoro-2-pentyloxy group, a 1,1,1-trifluoro-3-pentyloxy group, a 4,4,4-trifluoro-2-methyl-2-butoxy group, a 4,4,4-trifluoro-3-methyl-2-butoxy group, a 4,4,4-trifluoro-2-methyl-2-butoxy group, a 6,6,6-trifluoro-1-hexyloxy group, a 6,6,6-trifluoro-2-hexyloxy group, a 6,6,6-trifluoro-3-hexyloxy group, a 5,5,5-trifluoro-2-methyl-1-pentyloxy group, a 1,1,1-trifluoro-3-methyl-3-pentyloxy group, a 6,6,6-trifluoro-2-ethyl-1-butoxy group, or a 6,6,6-trifluoro-2,3-dimethyl-1-butoxy group, and is preferably a halogeno $C_1$-$C_4$ alkoxy group (said halogeno $C_1$-$C_4$ alkoxy group represents a $C_1$-$C_4$ alkoxy group substituted with 1 to 5 halogeno groups), more preferably a halogeno $C_1$-$C_2$ alkoxy group (said halogeno $C_1$-$C_2$ alkoxy group represents a $C_1$-$C_2$ alkoxy group substituted with 1 to 5 fluoro, chloro or bromo groups), still more preferably a trifluoromethoxy group, a 2,2,2-trifluoroethoxy group or a pentafluoroethoxy group, and most preferably a trifluoromethoxy group.

The "$C_1$-$C_6$ alkylthio group" in $Rf^3$, Substituent group βf and Substituent group γf of the general formula (If) is a mercapto group substituted by one $C_1$-$C_6$ alkyl group described above and can include a methylthio group, an ethylthio group, a 1-propylthio group, a 2-propylthio group, a 1-butylthio group, a 2-butylthio group, a 2-methyl-1-propylthio group, a 2-methyl-2-propylthio group, a 1-pentylthio group, a 2-pentylthio group, a 3-pentylthio group, a 2-methyl-2-butylthio group, a 3-methyl-2-butylthio group, a 1-hexylthio group, a 2-hexylthio group, a 3-hexylthio group, a 2-methyl-1-pentylthio group, a 3-methyl-1-pentylthio group, a 2-ethyl-1-butylthio group, a 2,2-dimethyl-1-butylthio group or a 2,3-dimethyl-1-butylthio group, and is preferably a $C_1$-$C_4$ alkylthio group, more preferably a $C_1$-$C_3$ alkylthio group (particularly a methylthio group, an ethylthio group or a propylthio group), further preferably a methylthio group or an ethylthio group, and most preferably a methylthio group.

The "$C_1$-$C_6$ alkylsulfinyl group" in $Rf^3$, Substituent group βf and Substituent group γf of the general formula (If) is a sulfinyl group (-SO-) substituted by one $C_1$-$C_6$ alkyl group described above and can include a methylsulfinyl group, an ethylsulfinyl group, a 1-propylsulfinyl group, a 2-propylsulfinyl group, a 1-butylsulfinyl group, a 2-butylsulfinyl group, a 2-methyl-1-propylsulfinyl group, a 2-methyl-2-propylsulfinyl group, a 1-pentylsulfinyl group, a 2-pentylsulfinyl group, a 3-pentylsulfinyl group, a 2-methyl-2-butylsulfinyl group, a 3-methyl-2-butylsulfinyl group, a 1-hexylsulfinyl group, a 2-hexylsulfinyl group, a 3-hexylsulfinyl group, a 2-methyl-1-pentylsulfinyl group, a 3-methyl-1-pentylsulfinyl group, a 2-ethyl-1-butylsulfinyl group, a 2,2-dimethyl-1-butylsulfinyl group or a 2,3-dimethyl-1-butylsulfinyl group, and is preferably a $C_1$-$C_4$ alkylsulfinyl group, more preferably a $C_1$-$C_3$ alkylsulfinyl group (particularly a methylsulfinyl group, an ethylsulfinyl group or a propylsulfinyl group), further preferably a methylsulfinyl group or an ethylsulfinyl group, and most preferably a methylsulfinyl group.

The "$C_1$-$C_6$ alkylsulfonyl group" in $Rf^3$, Substituent group βf and Substituent group γf of the general formula (If) is a sulfonyl group ($-SO_2-$) substituted by one $C_1$-$C_6$ alkyl group described above and can include a methanesulfonyl group, an ethanesulfonyl group, a 1-propanesulfonyl group, a 2-propanesulfonyl group, a 1-butanesulfonyl group, a 2-butanesulfonyl group, a 2-methyl-1-propanesulfonyl group, a 2-methyl-2-propanesulfonyl group, a 1-pentanesulfonyl group, a 2-pentanesulfonyl group, a 3-pentanesulfonyl group, a 2-methyl-2-butansulfonyl group, a 3-methyl-2-butanesulfonyl group, a 1-hexanesulfonyl group, a 2-hexanesulfonyl group, a 3-hexanesulfonyl group, a 2-methyl-1-pentanesulfonyl group, a 3-methyl-1-pentanesulfonyl group, a 2-ethyl-1-butanesulfonyl group, a 2,2-dimethyl-1-butanesulfonyl group or a 2,3-dimethyl-1-butanesulfonyl group, and is preferably a $C_1$-$C_4$ alkylsulfonyl group, more preferably a $C_1$-$C_3$ alkylsulfonyl group (particularly a methanesulfonyl group, an ethanesulfonyl group or a propanesulfonyl group), further preferably a methanesulfonyl group or an ethanesulfonyl group, and most preferably a methanesulfonyl group.

The "$C_1$-$C_6$ alkylamino group" in $Rf^3$, $Rf^{11}$, $Rf^{12}$, Substituent group βf and Substituent group γf of the general formula (If) is an amino group substituted by one $C_1$-$C_6$ alkyl group described above and can include a methylamino group, an ethylamino group, a 1-propylamino group, a 2-propylamino group, a 1-butylamino group, a 2-butylamino group, a 2-methyl-1-propylamino group, a 2-methyl-2-propylamino group, a 1-pentylamino group, a 2-pentylamino group, a 3-pentylamino group, a 2-methyl-2-butylamino group, a 3-methyl-2-butylamino group, a 1-hexylamino group, a 2-hexylamino group, a 3-hexylamino group, a 2-methyl-1-pentylamino group, a 3-methyl-1-pentylamino group, a 2-ethyl-1-butylamino group, a 2,2-dimethyl-1-butylamino group or a 2,3-dimethyl-1-butylamino group, and is preferably a $C_1$-$C_4$ alkylamino group, more preferably a $C_1$-$C_3$ alkylamino group (particularly a methylamino group, an ethylamino group

or a propylamino group), further preferably a methylamino group or an ethylamino group, and most preferably a methylamino group.

The "di($C_1$-$C_6$ alkyl)amino group" in $Rf^3$, $Rf^{11}$, $Rf^{12}$, Substituent group βf and Substituent group γf of the general formula (If) is an amino group substituted by two same or different $C_1$-$C_6$ alkyl groups described above and can include a dimethylamino group, a methylethylamino group, a methylpropylamino group [for example, a N-(1-propyl)-N-methylamino group or the like], a methylbutylamino group [for example, a N-(1-butyl)-N-methylamino group or the like], a diethylamino group, an ethylpropylamino group [for example, a N-(1-propyl)-N-ethylamino group or the like], a dipropylamino group [for example, a di(1-propyl)amino group, a di(2-propyl)amino group or the like], a dibutylamino group [for example, a di(1-butyl)amino group, a di(2-butyl)amino group or the like], a di(2-methyl-1-propyl)amino group, a dipentylamino group [for example, a di(1-pentyl)amino group, a di(2-pentyl)amino group, a di(3-pentyl)amino group or the like] or a dihexylamino group [for example, a di(l-hexyl)amino group, a di(2-hexyl)amino group, a di(3-hexyl)amino group or the like], and is preferably a di($C_1$-$C_4$ alkyl)amino group, more preferably a di($C_1$-$C_3$ alkyl)amino group, further preferably a dimethylamino group or a diethylamino group, and most preferably a dimethylamino group. Further, in the "di($C_1$-$C_6$ alkyl)amino group", said two alkyl groups may, together with the nitrogen atom of the amino group, form a 5- to 7-membered saturated heterocyclyl group containing 1 to 3 atoms selected from the group consisting of the nitrogen atom, an oxygen atom and a sulfur atom, and the 5- to 7-membered saturated heterocyclyl group can be, for example, a pyrrolidinyl group, a piperidyl group, a piperazinyl group, a morpholinyl group, a thiomorpholinyl group or a perhydroazepinyl group, and is preferably a 5-or 6-membered saturated heterocyclyl group containing 1 or 2 atoms selected from the group consisting of a nitrogen atom, an oxygen atom and a sulfur atom, more preferably a pyrrolidinyl group, a piperidyl group, a morpholinyl group or a thiomorpholinyl group, and further preferably a piperidyl group or a morpholinyl group.

The "($C_1$-$C_6$ alkoxy)carbonyl group" in $Rf^3$ and Substituent group βf of the general formula (If) is a carbonyl group (-CO-) substituted by one $C_1$-$C_6$ alkoxy group described above and can include a methoxycarbonyl group, an ethoxycarbonyl group, a 1-propoxycarbonyl group, a 2-propoxycarbonyl group, a 1-butoxycarbonyl group, a 2-butoxycarbonyl group, a 2-methyl-1-propoxycarbonyl group, a 2-methyl-2-propoxycarbonyl group, a 1-pentyloxycarbonyl group, a 2-pentyloxycarbonyl group, a 3-pentyloxycarbonyl group, a 2-methyl-2-butoxycarbonyl group, a 3-methyl-2-butoxycarbonyl group, a 1-hexyloxycarbonyl group, a 2-hexyloxycarbonyl group, a 3-hexyloxycarbonyl group, a 2-methyl-1-pentyloxycarbonyl group, a 3-methyl-1-pentyloxycarbonyl group, a 2-ethyl-1-butoxycarbonyl group, a 2,2-dimethyl-1-butoxycarbonyl group or a 2,3-dimethyl-1-butoxycarbonyl group, and is preferably a ($C_1$-$C_4$ alkoxy)carbonyl group, more preferably a methoxycarbonyl group or an ethoxycarbonyl group, and most preferably a methoxycarbonyl group.

The "halogeno $C_1$-$C_4$ alkoxy group" in $Rf^4$ and $Rf^5$ of the general formula (If) is the above $C_1$-$C_4$ alkoxy group substituted with 1 to 5 halogeno groups described above and can include a fluoromethoxy group, a difluoromethoxy group, a dichloromethoxy group, a dibromomethoxy group, a trifluoromethoxy group, a trichloromethoxy group, a 2-fluoroethoxy group, a 2-bromoethoxy group, a 2-chloroethoxy group, a 2-iodoethoxy group, a 2,2-difluoroethoxy group, a 2,2,2-trifluoroethoxy group, a 2,2,2-trichloroethoxy group, a pentafluoroethoxy group, a 3,3,3-trifluoro-1-propoxy group, a 1,1,1-trifluoro-2-propoxy group, a 1,1,1-trichloro-2-propoxy group, a 4,4,4-trifluoro-1-butoxy group, a 4,4,4-trifluoro-2-butoxy group, a 2-trifluoromethyl-1-propoxy group or a 2-trifluoromethyl-2-propoxy group, and is preferably a halogeno $C_1$-$C_2$ alkoxy group (said halogeno $C_1$-$C_2$ alkoxy group represents a $C_1$-$C_2$ alkoxy group substituted with 1 to 5 halogeno groups), more preferably a trifluoromethoxy group, a 2,2,2-trifluoroethoxy group or a pentafluoroethoxy group, and most preferably a trifluoromethoxy group.

The "$C_1$-$C_3$ alkyl group" in $Rf^6$ and $Rf^7$ of the general formula (If) is a straight or branched alkyl group having 1 to 3 carbon atoms and can include a methyl group, an ethyl group, a 1-propyl group or a 2-propyl group, and is preferably a methyl group or an ethyl group, and most preferably a methyl group.

The " ($C_3$-$C_8$ cycloalkyl) - ($C_1$-$C_6$ alkyl) oxy group" in $Rf^{11}$ of the general formula (If) is the above $C_1$-$C_6$ alkoxy group substituted by one $C_3$-$C_8$ cycloalkyl group described below and can include a cyclopropylmethoxy group, a cyclobutylmethoxy group, a cyclopentylmethoxy group, a cyclohexylmethoxy group, a cyclohexylmethoxy group, a 1-cyclopropylethoxyl group, a 2-cyclopropylethoxy group, a 2-cyclobutylethoxy group, a 2-cyclopentylethoxy group, a 2-cyclohexylethoxy group, a 2-cycloheptylethoxy group, a 3-cyclopropyl-1-propoxy group, a 2-cyclopropyl-1-propoxy group, a 2-cyclopropyl-2-propoxy group, a 3-cyclobutyl-1-propoxy group, a 3-cyclopentyl-1-propoxy group, a 3-cyclohexyl-1-propoxy group, a 4-cyclopropyl-1-butoxy group, a 4-cyclopropyl-2-butoxy group, a 3-cyclopropyl-2-methyl-1-propoxy group, a 3-cyclopropyl-2-methyl-2-propoxy group, a 4-cyclobutyl-1-butoxy group, a 5-cyclopropyl-1-pentyloxy group, a 5-cyclopropyl-2-pentyloxy group, a 5-cyclopropyl-3-pentyloxy group, a 4-cyclopropyl-2-methyl-2-butoxy group, a 4-cyclopropyl-3-methyl-2-butoxy group, a 6-cyclopropyl-1-hexyloxy group, a 6-cyclopropyl-2-hexyloxy group, a 6-cyclopropyl-3-hexyloxy group, a 5-cyclopropyl-2-methyl-1-pentyloxy group, a 5-cyclopropyl-3-methyl-1-pentyloxy group, a 4-cyclopropyl-2-ethyl-1-butoxy group, a 4-cyclopropyl-2,2-dimethyl-1-butoxy group or a 4-cyclopropyl-2,3-dimethyl-1-butoxy group, and is preferably a ($C_3$-$C_6$ cycloalkyl) - ($C_1$-$C_4$ alkyl)oxy group, more preferably a ($C_3$-$C_5$ cycloalkyl)-($C_1$-$C_2$ alkyl)oxy group, further preferably a ($C_3$-$C_4$ cycloalkyl) - ($C_1$-$C_2$ alkyl) oxy group, and most preferably a cyclopropylmethyloxy group.

The "$C_3$-$C_8$ cycloalkyloxy group" in Rf[11] and Substituent group γf of the general formula (If) is a hydroxyl group substituted by one $C_3$-$C_8$ cycloalkyl group described below and can include a cyclopropyloxy group, a cyclobutyloxy group, a cyclopentyloxy group, a cyclohexyloxy group, a cycloheptyloxy group or a cyclooctyloxy group, and is preferably a $C_3$-$C_6$ cycloalkyloxy group, more preferably a $C_3$-$C_4$ cycloalkyloxy group, and most preferably a cyclopropyloxy group.

The "[($C_3$-$C_8$ cycloalkyl)-($C_1$-$C_6$ alkyl)]amino group" in Rf[11] and Rf[12] of the general formula (If) is the above $C_1$-$C_6$ alkylamino group substituted by one $C_3$-$C_8$ cycloalkyl group described below and can include a cyclopropylmethylamino group, a cyclobutylmethylamino group, a cyclopentylmethylamino group, a cyclohexylmethylamino group, a cyclohexylmethylamino group, a 1-cyclopropylethylamino group, a 2-cyclopropylethylamino group, a 2-cyclobutylethylamino group, a 2-cyclopentylethylamino group, a 2-cyclohexylethylamino group, a 2-cycloheptylethylamino group, a 3-cyclopropyl-1-propylamino group, a 2-cyclopropyl-1-propylamino group, a 2-cyclopropyl-2-propylamino group, a 3-cyclobutyl-1-propylamino group, a 3-cyclopentyl-1-propylamino group, a 3-cyclohexyl-1-propylamino group, a 4-cyclopropyl-1-butylamino group, a 4-cyclopropyl-2-butylamino group, a 3-cyclopropyl-2-methyl-1-propylamino group, a 3-cyclopropyl-2-methyl-2-propylamino group, a 4-cyclobutyl-1-butylamino group, a 5-cyclopropyl-1-pentylamino group, a 5-cyclopropyl-2-pentylamino group, a 5-cyclopropyl-3-pentylamino group, a 4-cyclopropyl-2-methyl-2-butylamino group, a 4-cyclopropyl-3-methyl-2-butylamino group, a 6-cyclopropyl-1-hexylamino group, a 6-cylopropyl-2-hexylamino group, a 6-cyclopropyl-3-hexylamino group, a 5-cyclopropyl-2-methyl-1-pentylamino group, a 5-cyclopropyl-3-methyl-1-pentylamino group, a 4-cyclopropyl-2-ethyl-1-butylamino group, a 4-cyclopropyl-2,2-dimethyl-1-butylamino group or a 4-cyclopropyl-2,3-dimethyl-1-butylamino group, and is preferably a ($C_3$-$C_6$ cycloalkyl) - ($C_1$-$C_4$ alkyl) amino group, more preferably a ($C_3$-$C_5$ cycloalkyl)-($C_1$-$C_2$ alkyl)amino group, further preferably a ($C_3$-$C_4$ cycloalkyl)-($C_1$-$C_2$ alkyl)amino group, and most preferably a cyclopropylmethylamino group.

The "$C_3$-$C_8$ cycloalkylamino group" in Rf[11], Rf[12], Substituent group βf and Substituent group γf of the general formula (If) is an amino group substituted by one $C_3$-$C_8$ cycloalkyl group described below and can include a cyclopropylamino group, a cyclobutylamino group, a cyclopentylamino group, a cyclohexylamino group, a cycloheptylamino group or a cyclooctylamino group, and is preferably a $C_3$-$C_6$ cycloalkylamino group, more preferably a $C_3$-$C_4$ cycloalkylamino group, and most preferably a cyclopropylamino group.

The "di[($C_3$-$C_8$ cycloalkyl)-($C_1$-$C_6$ alkyl)]amino group" in Rf[11] and Rf[12] of the general formula (If) is an amino group substituted by two same or different ($C_3$-$C_8$ cycloalkyl)-($C_1$-$C_6$ alkyl) groups described below and can include a di(cyclopropylmethyl)amino group, a N-cyclopropylmethyl-N-cyclobutylmethylamino group, a N-cyclopropylmethyl-N-cyclopentylmethylamino group, a N-cyclopropylmethyl-N-cyclohexylmethylamino group, a N-cyclopropylmethyl-N-cycloheptylmethylamino group, a N-cyclopropylmethyl-N-cyclooctylmethylamino group, a N-cyclopropylmethyl-N-cyclopropylethylamino group, a N-cyclopropylmethyl-N-(3-cyclopropyl-1-propyl)amino group, a di(cyclobutylmethyl)amino group, a di(cyclopentylmethyl)amino group, a di(cyclohexylmethyl)amino group, a di(cycloheptylmethyl)amino group or a di(cyclooctylmethyl)amino group, and is preferably a di[($C_3$-$C_6$ cycloalkyl) - ($C_1$-$C_4$ alkyl)] amino group, more preferably a di[($C_3$-$C_5$ cycloalkyl)-($C_1$-$C_2$ alkyl)]amino group, further preferably a di[($C_3$-$C_4$ cycloalkyl)-($C_1$-$C_2$ alkyl)]amino group, and most preferably a di(cyclopropylmethyl)amino group.

The "di ($C_3$-$C_8$ cycloalkyl) amino group" in Rf[11], Rf[12], Substituent group βf and Substituent group γf of the general formula (If) is an amino group substituted by two same or different $C_3$-$C_8$ cycloalkyl groups described below and can include a dicyclopropylamino group, a N-cyclopropyl-N-cyclobutylamino group, a N-cyclopropyl-N-cyclopentylamino group, a N-cyclopropyl-N-cyclohexylamino group, a N-cyclopropyl-N-cycloheptylamino group, a N-cyclopropyl-N-cyclooctylamino group, a dicyclobutylamino group, a dicyclopentylamino group, a dicyclohexylamino group, a dicycloheptylamino group or a dicyclooctylamino group, and is preferably a di($C_3$-$C_6$ cycloalkyl)amino group, more preferably a di($C_3$-$C_4$ cycloalkyl)amino group, and most preferably a dicyclopropylamino group.

The "N-[($C_3$-$C_8$ cycloalkyl)-($C_1$-$C_6$ alkyl)]-N-($C_1$-$C_6$ alkyl)amino group" in Rf[11] and Rf[12] of the general formula (If) is an amino group substituted by one ($C_3$-$C_8$ cycloalkyl)-($C_1$-$C_6$ alkyl) group described below and one $C_1$-$C_6$ alkyl group described above and can include a N-cyclopropylmethyl-N-methylamino group, a N-cyclopropylmethyl-N-ethylamino group, a N-cyclopropylmethyl-N-propylamino group, a N-cyclopropylmethyl-N-butylamino group, a N-cyclopropylmethyl-N-pentylamino group, a N-cyclopropylmethyl-N-hexylamino group, a N-cyclopropylethyl-N-methylamino group, a N-(3-cyclopropyl-1-propyl)-N-methylamino group, a N-cyclobutylmethyl-N-methylamino group, a N-cyclopentylmethyl-N-methylamino group, a N-cyclohexylmethyl-N-methylamino group, a N-cycloheptylmethyl-N-methylamino group or a N-cyclooctylmethyl-N-methylamino group, and is preferably a N-[($C_3$-$C_6$ cycloalkyl) - ($C_1$-$C_4$ alkyl)]-N-($C_1$-$C_4$ alkyl) amino group, more preferably a N-[($C_3$-$C_4$cycloalkyl) - ($C_1$-$C_2$ alkyl)]-N-($C_1$-$C_2$ alkyl)amino group, further preferably a N-[($C_3$-$C_4$ cycloalkyl)methyl]-N-methylamino group, and most preferably a N-cyclopropylmethyl-N-methylamino group.

The "N- ($C_3$-$C_B$ cycloalkyl) -N- ($C_1$-$C_6$ alkyl) amino group" in Rf[11], Rf[12], Substituent group βf and Substituent group γf of the general formula (If) is an amino group substituted by one $C_3$-$C_8$ cycloalkyl group described below and one $C_1$-$C_6$ alkyl group described above and can include a N-cyclopropyl-N-methylamino group, a N-cyclopropyl-N-ethylamino group, a N-cyclopropyl-N-propylamino group, a N-cyclopropyl-N-butylamino group, a N-cyclopropyl-N-pentylamino group, a N-cyclopropyl-N-hexylamino group, a N-cyclobutyl-N-methylamino group, a N-cyclopentyl-N-methylamino group, a N-cyclohexyl-N-methylamino group, a N-cycloheptyl-N-methylamino group or a N-cyclooctyl-N-methylamino group, and is preferably a N-($C_3$-$C_6$ cycloalkyl)-N-($C_1$-$C_4$ alkyl)amino group, more preferably a N-($C_3$-$C_4$ cycloalkyl) -N- ($C_1$-$C_2$ alkyl) amino group, further preferably a N-($C_3$-$C_4$ cycloalkyl)-N-methylamino group, and most preferably a N-cyclopropyl-N-methylamino group.

The "N- [ ($C_3$-$C_8$ cycloalkyl) - ($C_1$-$C_6$ alkyl) ] -N- ($C_3$-$C_8$ cycloalkyl)amino group" in $Rf^{11}$ and $Rf^{12}$ of the general formula (If) is an amino group substituted by one ($C_3$-$C_8$ cycloalkyl) - ($C_1$-$C_6$ alkyl) group described below and one $C_3$-$C_8$ cycloalkyl group described below and can include a N-cyclopropylmethyl-N-cyclopropylamino group, a N-cyclobutylmethyl-N-cyclopropylamino group, a N-cyclopentylmethyl-N-cyclopropylamino group, a N-cyclohexylmethyl-N-cyclopropylamino group, a N-cycloheptylmethyl-N-cyclopropylamino group, a N-cyclooctylmethyl-N-cyclopropylamino group, a N-cyclopropylethyl-N-cyclopropylamino group, a N-(3-cyclopropyl-1-propyl)-N-cyclopropylamino group, a N-cyclopropylmethyl-N-cyclobutylamino group or a N-cyclopropylmethyl-N-cyclopentylamino group, and is preferably a N- [($C_3$-$C_6$ cycloalkyl) - ($C_1$-$C_4$ alkyl)]-N-($C_3$-$C_6$ cycloalkyl)amino group, more preferably a N-[($C_3$-$C_4$ cycloalkyl)-($C_1$-$C_2$ alkyl) ] -N-($C_3$-$C_4$ cycloalkyl) amino group, further preferably a N-[($C_3$-$C_4$ cycloalkyl)methyl] -N- ($C_3$-$C_4$ cycloalkyl)amino group, and most preferably a N-cyclopropylmethyl-N-cyclopropylamino group.

The "hydroxy ($C_1$-$C_6$ alkyl)amino group" in $Rf^{11}$ of the general formula (If) is an amino group substituted by one $C_1$-$C_6$ alkyl group described above and one hydroxy group and can include a hydroxy(methyl)amino group, a hydroxy (ethyl)amino group, a hydroxy(1-propyl)amino group, a hydroxy(2-propyl)amino group, a hydroxy(1-butyl)amino group, a hydroxy(2-butyl)amino group, a hydroxy(2-methyl-1-propyl)amino group, a hydroxy(2-methyl-2-propyl)amino group, a hydroxy(1-pentyl)amino group, a hydroxy(2-pentyl)amino group, a hydroxy(3-pentyl)amino group, a hydroxy(2-methyl-2-butyl)amino group, a hydroxy(3-methyl-2-butyl)amino group, a hydroxy(2-methyl-2-butyl)amino group, a hydroxy(1-hexyl)amino group, a hydroxy(2-hexyl)amino group, a hydroxy(3-hexyl)amino group, a hydroxy(2-methyl-1-pentyl)amino group, a hydroxy(3-methyl-3-pentyl)amino group, a hydroxy(2-ethyl-1-butyl)amino group, a hydroxy(2,3-dimethyl-1-butyl)amino group, a hydroxy(1-heptyl)amino group, a hydroxy(3-heptyl)amino group, a hydroxy(4-heptyl)amino group, a hydroxy(3-methyl-3-hexyl)amino group, a hydroxy(3-ethyl-3-pentyl)amino group, a hydroxy(3-octyl)amino group, a hydroxy(4-octyl)amino group, a hydroxy(3-ethyl-3-hexyl)amino group, a hydroxy(4-nonyl)amino group, a hydroxy(5-nonyl)amino group, a hydroxy(4-ethyl-4-heptyl)amino group, a hydroxy(4-decyl)amino group, a hydroxy(5-decyl)amino group or a hydroxy[4-(1-propyl)-4-heptyl]amino group, and is preferably a hydroxy($C_1$-$C_4$ alkyl)amino group, more preferably a hydroxy(methyl)amino group or a hydroxy(ethyl)amino group, and most preferably a hydroxymethylamino group.

The " ($C_3$-$C_8$ cycloalkyl) - ($C_1$-$C_6$ alkyl) group" in $Rf^{12}$, $Rf^{13}$, $Rf^{14}$, $Rf^{15}$ and Substituent group βf of the general formula (If) is the above $C_1$-$C_6$ alkyl group substituted by one $C_3$-$C_8$ cycloalkyl group described below and can include a cyclopropylmethyl group, a cyclobutylmethyl group, a cyclopentylmethyl group, a cyclohexylmethyl group, a cycloheptylmethyl group, a cyclooctylmethyl group, a 1-cyclopropylethyl group, a 2-cyclopropylethyl group, a 2-cyclobutylethyl group, a 2-cyclopentylethyl group, a 2-cyclohexylethyl group, a 2-cycloheptylethyl group, a 3-cyclopropyl-1-propyl group, a 2-cyclopropyl-1-propyl group, a 2-cyclopropyl-2-propyl group, a 3-cyclobutyl-1-propyl group, a 3-cyclopentyl-1-propyl group, a 3-cyclohexyl-1-propyl group, a 4-cyclopropyl-1-butyl group, a 4-cyclopropyl-2-butyl group, a 3-cyclopropyl-2-methyl-1-propyl group, a 3-cyclopropyl-2-methyl-2-propyl group, a 4-cyclobutyl-1-butyl group, a 5-cyclopropyl-1-pentyl group, a 5-cyclopropyl-2-pentyl group, a 5-cyclopropyl-3-pentyl group, a 4-cyclopropyl-2-methyl-2-butyl group, a 4-cyclopropyl-3-methyl-2-butyl group, a 6-cyclopropyl-1-hexyl group, a 6-cyclopropyl-2-hexyl group, a 6-cyclopropyl-3-hexyl group, a 5-cyclopropyl-2-methyl-1-pentyl group, a 5-cyclopropyl-3-methyl-1-pentyl group, a 4-cyclopropyl-2-ethyl-1-butyl group, a 4-cyclopropyl-2,2-dimethyl-1-butyl group or a 4-cyclopropyl-2,3-dimethyl-1-butyl group, and is preferably a ($C_3$-$C_6$ cycloalkyl)-($C_1$-$C_4$ alkyl) group, more preferably a ($C_3$-$C_5$ cycloalkyl)-($C_1$-$C_2$ alkyl) group, further preferably a ($C_3$-$C_4$ cycloalkyl)-($C_1$-$C_2$ alkyl) group, still more preferably a cyclopropylmethyl group or a cyclopropylethyl group, and most preferably a cyclopropylmethyl group.

The "$C_3$-$C_8$ cycloalkyl group" in $Rf^{12}$, $Rf^{13}$, $Rf^{14}$, $Rf^{15}$ and Substituent group βf of the general formula (If) is a cyclic alkyl group having 3 to 8 carbon atoms and can include a cyclopropyl group, a cyclobutyl group, a cyclopentyl group, a cyclohexyl group, a cycloheptyl group or a cyclooctyl group, and is preferably a $C_3$-$C_6$ cycloalkyl group, more preferably a $C_3$-$C_5$ cycloalkyl group, further preferably a $C_3$-$C_4$ cycloalkyl group (a cyclopropyl group or a cyclobutyl group), and most preferably a cyclopropyl group.

The "$C_1$-$C_4$ alkylene group" in $Xf^2$ of the general formula (If) is an alkylene group having 1 to 4 carbon atoms and can include a methylene group, an ethylene group [$-(CH_2)_2-$] , a methylmethylene group [-CH(Me)-], a trimethylene group [$-(CH_2)_3-$], a methylethylene group [$-CH(Me)CH_2-$ or $-CH_2CH(Me)-$], a tetramethylene group [$-(CH_2)_4-$], a methyltrimethylene group [$-CH(Me)CH_2CH_2-$,- $CH_2CH(Me)CH_2-$ or $-CH_2CH_2CH(Me)-$], and is preferably a $C_1$-$C_3$ alkylene group, more preferably a methylene group or an ethylene group, and most preferably a methylene group.

The "5- or 6-membered aromatic heterocyclyl group" in $Yf^1$ of the general formula (If) is a 5- or 6-membered aromatic heterocyclic group containing 1 to 4 atoms selected from the group consisting of a nitrogen atom, an oxygen atom and a sulfur atom and can include a furyl group, a thienyl group, a pyrrolyl group, an imidazolyl group, a pyrazolyl group, an oxazolyl group, an isoxazolyl group, a thiazolyl group, an isothiazolyl group, a triazolyl group, a tetrazolyl group, an oxadiazolyl group, a thiadiazolyl group, a tetrazolyl group, a pyranyl group, a pyridyl group, a pyridazinyl group, a pyrimidinyl group or a pyradinyl group, and is preferably a pyrrolyl group, a furyl group, a thienyl group, an imidazolyl

group, an oxazolyl group, a thiazolyl group or a pyridyl group, more preferably a thienyl group or an pyridyl group, and most preferably a pyridyl group.

The "6- to 10-membered aryl group" in $Yf^2$ of the general formula (If) is a 6- to 10-membered aromatic hydrocarbon group and is, for example, a phenyl group or a naphthyl group, and is preferably a phenyl group.

The "9- or 10-membered unsaturated cyclic hydrocarbon group" in $Yf^2$ of the general formula (If) is a group in which a 9- or 10-membered aromatic hydrocarbon group is partially reduced; which is not a saturated hydrocarbon group; and in which the cyclic group bound to $Y^1$ is a phenyl group. The 9- or 10-membered unsaturated cyclic hydrocarbon group can include an indanyl group or a tetrahydronaphthyl group, and is preferably an indanyl group.

The "5- to 10-membered aromatic heterocyclyl group" in $Yf^2$ of the general formula (If) is a 5- to 10-membered aromatic heterocyclic group containing 1 to 4 atoms selected from the group consisting of a nitrogen atom, an oxygen atom and a sulfur atom and can include a furyl group, a thienyl group, a pyrrolyl group, a pyrazolyl group, an imidazolyl group, an oxazolyl group, an isoxazolyl group, a thiazolyl group, an isothiazolyl group, a triazolyl group, an oxadiazolyl group, a thiadiazolyl group, a tetrazolyl group, pyridyl group, a pyridazinyl group, a pyrimidinyl group, a pyradinyl group, an azepinyl group, an azocinyl group, an azoninyl group, an indolyl group, a benzofuranyl group, a benzothienyl group, a benzoimidazolyl group, a benzoxazolyl group, a benzoisoxazolyl group, a benzothiazolyl group, a benzoisothiazolyl group, a quinolyl group, an isoquinolyl group, a quinoquixalinyl group or a quinazolinyl group, and is preferably a 5- or 6-membered aromatic heterocyclyl group, more preferably a pyrrolyl group, a furyl group, a thienyl group, an imidazolyl group, an oxazolyl group, a thiazolyl group, a pyridyl group or a pyrimidinyl group, further preferably a thienyl group, a thiazolyl group or a pyridyl group, and most preferably a pyridyl group.

The "9- or 10-membered unsaturated heterocyclyl group" in $Yf^2$ of the general formula (If) is a group in which a 9- or 10-membered aromatic heterocyclyl group is partially reduced; which is not a saturated heterocyclyl group; and in which the cyclic group bound to $Yf^1$ is an aromatic ring group. The 9- or 10-membered unsaturated heterocyclyl group can include an indolynyl group, a dihydrobenzofuryl group, a dihydrobenzothienyl group, a tetrahydroquinolyl group or a chromanyl group, and is preferably an indolynyl group, a dihydrobenzofuryl group or a dihydrobenzothienyl group.

The "hydroxy($C_1$-$C_6$ alkyl) group" in Substituent group βf and Substituent group γf of the general formula (If) is the above $C_1$-$C_6$ alkyl group substituted by one hydroxy group and can include a hydroxymethyl group, a hydroxyethyl group, a hydroxy(1-propyl) group, a hydroxy(2-propyl) group, a hydroxy(1-butyl) group, a hydroxy(2-butyl) group, a hydroxy(2-methyl-1-propyl) group, a hydroxy(2-methyl-2-propyl) group, a hydroxy(1-pentyl) group or a hydroxy(1-hexyl) group, and is preferably a hydroxy($C_1$-$C_4$ alkyl) group, more preferably a hydroxy($C_1$-$C_3$ alkyl) group (particularly a hydroxymethyl group, a hydroxyethyl group or a hydroxypropyl group), further preferably a hydroxymethyl group or a hydroxyethyl group, and most preferably a hydroxymethyl group.

The "carboxy($C_1$-$C_6$ alkyl) group" in Substituent group βf of the general formula (If) is the above $C_1$-$C_6$ alkyl group substituted by one carboxy group and can include a carboxymethyl group, a carboxyethyl group, a carboxy(1-propyl) group, a carboxy(2-propyl) group, a carboxy(1-butyl) group, a carboxy(2-butyl) group, a carboxy(2-methyl-1-propyl) group, a carboxy(2-methyl-2-propyl) group, a carboxy(1-pentyl) group or a carboxy(1-hexyl) group, and is preferably a carboxy($C_1$-$C_4$ alkyl) group, more preferably a carboxy($C_1$-$C_3$ alkyl) group (particularly a carboxymethyl group, a carboxyethyl group or a carboxypropyl group), further preferably a carboxymethyl group or a carboxyethyl group, and most preferably a carboxymethyl group.

The "($C_1$-$C_6$ alkoxy) carbonyl-($C_1$-$C_6$ alkyl) group" in Substituent group βf of the general formula (If) is the above $C_1$-$C_6$ alkyl group substituted by one ($C_1$-$C_6$ alkoxy)carbonyl group described below and can include a methoxycarbonylmethyl group, an ethoxycarbonylmethyl group, a propoxycarbonylmethyl group, a butoxycarbonylmethyl group, a pentyloxycarbonylmethyl group, a hexyloxycarbonylmethyl group, a methoxycarbonylethyl group, a methoxycarbonylpropyl group, a methoxycarbonylbutyl group, a methoxycarbonylpentyl group or a methoxycarbonylhexyl group, and is preferably a ($C_1$-$C_4$ alkoxy)carbonyl-($C_1$-$C_4$ alkyl) group, more preferably a ($C_1$-$C_2$ alkoxy)carbonyl-($C_1$-$C_2$ alkyl) group, further preferably a methoxycarbonylmethyl group or a methoxycarbonylethyl group, and most preferably a methoxycarbonylmethyl group.

The "$C_2$-$C_7$ alkenyl group" in Substituent group βf of the general formula (If) is a straight or branched alkenyl group having 2 to 7 carbon atoms (which may have one or more carbon-carbon double bonds) and can include a vinyl group, a 2-propenyl group (allyl group), a 2-butenyl group, a 2-pentenyl group, a 3-methyl-2-butenyl group, a 2-hexenyl group, a 3-methyl-2-pentenyl group, a 2-heptenyl group or a 3-ethyl-2-pentenyl group, and is preferably a $C_2$-$C_5$ alkenyl group, more preferably a $C_2$-$C_4$ alkenyl group, and most preferably a vinyl group or a 2-propenyl group.

The "$C_2$-$C_7$ alkynyl group" in Substituent group βf of the general formula (If) is a straight or branched alkynyl group having 2 to 7 carbon atoms (which may have one or more carbon-carbon triple bonds) and can include an ethynyl group, a 2-propynyl group, a 2-butynyl group, a 2-pentynyl group, a 2-hexynyl group or a 2-heptynyl group, and is preferably a $C_2$-$C_5$ alkynyl group, more preferably a $C_2$-$C_4$ alkynyl group, and most preferably an ethynyl group or a 2-propynyl group.

The "($C_1$-$C_6$ alkyl)carbonylamino group" in Substituent group βf of the general formula (If) is a group in which the carbon atom of a carbonylamino group (-CONH-) is substituted by one $C_1$-$C_6$ alkyl group described above and can

include a methylcarbonylamino group, an ethylcarbonylamino group, a (1-propyl)carbonylamino group, a (2-propyl) carbonylamino group, a (1-butyl)carbonylamino group, a (2-butyl)carbonylamino group, a (2-methyl-1-propyl)carbonylamino group, a (2-methyl-2-propyl)carbonylamino group, a (1-pentyl)carbonylamino group or a (1-hexyl)carbonylamino group, and is preferably a ($C_1$-$C_4$ alkyl) carbonylamino group, more preferably a ($C_1$-$C_3$ alkyl)carbonylamino group, further preferably a methylcarbonylamino group or an ethylcarbonylamino group, and most preferably a methylcarbonylamino group.

The "($C_3$-$C_8$ cycloalkyl)carbonylamino group" in Substituent group βf of the general formula (If) is a group in which the carbon atom of a carbonylamino group (-CONH-) is substituted by one $C_3$-$C_8$ cycloalkyl group described above and can include a cyclopropylcarbonylamino group, a cyclobutylcarbonylamino group, a cyclopentylcarbonylamino group, a cyclohexylcarbonylamino group, a cycloheptylcarbonylamino group or a cyclooctylcarbonylamino group, and is preferably a ($C_3$-$C_6$ cycloalkyl)carbonylamino group, more preferably a ($C_3$-$C_5$ cycloalkyl)carbonylamino group, further preferably a ($C_3$-$C_4$ cycloalkyl)carbonylamino group (a cyclopropylcarbonylamino group or a cyclobutylcarbonylamino group), and most preferably a cyclopropylcarbonylamino group.

The "N-[($C_1$-$C_6$ alkyl) carbonyl] -N- ($C_1$-$C_6$ alkyl) amino group" in Substituent group βf of the general formula (If) is a group in which the nitrogen atom of the above ($C_1$-$C_6$ alkyl)carbonylamino group is substituted by one $C_1$-$C_6$ alkyl group described above and can include a N-methylcarbonyl-N-methylamino group, a N-ethylcarbonyl-N-methylamino group, a N-propylcarbonyl-N-methylamino group, a N-butylcarbonyl-N-methylamino group, a N-pentylcarbonyl-N-methylamino group, a N-hexylcarbonyl-N-methylamino group, a N-methylcarbonyl-N-ethylamino group, a N-methylcarbonyl-N-propylamino group, a N-methylcarbonyl-N-butylamino group, a N-methylcarbonyl-N-pentylamino group or a N-methylcarbonyl-N-hexylamino group, and is preferably a N-[($C_1$-$C_4$ alkyl)carbonyl]-N-($C_1$-$C_4$ alkyl) amino group, more preferably a N-[($C_1$-$C_2$ alkyl) carbonyl] -N-($C_1$-$C_2$ alkyl) amino group, further preferably a N-methylcarbonyl-N-methylamino group or a N-ethylcarbonyl-N-methylamino group, and most preferably a N-methylcarbonyl-N-methylamino group.

The "N- [($C_3$-$C_8$ cycloalkyl) carbonyl] -N- ($C_1$-$C_6$ alkyl)amino group" in Substituent group βf of the general formula (If) is a group in which the nitrogen atom of the above ($C_3$-$C_8$ cycloalkyl)carbonylamino group is substituted by one $C_1$-$C_6$ alkyl group described above and can include a N-cyclopropylcarbonyl-N-methylamino group, a N-cyclobutylcarbonyl-N-methylamino group, a N-cyclopentylcarbonyl-N-methylamino group, a N-cyclohexylcarbonyl-N-methylamino group, a N-cycloheptylcarbonyl-N-methylamino group, a N-cyclooctylcarbonyl-N-methylamino group, a N-cyclopropylcarbonyl-N-ethylamino group, a N-cyclopropylcarbonyl-N-propylamino group, a N-cyclopropylcarbonyl-N-butylamino group, a N-cyclopropylcarbonyl-N-pentylamino group or a N-cyclopropylcarbonyl-N-hexylamino group, and is preferably a N-[($C_3$-$C_6$ cycloalkyl) carbonyl] -N- ($C_1$-$C_4$ alkyl) amino group, more preferably a N-[($C_3$-$C_5$ cycloalkyl) carbonyl]-N- ($C_1$- $C_2$ alkyl)amino group, further preferably a N-[($C_3$-$C_4$ cycloalkyl) carbonyl] -N-($C_1$-$C_2$ alkyl) amino group, and most preferably a N-cyclopropylcarbonyl-N-methylamino group.

The "$C_1$-$C_6$ alkylsulfonylamino group" in Substituent group βf of the general formula (If) is an amino group substituted by one $C_1$-$C_6$ alkylsulfonyl group described above and can include a methanesulfonylamino group, an ethanesulfonylamino group, a 1-propanesulfonylamino group, a 2-propanesulfonylamino group, a 1-butanesulfonylamino group, a 2-butanesulfonylamino group, a 2-methyl-1-propanesulfonylamino group, a 2-methyl-2-propanesulfonylamino group, a 1-pentanesulfonylamino group, a 2-pentanesulfonylamino group, a 3-pentanesulfonylamino group, a 2-methyl-2-butanesulfonylamino group, a 3-methyl-2-butanesulfonylamino group, a 1-hexanesulfonylamino group, a 2-hexanesulfonylamino group, a 3-hexanesulfonylamino group, a 2-methyl-1-pentanesulfonylamino group, a 3-methyl-1-pentanesulfonylamino group, a 2-ethyl-1-butanesulfonylamino group, a 2,2-dimethyl-1-butanesulfonylamino group or a 2,3-dimethyl-1-butanesulfonylamino group, and is preferably a $C_1$-$C_4$ alkylsulfonylamino group, more preferably a methanesulfonylamino group or an ethanesulfonylamino group, and most preferably a methanesulfonylamino group.

The "N- ($C_1$-$C_6$ alkylsulfonyl) -N- ($C_1$-$C_6$ alkyl) amino group" in Substituent group βf of the general formula (If) is an amino group substituted by one $C_1$-$C_6$ alkylsulfonyl group described above and one $C_1$-$C_6$ alkyl group described above and can include a N-methanesulfonyl-N-methylamino group, a N-methanesulfonyl-N-ethylamino group, a N-methanesulfonyl-N-propylamino group, a N-methanesulfonyl-N-butylamino group, a N-methanesulfonyl-N-pentylamino group, a N-methanesulfonyl-N-hexylamino group, a N-ethanesulfonyl-N-methylamino group, a N-propanesulfonyl-N-methylamino group, a N-butanesulfonyl-N-methylamino group, a N-pentanesulfonyl-N-methylamino group or a N-hexanesulfonyl-N-methylamino group, and is preferably a N-($C_1$-$C_4$ alkylsulfonyl)-N-($C_1$-$C_4$ alkyl)amino group, more preferably a N-($C_1$-$C_2$ alkylsulfonyl)-N-($C_1$-$C_2$ alkyl) amino group, further preferably a N-methanesulfonyl-N-methylamino group or a N-ethanesulfonyl-N-methylamino group, and most preferably a N-methanesulfonyl-N-methylamino group.

The "N-($C_1$-$C_6$ alkylsulfonyl)-N-($C_3$-$C_8$ cycloalkyl) amino group" in Substituent group βf of the general formula (If) is an amino group substituted by one $C_1$-$C_6$ alkylsulfonyl group described above and one $C_3$-$C_8$ cycloalkyl group and can include a N-methanesulfonyl-N-cyclopropylamino group, a N-methanesulfonyl-N-cyclobutylamino group, a N-methanesulfonyl-N-cyclopentylamino group, a N-methanesulfonyl-N-cyclohexylamino group, a N-ethanesulfonyl-N-cyclopropylamino group, a N-propanesulfonyl-N-cyclopropylamino group, a N-butanesulfonyl-N-cyclopropylamino group, a N-pentanesulfonyl-N-cyclopropylamino group or a N-hexylsulfonyl-N-cyclopropylamino group, and is preferably a

N-($C_1$-$C_4$ alkylsulfonyl)-N-($C_3$-$C_6$ cycloalkyl) amino group, more preferably a N-($C_1$-$C_2$ alkylsulfonyl)-N-($C_3$-$C_4$ cycloalkyl) amino group, further preferably a N-methanesulfonyl-N-cyclopropylamino group or a N-ethanesulfonyl-N-cyclopropylamino group, and most preferably a N-methanesulfonyl-N-cyclopropylamino group.

The "($C_1$-$C_6$ alkyl)carbonyl group" in Substituent group βf of the general formula (If) is a carbonyl group (-CO-) substituted by one $C_1$-$C_6$ alkyl group described above and can include a methylcarbonyl group (an acetyl group), an ethylcarbonyl group, a (1-propyl)carbonyl group, a (2-propyl)carbonyl group, a (1-butyl)carbonyl group, a (2-butyl)carbonyl group, a (2-methyl-1-propyl)carbonyl group, a (2-methyl-2-propyl)carbonyl group, a (1-pentyl)carbonyl group or a (1-hexyl)carbonyl group, and is preferably a ($C_1$-$C_4$ alkyl)carbonyl group, more preferably a ($C_1$-$C_3$ alkyl)carbonyl group, further preferably a methylcarbonyl group or an ethylcarbonyl group, and most preferably a methylcarbonyl group.

The "($C_1$-$C_6$ alkylamino)carbonyl group" in Substituent group βf of the general formula (If) is a carbonyl group (-CO-) substituted by one $C_1$-$C_6$ alkylamino group described above and can include a methylaminocarbonyl group, an ethylaminocarbonyl group, a (1-propylamino)carbonyl group, a (2-propylamino)carbonyl group, a (1-butylamino)carbonyl group, a (2-butylamino)carbonyl group, a (2-methyl-1-propylamino)carbonyl group, a (2-methyl-2-propylamino)carbonyl group, a (1-pentylamino)carbonyl group or a (1-hexylamino)carbonyl group, and is preferably a ($C_1$-$C_4$ alkylamino) carbonyl group, more preferably a ($C_1$-$C_3$ alkylamino)carbonyl group, further preferably a methylaminocarbonyl group or an ethylaminocarbonyl group, and most preferably a methylaminocarbonyl group.

The "($C_3$-$C_8$ cycloalkylamino)carbonyl group" in Substituent group βf of the general formula (If) is a carbonyl group (-CO-) substituted by one $C_3$-$C_8$ cycloalkylamino group described above and can include a cyclopropylaminocarbonyl group, a cyclobutylaminocarbonyl group, a cyclopentylaminocarbonyl group, a cyclohexylaminocarbonyl group, a cycloheptylaminocarbonyl group or a cyclooctylaminocarbonyl group, and is preferably a $C_3$-$C_6$ cycloalkylaminocarbonyl group, more preferably a $C_3$-$C_4$ cycloalkylaminocarbonyl group, and most preferably a cyclopropylaminocarbonyl group.

The "di($C_1$-$C_6$ alkyl)aminocarbonyl group" in Substituent group βf of the general formula (If) is a carbonyl group (-CO-) substituted by one di($C_1$-$C_6$ alkyl)amino group described above and can include a dimethylaminocarbonyl group, a (N-methyl-N-ethylamino)carbonyl group, a (N-methyl-N-propylamino)carbonyl group [for example, an [N-(1-propyl)-N-methylamino]carbonyl group or the like], a (N-methyl-N-butylamino)carbonyl group [for example, an [N-(1-butyl)-N-methylamino]carbonyl group or the like], a (N-methyl-N-pentylamino)carbonyl group, a (N-methyl-N-hexylamino)carbonyl group, a diethylaminocarbonyl group, a dipropylaminocarbonyl group [for example, a di(1-propyl)aminocarbonyl group, a di(2-propyl)aminocarbonyl group or the like], a dibutylaminocarbonyl group, a dipentylaminocarbonyl group or a dihexylaminocarbonyl group, and is preferably a di($C_1$-$C_4$ alkyl)aminocarbonyl group (said alkyl groups are the same or different), more preferably a di($C_1$-$C_2$ alkyl)aminocarbonyl group (said alkyl groups are the same or different), further preferably a dimethylaminocarbonyl group or a diethylaminocarbonyl group, and most preferably a dimethylaminocarbonyl group. Further, in the di($C_1$-$C_6$ alkyl)aminocarbonyl group, said two alkyl groups may, together with the nitrogen atom of the amino group, form a 5- to 7-membered saturated heterocyclyl group containing 1 to 3 atoms selected from the group consisting of a nitrogen atom, an oxygen atom and a sulfur atom. In this case, the di($C_1$-$C_6$ alkyl)aminocarbonyl group can include a pyrrolidinylcarbonyl group, a piperidylcarbonyl group, a piperazinylcarbonyl group, a morpholinylcarbonyl group or a thiomorpholinylcarbonyl group, and is preferably a pyrrolidinylcarbonyl group, a piperidylcarbonyl group or a morpholinylcarbonyl group.

The "N- ($C_3$-$C_8$ cycloalkyl) -N- ($C_1$-$C_6$ alkyl) aminocarbonyl group" in Substituent group βf of the general formula (If) is a carbonyl group (-CO-) substituted by one N-($C_3$-$C_8$ cycloalkyl)-N-($C_1$-$C_6$ alkyl)amino group described above and can include a N-cyclopropyl-N-methylaminocarbonyl group, a N-cyclopropyl-N-ethylaminocarbonyl group, a N-cyclopropyl-N-propylaminocarbonyl group, a N-cyclopropyl-N-butylaminocarbonyl group, a N-cyclopropyl-N-pentylaminocarbonyl group, a N-cyclopropyl-N-hexylaminocarbonyl group, a N-cyclobutyl-N-methylaminocarbonyl group, a N-cyclopentyl-N-methylaminocarbonyl group, a N-cyclohexyl-N-methylaminocarbonyl group, a N-cycloheptyl-N-methylaminocarbonyl group or a N-cyclooctyl-N-methylaminocarbonyl group, and is preferably a N-($C_3$-$C_6$ cycloalkyl)-N-($C_1$-$C_4$ alkyl)aminocarbonyl group, more preferably a N-($C_3$-$C_4$ cycloalkyl) -N- ($C_1$-$C_2$ alkyl)aminocarbonyl group, further preferably a N-($C_3$-$C_4$ cycloalkyl)-N-methylaminocarbonyl group, and most preferably a N-cyclopropyl-N-methylaminocarbonyl group.

The " ($C_1$-$C_6$ alkoxy) - ($C_1$-$C_6$ alkyl) group" in Substituent group γf of the general formula (If) is the above $C_1$-$C_6$ alkyl group substituted by one $C_1$-$C_6$ alkoxy group described above and can include a methoxymethyl group, an ethoxymethyl group, a propoxymethyl group, a butoxymethyl group, a pentyloxymethyl group, a hexyloxymethyl group, a methoxyethyl group, a methoxypropyl group, a methoxybutyl group, a methoxypentyl group or a methoxyhexyl group, and is preferably a ($C_1$-$C_4$ alkoxy) - ($C_1$-$C_4$ alkyl) group, more preferably a ($C_1$-$C_2$ alkoxy)-($C_1$-$C_2$ alkyl) group, further preferably a methoxymethyl group or a methoxyethyl group, and most preferably a methoxymethyl group.

The "mercapto($C_1$-$C_6$ alkyl) group" in Substituent group γf of the general formula (If) is the above $C_1$-$C_6$ alkyl group substituted by one mercapto group and can include a mercaptomethyl group, a mercaptoethyl group, a mercapto (1-propyl) group, a mercapto(2-propyl) group, a mercapto(1-butyl) group, a mercapto(2-butyl) group, a mercapto(2-methyl-1-propyl) group, a mercapto(2-methyl-2-propyl) group, a mercapto(1-pentyl) group or a mercapto(1-hexyl) group,

and is preferably a mercapto($C_1$-$C_4$ alkyl) group, more preferably a mercapto($C_1$-$C_3$ alkyl) group (particularly a mercaptomethyl group, a mercaptoethyl group or a mercaptopropyl group), further preferably a mercaptomethyl group or a mercaptoethyl group, and most preferably a mercaptomethyl group.

The " ($C_1$-$C_6$ alkylthio) - ($C_1$-$C_6$ alkyl) group" in Substituent group γf of the general formula (If) is the above $C_1$-$C_6$ alkyl group substituted by one $C_1$-$C_6$ alkylthio group described above and can include a methylthiomethyl group, an ethylthiomethyl group, a propylthiomethyl group, a butylthiomethyl group, a pentylthiomethyl group, a hexylthiomethyl group, a methylthioethyl group, a methylthiopropyl group, a methylthiobutyl group, a methylthiopentyl group or a methylthiohexyl group, and is preferably a ($C_1$-$C_4$ alkylthio) - ($C_1$-$C_4$ alkyl) group, more preferably a ($C_1$-$C_2$ alkylthio) - ($C_1$-$C_2$ alkyl) group, further preferably a methylthiomethyl group or a methylthioethyl group, and most preferably a methylthiomethyl group.

The " ($C_1$-$C_6$ alkylsulfinyl) - ($C_1$-$C_6$ alkyl) group" in Substituent group γf of the general formula (If) is the above $C_1$-$C_6$ alkyl group substituted by one $C_1$-$C_6$ alkylsulfinyl group described above and can include a methylsulfinylmethyl group, an ethylsulfinylmethyl group, a propylsulfinylmethyl group, a butylsulfinylmethyl group, a pentylsulfinylmethyl group, a hexylsulfinylmethyl group, a methylsulfinylethyl group, a methylsulfinylpropyl group, a methylsulfinylbutyl group, a methylsulfinylpentyl group or a methylsulfinylhexyl group, and is preferably a ($C_1$-$C_4$ alkylsulfinyl) - ($C_1$-$C_4$ alkyl) group, more preferably a ($C_1$-$C_2$ alkylsulfinyl)-($C_1$-$C_2$ alkyl) group, further preferably a methylsulfinylmethyl group or a methylsulfinylethyl group, and most preferably a methylsulfinylmethyl group.

The " ($C_1$-$C_6$ alkylsulfonyl) - ($C_1$-$C_6$ alkyl) group" in Substituent group γf of the general formula (If) is the above $C_1$-$C_6$ alkyl group substituted by one $C_1$-$C_6$ alkylsulfonyl group described above and can include a methanesulfonylmethyl group, an ethanesulfonylmethyl group, a propanesulfonylmethyl group, a butanesulfonylmethyl group, a pentanesulfonylmethyl group, a hexanesulfonylmethyl group, a methanesulfonylethyl group, a methanesulfonylpropyl group, a methanesulfonylbutyl group, a methanesulfonylpentyl group or a methanesulfonylhexyl group, and is preferably a ($C_1$-$C_4$ alkylsulfonyl) - ($C_1$-$C_4$ alkyl) group, more preferably a ($C_1$-$C_2$ alkylsulfonyl) - ($C_1$-$C_2$ alkyl) group, further preferably a methanesulfonylmethyl group or a methanesulfonylethyl group, and most preferably a methanesulfonylmethyl group.

The "amino($C_1$-$C_6$ alkyl) group" in Substituent group γf of the general formula (If) is the above $C_1$-$C_6$ alkyl group substituted by one amino group and can include an aminomethyl group, an aminoethyl group, an amino(1-propyl) group, an amino(2-propyl) group, an amino(1-butyl) group, an amino(2-butyl) group, an amino(2-methyl-1-propyl) group, an amino(2-methyl-2-propyl) group, an amino(1-pentyl) group or an amino(1-hexyl) group, and is preferably an amino($C_1$-$C_4$ alkyl) group, more preferably an amino ($C_1$-$C_3$ alkyl) group (particularly an aminomethyl group, an aminoethyl group or an aminopropyl group), further preferably an aminomethyl group or an aminoethyl group, and most preferably an aminomethyl group.

The "($C_1$-$C_6$ alkylamino) - ($C_1$-$C_6$ alkyl) group" in Substituent group γf of the general formula (If) is the above $C_1$-$C_6$ alkyl group substituted by one $C_1$-$C_6$ alkylamino group described above and can include a methylaminomethyl group, an ethylaminomethyl group, a (1-propylamino)methyl group, a (2-propylamino)methyl group, a (1-butylamino) methyl group, a (2-butylamino)methyl group, a (2-methyl-2-propylamino)methyl group, a methylaminoethyl group, an ethylaminoethyl group, a (1-propylamino)ethyl group, a (2-propylamino)ethyl group, a (1-butylamino)ethyl group, a (2-butylamino)ethyl group, a (2-methyl-2-propylamino)ethyl group, a methylamino(1-propyl) group, an ethylamino(1-propyl) group, a (1-propylamino)-(1-propyl) group, a (1-butylamino)-(1-propyl) group, a methylamino(1-butyl) group, an ethylamino(1-butyl) group, a (1-propylamino)-(1-butyl) group, a (1-butylamino)-(1-butyl) group, a methylamino(1-pentyl) group or a methylamino(1-hexyl) group, and is preferably a ($C_1$-$C_4$ alkylamino)-($C_1$-$C_4$ alkyl) group, more preferably a ($C_1$-$C_2$ alkylamino) - ($C_1$-$C_2$ alkyl) group, further preferably a methylaminomethyl group, an ethylaminomethyl group or a methylaminoethyl group, and most preferably a methylaminomethyl group.

The " ($C_3$-$C_8$ cycloalkylamino) - ($C_1$-$C_6$ alkyl) group" in Substituent group γf of the general formula (If) is one $C_1$-$C_6$ alkyl group described above substituted by one $C_3$-$C_8$ cycloalkylamino group described above and can include a cyclopropylaminomethyl group, a cyclobutylaminomethyl group, a cyclopentylaminomethyl group, a cyclohexylaminomethyl group, a cycloheptylaminomethyl group, a cyclooctylaminomethyl group, a cyclopropylaminoethyl group, a cyclopropylaminopropyl group, a cyclopropylaminobutyl group, a cyclopropylaminopentyl group or a cyclopropylaminohexyl group, and is preferably a ($C_3$-$C_6$ cycloalkylamino)-($C_1$-$C_4$ alkyl) group, more preferably a ($C_3$-$C_4$ cycloalkylamino)-($C_1$-$C_2$ alkyl) group, further preferably a cyclopropylaminomethyl group or a cyclopropylaminoethyl group, and most preferably a cyclopropylaminomethyl group.

The "di ($C_1$-$C_6$ alkyl) amino- ($C_1$-$C_6$ alkyl) group" in Substituent group γf of the general formula (If) is a $C_1$-$C_6$ alkyl group substituted by one di($C_1$-$C_6$ alkyl)amino group described above and can include a dimethylaminomethyl group, a (N-methyl-N-ethylamino)methyl group, a (N-methyl-N-propylamino)methyl group, a (N-methyl-N-butylamino) methyl group, a (N-methyl-N-pentylamino)methyl group, a (N-methyl-N-hexylamino)methyl group, a diethylaminomethyl group, a dimethylaminoethyl group, a dimethylaminopropyl group, a dimethylaminobutyl group, a dimethylaminopentyl group or a dimethylaminohexyl group, and is preferably a di($C_1$-$C_4$ alkyl)amino-($C_1$-$C_4$ alkyl) group (said alkyl groups are the same or different), more preferably a di($C_1$-$C_2$ alkyl)amino-($C_1$-$C_2$ alkyl) group (said alkyl groups are the same or different), further preferably a dimethylaminomethyl group, a dimethylaminoethyl group or a (N-methyl-N-ethylamino)methyl group, still more preferably a dimethylaminomethyl group or a (N-methyl-N-ethylamino)methyl group, and most preferably a dimethylaminomethyl group. In the di($C_1$-$C_6$ alkyl)amino-($C_1$-$C_6$ alkyl) group, said two alkyl groups in the di($C_1$-$C_6$ alkyl)amino moiety may, together with the nitrogen atom of the amino group, form a 5- to 7-membered saturated heterocyclyl group containing 1 to 3 atoms selected from the group consisting of a nitrogen atom, an oxygen atom and a sulfur atom. In this case, the di($C_1$-$C_6$ alkyl)aminocarbonyl group can include a pyrrolidinylmethyl group, a piperidylmethyl group, a piperazinylmethyl group, a morpholinylmethyl group or a thiomorpholinylmethyl group, and is preferably a pyrrolidinylmethyl group, a piperidylmethyl group or a morpholinylmethyl group.

The "di($C_3$-$C_8$ cycloalkyl)amino-($C_1$-$C_6$ alkyl) group" in Substituent group γf of the general formula (If) is a $C_1$-$C_6$ alkyl group substituted by one di($C_3$-$C_8$ cycloalkyl)amino group described above and can include a dicyclopropylaminomethyl group, a (N-cyclopropyl-N-cyclobutylamino)methyl group, a (N-cyclopropyl-N-cyclopentylamino)methyl group, a (N-cyclopropyl-N-cyclohexylamino)methyl group, a (N-cyclopropyl-N-cycloheptylamino)methyl group, a (N-cyclopropyl-N-cyclooctylamino)methyl group, a dicyclobutylaminomethyl group, a dicyclopentylaminomethyl group, a dicyclohexylaminomethyl group, a dicycloheptylaminomethyl group, a dicyclooctylaminomethyl group, a dicyclopropylaminoethyl group, a dicyclopropylaminopropyl group, a dicyclopropylaminobutyl group, a dicyclopropylaminopentyl group or a dicyclopropylaminohexyl group, and is preferably a di($C_3$-$C_6$ cycloalkyl) amino- ($C_1$-$C_4$ alkyl) group, more preferably a di ($C_3$-$C_4$ cycloalkyl)amino-($C_1$-$C_2$ alkyl) group, and most preferably a dicyclopropylaminomethyl group.

The "[N-($C_3$-$C_8$ cycloalkyl)-N-($C_1$-$C_6$ alkyl) amino] - ($C_1$-$C_6$ alkyl) group" in Substituent group γf of the general formula (If) is a $C_1$-$C_6$ alkyl group substituted by one N-($C_3$-$C_8$ cycloalkyl) -N- ($C_1$-$C_6$ alkyl) amino group described above and can include a (N-cyclopropyl-N-methylamino)methyl group, a (N-cyclopropyl-N-ethylamino)methyl group, a (N-cyclopropyl-N-propylamino)methyl group, a (N-cyclopropyl-N-butylamino)methyl group, a (N-cyclopropyl-N-pentylamino)methyl group, a (N-cyclopropyl-N-hexylamino)methyl group, a (N-cyclobutyl-N-methylamino)methyl group, a (N-cyclopentyl-N-methylamino)methyl group, a (N-cyclohexyl-N-methylamino)methyl group, a (N-cycloheptyl-N-methylamino)methyl group, a (N-cyclooctyl-N-methylamino)methyl group, a (N-cyclopropyl-N-methylamino)ethyl group, a (N-cyclopropyl-N-methylamino)propyl group, a (N-cyclopropyl-N-methylamino)butyl group, a (N-cyclopropyl-N-methylamino) pentyl group or a (N-cyclopropyl-N-methylamino)hexyl group, and is preferably a [N-($C_3$-$C_6$ cycloalkyl)-N-($C_1$-$C_4$ alkyl) amino]-($C_1$-$C_4$ alkyl) group, more preferably a [N-($C_3$-$C_4$ cycloalkyl)-N-($C_1$-$C_2$ alkyl)amino]-($C_1$-$C_2$ alkyl) group, and most preferably a (N-cyclopropyl-N-methylamino)methyl group.

The "$C_3$-$C_8$ cycloalkylthio group" in Substituent group γf of the general formula (If) is a mercapto group substituted by one $C_3$-$C_8$ cycloalkyl group described above and can include a cyclopropylthio group, a cyclobutylthio group, a cyclopentylthio group, a cyclohexylthio group, a cycloheptylthio group or a cyclooctylthio group, and is preferably a $C_3$-$C_6$ cycloalkylthio group, more preferably a $C_3$-$C_5$ cycloalkylthio group, further preferably a $C_3$-$C_4$ cycloalkylthio group (a cyclopropylthio group or a cyclobutylthio group), and most preferably a cyclopropylthio group.

The "$C_3$-$C_8$ cycloalkylsulfinyl group" in Substituent group γf of the general formula (If) is a sulfinyl group (-SO-) substituted by one $C_3$-$C_8$ cycloalkyl group described above and can include a cyclopropylsulfinyl group, a cyclobutylsulfinyl group, a cyclopentylsulfinyl group, a cyclohexylsulfinyl group, a cycloheptylsulfinyl group or a cyclooctylsulfinyl group, and is preferably a $C_3$-$C_6$ cycloalkylsulfinyl group, more preferably a $C_3$-$C_5$ cycloalkylsulfinyl group, further preferably a $C_3$-$C_4$ cycloalkylsulfinyl group (a cyclopropylsulfinyl group or a cyclobutylsulfinyl group), and most preferably a cyclopropylsulfinyl group.

The "$C_3$-$C_8$ cycloalkylsulfonyl group" in Substituent group γf of the general formula (If) is a sulfonyl group (-$SO_2$-) substituted by one $C_3$-$C_8$ cycloalkyl group described above and can include a cyclopropanesulfonyl group, a cyclobutanesulfonyl group, a cyclopentanesulfonyl group, a cyclohexanesulfonyl group, a cycloheptanesulfonyl group or a cyclooctanesulfonyl group, and is preferably a $C_3$-$C_6$ cycloalkylsulfonyl group, more preferably a $C_3$-$C_5$ cycloalkylsulfonyl group, further preferably a $C_3$-$C_4$ cycloalkylsulfonyl group (a cyclopropanesulfonyl group or a cyclobutanesulfonyl group), and most preferably a cyclopropanesulfonyl group.

In the general formula (If), $Xf^1$ is preferably a group having the formula: -NH-, -O- or -S-, more preferably a group having the formula: -O-.

In the general formula (If), when $Yf^1$ is a phenyl group or a substituted phenyl group, the substitution positions at which $Xf^1$ and $Yf^2$ bind to $Yf^1$ are preferably the 1- and 3-positions (represented by the following $Yf^{1a}$) or the 1- and 4-positions (represented by the following $Yf^{1b}$), and more preferably the 1- and 4-positions. When $Yf^1$ is a thienyl group or a substituted thienyl group, the substitution positions of $Xf^1$ and $Yf^2$ are preferably the 2-and 4-positions or the 2- and 5-positions (represented by the following $Yf^{1c}$), and more preferably the 2- and 5-positions. When $Yf^1$ is a pyridyl group or a substituted pyridyl group, the substitution positions of $Xf^1$ and $Yf^2$ are preferably the 2- and 4-positions, the 2- and 5-positions (represented by the following $Yf^{1d}$), the 3- and 5-positions or the 3- and 6-positions (represented by the following $Yf^{1e}$), and more preferably the 2- and 5-positions or the 3- and 6-positions.

[Chemical formula 12]

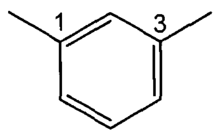
( $Yf^{1a}$ )

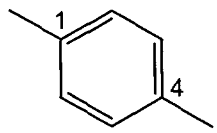
( $Yf^{1b}$ )

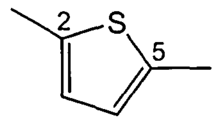
( $Yf^{1c}$ )

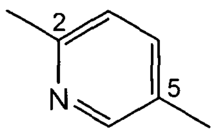
( $Yf^{1d}$ )

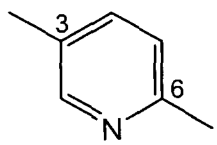
( $Yf^{1e}$ )

In the general formula (If), when $Yf^2$ is a phenyl group or a substituted phenyl group, the substitution positions at which $Yf^1$ and $Rf^8$ bind to $Yf^2$ are preferably the 1- and 3-positions (represented by the following $Yf^{2a}$) or the 1- and 4-positions (represented by the following $Yf^{2b}$), and more preferably the 1- and 4-positions. When $Yf^2$ is a thienyl group or a substituted thienyl group, the substitution positions of $Yf^1$ and $Rf^8$ are preferably the 2-and 4-positions (represented by the following $Yf^{2c}$), the 2-and 5-positions (represented by the following $Yf^{2d}$) or the 3- and 5-positions (represented by the following $Yf^{2e}$), and more preferably the 2- and 5-positions. When $Yf^2$ is a thiazolyl group or a substituted thiazolyl group, the substitution positions of $Yf^1$ and $Rf^8$ are preferably the 2-and 4-positions (represented by the following $Yf^{2f}$), the 2-and 5-positions (represented by the following $Yf^{2g}$) or the 5- and 2-positions (represented by the following $Yf^{2h}$), and more preferably the 2- and 5-positions. When $Yf^1$ is a pyridyl group or a substituted pyridyl group, the substitution positions of $Yf^1$ and $Rf^8$ are preferably the 2-and 4-positions (represented by the following $Yf^{2i}$), the 2-and 5-positions (represented by the following $Yf^{2j}$) or the 3- and 5-positions (represented by the following $Yf^{2k}$), and more preferably the 2- and 5-positions.

[Chemical formula 13]

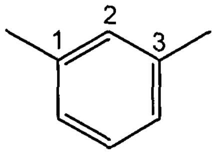

( $Yf^{2a}$ )

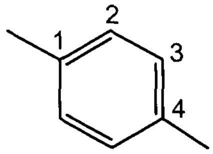

( $Yf^{2b}$ )

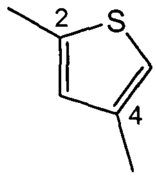

( $Yf^{2c}$ )

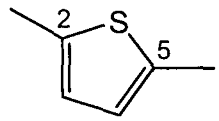

( $Yf^{2d}$ )

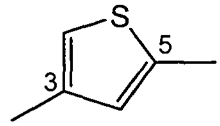

( $Yf^{2e}$ )

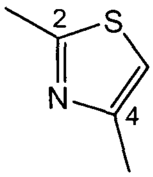

( $Yf^{2f}$ )

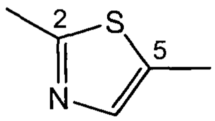

( $Yf^{2g}$ )

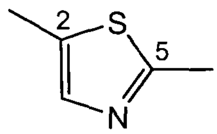

( $Yf^{2h}$ )

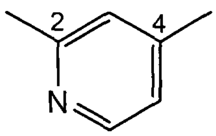

( $Yf^{2i}$ )

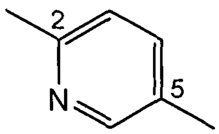

( $Yf^{2j}$ )

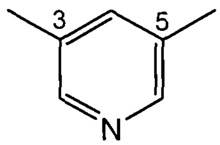

( $Yf^{2k}$ )

In the general formula (If), when $Yf^2$ is a substituted phenyl group, said substituent of $Yf^2$ is preferably a group selected from Substituent group βf1, more preferably a group selected from Substituent group βf2, further preferably a group selected from Substituent group βf3, and most preferably a methyl group, a fluoro group or a chloro group. When $Yf^2$ is the above $Yf^{2b}$, the substitution position of said substituent of $Yf^2$ is preferably the 2- or 3-position, and more preferably the 3-position. Said substituent of $Yf^2$ is particularly preferably a 2-methyl group, a 3-fluoro group or a 3-chloro group.

Further, in the general formula (If), the following compounds are preferable:

(i) a compound in which $Rf^8$ is a group having the formula: $-Xt^{2e}Rt^{10e}$ [wherein $Rf^{10e}$ represents a group having the formula: $-CORf^{11e}$ (wherein $Rf^{11e}$ represents a hydroxyl group or a methoxy group), and $Xf^{2e}$ represents a methylene group or a substituted methylene group (said substituent is one hydroxymethyl group, or two substituents together with each other form an ethylene group)], and
$Yf^2$ is a substituted phenyl group represented by the above $Yf^{2a}$ (said substituent is one group selected from Substituent group βf3 and the substitution positions at which $Yf^1$, $Rf^8$ and the group selected from Substituent group βf3 that bind to said phenyl group are the 1-, 3- and 2-positions, respectively); or
(ii) a compound in which $Rf^8$ is a group having the formula: $-SO_2Me$ and $Yf^2$ is a phenyl group represented by the above $Yf^{2a}$ (the substitution positions at which $Yf^1$ and $Rf^8$ bind to said phenyl group are the 1- and 3-positions).

The compounds represented by the general formula (If) of the present invention can be prepared according to the following Method Af to Method Pf.

[Chemical formula 14]

Method Af

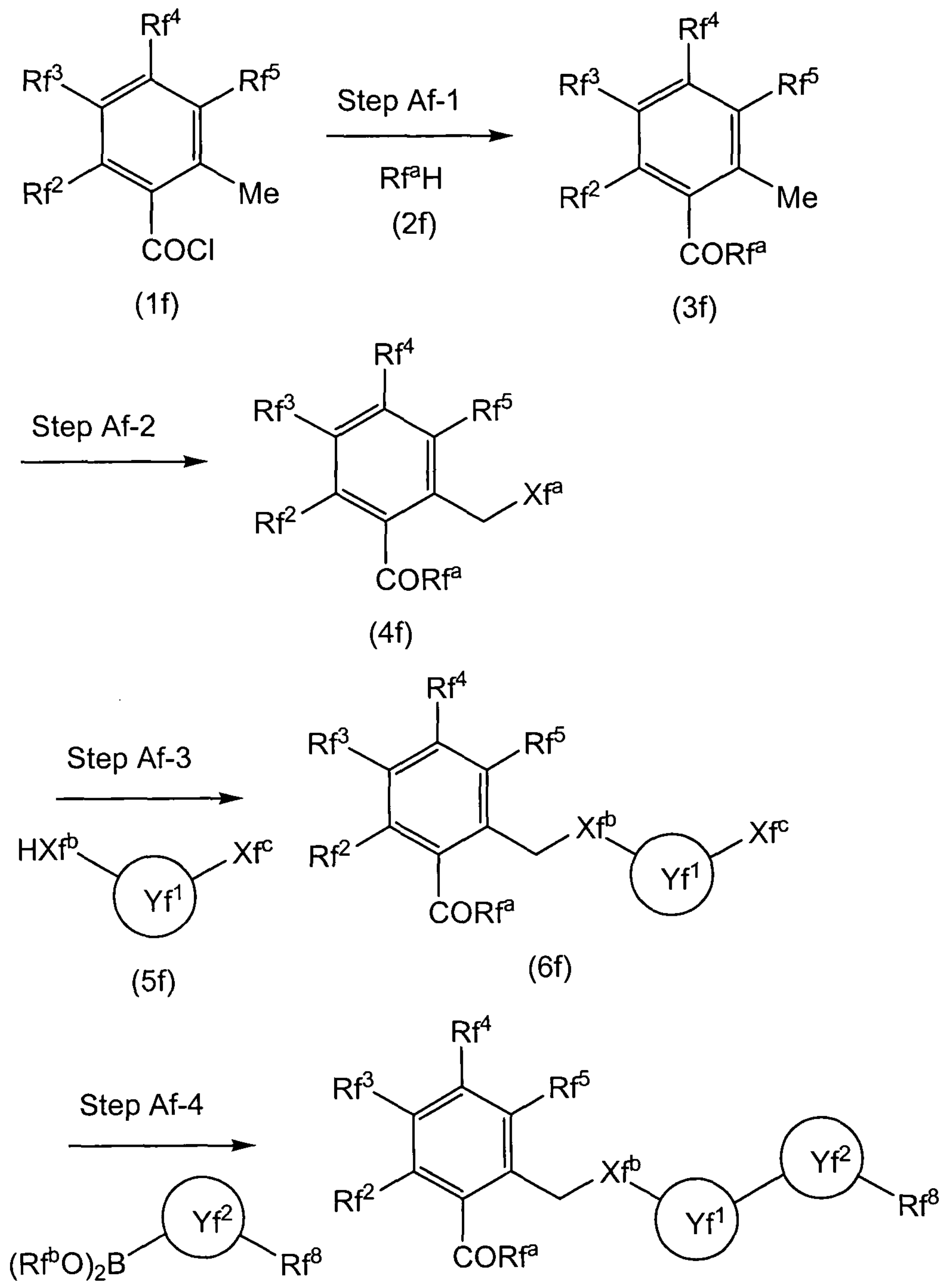


(1f) (2f) (3f)

(4f)

(5f) (6f)

(7f) (If-a)

[Chemical formula 15]

Method Bf

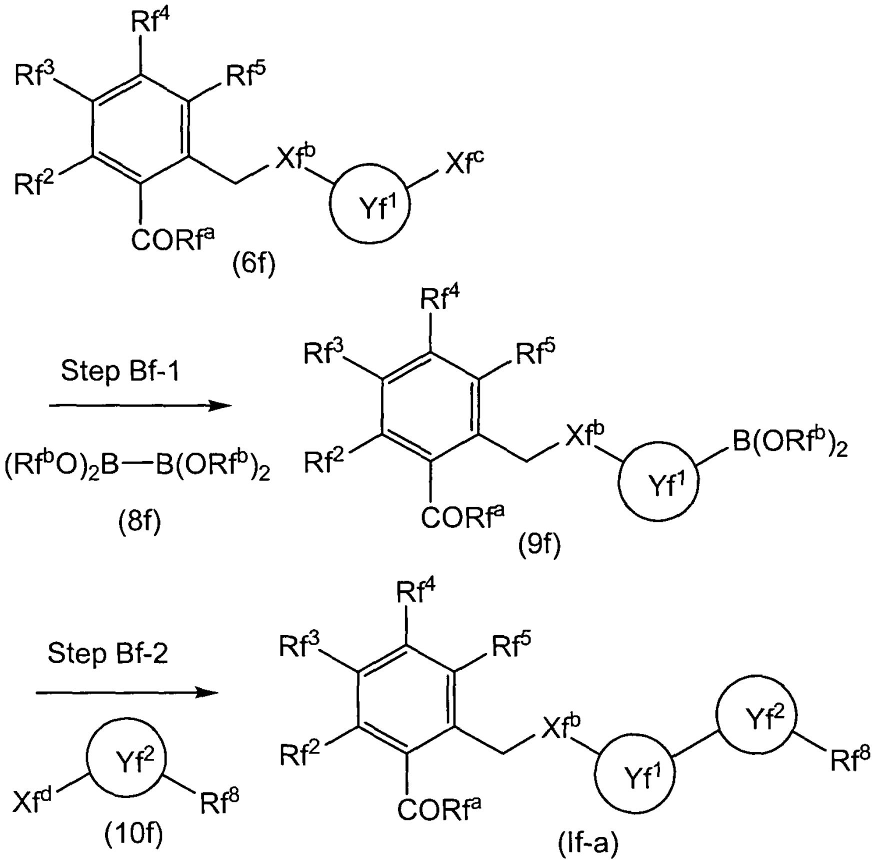


(6f) (8f) (9f) (10f) (If-a)

Method Cf

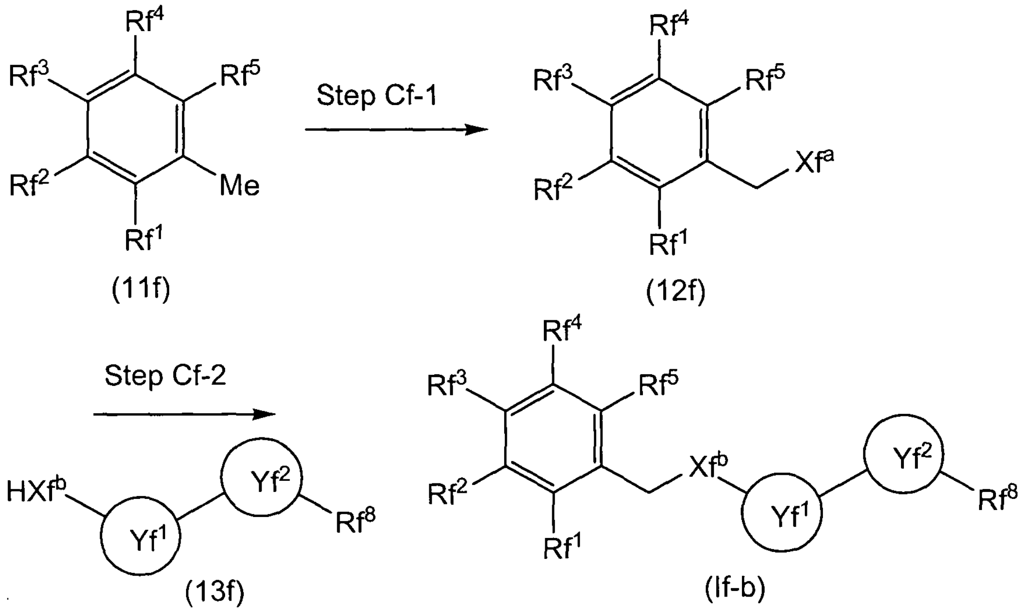


(11f) (12f) (13f) (If-b)

[Chemical formula 16]

Method Df

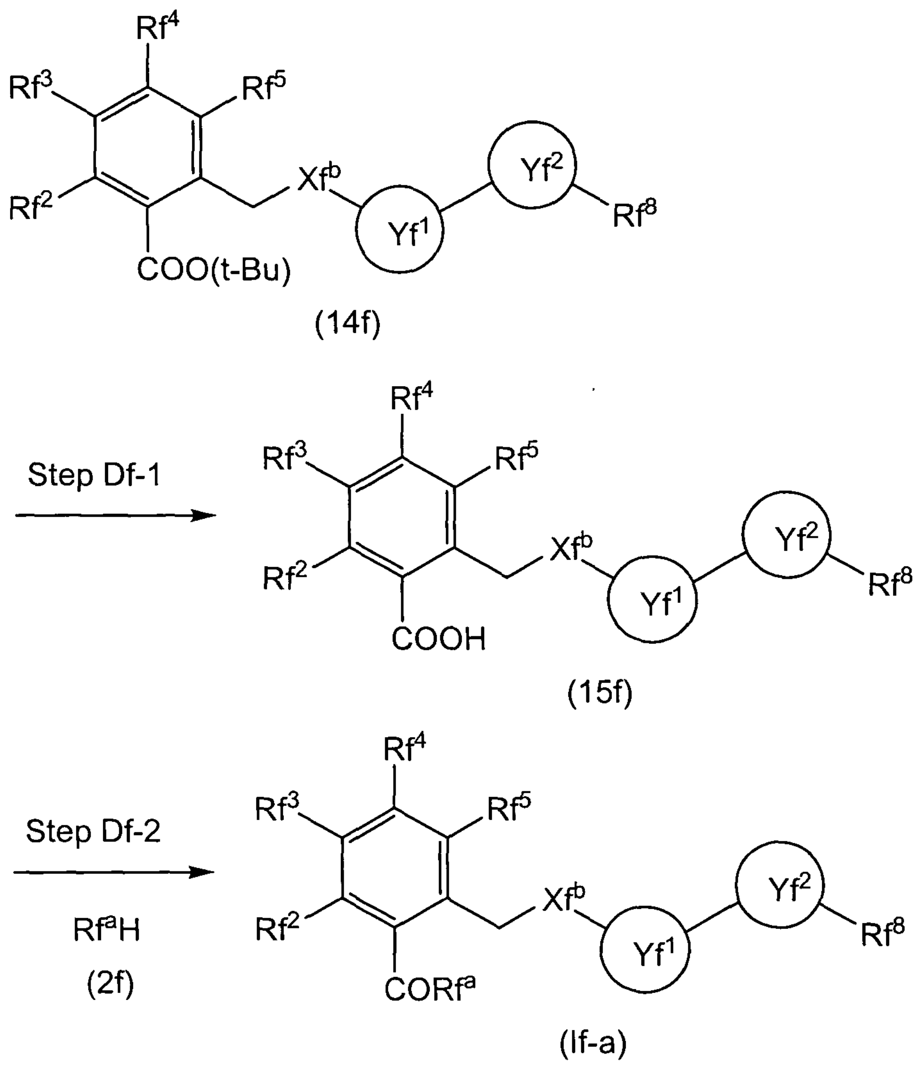


(14f)

(15f)

(If-a)

[Chemical formula 17]

Method Ef

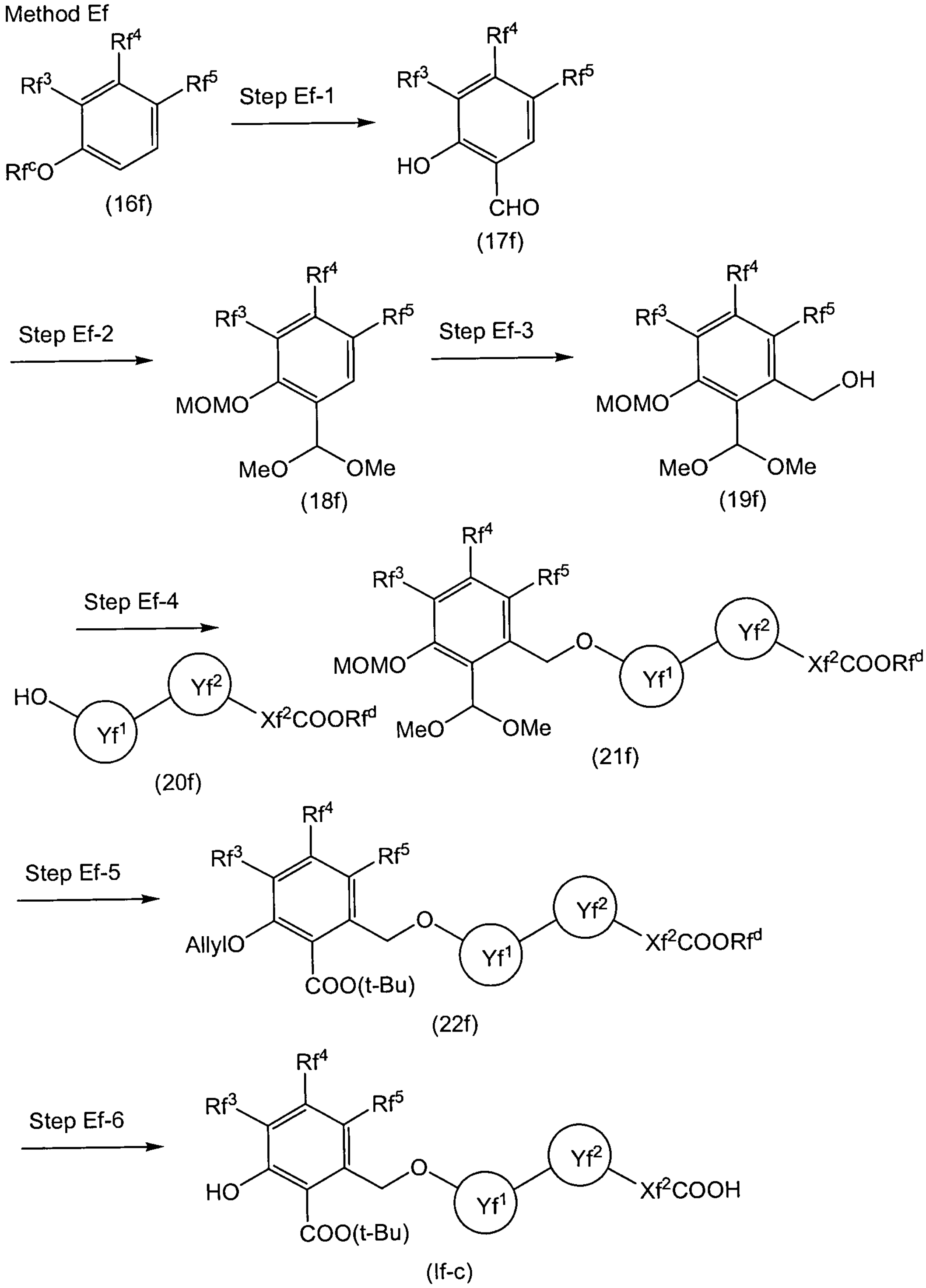


(16f) (17f) (18f) (19f) (20f) (21f) (22f) (If-c)

[Chemical formula 18]

Method Ff

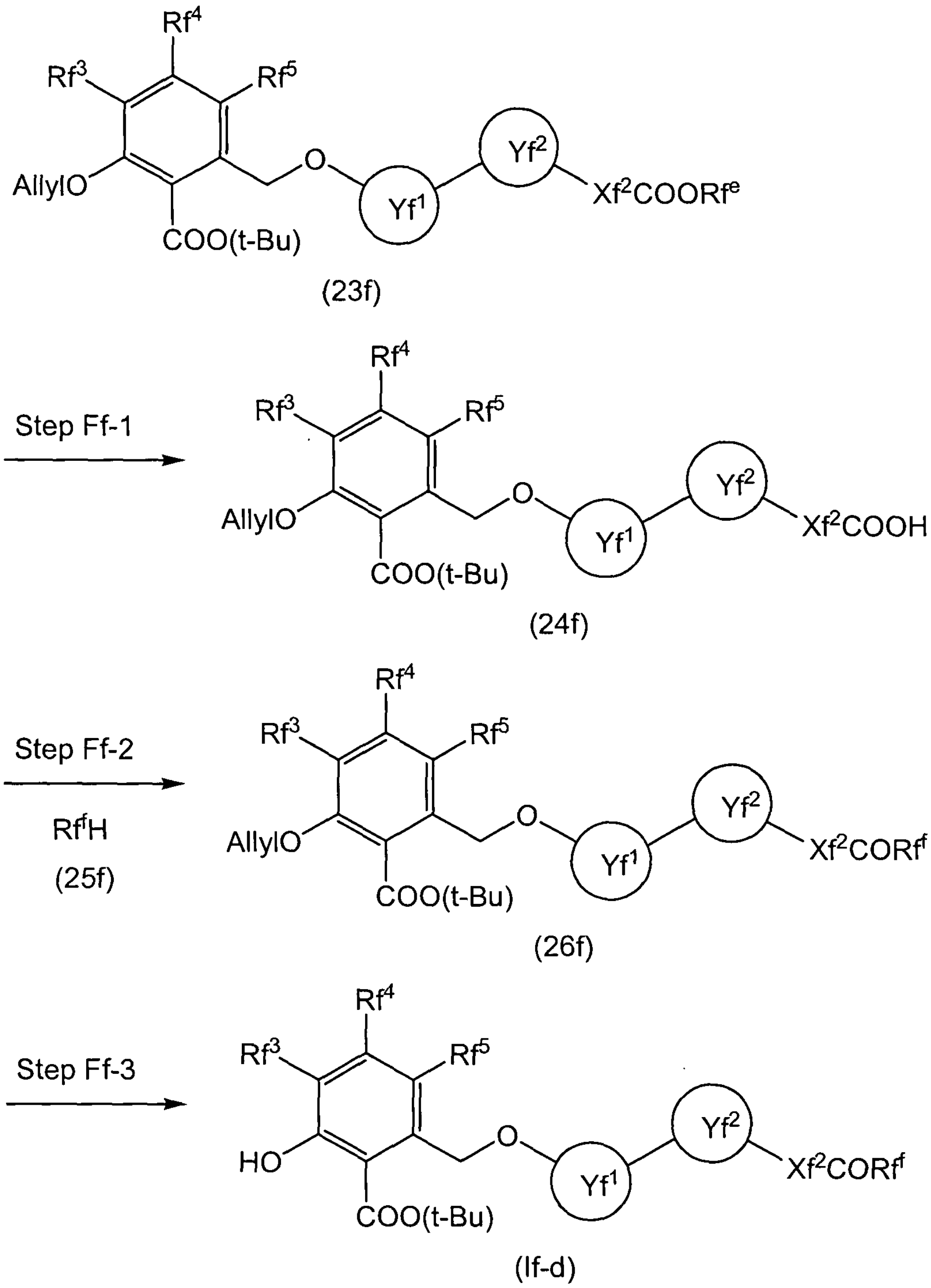


(23f)

(24f)

(26f)

(If-d)

[Chemical formula 19]

Method Gf

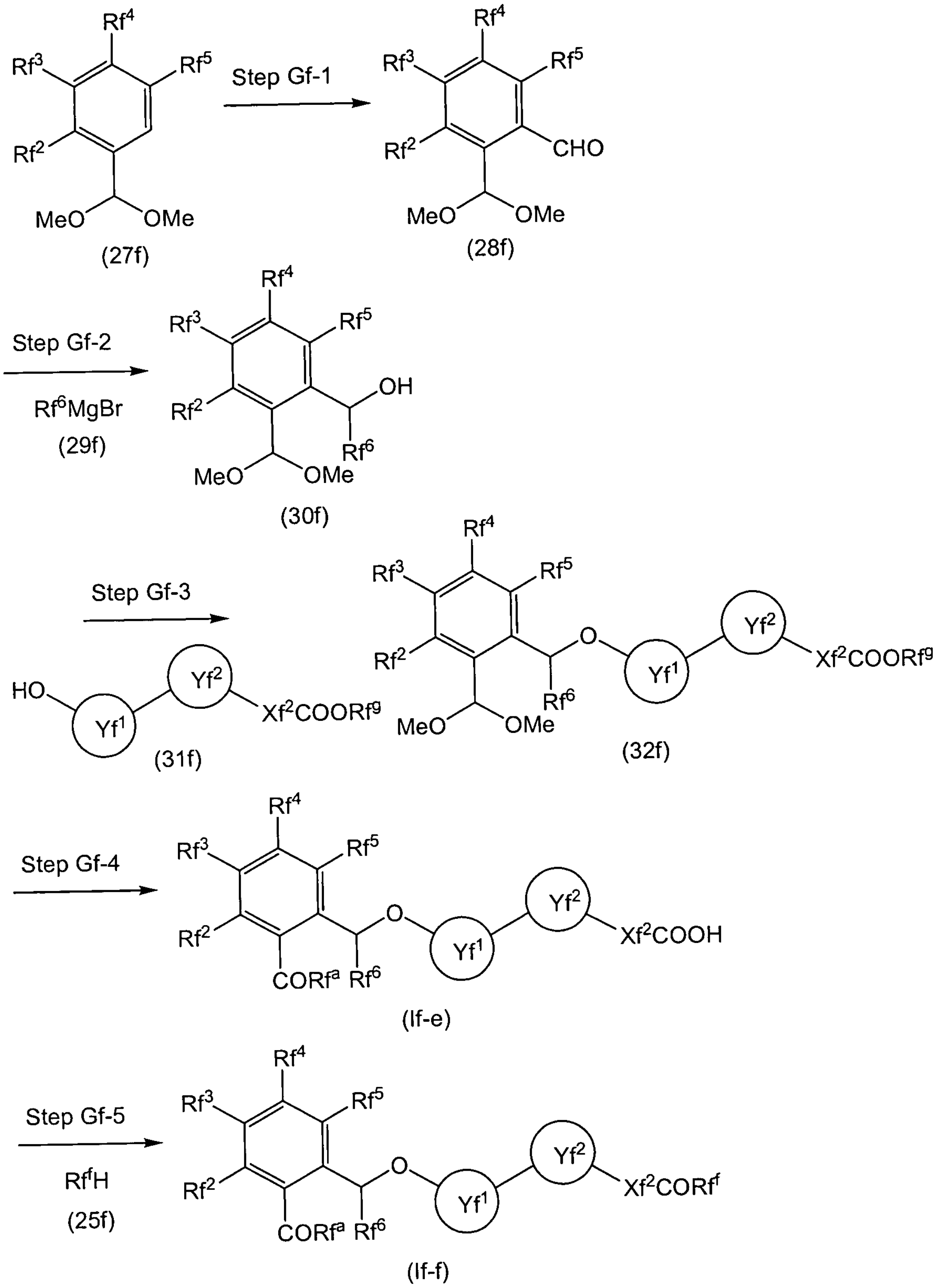


(27f) (28f) (30f) (31f) (32f) (If-e) (If-f)

[Chemical formula 20]

Metho Hf

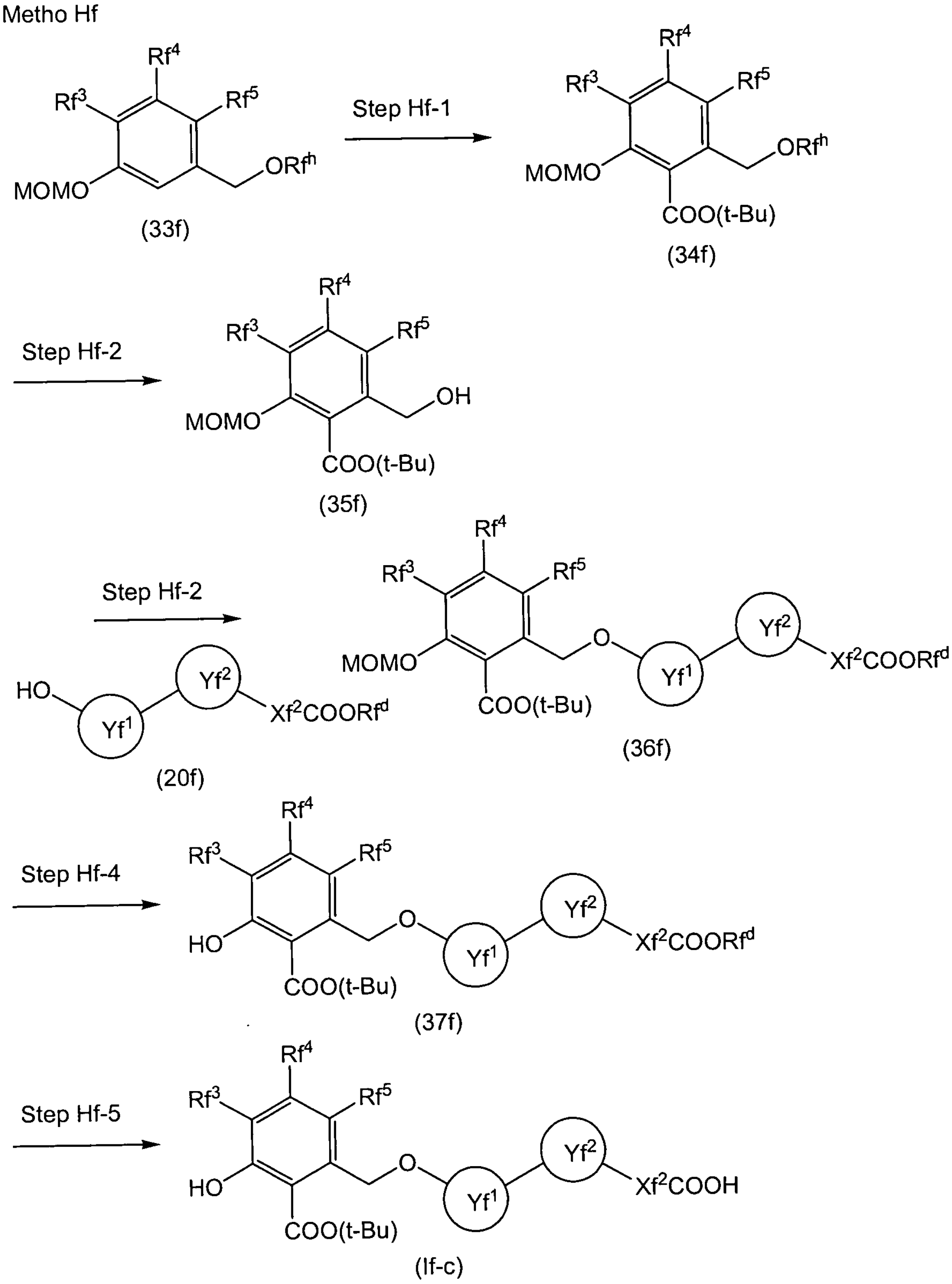


(33f) (34f) (35f) (20f) (36f) (37f) (If-c)

[Chemical formula 21]

Process If

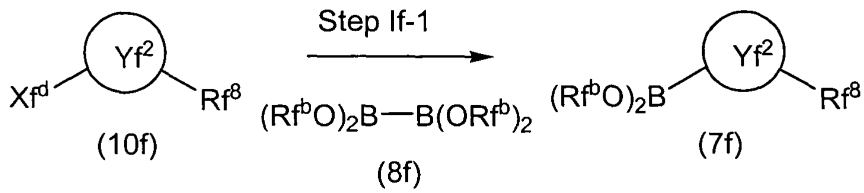


Method Jf

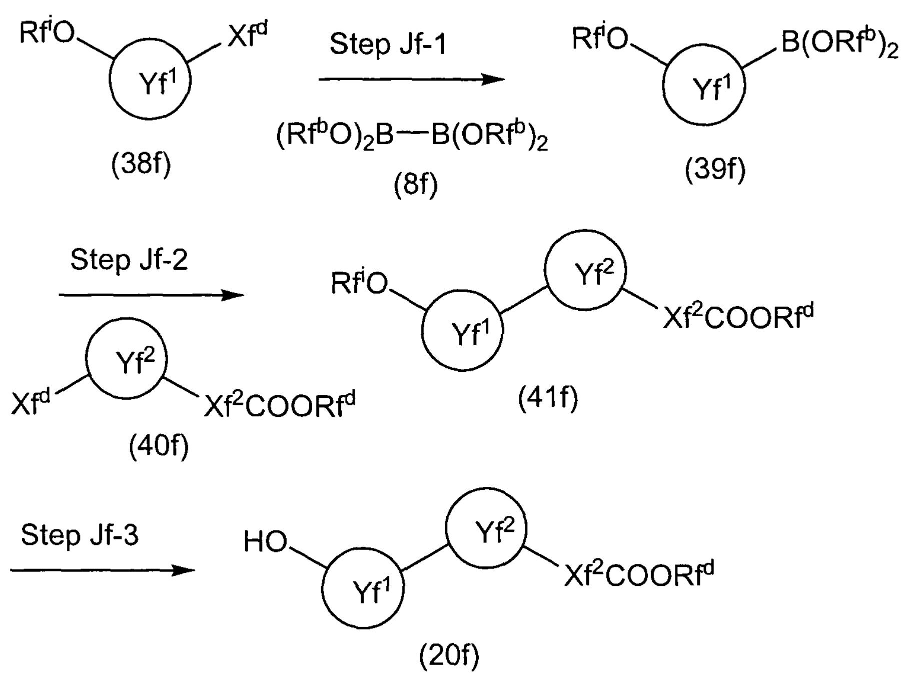


Method Kf

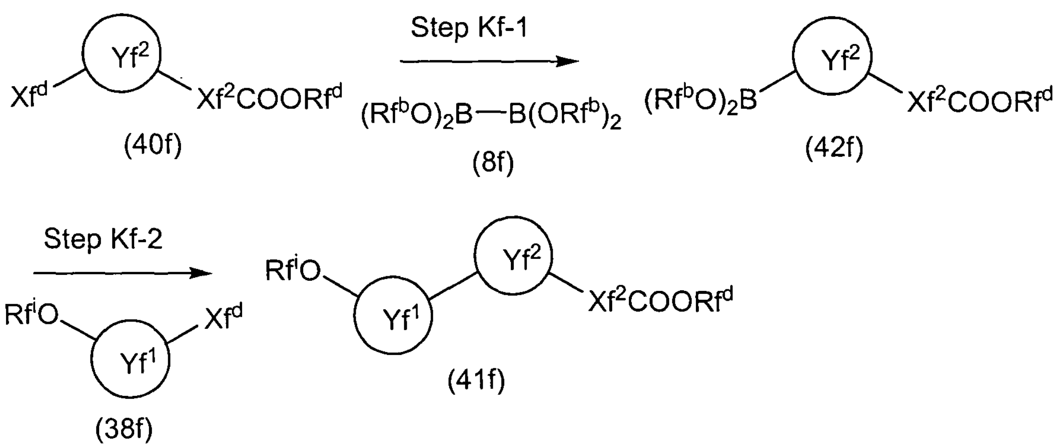


[Chemical formula 22]

Method Lf

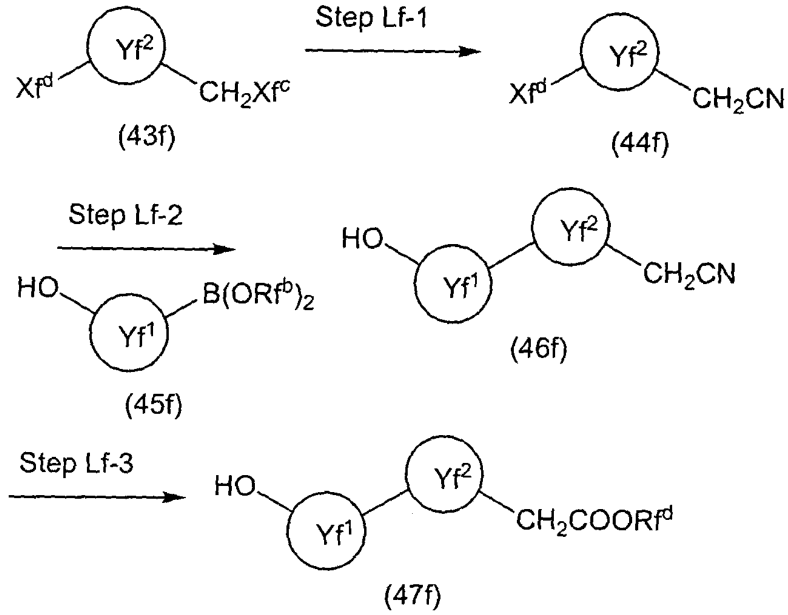


(43f) (44f) (45f) (46f) (47f)

Method Mf

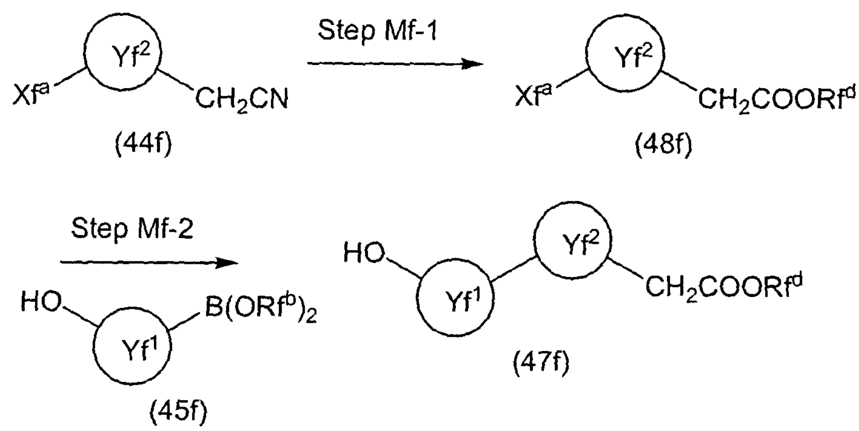


(44f) (48f) (45f) (47f)

Method Nf

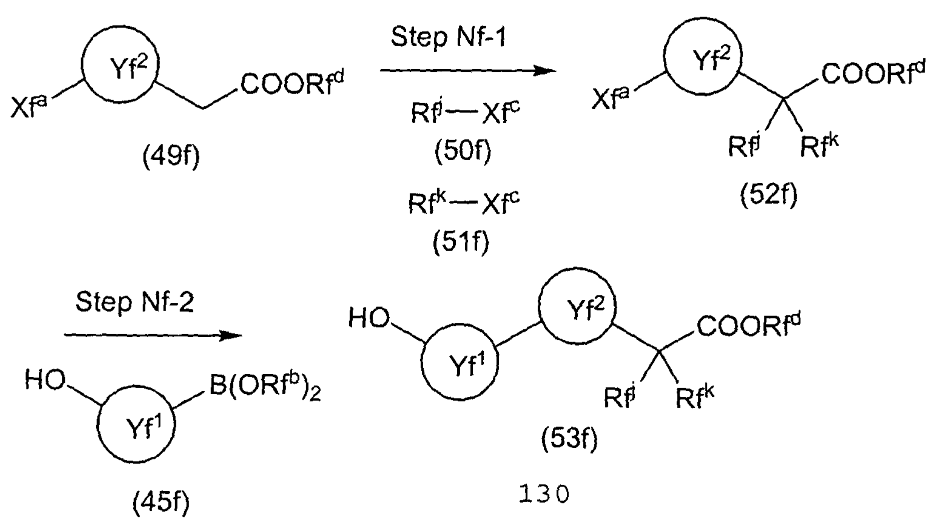


(49f) (50f) (51f) (52f) (45f) (53f)

130

[Chemical formula 23]

Method Of

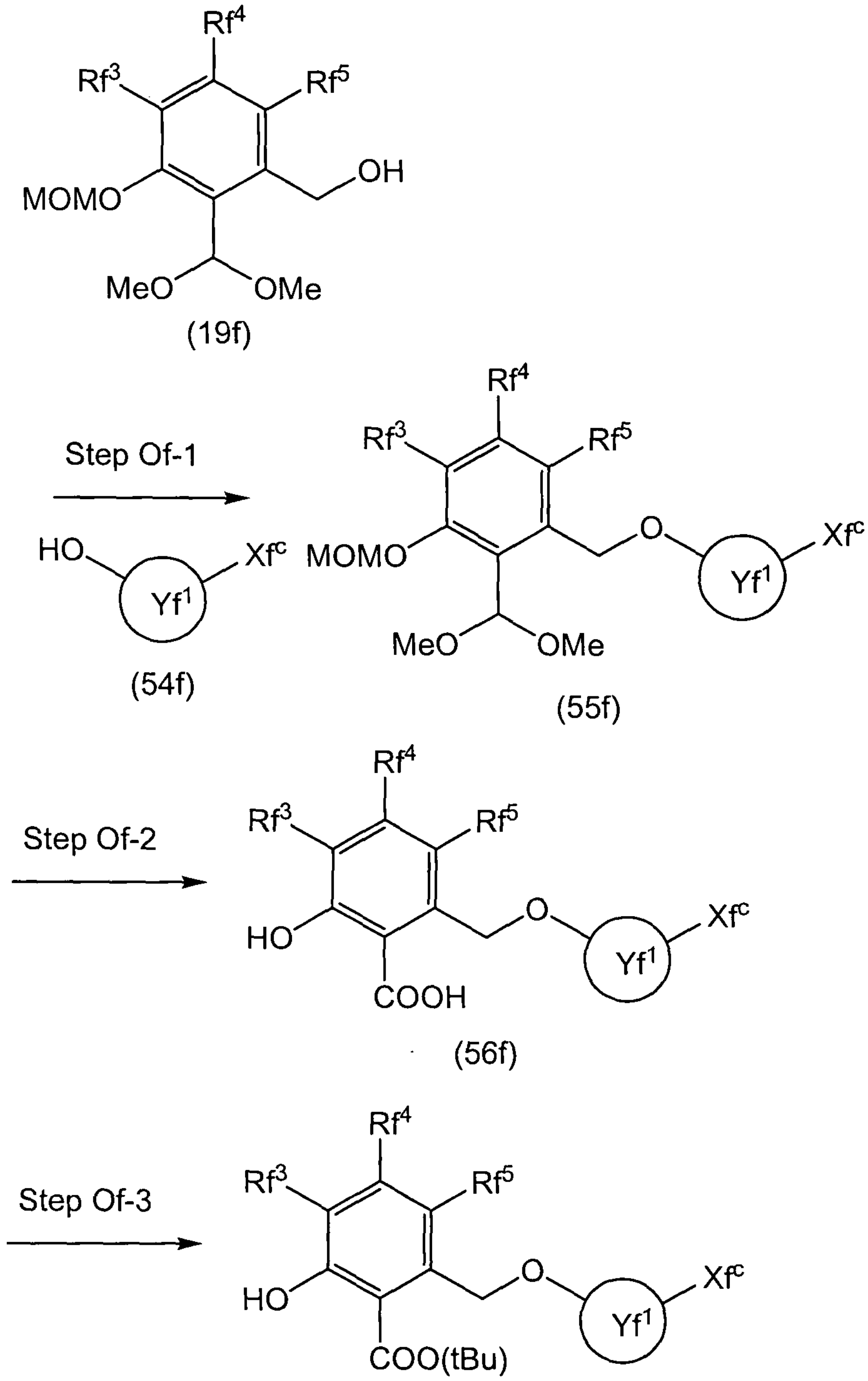


(19f)

(54f)

(55f)

(56f)

(57f)

[Chemical formula 24]

Method Pf

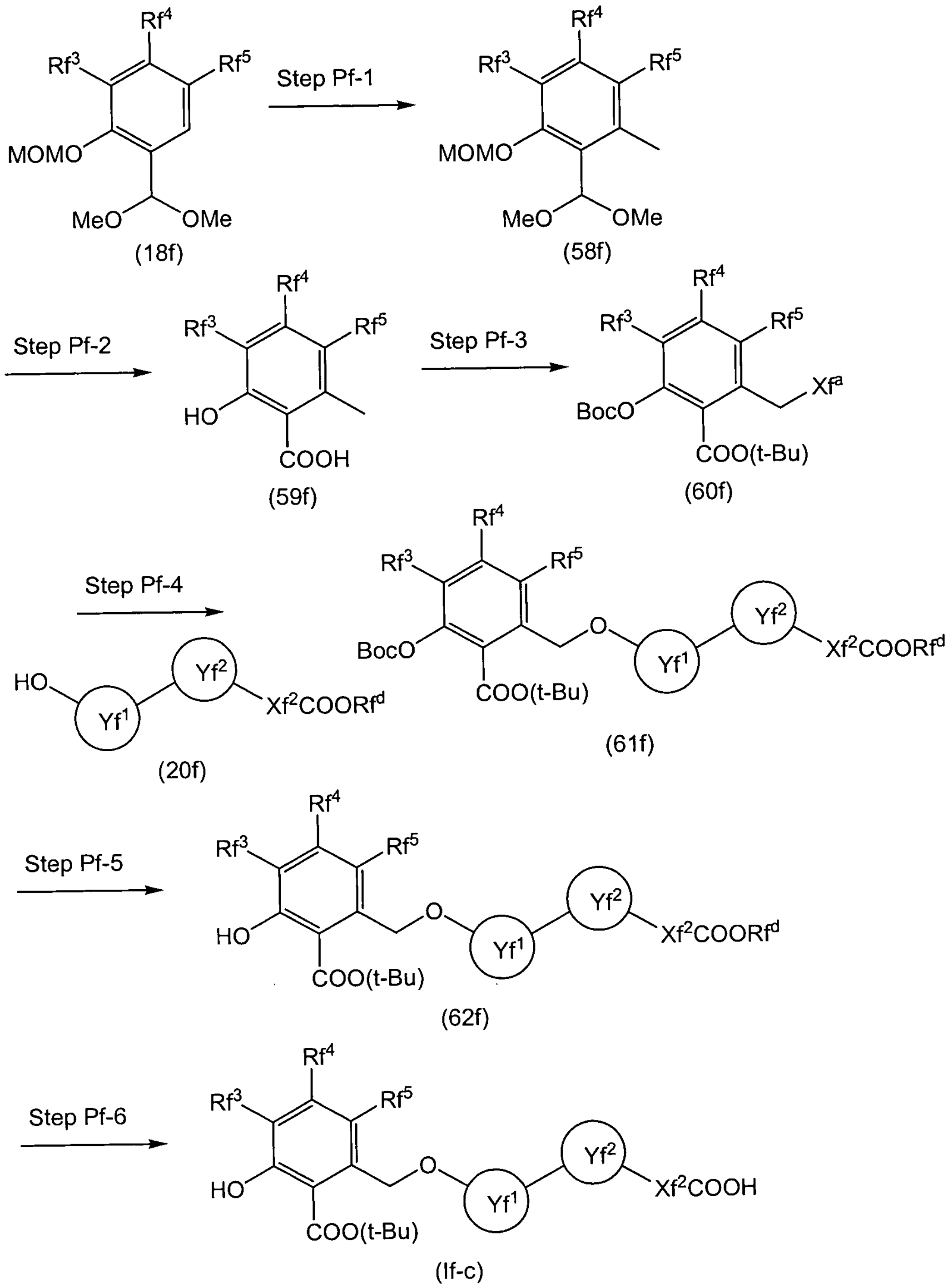


(18f) (58f) (59f) (60f) (20f) (61f) (62f) (If-c)

In the structural formulae of the compounds of the above Method Af to Method Pf, $Rf^1$, $Rf^2$, $Rf^3$, $Rf^4$, $Rf^5$, $Rf^6$, $Rf^8$, $Xf^2$, $Yf^1$, and $Yf^2$ have the same meanings as defined above, $Rf^a$ represents a $C_1$-$C_{10}$ alkoxy group as in $Rf^9$, a

halogeno $C_1$-$C_{10}$ alkoxy group, a phenyl-($C_1$-$C_{10}$ alkoxy) group, a $C_1$-$C_{10}$ alkylamino group or a di($C_1$-$C_{10}$ alkyl)amino group, $Rf^b$ represents a hydrogen atom or a $C_1$-$C_6$ alkyl group, or two $Rf^b$s together may form an ethylene group or a trimethylene group (said ethylene group or trimethylene group may be substituted with 1 to 4 methyl groups), $Rf^c$ represents a tetrahydrofuranyl group, a tetrahydropyranyl group or a methoxymethyl group, $Rf^d$ represents a $C_1$-$C_6$ alkyl group or an allyl group, $Rf^e$ represents a $C_1$-$C_6$ alkyl group, $Rf^f$ represents a $C_1$-$C_6$ alkoxy group, an amino group, a $C_1$-$C_6$ alkylamino group or a di($C_1$-$C_6$ alkyl)amino group as in $Rf^{11}$, $Rf^g$ represents an allyl group, $Rf^h$ represents a silyl group substituted by three groups selected from the group consisting of a $C_1$-$C_6$ alkyl group and a phenyl group (preferably a tert-butyldimethylsilyl group, a tert-butyldiphenylsilyl group or a triisopropylsilyl group), $Rf^i$ represents a protective group of a hydroxyl group, preferably a silyl group substituted by 3 groups selected from the group consisting of a $C_1$-$C_6$ alkyl group and a phenyl group (particularly a tert-butyldimethylsilyl group, a tert-butyldiphenylsilyl group or a triisopropylsilyl group), a tetrahydrofuranyl group, a tetrahydropyranyl group, a methoxymethyl group or an allyl group, $Rf^j$ and $Rf^k$ represent a $C_1$-$C_4$ alkyl group or, together with each other, may form an ethylene group or a trimethylene group, $Xf^a$ and $Xf^c$ represent a chloro group, a bromo group or an iodo group, $Xf^b$ represents a group having the formula: -NH-, -$NRf^{12}$, -O- or -S-, $Xf^d$ represents a chloro group, a bromo group, an iodo group or a trifluoromethanesulfonyloxy group, Allyl represents an allyl group, Boc represents a tert-butoxycarbonyl group, t-Bu represents a tert-butyl group and MOM represents a methoxymethyl group.

In the reactions of the respective steps of the following Method Af to Method Pf, in cases where the compound becoming the reaction substrate has a group which inhibits the desired reaction such as an amino group, a hydroxyl group or a carboxyl group, introduction of a protective group to those groups may be appropriately carried out, if necessary and removal of the introduced protective group may be appropriately carried out, if necessary. Such a protective group is not particularly limited if the group is a protective group which is usually used to progress the reaction and can be a protective group, for example, described in T. H. Greene, P. G. Wuts, Protective Groups in Organic Synthesis, Third Edition, 1999, John Wiley & Sons,. Inc. or the like. The introduction reaction of those protective groups and the removal reaction of the protective groups can be carried out according to usually used methods such as methods described in the above literature.

The solvent used in the reactions of the respective steps of the following Method Af to Method Pf is not particularly limited if the solvent does not inhibit the reaction and dissolves the starting material to some extent, and is selected from the following solvent group. The solvent group comprises aliphatic hydrocarbons such as hexane, pentane, petroleum ether and cyclohexane; aromatic hydrocarbons such as benzene, toluene and xylene; halogenated hydrocarbons such as methylene chloride, chloroform, carbon tetrachloride, dichloroethane, chlorobenzene and dichlorobenzene; ethers such as diethyl ether, diisopropyl ether, tetrahydrofuran, dioxane, dimethoxyethane and diethyleneglycol dimethyl ether; ketones such as acetone, methyl ethyl ketone, methyl isobutyl ketone and cyclohexanone; esters such as ethyl acetate, propyl acetate and butyl acetate; nitriles such as acetonitrile, propionitrile, butyronitrile and isobutyronitrile; carboxylic acids such as acetic acid and propionic acid; alcohols such as methanol, ethanol, 1-propanol, 2-propanol, 1-butanol, 2-butanol, 2-methyl-1-propanol and 2-methyl-2-propanol; amides such as formamide, dimethylformamide, dimethylacetamide, N-methyl-2-pyrrolidone and hexamethylphosphorotriamide; sulfoxides such as dimethyl sulfoxide and sulfolane; water; and mixtures of these.

The acid used in the reactions of the respective steps of the following Method Af to Method Pf is not particularly limited if the acid does not inhibit the reaction, and is selected from the following acid group. The acid group comprises organic acids such as acetic acid, propionic acid, trifluoroacetic acid and pentafluoropropionic acid, organic sulfonic acids such as p-toluenesulfonic acid, camphor sulfonic acid and trifluoromethanesulfonic acid, and inorganic acids such as hydrochloric acid, hydrobromic acid, hydroiodic acid, phosphoric acid, sulfuric acid and nitric acid.

The base used in the reactions of the respective steps of the following Method Af to Method Pf is not particularly limited if the base does not inhibit the reaction, and is selected from the following base group. The base group comprises alkali metal carbonates such as lithium carbonate, sodium carbonate, potassium carbonate and cesium carbonate; alkali metal hydrogencarbonates such as lithium hydrogencarbonate, sodium hydrogencarbonate and potassium hydrogencarbonate; alkali metal hydroxides such as lithium hydroxide, sodium hydroxide and potassium hydroxide; alkaline earth metal hydroxides such as calcium hydroxide and barium hydroxide; alkali metal hydrides such as lithium hydride, sodium hydride and potassium hydride; alkali metal amides such as lithium amide, sodium amide and potassium amide; alkali metal alkoxides such as lithium methoxide, sodium methoxide, sodium ethoxide, sodium tert-butoxide and potassium tert-butoxide; lithium alkylamides such as lithium diisopropylamide; lithium silylamides such as lithium bis-trimethylsilylamide and sodium bis-trimethylsilylamide; alkyl lithiums such as n-butyl lithium, sec-butyl lithium and tert-butyl lithium; and organic amines such as triethylamine, tributylamine, diisopropylethylamine, N-methylpiperidine, N-methylmorpholine, N-ethylmorpholine, pyridine, picoline, 4-(N,N-dimethylamino)pyridine, 4-pyrrolidinopyridine, 2,6-di(tert-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU).

In the reactions of the respective steps of the following Method Af to Method Pf, the reaction temperature varies depending on the solvent, the starting material, the reagent, etc. and the reaction time varies depending on the solvent,

the starting material, the reagent, the reaction temperature, etc.

In the reactions of the respective steps of the following Method Af to Method Pf, after completion of the reaction, the desired compounds of the respective steps are isolated from the reaction mixture according to usually used methods. For example, the desired compound is obtained by (i) removing the insolubles such as a catalyst by filtration, if necessary, (ii) adding water and a solvent immiscible with water (for example, methylene chloride, diethyl ether, ethyl acetate, etc.) to the reaction mixture followed by extracting the desired compound, (iii) washing the organic layer with water and drying it using a drying agent such as anhydrous magnesium sulfate, and (iv) evaporating the solvent. The obtained desired compound can be further purified, if necessary, by usually used methods, for example, recrystallization, reprecipitation or silica gel column chromatography. Further, the desired compounds of the respective steps can be used for the subsequent reaction as such without purification.

(Method Af)

Method Af is a method to prepare the compound (If-a) in which $Rf^1$ is $-CORf^a$, $Rf^6$ and $Rf^7$ are hydrogen atoms and $Xf^1$ is $Xf^b$ in the formula (If).

(Step Af-1)

Step Af-1 is a step to prepare the compound (3f) by reacting the compound (If), which is publicly known or easily obtained from publicly known compounds, with the compound (2f) in the presence or absence of a base.

In Step Af-1, in cases where $Rf^a$ of the compound (2f) is a $C_1$-$C_{10}$ alkoxy group, a halogeno $C_1$-$C_{10}$ alkoxy group or a phenyl-($C_1$-$C_{10}$ alkoxy) group, the compound (2fa) $Rf^aXf^e$ (wherein $Xf^e$ represents alkali metal, preferably sodium or potassium) can be used instead of the compound (2f) in the absence of a base.

The base used is selected from the above base group, and is not particularly limited if it is usually used in an esterification reaction or an amidation reaction, and is preferably an organic amine, more preferably triethylamine.

The solvent used is selected from the above solvent group and is preferably an ether, more preferably tetrahydrofuran.

The reaction temperature is usually from -20 to 100˚C, preferably from 0 to 50˚C.

The reaction time is usually from 10 minutes to 6 hours, preferably from 30 minutes to 3 hours.

Step Af-1 can be also carried out according to a method similar to Step Df-2 using a carboxylic acid compound instead of the compound (1f).

(Step Af-2)

Step Af-2 is a step to prepare the compound (4f) by halogenating the compound (3f) obtained in Step Af-1 with a halogenating agent.

The halogenating agent used is not particularly limited if it is usually used in a halogenation reaction, and can include an N-halogeno-succinimide such as N-chlorosuccinimide, N-bromosuccinimide, N-iodosuccinimide, or a halogen such as bromine and iodine, and is preferably an N-halogeno-succinimide, more preferably N-bromosuccinimide. Step Af-2 can be carried out, if necessary, in the presence of a radical reaction initiator such as azoisobutyronitrile (preferably azoisobutyronitrile or benzoyl peroxide).

The solvent used is selected from the above solvent group and is preferably an aromatic hydrocarbon or a halogenated hydrocarbon, more preferably benzene or carbon tetrachloride.

The reaction temperature is usually from 20 to 200˚C, preferably from 50 to 150˚C.

The reaction time is usually from 30 minutes to 12 hours, preferably from 30 minutes to 6 hours.

(Step Af-3)

Step Af-3 is a step to prepare the compound (6f) by reacting the compound (4f) obtained in Step Af-2 with the compound (5f), which is publicly known or easily obtained from publicly known compounds, in the presence of a base.

The base used is selected from the above base group, and is not particularly limited if it is usually used in the alkylation reaction of phenols, and is preferably an alkali metal carbonate, an alkali metal hydrogencarbonate or an alkali metal hydride, more preferably potassium carbonate or cesium carbonate.

The solvent used is selected from the above solvent group and is preferably an amide, more preferably dimethylformamide.

The reaction temperature is usually from -20 to 100˚C, preferably from 0 to 50˚C.

The reaction time is usually from 30 minutes to 48 hours, preferably from 1 hour to 48 hours.

(Step Af-4)

Step Af-4 is a step to prepare the compound (If-a) by reacting the compound (6f) obtained in Step Af-3 with the compound (7f) in the presence of a palladium catalyst and a base. The compound (7f) is publicly known, is easily obtained from publicly known compounds, or can be prepared by Method If.
The palladium catalyst used is not particularly limited if it is usually used in carbon-carbon bond formation reactions, and can be a palladium catalyst described in J. Tsuji, Palladium Reagents and Catalysis: New perspectives for the 21st Century, 2004, John Wiley & Sons, Inc. or the like. The palladium catalyst used can include tetrakis(triphenylphosphine)palladium (0), bis[1,2-bis(diphenylphosphino)ethane]palladium (0), tris(dibenzylideneacetone)dipalladium (0), bis(tri-t-butylphosphine)palladium (0), bis(tricyclohexylphosphine)palladium (0), palladium chloride (II), palladium acetate (II), dichlorobis(triphenylphosphine)palladium (II), dichlorobis[methylenebis(diphenylphosphine)]dipalladium-dichloromethane adduct, [1,2-bis(diphenylphosphino)ethane]dichloropalladium (II), [1,1'-bis(diphenylphosphino)ferrocene]dichloropalladium (II)-dichloromethane adduct, palladium (II) acetylacetonate, bis(benzonitrile)palladium (II) chloride, bis(acetato)bis(triphenylphosphine)palladium (II), bis(acetonitrile)dichloropalladium (II), bis(benzonitrile)dichloropalladium (II), trans-benzyl(chloro)bis(triphenylphosphine)palladium (II), palladium-carbon, palladium hydroxide or palladium hydroxide-carbon, and is preferably tetrakis(triphenylphosphine)palladium (0), palladium acetate (II), tris (dibenzylideneacetone)dipalladium (0) or [1,1'-bis(diphenylphosphino)ferrocene]dichloropalladium (II)-dichloromethane adduct.
In Step Af-4, a phosphorus ligand capable of being coordinated onto the palladium catalyst may be appropriately used, if necessary. The phosphorus ligand used can be a phosphorus ligand described in J. Tsuji, Palladium Reagents and Catalysis: New perspectives for the 21st Century, 2004, John Wiley & Sons, Inc. or the like. The phosphorus ligand used can include triphenylphosphine, tri-o-tolylphosphine, tri-m-tolylphosphine, tri-p-tolylphosphine, tris(2,6-dimethoxyphenyl)phosphine, tris[2-(diphenylphosphino)ethyl]phosphine, bis(2-methoxyphenyl)phenylphosphine, 2-(di-t-butylphosphino)biphenyl, 2-(dicyclohexylphosphino)biphenyl, 2-(diphenylphosphino)-2'-(N,N-dimethylamino)biphenyl, tri-t-butylphosphine, bis(diphenylphosphino)methane, 1,2-bis(diphenylphosphino)ethane, 1,2-bis(dimethylphosphino) ethane, 1,3-bis(diphenylphosphino)propane, 1,4-bis(diphenylphosphino)butane, 1,5-bis(diphenylphosphino)pentane, 1,6-bis(diphenylphosphino)hexane, 1,2-bis(dimethylphosphino)ethane, 1,1'-bis(diphenylphosphino)ferrocene, bis(2-diphenylphosphinoethyl)phenylphosphine, 2-(dicyclohexylphosphino-2',6'-dimethoxy-1,1'-biphenyl (S-PHOS), 2-(dicyclohexylphosphino-2',4',6'-tri-iso-propyl-1,1'-biphenyl (X-PHOS) or bis(2-diphenylphosphinophenyl) ether (DPEphos), and is preferably triphenylphosphine, tri-o-tolylphosphine, 1,3-bis(diphenylphosphino)propane, 2-(dicyclohexylphosphino-2',6'-dimethoxy-1,1'-biphenyl or bis(2-diphenylphosphinophenyl) ether.
The base used can be a base selected from the above base group or an alkali metal phosphate, and is preferably an alkali metal carbonate or an alkali metal phosphate, more preferably sodium carbonate, potassium carbonate or potassium phosphate.
The solvent used is selected from the above solvent group and is preferably a hydrocarbon, an ether, an alcohol, an amide, water or a mixture of these, more preferably toluene, tetrahydrofuran, ethanol, dimethylacetamide, water or a mixture of these, most preferably a mixture of toluene and ethanol, a mixture of tetrahydrofuran and water or a mixture of dimethylacetamide and water.
The reaction temperature is usually from 20 to 200˚C, preferably from 50 to 150˚C.
The reaction time is usually from 1 hour to 48 hours, preferably from 3 hours to 24 hours.

(Method Bf)

Method Bf is prepare the compound (If-a) in which $Rf^1$ is $-CORf^a$, $Rf^6$ and $Rf^7$ are hydrogen atoms and $Xf^1$ is $Xf^b$ in the formula (If).

(Step Bf-1)

Step Bf-1 is a step to prepare the compound (9f) by reacting the compound (6f) obtained in Step Af-3 with the compound (8f), which is publicly known or easily obtained from publicly known compounds, in the presence of a palladium reagent and a base.
The palladium catalyst used can be a catalyst similar to the one indicated in Step Af-4, and is preferably the [1,1'-bis(diphenylphosphino)ferrocene]-dichloropalladium (II)-dichloromethane adduct. Further, a phosphorus ligand can be appropriately used, if necessary, similarly to Step Af-4.
The base used is a base indicated in the above base group, or an alkali metal salt of acetic acid, such as sodium acetate and potassium acetate, and is preferably an alkali metal salt of acetic acid, more preferably potassium acetate.
The solvent used is selected from the above solvent group, and is preferably an ether, a sulfoxide or a mixture

of these, more preferably tetrahydrofuran, dioxane, dimethyl sulfoxide or a mixture of these, further preferably dimethyl sulfoxide or dioxane.

The reaction temperature is usually from 20 to 200˚C, preferably from 50 to 150˚C.

The reaction time is usually from 30 minutes to 24 hours, preferably from 2 hours to 12 hours.

(Step Bf-2)

Step Bf-2 is a step to prepare the compound (If-a) by reacting the compound (9f) obtained in Step Bf-1 with the compound (10f), which is publicly known or easily obtained from publicly known compounds, in the presence of a palladium catalyst and a base.

Step Bf-2 can be carried out according to a method similar to Step Af-4.

(Method Cf)

Method Cf is a method to prepare the compound (If-b) in which $Rf^6$ and $Rf^7$ are hydrogen atoms and $Xf^1$ is $Xf^b$ in the formula (If).

(Step Cf-1)

Step Cf-1 is a step to prepare the compound (12f) by halogenating the compound (11f), which is publicly known or easily obtained from publicly known compounds, with a halogenating agent.

Step Cf-1 can be carried out according to a method similar to Step Af-2.

(Step Cf-2)

Step Cf-2 is a step to prepare the compound (If-b) by reacting the compound (12f) obtained in Step Cf-1 with the compound (13f) in the presence of a base. The compound (13f) is publicly known, is easily obtained from publicly known compounds, or can be obtained by Method Jf, Method Lf or Method Mf.

Step Cf-2 can be carried out according to a method similar to Step Af-3.

The compound (If-b-2) in which $Rf^6$ and $Rf^7$ are hydrogen atoms and $Xf^1$ is a group having the formula: -SO- or $-SO_2-$ in the formula (If) can be prepared by oxidizing the compound (If-b-1) in which $Xf^b$ is a group having the formula: -S- in the compound (If-b) obtained in Step Cf-2 in a solvent (preferably methylene chloride, etc.) with 1 mole or 2 moles of meta-chloroperbenzoic acid.

(Method Df)

Method Df is a method to prepare the compound (If-a) in which $Rf^1$ is $-CORf^a$, $Rf^6$ and $Rf^7$ are hydrogen atoms and $Xf^1$ is $Xf^b$ in the formula (If).

(Step Df-1)

Step Df-1 is a step to prepare the compound (15f) by treating the compound (14f) obtained by Method Af, Method Bf or Method Cf with an acid.

The acid used is selected from the above acid group, and is not particularly limited if it is used in elimination reactions of a tert-butyl group, and is preferably trifluoroacetic acid or hydrochloric acid, more preferably trifluoroacetic acid.

The solvent used is selected from the above solvent group and is preferably a halogenated hydrocarbon, more preferably methylene chloride.

The reaction temperature is usually from 0 to 100˚C, preferably from 20 to 60˚C.

The reaction time is usually from 1 hour to 48 hours, preferably from 1 hour to 24 hours.

(Step Df-2)

Step Df-2 is to prepare the compound (If-a) by reacting the compound (15f) obtained in Step Df-1 with the compound (2f), which is publicly known or easily obtained from publicly known compounds, in the presence of a condensing agent.

The condensing agent used is not particularly limited if it is usually used in condensation reactions of a carboxylic acid and an amine or a carboxylic acid and an alcohol, and can be a condensing agent described in R. C. Larock,

Comprehensive Organic Transformations, Second Edition, 1999, John Wiley & Sons, Inc. or the like. The condensing agent used can include (i) a combination of a phosphoric acid ester such as diethylphosphoryl cyanide and diphenylphosphoryl azide and the base described below;
(ii) a carbodiimide such as 1,3-dicyclohexylcarbodiimide, 1,3-diisopropylcarbodiimide and 1-ethyl-3-(3-dimethylaminopropyl)carbodiimide (WSC); a combination of the above carbodiimide and the base described below; a combination of the above carbodiimide and an N-hydroxy compound such as N-hydroxysuccinimide, 1-hydroxybenzotriazole and N-hydroxy-5-norbornene-2,3-dicarboxyimide;
(iii) a combination of a disulfide such as 2,2'-dipyridyl disulfide and 2,2'-dibenzothiazolyl disulfide and a phosphine such as triphenylphosphine and tributylphosphine;
(iv) a combination of a 2-halogeno-1-lower alkylpyridinium halide such as 2-chloro-1-methylpyridinium iodide and 2-bromo-1-ethylpyridinium chloride and the base described below; or
(v) an imidazole such as 1,1'-oxalyldiimidazole and N,N'-carbonyldiimidazole; or
(vi) a combination of a sulfonyl chloride such as p-toluenesulfonyl chloride, 2,4,6-trimethylsulfonyl chloride and 2,4,6-triisopropylsulfonyl chloride and the base described below, and is preferably a combination of a carbodiimide and a base, a combination of a 2-halogeno-1-lower alkylpyridinium halide and a base or a combination of a sulfonyl chloride and a base, more preferably a combination of 1-ethyl-3-(3-dimethylaminopropyl)carbodiimide and a base, a combination of 2-chloro-1-methylpyridinium iodide and a base or a combination of 2,4,6-triisopropylsulfonyl chloride and a base.
The base used in combination with the above condensing agent is preferably an organic amine from the above base group, more preferably triethylamine, diisopropylethylamine, pyridine, 4-(N,N-dimethylamino)pyridine or a mixture of these, most preferably triethylamine, 4-(N,N-dimethylamino)pyridine or a mixture of these. In cases where the compound (12f) is an amine in Step Df-2, an excess amount of the compound (12f) can be used as a base.
The solvent used is selected from the above solvent group and is preferably a halogenated hydrocarbon, more preferably methylene chloride.
The reaction temperature is usually from 0 to 100˚C, preferably from 20 to 60˚C.
The reaction time is usually from 1 hour to 48 hours, preferably from 3 hours to 24 hours.
Further, Step Df-2 can be also carried out by converting the compound (15f) to the acid chloride with oxalyl chloride, thionyl chloride, etc. in a solvent (preferably methylene chloride, etc.) followed by reacting the acid chloride with the compound (2f) or the above compound (2fa) in the presence of a base (preferably triethylamine, etc.).

(Method Ef)

Method Ef is a method to prepare the compound (If-c) in which $Rf^1$ is -COO (t-Bu), $Rf^2$ is a hydroxyl group, $Rf^6$ and $Rf^7$ are hydrogen atoms and $Xf^1$ is a group having the formula: -O- and $Rf^8$ is $-Xf^2COOH$ in the formula (If).

(Step Ef-1)

Step Ef-1 is a step to prepare the compound (17f) by reacting the compound (16f), which is publicly known or easily obtained from publicly known compounds, with dimethylformamide in the presence of an alkyllithium and a base.
The alkyllithium used is selected from the alkyllithiums indicated in the above base group, and is preferably n-butyllithium.
The base used can be one having the property of being coordinated onto a lithium ion, and is preferably tetramethylethylenediamine.
The solvent used is selected from the above solvent group and is preferably an ether, more preferably diethyl ether.
The reaction temperature is usually from -80 to 50˚C, preferably from -50 to 20˚C.
The reaction time is usually from 10 minutes to 6 hours, preferably from 30 minutes to 3 hours.
In Step Ef-1, a compound in which the hydroxyl group is $-ORf^c$ in the compound (17f) is sometimes obtained depending on the kind of the compound (16f). In this case, the compound (17f) can be obtained by treating the obtained compound with an acid (preferably the inorganic acid, more preferably hydrochloric acid) to carry out the elimination reaction of $Rf^c$.

(Step Ef-2)

Step Ef-2 comprises
(Step Ef-2a): a step in which the compound (17f) obtained in Step Ef-1 is reacted with methyl ortho-formate in the presence of an acid; and
(Step Ef-2b): a step in which the compound obtained in Step Ef-2a is reacted with chloromethyl methyl ether in the presence of a base to prepare the compound (18f).

(Step Ef-2a)

The acid used is selected from the above acid group and is preferably an organic sulfonic acid, more preferably camphor sulfonic acid.
The solvent used is selected from the above solvent group and is preferably an alcohol, more preferably methanol.
The reaction temperature is usually from 0 to 150˚C, preferably from 20 to 100˚C.
The reaction time is usually from 1 hour to 24 hours, preferably from 2 hours to 12 hours.

(Step Ef-2b)

The base used is selected from the above base group and is preferably an organic amine, more preferably diisopropylethylamine.
The solvent used is selected from the above solvent group and is preferably a halogenated hydrocarbon, more preferably methylene chloride.
The reaction temperature is usually from -20 to 100˚C, preferably from 0 to 50˚C.
The reaction time is usually from 1 hour to 48 hours, preferably from 3 hours to 24 hours.

(Step Ef-3)

Step Ef-3 comprises
(Step Ef-3a): a step in which the compound (18f) obtained in Step Ef-2 is reacted with dimethylformamide in the presence of an alkyllithium and a base; and
(Step Ef-3b): a step in which the compound obtained in Step Ef-3a is reduced by a reducing agent to prepare the compound (19f).

(Step Ef-3a)

The alkyllithium used is selected from the alkyllithiums indicated in the above base group and is preferably n-butyllithium. In Step Ef-3a, the ratio of the number of moles of the compound (18) and n-butyllithium is preferably 1:1 to 1:3, more preferably 1:1.5 to 1:2.5.
The base used can be one having the property of being coordinated onto a lithium ion, and is preferably tetramethylethylenediamine. In Step Ef-3a, the ratio of the number of moles of the compound (18f) and tetramethylethylenediamine is preferably 1:1 to 1:3, more preferably 1:1 to 1:2.5.
The solvent used is selected from the above solvent group and is preferably an ether, more preferably diethyl ether or tetrahydrofuran.
The reaction temperature is usually from -80 to 60˚C, preferably from -50 to 40˚C.
The reaction time is usually from 30 minutes to 10 hours, preferably from 30 minutes to 6 hours.

(Step Ef-3b)

The reducing agent used is not particularly limited if it is usually used in reduction reactions of a formyl group, and can include an alkali metal borohydride such as sodium borohydride, sodium triacetoxy borohydride, sodium cyano borohydride and lithium borohydride, and is preferably sodium borohydride.
The solvent used is selected from the above solvent group and is preferably an ether, an alcohol or a mixture of these, more preferably tetrahydrofuran, methanol or a mixture of these, most preferably a mixture of tetrahydrofuran and methanol.
The reaction temperature is usually from 0 to 100˚C, preferably from 20 to 60˚C.
The reaction time is usually from 10 minutes to 6 hours, preferably from 30 minutes to 3 hours.

(Step Ef-4)

Step Ef-4 is a step to prepare the compound (21f) by reacting the compound (19f) obtained in Step Ef-3 with the compound (20f) in the presence of an azodicarboxylate reagent and a phosphine reagent. The compound (20f) is publicly known, is easily obtained from publicly known compounds, or can be obtained by Method Jf, Method Lf or Method Mf.
The azodicarboxylate reagent used is not particularly limited if it is usually used for the Mitsunobu reaction and can include a dialkylazodicarboxylate such as dimethylazodicarboxylate, diethylazodicarboxylate, dipropylazodicarboxylate, diisopropylazodicarboxylate and di(tert-butyl)azodicarboxylate; bis(2,2,2-trichloroethyl)azodicarboxylate; diphenylazodicarboxylate; 1,1'-(azodicarbonyl)dipiperidine; N,N,N',N'-(tetramethylazodicarboxamide); or dibenzylazodicarboxylate, and is preferably a dialkylazodicarboxylate or 1,1'-(azodicarbonyl)dipiperidine, more preferably diethylazodicarboxylate or 1,1'-(azodicarbonyl)dipiperidine. As the azodicarboxylate reagent, an azodicarboxylate reagent immobilized onto a polymer such as polystyrene [preferably an azodicarboxylate reagent immobilized onto polystyrene such as ethoxycarbonylazocarboxymethyl polystyrene (Nova Biochem Inc.; Product number 01-64-0371)] can be also used.

The phosphine reagent used is not particularly limited if it is usually used for the Mitsunobu reaction and can include triphenylphosphine, tritolylphosphine, tris(methoxyphenyl)phosphine, tris(chlorophenyl)phosphine, tri-n-butylphosphine or 2-(di-t-butylphosphino)biphenyl, and is preferably triphenylphosphine or tri-n-butylphosphine. As the phosphine reagent, a phosphine reagent immobilized onto a polymer such as polystyrene (preferably triphenylphosphine immobilized onto polystyrene such as triphenylphosphine polystyrene) can be also used.

The solvent used is selected from the above solvent group and is preferably an aromatic hydrocarbon or an ether, more preferably tetrahydrofuran.

The reaction temperature is usually from 0 to 100˚C, preferably from 20 to 60˚C.

The reaction time is usually from 10 minutes to 12 hours, preferably from 30 minutes to 6 hours.

(Step Ef-5)

Step Ef-5 comprises

(Step Ef-5a): a step in which in the compound (21f) obtained in Step Ef-4, the dimethoxymethyl group is converted to a formyl group and the methoxymethyl group is eliminated, in the presence of an acid;

(Step Ef-5b): a step in which the hydroxyl group of the compound obtained in Step Ef-5a is reacted with allyl bromide in the presence of a base;

(Step Ef-5c): a step in which the compound obtained in Step Ef-5b is oxidized by sodium hypochlorite ($NaClO_2$) in the presence of sodium dihydrogenphosphate and 2-methyl-2-butene; and

(Step Ef-5d): a step in which the compound obtained in Step Ef-5c is reacted with N,N-dimethylformamide di-tert-butyl acetal [$Me_2NC[O(t\text{-}Bu)]_2$] to prepare the compound (22f).

(Step Ef-5a)

The acid used is selected from the above acid group and is preferably an organic sulfonic acid or an inorganic acid, more preferably p-toluenesulfonic acid or hydrochloric acid.

The solvent used is selected from the above solvent group and is preferably an ether or a ketone, more preferably tertahydrofuran or acetone.

The reaction temperature is usually from 0 to 100˚C, preferably from 20 to 60˚C.

The reaction time is usually from 10 minutes to 24 hours, preferably from 30 minutes to 12 hours. (Step Ef-5b)

The base used is selected from the above base group and is preferably an alkali metal carbonate, more preferably potassium carbonate.

The solvent used is selected from the above solvent group and is preferably an amide, more preferably dimethylformamide.

The reaction temperature is usually from 0 to 100˚C, preferably from 20 to 60˚C.

The reaction time is usually from 10 minutes to 24 hours, preferably from 30 minutes to 12 hours.

(Step Ef-5c)

The solvent used is selected from the above solvent group and is preferably an ether, an alcohol, water or a mixture of these, more preferably a mixture of 1,4-dioxane and water, a mixture of 2-methyl-2-propanol and water or a mixture of 1,4-dioxane/2-methyl-2-propanol/water.

The reaction temperature is usually from 0 to 100˚C, preferably from 20 to 60˚C.

The reaction time is usually from 10 minutes to 6 hours, preferably from 30 minutes to 3 hours.

(Step Ef-5d)

The solvent used is selected from the above solvent group and is preferably an aromatic hydrocarbon, more preferably toluene.

The reaction temperature is usually from 50 to 200˚C, preferably from 80 to 150˚C.

The reaction time is usually from 30 minutes to 24 hours, preferably from 1 hour to 12 hours.

(Step Ef-6)

Step Ef-6 comprises
(Step Ef-6a): a step in which in the allyloxy group of the compound (22f) obtained in Step Ef-5, the allyl group is eliminated in the presence of a palladium reagent;
(Step Ef-6b): a step in which the compound obtained in Step Ef-6a is hydrolyzed in the presence of a base to prepare the compound (If-c).
In Step Ef-6, in cases where $Rf^d$ of the compound (22f) is an allyl group, since $Rf^d$ is simultaneously eliminated in Step Ef-6a, it is not needed to carry out Step Ef-6b.

(Step Ef-6a)

The palladium reagent used is not particularly limited if it is usually used for elimination reactions of the allyl group and can be, for example, one similar to that indicated in Step Af-4, and is preferably tetrakis(triphenylphosphine) palladium (0).
In Step Ef-6a, a scavenger can be appropriately used, if necessary. The scavenger used can include pyrrolidine, piperidine, morpholine, diethylamine, formic acid, acetic acid, 2-ethylhexanoic acid, 2-methylhexanoic acid sodium salt, 5,5-dimethyl-1,3-cyclohexane-dione, dimethyl malonate or tributyltin hydride, and is preferably pyrrolidine or morpholine.
The solvent used is selected from the above solvent group and is preferably an ether or a mixture of an ether and water, more preferably a mixture of dioxane and water.
The reaction temperature is usually from 0 to 100˚C, preferably from 20 to 60˚C.
The reaction time is usually from 10 minutes to 12 hours, preferably from 30 minutes to 6 hours.

(Step Ef-6b)

The base used can be an alkali metal hydroxide from the above base group, preferably sodium hydroxide or potassium hydroxide.
The solvent used is selected from the above solvent group and is preferably an ether, an alcohol or a mixture of these, more preferably tetrahydrofuran, methanol or a mixture of these. In Step Ef-6b, water is necessarily used and water alone can be also used as the solvent.
The reaction temperature is usually from 0 to 150˚C, preferably from 20 to 100˚C.
The reaction time is usually from 1 hour to 36 hours, preferably from 2 hours to 24 hours.

(Method Ff)

Method Ff is a method to prepare the compound (If-d) in which $Rf^1$ is -COO(t-Bu), $Rf^2$ is a hydroxyl group, $Rf^6$ and $Rf^7$ are hydrogen atoms, $Xf^1$ is a group having a formula: - O- and $Rf^8$ is $-Xf^2CORf^f$ in the formula (If).

(Step Ff-1)

Step Ff-1 is a step to prepare the compound (24f) by carrying out an elimination reaction of $Rf^e$ in the compound (23f) obtained in Step Ef-5.
Step Ff-1 can be carried out according to a method similar to Step Ef-6b.

(Step Ff-2)

Step Ff-2 is a step to prepare the compound (26f) by reacting the compound (24f) obtained in Step Ff-1 with the compound (25f), which is publicly known or easily obtained from publicly known compounds, in the presence of a condensing agent.
Step Ff-2 can be carried out according to a method similar to Step Df-2.

(Step Ff-3)

Step Ff-3 is a step to prepare the compound (If-d) by eliminating the allyl group in the presence of a palladium reagent in the allyloxy group of the compound (26f) obtained in Step Ff-2.
Step Ff-3 can be carried out according to a method similar to Step Ef-6a.

(Method Gf)

Method Gf is a method to prepare the compound (If-e) or the compound (If-f) in which $Rf^1$ is $-CORf^a$, $Rf^7$ is a hydrogen atom, $Xf^1$ is a group having a formula: -O- and $Rf^8$ is $-Xf^2COOH$ or $-Xf^2CORf^f$ in the formula (If).

(Step Gf-1)

Step Gf-1 is a step to prepare the compound (28f) by reacting the compound (27f), which is publicly known or easily obtained from publicly known compounds, with dimethylformamide in the presence of an alkyllithium and a base.
Step Gf-1 can be carried out according to a method similar to Step Ef-3a.

(Step Gf-2)

Step Gf-2 is a step to prepare the compound (30f) by reacting the compound (28f) obtained in Step Gf-1 with the compound (29f).
The solvent used is selected from the above solvent group and is preferably an ether, more preferably tetrahydrofuran.
The reaction temperature is usually from 0 to 100˚C, preferably from 20 to 60˚C.
The reaction time is usually from 30 minutes to 24 hours, preferably from 1 hour to 12 hours.
In Step Gf-2, the compound: $Rf^6MgCl$ can be also used instead of the compound (29f).

(Step Gf-3)

Step Gf-3 is to prepare the compound (32f) by reacting the compound (30f) obtained in Step Gf-2 with the compound (31f) in the presence of an azodicarboxylate reagent and a phosphine reagent. The compound (31f) is publicly known, is easily obtained from publicly known compounds, or can be obtained by Method Jf, Method Lf or Method Mf.
Step Gf-3 can be carried out according to a method similar to Step Ef-4.

(Step Gf-4)

Step Gf-4 comprises
(Step Gf-4a): a step in which the dimethoxymethyl group is converted to a formyl group in the presence of an acid in the compound (32f) obtained in Step Gf-3;
(Step Gf-4b): a step in which the compound obtained in Step Gf-4a is oxidized by sodium hypochlorite ($NaClO_2$) in the presence of sodium dihydrogenphosphate and 2-methyl-2-butene;
(Step Gf-4c): a step in which the compound obtained in Step Gf-4b is reacted with the above compound (2f), which is publicly known or easily obtained from publicly known compounds, in the presence of a condensing agent; and
(Step Gf-4d): a step in which in the $-COORf^g$ group of the compound obtained in Step Gf-4c, the $Rf^g$ group is eliminated in the presence of a palladium reagent to prepare the compound (If-e).
Step Gf-4a, Step Gf-4b, Step Gf-4c and Step Gf-4d can be carried out according to methods similar to Step Ef-5a, Step Ef-5c, Step Df-2 and Step Ef-6a, respectively.

(Step Gf-5)

Step Gf-5 is a step to prepare the compound (If-f) by reacting the compound (If-e) obtained in Step Gf-4 with the compound (25f), which is publicly known or easily obtained from publicly known compounds, in the presence of a condensing agent.
Step Gf-5 can be carried out according to a method similar to Step Df-2.

(Method Hf)

Method Hf is a method to prepare the compound (If-c) in which $Rf^1$ is -COO(t-Bu), $Rf^2$ is a hydroxyl group, $Rf^6$ and $Rf^7$ are hydrogen atoms, $Xf^1$ is a group having the formula: -O- and $Rf^8$ is $-Xf^2COOH$ in the formula (If).

(Step Hf-1)

Step Hf-1 is a step to prepare the compound (34f) by reacting the compound (33f), which is publicly known or easily obtained from publicly known compounds, with di-tert-butyl dicarbonate [$(t\text{-}BuOCO)_2O$] in the presence of an alkyl

lithium and a base.

The alkyl lithium used is selected from the alkyl lithiums indicated in the above base group and is preferably n-butyl lithium.

The base used can be one having the property of being coordinated onto a lithium ion, and is preferably tetramethylethylenediamine.

The solvent used is selected from the above solvent group and is preferably an ether, more preferably diethyl ether.

The reaction temperature is usually from -80 to 50°C, preferably from -50 to 20°C.

The reaction time is usually from 10 minutes to 6 hours, preferably from 30 minutes to 3 hours.

(Step Hf-2)

Step Hf-2 is a step to prepare the compound (35f) by carrying out an elimination reaction of the silyl group ($Rf^h$) in the compound (34f) obtained in Step Hf-1.

The reagent used is not particularly limited if it is usually used in elimination reactions of the silyl group, and can include an acid from the above acid group, a reagent producing fluorine ion ($F^-$) such as tetra-n-butylammonium fluoride, potassium fluoride or a mixture of these, and is preferably acetic acid, tetra-n-butylammonium fluoride or a mixture of these, more preferably a mixture of acetic acid and tetra-n-butylammonium fluoride.

The solvent used is selected from the above solvent group and is preferably an ether, more preferably tetrahydrofuran. As a combination of the reagent and the solvent used in Step Hf-2, a mixture of acetic acid, tetrahydrofuran and water is also preferable.

The reaction temperature is usually from 0 to 150°C, preferably from 20 to 100°C.

The reaction time is usually from 30 minutes to 12 hours, preferably from 1 hour to 6 hours.

(Step Hf-3)

Step Hf-3 is a step to prepare the compound (36f) by reacting the compound (35f) obtained in Step Hf-2 with the compound (20f) in the presence of an azodicarboxylate reagent and a phosphine reagent. The compound (20f) is publicly known, is easily obtained from publicly known compounds, or can be obtained by Method Jf, Method Lf or

Method Mf.

Step Hf-3 can be carried out according to a method similar to Step Ef-4.

(Step Hf-4)

Step Hf-4 is a step to prepare the compound (37f) by carrying out an elimination reaction of the methoxymethyl group in the compound (36f) obtained in Step Hf-3.

The reagent used is not particularly limited if it is usually used in elimination reactions of the methoxymethyl group and does not affect the -COO(t-Bu) group, and can include a combination of a silyl halide such as trimethylsilyl chloride and trimethylsilyl bromide and an ammonium halide such as tetra-n-butylammonium chloride and tetra-n-butylammonium bromide, and is preferably a combination of trimethylsilyl chloride and tetra-n-butylammonium bromide.

The solvent used is selected from the above solvent group and is preferably a halogenated hydrocarbon, more preferably methylene chloride.

The reaction temperature is usually from 0 to 150°C, preferably from 20 to 100°C.

The reaction time is usually from 30 minutes to 24 hours, preferably from 2 hours to 12 hours.

(Step Hf-5)

Step Hf-5 is a step to prepare the compound (If-c) by carrying out an elimination reaction of the $Rf^d$ group in the compound (37f) obtained in Step Hf-4.

Step Hf-5 can be carried out according to a method similar to Step Ef-6b. Further, in cases where $Rf^d$ of the compound (37f) is an allyl group, Step Hf-5 can be also carried out according to a method similar to Step Ef-6a.

(Method If)

Method If is a method to prepare the compound (7f) used in Step Af-4.

(Step If-1)

Step If-1 is a step to prepare the compound (7f) by reacting the compound (10f), which is publicly known or easily obtained from publicly known compounds, with the compound (8f), which is publicly known or easily obtained from publicly known compounds, in the presence of a palladium reagent and a base.
Step If-1 can be carried out according to a method similar to Step Bf-1.

(Method Jf)

Method Jf is a method to prepare the compound (20f) used in Step Ef-4 or Step Hf-3.

(Step Jf-1)

Step Jf-1 is a step to prepare the compound (39f) by reacting the compound (38f), which is publicly known or easily obtained from publicly known compounds, with the compound (8f), which is publicly known or easily obtained from publicly known compounds, in the presence of a palladium reagent and a base.
Step Jf-1 can be carried out according to a method similar to Step Bf-1.

(Step Jf-2)

Step Jf-2 is a step to prepare the compound (41f) by reacting the compound (39f) obtained in Step Jf-1 with the compound (40f), which is publicly known or easily obtained from publicly known compounds, in the presence of a palladium catalyst and a base.
Step Jf-2 can be carried out according to a method similar to Step Af-4.

(Step Jf-3)

Step Jf-3 is a step to prepare the compound (20f) by carrying out elimination of the $Rf^i$ group in the compound (41f) obtained in Step Jf-2.
Step Jf-3 can be carried out according to usually used methods (for example, a method described in T. H. Greene, P. G. Wuts, Protective Groups in Organic Synthesis, Third Edition, 1999, John Wiley & Sons, Inc. or the like) depending on the kind of the $Rf^i$ group.

(Method Kf)

Method Kf is a method to prepare the compound (41f) used in Step Jf-3.

(Step Kf-1)

Step Kf-1 is a step to prepare the compound (42f) by reacting the compound (40f), which is publicly known or easily obtained from publicly known compounds, with the compound (8f), which is publicly known or easily obtained from publicly known compounds, in the presence of a palladium reagent and a base.
Step Kf-1 can be carried out according to a method similar to Step Bf-1.

(Step Kf-2)

Step Kf-2 is a step to prepare the compound (41f) by reacting the compound (42f) obtained in Step Kf-1 with the compound (38f), which is publicly known or easily obtained from publicly known compounds, in the presence of a palladium catalyst and a base.
Step Kf-2 can be carried out according to a method similar to Step Af-4.

(Method Lf)

Method Lf is a method to prepare the compound (47f) in which $Xf^2$ is a methylene group in the compound (20f) used in Step Ef-4 or Step Hf-3.

(Step Lf-1)

Step Lf-1 is to prepare the compound (44f) by reacting the compound (43f), which is publicly known or easily obtained from publicly known compounds, with a cyanating reagent.
The cyanating reagent used is not particularly limited if it is usually used in cyanation reactions of a halogenated alkyl and can be, for example, an alkali metal cyanide, and is preferably sodium cyanide or potassium cyanide.
The solvent used is selected from the above solvent group and is preferably an alcohol, water or a mixture of these, more preferably ethanol, water or a mixture of these, more preferably a mixture of ethanol and water.
The reaction temperature is usually from 0 to 150˚C, preferably from 20 to 100˚C.
The reaction time is usually from 30 minutes to 24 hours, preferably from 2 hours to 12 hours.

(Step Lf-2)

Step Lf-2 is to prepare the compound (46f) by reacting the compound (44f) obtained in Step Lf-1 with the compound (45f), which is publicly known or easily obtained from publicly known compounds, in the presence of a palladium catalyst and a base.
Step Lf-2 can be carried out according to a method similar to Step Af-4.

(Step Lf-3)

Step Lf-3 comprises
(Step Lf-3a): a step in which the compound (46f) obtained in Step Lf-2 is hydrolyzed in the presence of an acid; and
(Step Lf-3b): a step in which the compound obtained in Step Lf-3a is reacted with the compound: $Rf^dOH$ in the presence of an acid to prepare the compound (47f).

(Step Lf-3a)

The acid used is an acid selected from the above acid group or a mixture of these, and is preferably hydrochloric acid or a mixture of hydrochloric acid and acetic acid, more preferably a mixture of hydrochloric acid and acetic acid.
The solvent used is selected from the above solvent group and is preferably acetic acid, water or a mixture of these, more preferably water. In Step Lf-3a, water is necessarily used and water alone can be also used as the solvent.
The reaction temperature is usually from 20 to 180˚C, preferably from 50 to 150˚C.
The reaction time is usually from 1 hour to 72 hours, preferably from 2 hours to 48 hours.
Step Lf-3a can be also carried out according to a method similar to Step Mf-1a.

(Step Lf-3b)

The acid used is selected from the above acid group and is preferably hydrochloric acid or sulfuric acid, more preferably sulfuric acid.
The solvent used is selected from the above solvent group and is preferably an alcohol. In Step Lf-3b, the compound: $Rf^dOH$ is preferably used as the solvent.
The reaction temperature is usually from 20 to 180˚C, preferably from 50 to 150˚C.
The reaction time is usually from 1 hour to 36 hours, preferably from 2 hours to 24 hours.
Step Lf-3b can be also carried out according to a method similar to Step Mf-1b.

(Method Mf)

Method Mf is a method to prepare the compound (47f) in which $Xf^2$ is a methylene group in the compound (20f) used in Step Ef-4 or Step Hf-3.

(Step Mf-1)

Step Mf-1 comprises
(Step Mf-1a): a step in which the compound (44f) obtained in Step Lf-1 is hydrolyzed in the presence of a base; and
(Step Mf-1b): a step in which the compound obtained in Step Mf-1a is reacted with the compound: $Rf^dXf^d$ in the presence of a base to prepare the compound (48f).

(Step Mf-1a)

The base used can be an alkali metal hydroxide or an alkaline earth metal hydroxide from the above base group, and is preferably sodium hydroxide or potassium hydroxide.
The solvent used is selected from the above solvent group and is preferably an alcohol, water or a mixture of these, more preferably a mixture of an alcohol and water, further preferably a mixture of ethylene glycol and water. In Step Mf-1a, water is necessarily used and water alone can be also used as the solvent.
The reaction temperature is usually from 50 to 200˚C, preferably from 80 to 160˚C.
The reaction time is usually from 1 hour to 72 hours, preferably from 2 hours to 48 hours.
Step Mf-1a can be also carried out according to a method similar to Step Lf-3a.

(Step Mf-1b)

The base used is selected from the above base group and is preferably an alkali metal carbonate, an alkali metal hydrogencarbonate or an alkali metal hydride, more preferably an alkali metal carbonate, further preferably sodium carbonate or potassium carbonate.
The solvent used is selected from the above solvent group and is preferably an amide, more preferably dimethylformamide.
The reaction temperature is usually from 0 to 150˚C, preferably from 20 to 100˚C.
The reaction time is usually from 1 hour to 24 hours, preferably from 2 hours to 12 hours.
Step Mf-1b can be also carried out according to a method similar to Step Lf-3b.

(Step Mf-2)

Step Mf-2 is a step to prepare the compound (47f) by reacting the compound (48f) obtained in Step Mf-1 with the compound (45f), which is publicly known or easily obtained from publicly known compounds, in the presence of a palladium catalyst and a base.
Step Mf-2 can be carried out according to a method similar to Step Af-4.

(Method Nf)

Method Nf is a method to prepare the compound (53f) in which $Xf^2$ is a methylene group substituted by $Rf^j$ and $Rf^k$ in the compound (20f) used in Step Ef-4 or Step Hf-3.

(Step Nf-1)

Step Nf-1 is a step to prepare the compound (52f) by successively reacting the compound (49f), which is publicly known or easily obtained from publicly known compounds, with the compound (50f) and subsequently the compound (51f) in the presence of a base. Step Nf-1 can be also carried out using the compound: $Xf^c$-$Rf^1$-$Xf^c$ (wherein $Rf^1$ represents an ethylene group or a trimethylene group) instead of the compound (50f) and the compound (51f).
The base used is selected from the above base group and is preferably an alkali metal hydride, more preferably sodium hydride.
The solvent used is selected from the above solvent group and is preferably an amide, more preferably dimethylformamide.
The reaction temperature is usually from 0 to 150˚C, preferably from 20 to 100˚C.
The reaction time is usually from 30 minutes to 12 hours, preferably from 1 hour to 6 hours.

(Step Nf-2)

Step Nf-2 is a step to prepare the compound (53f) by reacting the compound (52f) obtained in Step Nf-1 with the compound (45f), which is publicly known or easily obtained from publicly known compounds, in the presence of a palladium catalyst and a base.
Step Nf-2 can be carried out according to a method similar to Step Af-4.

(Method Of)

Method Of is a method to prepare the compound (57f) in which $Rf^a$ is a t-butoxy group, $Rf^2$ is a hydroxyl group and $Xf^b$ is a group having a formula: -O- in the compound (6f) used in Step Af-4 or Step Bf-1.

(Step Of-1)

Step Of-1 is a step to prepare the compound (55f) by reacting the compound (19f) obtained in Step Ef-3 with the compound (54f) in the presence of an azodicarboxylate reagent and a phosphine reagent. The compound (54f) is publicly known or is easily obtained from publicly known compounds.
Step Of-1 can be carried out according to a method similar to Step Ef-4.

(Step Of-2)

Step Of-2 comprises
(Step Of-2a): a step in which in the compound (55f) obtained in Step Of-1, the dimethoxymethyl group is converted to a formyl group and the methoxymethyl group is eliminated, in the presence of an acid; and
(Step Of-2b): a step in which the compound obtained in Step Of-2a is oxidized by sodium hypochlorite ($NaClO_2$) in the presence of sodium dihydrogenphosphate and 2-methyl-2-butene.
Step Of-2a can be carried out according to a method similar to Step Ef-5a.
Step Of-2b can be carried out according to a method similar to Step Ef-5c.

(Step Of-3)

Step Of-3 comprises
(Step Of-3a): a step in which the compound (56f) obtained in Step Of-2 is reacted with di-tert-butyl dicarbonate [$(tBuO-CO)_2$] in the presence of a base; and
(Step Of-3b): a step in which the Boc group on the hydroxyl group of the compound obtained in Step Of-3a is eliminated in the presence of a base.

(Step Of-3a)

The base used is selected from the above base group and is preferably an organic amine, more preferably 4-(N,N-dimethylamino)pyridine.
The solvent used is selected from the above solvent group and is preferably an ether, an alcohol or a mixture of these, more preferably tetrahydrofuran, 2-methyl-2-propanol or a mixture of these.
The reaction temperature is usually from 0 to 150˚C, preferably from 20 to 100˚C.
The reaction time is usually from 30 minutes to 24 hours, preferably from 1 hour to 12 hours.

(Step Of-3b)

The base used is preferably pyrrolidine or piperidine, more preferably pyrrolidine.
The solvent used is selected from the above solvent group and is preferably an ether, more preferably tetrahydrofuran.
The reaction temperature is usually from 0 to 150˚C, preferably from 20 to 100˚C.
The reaction time is usually from 10 minutes to 12 hours, preferably from 30 minutes to 6 hours.

(Method Pf)

Method Pf is a method to prepare the compound (If-c) in which $Rf^1$ is -COO(t-Bu), $Rf^2$ is a hydroxyl group, $Rf^6$ and $Rf^7$ are hydrogen atoms, $Xf^1$ is a group having the formula: -O- and $Rf^8$ is $-Xf^2COOH$ in the formula (If).

(Step Pf-1)

Step Pf-1 is a step to react the compound (18f) obtained in Step Ef-2 with methyl iodide in the presence of an alkyl lithium and a base.
Step Pf-1 can be carried out according to a method similar to Step Ef-3a.

(Step Pf-2)

Step Pf-2 comprises
(Step Pf-2a): a step in which in the compound (58f) obtained in Step Pf-1, the dimethoxymethyl group is converted to a formyl group and the methoxymethyl group is eliminated, in the presence of an acid; and

(Step Pf-2b): a step in which the compound obtained in Step Pf-2a is oxidized by sodium hypochlorite ($NaClO_2$) in the presence of sodium dihydrogenphosphate and 2-methyl-2-butene.

Step Pf-2a can be carried out according to a method similar to Step Ef-5a.

Step Pf-2b can be carried out according to a method similar to Step Ef-5c.

(Step Pf-3)

Step Pf-3 comprises

(Step Pf-3a): a step in which the compound (59f) obtained in Step Pf-2 is reacted with di-tert-butyl dicarbonate [$(tBuOCO)_2 0$] in the presence of a base; and

(Step Pf-3b): a step in which the compound obtained in Step Pf-3a is halogenated by a halogenating agent to prepare the compound (60f).

Step Pf-3a can be carried out according to a method similar to Step Of-3a.

Step Pf-3b can be carried out according to a method similar to Step Af-2.

(Step Pf-4)

Step Pf-4 is a step to prepare the compound (61f) by reacting the compound (60f) obtained in Step Pf-3 with the compound (20f) in the presence of a base. The compound (20f) is publicly known, is easily obtained from publicly known compounds or can be obtained by Method Jf, Method Lf or Method Mf.

Step Pf-4 can be carried out according to a method similar to Step Af-3.

(Step Pf-5)

Step Pf-5 is a step to eliminate the Boc group on the hydroxyl group of the compound (61f) obtained in Step Pf-4 in the presence of a base.

Step Pf-5 can be carried out according to a method similar to Step Of-3b.

(Step Pf-6)

Step Pf-6 is a step to prepare the compound (If-c) by hydrolyzing the compound (62f) obtained in Step Pf-5 in the presence of a base.

Step Pf-5 can be carried out according to a method similar to Step Ef-6b.

Further, substituent introduction reactions or the like under the following reaction conditions can be appropriately applied, if necessary, to the above Method Af to Method Pf;

(a) bromination of the 2-position of a thiophene ring: N-bromosuccinimide, acetic acid (Jackson, P. M., J. Chem. Soc, Perkin Trans. 1, 1990, vol. 11, p. 2909-2918);

(b) introduction of a methoxycarbonylmethyl group onto a nitrogen in a pyrazole ring: methyl bromoacetate, potassium carbonate; or

(c) introduction of a hydroxymethyl group to a benzyl position: paraformaldehyde, sodium hydrogencarbonate.

In cases where the compounds represented by the formulae (Ia) to (If) or pharmacologically acceptable esters thereof of the present invention have a basic group, they can be converted to a salt by reacting with an acid and in cases where the compounds represented by the formulae (Ia) to (If) or pharmacologically acceptable esters thereof of the present invention have an acidic group, they can be converted to a salt by reacting with a base. In cases where these salts are used for treatment of a disease, these must be pharmacologically acceptable ones.

Salts formed with the basic group of the compounds represented by the formulae (Ia) to (If) of the present invention can include preferably inorganic acid salts such as hydrohalogenic acid salts including hydrochloride, hydrobromide and hydroiodide; nitrates; perchlorates; sulfates; or phosphates; salts with $C_1$-$C_6$ alkanesulfonic acid which may be substituted by a fluorine atom such as methanesulfonate, trifluoromethanesulfonate and ethanesulfonate; salts with $C_6$-$C_{10}$ arylsulfonic acid which may be substituted by $C_1$-$C_4$ alkyl such as benzene sulfonate and p-toluenesulfonate; organic acid salts such as acetate; malates; fumarates; succinates; citrates; tartrates; oxalates; or maleates; or amino acid salts such as glycine salt, lysine salt, arginine salt, ornithine salt, glutamate and aspartate, more preferably hydrohalogenic acid salts.

Salts formed with the acidic group of the compounds represented by the formulae (Ia) to (If) of the present invention can include preferably metal salts such as alkali metal salts including sodium salt, potassium salt and lithium salt; alkaline earth metal salts including calcium salt and magnesium salt; aluminum salt; iron salt; zinc salt; copper salt;

nickel salt; or cobalt salt; amine salts such as inorganic amine salts including ammonium salt; or organic amine salts including t-octylamine salt, dibenzylamine salt, morpholine salt, glucosamine salt, phenylglycinealkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt, tris(hydroxymethyl)aminomethane salt, choline salt and tromethamine salt [2-amino-2-(hydroxymethyl)propan-1,3-diol salt]; or amino acid salts such as glycine salt, lysine salt, arginine salt, ornithine salt, glutamate and aspartate, and more preferably alkali metal salts.

The compounds represented by the formulae (Ia) to (If) or pharmacologically acceptable salts or esters thereof of the present invention may form hydrates by allowing to stand in an atmosphere or adsorbing a moisture content at the time of recrystallization and these hydrates are also included in the present invention. Further, the compounds of the present invention may form solvates by taking in other solvents and these solvates are also included in the present invention.

In cases where the compound of the present invention has one or more asymmetric center, an optical isomer (including a diastereomer) can exist and these isomers and mixtures thereof are described by a single formula such as the formulae (Ia) to (If). The present invention includes any of these respective isomers and mixtures thereof (including racemates) in optional ratio.

The present invention includes esters of the compounds represented by the formulae (Ia) to (If). These esters are compounds in which the hydroxyl group or the carboxyl group of the compounds represented by the formulae (Ia) to (If) is modified by addition of a protective group according to methods well-known in the field (for example, "Protective Groups in Organic Synthesis, Second Edition", Theodora W. Greene and Peter G. M. Wuts, 1991, John Wiley & Sons, Inc.).

The nature of these protective groups is not particularly limited. However, in cases where the ester is used for treatment of a disease, it must be pharmacologically acceptable and, for example, the protective group must be eliminated in a metabolism process (for example, hydrolysis) when said compound is administered into a living body of a mammal to be able to produce compounds represented by the formulae (Ia) to (If) or salts thereof. Namely, a pharmacologically acceptable ester is a "prodrug" of the compounds represented by the formulae (Ia) to (If) of the present invention.

It is easily determined whether or not an ester of the compounds represented by the formulae (Ia) to (If) of the present invention is pharmacologically acceptable. In cases where, when said compound is intraveneously administered to an experimental animal such as a rat or a mouse, blood or a body fluid of the animal is measured, the compounds represented by the formulae (Ia) to (If) or pharmacologically acceptable salts thereof of the present invention are detected, it is determined that said compound is a pharmacologically acceptable ester.

The compounds represented by the formulae (Ia) to (If) of the present invention can be converted to an ester and the ester can be a compound in which, for example, the hydroxyl group of said compound is esterified. The ester residual group is a protective group which can be eliminated in a metabolism process (for example, hydrolysis) in the living body.

The ester group which can be eliminated in a metabolism process (for example, hydrolysis) in the living body is an ester group which is eliminated in a metabolism process (for example, hydrolysis) to produce the compounds represented by the formulae (Ia) to (If) or the salts thereof when administered into the living body of a mammal. The protective group as such an ester residual group can include preferably the following groups:

(i) a 1- (acyloxy) - ($C_1$-$C_6$ alkyl) group such as a 1-[($C_1$-$C_6$ alkyl)carbonyloxy]-($C_1$-$C_6$ alkyl) group, a 1-[($C_3$-$C_8$ cycloalkyl) carbonyloxy] - ($C_1$-$C_6$ alkyl) group or a 1- [($C_6$-$C_{12}$ aryl) carbonyloxy] - ($C_1$-$C_6$ alkyl) group;
(ii) a substituted carbonyloxyalkyl group such as a ($C_1$-$C_6$ alkoxy)carbonyloxyalkyl group or an oxodioxolenylmethyl group which may be substituted (said substituent is a group selected from the group consisting of a $C_1$-$C_6$ alkyl group and an aryl group which may be substituted by $C_1$-$C_6$ alkyl or halogeno);
(iii) a phthalidyl group which may be substituted by $C_1$-$C_6$ alkyl or $C_1$-$C_6$ alkoxy;
(iv) an aliphatic acyl group indicated in a general protective group of an hydroxyl group;
(v) an aromatic acyl group indicated in the general protective group of an hydroxyl group;
(vi) a half ester residual group of succinic acid;
(vii) a phosphoric acid ester residual group;
(viii) an ester formation residual group of an amino acid such as glutamate and aspartate;
(ix) a carbamoyl group which may be substituted by 1 or 2 $C_1$-$C_6$ alkyl groups; or
(x) a 1-(acyloxy)alkoxycarbonyl group (said acyloxy group represents the above aliphatic acyloxy group or the above aromatic acyloxy group).

Of the above protective groups which are used to produce the compounds represented by the formulae (Ia) to (If) having the modified hydroxyl group and can be eliminated in a metabolism process (for example, hydrolysis) in the living body, an aliphatic acyl group (particularly, a $C_1$-$C_{25}$ alkylcarbonyl group) and a substituted carbonyloxyalkyl

group are preferable.

It is known that in addition to the compounds represented by the above formulae (Ia) to (If), the compounds included in the formulae described in, for example, International Patent Publication WO02/062302, International Patent Publication WO03/039480, International Patent Publication WO03/090746, International Patent Publication WO02/46141, International Patent Publication WO03/103651, International Patent Publication WO03/084544, International Patent Publication WO02/046181, International Patent Publication WO02/046172, International Patent Publication WO02/024632, International Patent Publication WO2004/009091, International Patent Publication WO03/031408, International Patent Publication WO03/045382, International Patent Publication WO03/053352, International Patent Publication WO2004/011448, International Patent Publication WO03/099769, International Patent Publication WO03/099775, International Patent Publication WO03/059874, International Patent Publication WO03/082192, International Patent Publication WO03/082802, International Patent Publication WO03/082205, International Patent Publication WO01/60818, International Patent Publication WO00/54759, International Patent Publication WO03/063796, International Patent Publication WO03/063576, International Patent Publication WO03/059884, International Patent Publication WO01/41704, International Patent Publication WO03/090869, International Patent Publication WO2004/024161, International Patent Publication W02004/024162, International Patent Publication W02004/026816, International Patent Publication WO03/090732, International Patent Publication W02004/043939, International Patent Publication W02004/072041, International Patent Publication W02004/072042, International Patent Publication WO2004/072046, International Patent Publication WO2004/076418, International Patent Publication WO2004/103376, International Patent Publication W02005/005416, International Patent Publication WO2005/005417, International Patent Publication WO2005/016277, International Patent Publication WO2005/023188, International Patent Publication WO2005/023196, International Patent Publication WO2005/023247 and US Patent Publication US2004/0152681 and the compounds specifically disclosed in these publications are also the LXR ligands. These LXR ligands can be used as an active ingredient of a medicament of the present invention. All matters disclosed in the above respective publications and International Patent Publication WO03/106435, International Patent Publication W02005/023782, PCT/JP2005/009142 specification, Japanese Patent Application No. 2005-146390 specification, PCT/JP2005/011928 specification, Japanese Patent Application No. 2005-189264 specification, Japanese Patent Application No. 2005-110908 specification, Japanese Patent Application No. 2004-311821 specification and Japanese Patent Application No. 2005-187686 specification are included in the disclosure of the present specification by reference.

An LXR ligand in a form of a salt such as an acid addition salt or a base addition salt may be used as an active ingredient of a medicament of the present invention. The acid addition salt can include inorganic acid salts such as hydrohalogenic acid salts including hydrochloride, hydrobromide and hydroiodide, nitrates, perchlorates, sulfates and phosphates; organic acid salts such as salts with a $C_1$-$C_6$ alkanesulfonic acid which may be substituted by a fluorine atom including methanesulfonate, trifluoromethanesulfonate and ethanesulfonate, salts with a $C_6$-$C_{10}$ arylsulfonic acid which may be substituted by $C_1$-$C_4$ alkyl including benzenesulfonate and p-toluenesulfonate, acetate, malate, fumarate, succinate, citrate, tartarate, oxalate and maleate; or amino acid salts such as glycine salt, lysine salt, arginine salt, ornithine salt, glutamate and aspartate. The base addition salt can include metal salts such as alkali metal salts including sodium salt, potassium salt and lithium salt; alkaline earth metal salts including calcium salt and magnesium salt; aluminum salt, iron salt, zinc salt, copper salt, nickel salt and cobalt salt; inorganic amine salts such as ammonium salt; organic amine salts such as a t-octylamine salt, dibenzylamine salt, morpholine salt, glucosamine salt, phenylglycinealkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt, tris(hydroxymethy)aminomethane salt, choline salt and tromethamine salt [2-amino-2-(hydroxymethyl)propan-1,3-diol salt]; or amino acid salts such as glycine salt, lysine salt, arginine salt, ornithine salt, glutamate and aspartate.

An LXR ligand in a form of hydrate or solvate can be also used in addition to the LXR ligand in a free form or a salt form as an active ingredient of a medicament of the present invention. In cases where the LXR ligand has one or more asymmetric center, an optical isomer (including a diastereomer) exists in said ligand and these isomers or optional mixtures thereof and racemates and the like may be used as an active ingredient of a medicament of the present invention. Further, in cases where the LXR ligand is a substance including one or more double bonds or ring structure and a geometrical isomer based on its (their) double bonds or ring structures exists, the respective isomers and mixtures of these at an optional ratio may be used as an active ingredient of a medicament of the present invention.

The preferable compounds as an active ingredient of a medicament of the present invention are shown below but an active ingredient of a medicament of the present invention is not limited to the following compounds.

N-(2,2,2-Trifluoroethyl)-N-{4-[2,2,2-trifluoro-1-hydroxy-1-(trifluoromethyl)ethyl]phenyl}benzenesulfonamide,
3-Chloro-4-(3-(2-propyl-3-trifluoromethyl-6-benz-[4,5]-isoxazoloxy)propylthio)phenylacetic acid,
2-(3-{3-[[2-Chloro-3-(trifluoromethyl)benzyl](2,2-diphenylethyl)amino]propoxy}-phenyl)acetic acid,
2-Benzyl-6,7-dimethoxy-3-[4-[2,2,2-trifluoro-1-hydroxy-1-(trifluoromethyl)ethyl]phenyl]-4(3H)-quinazolinone,
2-Benzyl-6-(2-hydroxyethoxy)-3-[4-[2,2,2-trifluoro-1-hydroxy-1- (trifluoromethyl) ethyl] phenyl] -4 (3H) - quinazolinone,

2-Benzyl-6-(2-pyridyl)-3-[4-[2,2,2-trifluoro-1-hydroxy-1-(trifluoromethyl)ethyl]phenyl]-4(3H)-quinazolinone,
6-(1H- Imidazol- 1- yl)- 2-(4- methylbenzyl)- 3- {2- methyl- 4- [2,2,2- trifluoro- 1- hydroxy- 1-(trifluoromethyl) ethyl] phenyl}- 4 (3H)-quinazolinone,
2- Benzyl- 6- fluoro- 3- {3- methoxy- 4-[2,2,2- trifluoro- 1- hydroxy- 1-(trifluoromethyl) ethyl] phenyl}- 7-(4- morpholinyl)- 4 (3H)-quinazolinone,
3- {2- Methyl- 4-[2,2,2- trifluoro- 1- hydroxy- 1-(trifluoromethyl) ethyl] phenyl}- 2-(3- pyridylmethyl)- 6-(1H- 1,2,4- triazol- 1- yl)- 4 (3H)-quinazolinone,
N,N-Dimethyl-3β-hydroxycholenamide,
6- Chloro- 7- methoxy- 3- {2- methyl- 5-[2,2,2- trifluoro- 1- hydroxy- 1-(trifluoromethyl) ethyl] phenyl}- 2-(3- thienylmethyl)- 4 (3H)-quinazolinone,
2-(3-Fluorobenzyl)-6,7-dimethoxy-3-{2-methyl-5-[2,2,2-trifluoro-1-hydroxy-1-(trifluoromethyl)ethyl]phenyl}-4(3H)-quinazolinone,
tert-Butyl 2-({4-[acetyl(methyl)amino]phenoxy}methyl)-6-fluoro-1H-indol-1-carboxylate,
tert-Butyl 2-({4-[(cyclopropylcarbonothioyl)(methyl)amino]phenoxy}methyl)-4,6-difluoro-1H-indol-1-carboxylate,
tert-Butyl 6-({4-[(cyclopropylcarbonyl)(methyl)amino]phenoxy}methyl)-2-hydroxy-3-(trifluoromethyl)benzoate,
tert-Butyl 2-hydroxy-6-({4-[methyl(methylsulfonyl)amino]phenoxy}methyl)-3-(trifluoromethyl)benzoate,
tert-Butyl 6-({4-[acetyl(methyl)amino]phenoxy}methyl)-3-ethyl-2-hydroxybenzoate,
tert-Butyl 6-({4-[(cyclopropylacetyl)(methyl)amino]phenoxy}methyl)-2-hydroxy-3-(trifluoromethyl)benzoate,
(4'-{[1-(tert-Butoxycarbonyl)-6-fluoro-1H-indol-2-yl]methoxy}-1,1'-biphenyl-4-yl)acetic acid,
(4'-{[1-(tert-Butoxycarbonyl)-6-fluoro-1H-indol-2-yl]methoxy}-3-chloro-1,1'-biphenyl-4-yl)acetic acid,
[5- (4-{ [1- (tert-Butoxycarbonyl) -6-fluoro-1H-indol-2-yl]methoxy}phenyl)-2-thienyl]acetic acid,
(4'-{[1-(tert-Butoxycarbonyl)-6-fluoro-1H-indol-2-yl]methoxy}-2-chloro-1,1'-biphenyl-4-yl)acetic acid,
(4'-{[2-(tert-Butoxycarbonyl)-3-hydroxy-4-(trifluoromethyl)benzyl]oxy}-1,1'-biphenyl-4-yl)acetic acid,
(4'-{[2-(tert-Butoxycarbonyl)-3-hydroxy-4-(trifluoromethyl)benzyl]oxy}-3-chloro-1,1'-biphenyl-4-yl)acetic acid,
(4'-{[2-(tert-Butoxycarbonyl)-3-hydroxy-4-(trifluoromethyl)benzyl]oxy}-2-ethyl-1,1'-biphenyl-4-yl)acetic acid,
(4'-{[2-(tert-Butoxycarbonyl)-3-hydroxy-4-(trifluoromethyl)benzyl]oxy}-2-methyl-1,1'-biphenyl-3-yl)acetic acid,
1-(4'-{[2-(tert-Butoxycarbonyl)-3-hydroxy-4-(trifluoromethyl)benzyl]oxy}-3-fluoro-1,1'-biphenyl-4-yl)cyclopropanecarboxylic acid,
(4'-{[2-(tert-Butoxycarbonyl)-3-hydroxy-4-isopropylbenzyl]oxy}-1,1'-biphenyl-4-yl)acetic acid,
(4'-{[2-(tert-Butoxycarbonyl)-4-fluoro-3-hydroxybenzyl]oxy}-1,1'-biphenyl-4-yl)acetic acid,
(4'-{[2-(tert-Butoxycarbonyl)-4-chloro-3-hydroxybenzyl]oxy}-1,1'-biphenyl-4-yl)acetic acid,
[5-(4-{[2-(tert-Butoxycarbonyl)-3-hydroxy-4-(trifluoromethyl)benzyl]oxy}phenyl)-2-thienyl]acetic acid,
(4'-{[2-(tert-Butoxycarbonyl)-3-hydroxy-4-(trifluoromethyl)benzyl]oxy}-2-nitro-1,1'-biphenyl-4-yl)acetic acid,
(4'-{[2-(tert-Butoxycarbonyl)-3-hydroxy-4-(trifluoromethyl) benzyl] oxy}-3-fluoro-1, 1'-biphenyl-4-yl)acetic acid,
(4'-{[2-(tert-Butoxycarbonyl)-3-hydroxy-4-(trifluoromethyl)benzyl]oxy}-2-methyl-1,1'-biphenyl-4-yl)acetic acid,
(4'-{[2-(tert-Butoxycarbonyl)-3-hydroxy-4-(trifluoromethyl)benzyl]oxy}-2-methoxy-1,1'-biphenyl-4-yl)acetic acid,
(4'-{[2-(tert-Butoxycarbonyl)-3-hydroxy-4-(trifluoromethyl)benzyl]oxy}-2-chloro-1,1'-biphenyl-4-yl)acetic acid,
(4'-{[2-(tert-Butoxycarbonyl)-3-hydroxy-4-(trifluoromethyl)benzyl]oxy}-2-trifluoromethyl-1,1'-biphenyl-4-yl)acetic acid,
1-(4'-{[2-(tert-Butoxycarbonyl)-3-hydroxy-4-(trifluoromethyl)benzyl]oxy}-1,1'-biphenyl-4-yl)cyclopropanecarboxylic acid,
tert-Butyl 2-hydroxy-6-({[3'-(methylsulfonyl)-1,1'-biphenyl-4-yl]oxy}methyl)-3-(trifluoromethyl)benzoate,
(2-Amino-4'-{[2-(tert-butoxycarbonyl)-3-hydroxy-4-(trifluoromethyl)benzyl]oxy}-1,1'-biphenyl-4-yl)acetic acid,
[4'-{[2-(tert-Butoxycarbonyl)-3-hydroxy-4-(trifluoromethyl)benzyl]oxy}-2-(dimethylamino)-1,1'-biphehyl-4-yl]acetic acid,
2- (4'-{[2- (tert-Butoxycarbonyl) -3-hydroxy-4-(trifluoromethyl)benzyl]oxy}-1,1'-biphenyl-4-yl)-3-hydroxypropanoic acid,
(4'-{[2-(tert-Butoxycarbonyl)-3-hydroxy-4-(trifluoromethyl)benzyl]oxy}-2-isopropyl-1,1'-biphenyl-4-yl)acetic acid,
4'-{[2-(tert-Butoxycarbonyl)-3-hydroxy-4-(trifluoromethyl)benzyl]oxy}-1,1'-biphenyl-4-carboxylic acid,
(4'-{[2-(tert-Butoxycarbonyl)-3-hydroxy-4-(trifluoromethyl)benzyl]oxy}-2-formyl-1,1'-biphenyl-4-yl)acetic acid,
(4'-{[2-(tert-Butoxycarbonyl)-3-hydroxy-4-(trifluoromethyl)benzyl]oxy}-2-(hydroxymethyl)-1,1'-biphenyl-4-yl)acetic acid, or
(4'-{[2-(tert-Butoxycarbonyl)-3-hydroxy-4-(trifluoromethyl)benzyl]oxy}-2-cyano-1,1'-biphenyl-4-yl)acetic acid.

Further, the following compounds are particularly preferable as an active ingredient of a medicament of the present invention.

2-Benzyl-6,7-dimethoxy-3-[4-[2,2,2-trifluoro-1-hydroxy-1-(trifluoromethyl)ethyl]phenyl]-4(3H)-quinazolinone,
2-Benzyl-6-(2-hydroxyethoxy)-3-[4-[2,2,2-trifluoro-1-hydroxy-1-(trifluoromethyl)ethyl]phenyl]-4(3H)-quinazolinone,
3-{2-Methyl-4-[2,2,2-trifluoro-1-hydroxy-1-(trifluoromethyl)ethyl]phenyl}-2-(3-pyridylmethyl)-6-(1H-1,2,4-triazoly-1-yl)-4 (3H)-quinazolinone,
tert-Butyl 2-({4-[(cyclopropylcarbonothioyl)(methyl)amino]phenoxy}methyl)-4,6-difluoro-1H-indol-1-carboxylate,
tert-Butyl 6-({4-[(cyclopropylcarbonyl)(methyl)amino]phenoxy}methyl)-2-hydroxy-3-(trifluoromethyl)benzoate,
tert-Butyl 2-hydroxy-6-({4-[methyl(methylsulfonyl)amino]phenoxy}methyl)-3-(trifluoromethyl)benzoate,
(4'-{[1-(tert-Butoxycarbonyl)-6-fluoro-1H-indol-2-yl]methoxy}-1,1'-biphenyl-4-yl)acetic acid,

(4'-{[2-(tert-Butoxycarbonyl)-3-hydroxy-4-(trifluoromethyl)benzyl]oxy}-1,1'-biphenyl-4-yl)acetic acid,
(4'-{[2-(tert-Butoxycarbonyl)-3-hydroxy-4-(trifluoromethyl)benzyl]oxy}-3-chloro-1,1'-biphenyl-4-yl)acetic acid,
(4'-{[2-(tert-Butoxycarbonyl)-3-hydroxy-4-(trifluoromethyl)benzyl]oxy}-2-ethyl-1,1'-biphenyl-4-yl)acetic acid,
(4'-{[2-(tert-Butoxycarbonyl)-3-hydroxy-4-(trifluoromethyl)benzyl]oxy}-2-methyl-1,1'-biphenyl-3-yl)acetic acid,
(4'-{[2-(tert-Butoxycarbonyl)-3-hydroxy-4-(trifluoromethyl)benzyl]oxy}-3-fluoro-1,1'-biphenyl-4-yl)acetic acid,
(4'-{[2-(tert-Butoxycarbonyl)-3-hydroxy-4-(trifluoromethyl)benzyl]oxy}-2-methoxy-1,1'-biphenyl-4-yl)acetic acid,
(4'-{[2-(tert-Butoxycarbonyl)-3-hydroxy-4-(trifluoromethyl)benzyl]oxy}-2-chloro-1,1'-biphenyl-4-yl)acetic acid,
tert-Butyl 2-hydroxy-6-({[3'-(methylsulfonyl)-1,1'-biphenyl-4-yl]oxy}methyl)-3-(trifluoromethyl)benzoate,
2-(4'-{[2-(tert-Butoxycarbonyl)-3-hydroxy-4-(trifluoromethyl)benzyl]oxy}-1,1'-biphenyl-4-yl)-3-hydroxypropanoic acid,
(4'-{[2-(tert-Butoxycarbonyl)-3-hydroxy-4-(trifluoromethyl)benzyl]oxy}-2-formyl-1,1'-biphenyl-4-yl)acetic acid,
(4'-{[2-(tert-Butoxycarbonyl)-3-hydroxy-4-(trifluoromethyl)benzyl]oxy}-2-(hydroxymethyl)-1,1'-biphenyl-4-yl)acetic acid, or
(4'-{[2-(tert-Butoxycarbonyl)-3-hydroxy-4-(trifluoromethyl)benzyl]oxy}-2-cyano-1,1'-biphenyl-4-yl)acetic acid.

A medicament provided by the present invention has an activity of suppressing production of tissue factor, and has an activity of decreasing thrombus formation in bodies of warm-blooded animals. Thus, a medicament of the present invention is useful for, for example, the treatment and/or prophylaxis of vascular restenosis following angioplasty, endarterectomy, percutaneous transluminal coronary angioplasty (PTCA) or stent implantation, or the treatment and/or prophylaxis of blood coagulation diseases. The application target of a medicament of the present invention is not limited to the aforementioned conditions.

A medicament comprising an LXR ligand as an active ingredient is one which allows the administration of an LXR ligand itself, or one which is mixed with a suitable pharmacologically acceptable vehicle or diluent and so forth and allows to be administered either orally in the form of a tablet, capsule, granules, powder or syrup and the like, or parenterally in the form of an injection, suppository, patch or externally applied preparation and the like. These formulations can be produced by known methods using additives such as excipients, lubricants, binders, disintegration agents, emulsifiers, stabilizers, corrigents and diluents.

Examples of excipients include organic excipients and inorganic excipients. Examples of organic excipients include sugar derivatives such as lactose, sucrose, glucose, mannitol and sorbitol; starch derivatives such as cornstarch, potato starch, alpha starch and dextrin; cellulose derivatives such as crystalline cellulose; gum arabic; dextran; and, pullulan. Examples of inorganic excipients include silicate derivatives such as light silicic anhydride, synthetic aluminum silicate, calcium silicate or magnesium aluminometasilicate; phosphates such as calcium hydrogenphosphate; carbonates such as calcium carbonate; and sulfates such as calcium sulfate.

Examples of lubricants include stearic acid; metal stearates such as calcium stearate or magnesium stearate; talc; colloidal silica; waxes such as bees wax and spermaceti; boric acid; adipic acid; sulfates such as sodium sulfate; glycol; fumaric acid; sodium benzoate, DL-leucine; lauryl sulfates such as sodium lauryl sulfate and magnesium lauryl sulfate; silicic acids such as silicic anhydride or silicic acid hydrate; and the aforementioned starch derivatives.

Examples of binders include hydroxypropyl cellulose, hydroxypropyl methyl cellulose, polyvinyl pyrrolidone, polyethylene glycol, and derivatives described in the aforementioned excipients.

Examples of disintegration agents include cellulose derivatives such as low substituted hydroxypropyl cellulose, carboxymethyl cellulose, calcium carboxymethyl cellulose or internally crosslinked sodium carboxymethyl cellulose; chemically modified starch and cellulose derivatives such as carboxymethyl starch and sodium carboxymethyl starch; and crosslinked polyvinyl pyrrolidone.

Examples of emulsifiers include colloidal clays such as bentonite or veegum; metal hydroxides such as magnesium hydroxide or aluminum hydroxide; anionic surfactants such as sodium lauryl sulfate and calcium stearate; cationic surfactants such as benzalkonium chloride; and nonionic surfactants such as polyoxyethylene alkyl ether, polyoxyethylene sorbitan fatty acid esters or sucrose fatty acid esters.

Examples of stabilizers include para-hydroxybenzoic acid esters such as methyl paraben and propyl paraben; alcohols such as chlorobutanol, benzyl alcohol or phenyl ethyl alcohol; benzalkonium chloride; phenols such as phenol and cresol; thimerosal; dehydroacetic acid; and sorbic acid.

Examples of corrigents include usually used sweeteners, acidulants and fragrances.

A medicament of the present invention can be administered to warm-blooded animals (including humans), and can be administered particularly preferably to humans. Although there are no particular limitations on the dose of an LXR ligand, said dose is preferably suitably selected depending on the type of an LXR ligand, type of disease, and body weight or age of the patient. A preferred dose of an LXR ligand has a lower limit of 0.01 mg/kg (and preferably 0.05 mg/kg) and an upper limit of 500 mg/kg (and preferably 100 mg/kg) per administration for human adult in a case of oral administration, and a lower limit of 0.001 mg/kg (and preferably 0.005 mg/kg) and an upper limit of 100 mg/kg (and preferably 20 mg/kg) for human adult in a case of intravenous administration. An LXR ligand is preferably administered 1 to 6 times per day corresponding to depending on the disease and symptoms thereof.

[Examples]

The following Examples indicate test methods and results of the inhibitory activity of tissue factor production of compounds. Test Examples indicate examples of methods for measuring an activity of compounds as an LXR ligand (and preferably an LXR agonist). The Reference Examples indicate methods for preparation of a compound tested for an activity of tissue factor production. Formulation Examples indicate examples of methods for preparing formulations of medicaments of the present invention.

Compounds A to J in Table 7 of Example 1 and Table 8 of Example 2 indicate the following compounds.Compound A: N-(2,2,2-Trifluoroethyl)-N-{4-[2,2,2-trifluoro-1-hydroxy-1-(trifluoromethyl)ethyl]phenyl}benzenesulfonamide (Compound 12 described on page 55 of International Patent Publication WO2000/054759)

[Chemical formula 25]

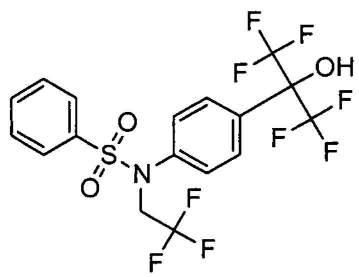

Compound B: 3-Chloro-4-(3-(2-propyl-3-trifluoromethyl-6-benz-[4,5]-isoxazoloxy)propylthio)phenylacetic acid (a compound described in Example 20 on page 70 of International Patent Publication WO1997/028137; an activity for an LXR ligand is described in Endocrinology, 143, pp. 2548-2558, 2002)

[Chemical formula 26]

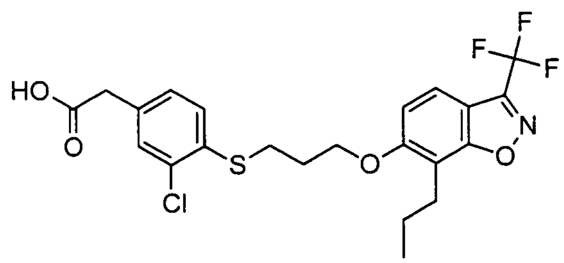

Compound C: 2-(3-{3-[[2-Chloro-3-(trifluoromethyl)benzyl](2,2-diphenylethyl)amino]propoxy}-phenyl)acetic acid (a compound described in Example 16 on page 46 of International Patent Publication WO2002/24632)

[Chemical formula 27]

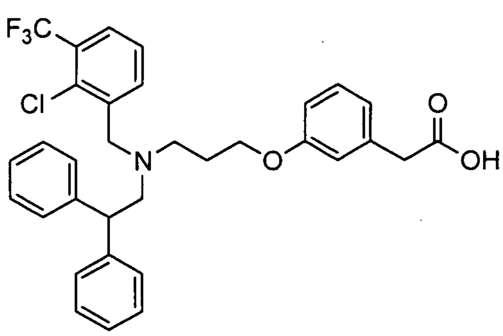

Compound D: 2-Benzyl-6,7-dimethoxy-3-[4-[2,2,2-trifluoro-1-hydroxy-1- (trifluoromethyl) ethyl] phenyl] -4 (3H) - quinazolinone (a compound described in Example 54 on page 298 of International Patent Publication WO2003/106435);
Compound E: 2-Benzyl-6-(2-hydroxyethoxy)-3-[4-[2,2,2-trifluoro-1-hydroxy-1-(trifluoromethyl)ethyl]phenyl]-4(3H)-quinazolinone (a compound described in Example 1 of International Patent Publication WO2005/023782);
Compound F: 2-Benzyl-6-(2-pyridyl)-3-[4-[2,2,2-trifluoro-1-hydroxy-1- (trifluoromethyl) ethyl] phenyl] -4 (3H) - quinazolinone (a compound described in Example 102 of International Patent Publication WO2005/023782);
Compound G: 6-(1H-Imidazol-1-yl)-2-(4-methylbenzyl)-3-{2-methyl-4-[2,2,2-trifluoro-1-hydroxy-1-(trifluoromethyl)ethyl] phenyl}-4(3H)-quinazolinone (a compound described in Example 232 of International Patent Publication WO2005/023782);
Compound H: 2-Benzyl-6-fluoro-3-{3-methoxy-4-[2,2,2-trifluoro-1-hydroxy-1-(trifluoromethyl)ethyl]phenyl}-7-(4-morpholinyl)-4(3H)-quinazolinone (a compound described in Example 193 of International Patent Publication W02005/023782);
Compound I: 3-{2-Methyl-4-[2,2,2-trifluoro-1-hydroxy-1-(trifluoromethyl)ethyl]phenyl}-2-(3-pyridylmethyl)-6-(1H-1,2,4-triazol-1-yl)-4(3H)-quinazolinone (a compound described in Example 233 of International Patent Publication WO2005/023782);
Compound J: N,N-Dimethyl-3β-hydroxycholenamide (a compound described in J. Med. Chem., 2001, Vol. 44, pp. 886-897).

[Chemical formula 28]

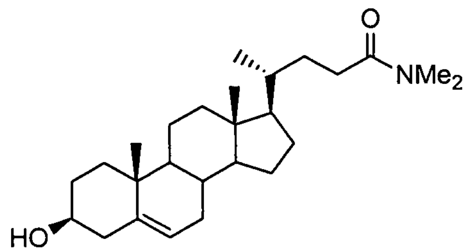

Compounds D to I are compounds represented by the following structural formula. Compound D is included in compounds represented by the aforementioned general formula (Ia). Compounds E to I are included in compounds represented by the aforementioned general formula (Ib).

[Table 1]

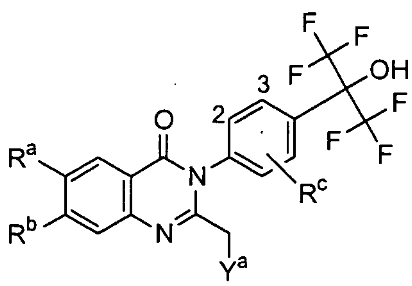

| | $R^a$ | $R^b$ | $R^c$ | $Y^a$ |
|---|---|---|---|---|
| Compound D | OMe | OMe | H | phenyl |
| Compound E | $HO(CH_2)_2O$ | H | H | phenyl |
| Compound F | 2-pyridyl | H | H | phenyl |
| Compound G | 1H-imidazol-1-yl | H | 2-Me | 4-methylphenyl |
| Compound H | F | 4-morpholinyl | 3-OMe | phenyl |
| Compound I | 1H-1,2,4-triazol-1-yl | H | 2-Me | 3-pyridyl |

The compounds indicated in Reference Examples 1 and 2 are compounds represented by the following structural formula. The compounds indicated in Reference Examples 1 and 2 are included in the compounds represented

by the aforementioned general formula (Ia).

[Table 2]

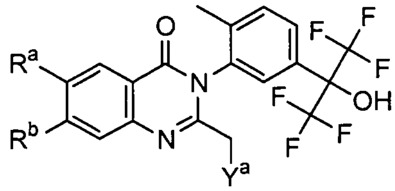

| Reference Example No. | $R^a$ | $R^b$ | $Y^a$ |
|---|---|---|---|
| Reference Example 1 | Cl | OMe | 3-thienyl |
| Reference Example 2 | OMe | OMe | 3-F-phenyl |

The compounds represented in Reference Examples 3 to 37 are compounds represented by the following structural formula. The compounds indicated in Reference Examples 3 and 4 are included in compounds represented by the aforementioned general formula (Ic). The compounds indicated in Reference Examples 5 to 8 are included in compounds represented by the aforementioned formula (Id). The compounds indicated in Reference Examples 9 to 12 are included in compounds represented by the aforementioned general formula (Ie). The compounds indicated in Reference Examples 13 to 37 are compounds included in the compounds represented by the aforementioned general formula (If). The abbreviations used in the following Tables 3 to 6 indicate the following groups;
cbx-cPr: 1-carboxy-1-cyclopropyl
cPr: cyclopropyl
Et: ethyl
iPr: 2-propyl
Me: methyl
t-Bu: 2-methyl-2-propyl.

[Table 3]

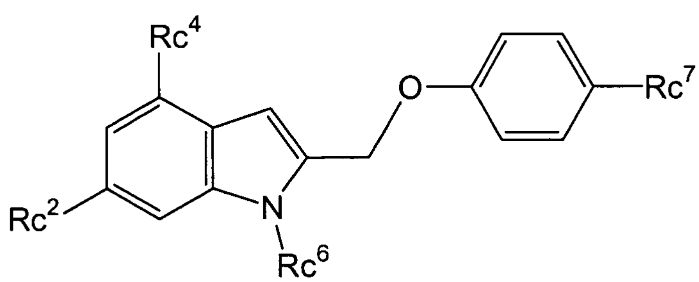

| Reference Example No. | $Rc^2$ | $Rc^4$ | $Rc^6$ | $Rc^7$ |
|---|---|---|---|---|
| Reference Example 3 | F | H | COO(t-Bu) | $N(Me)COCH_3$ |
| Reference Example 4 | F | F | COO(t-Bu) | N(Me)CScPr |

[Table 4]

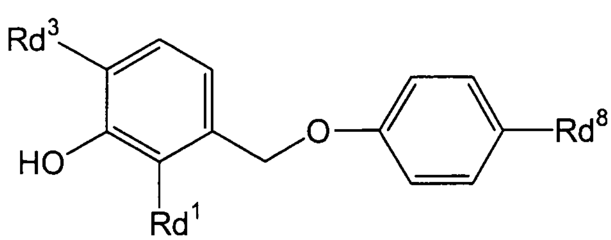

(continued)

| Reference Example No. | $Rd^1$ | $Rd^3$ | $Rd^8$ |
|---|---|---|---|
| Reference Example 5 | COO(t-Bu) | $CF_3$ | N(Me)COcPr |
| Reference Example 6 | COO(t-Bu) | $CF_3$ | N(Me)$SO_2$Me |
| Reference Example 7 | COO(t-Bu) | Et | N(Me)COMe |
| Reference Example 8 | COO(t-Bu) | $CF_3$ | N(Me)$COCH_2$cPr |

[Table 5]

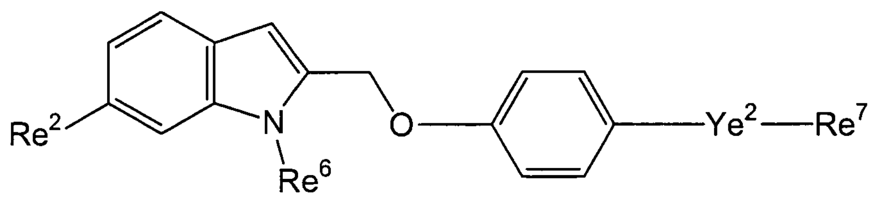

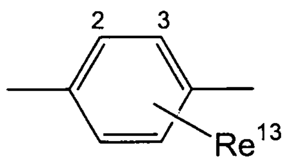

(Ye$^{2a}$)

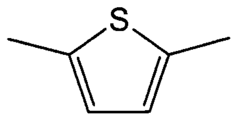

(Ye$^{2b}$)

| Reference Example No. | $Re^2$ | $Re^6$ | $Ye^2$ | $Re^7$ | $Re^{13}$ |
|---|---|---|---|---|---|
| Reference Example 9 | F | COO(t-Bu) | $Ye^{2a}$ | $CH_2COOH$ | H |
| Reference Example 10 | F | COO(t-Bu) | $Ye^{2a}$ | $CH_2COOH$ | 3-Cl |
| Reference Example 11 | F | COO(t-Bu) | $Ye^{2b}$ | $CH_2COOH$ | |
| Reference Example 12 | F | COO(t-Bu) | $Ye^{2a}$ | $CH_2COOH$ | 2-Cl |

[Table 6]

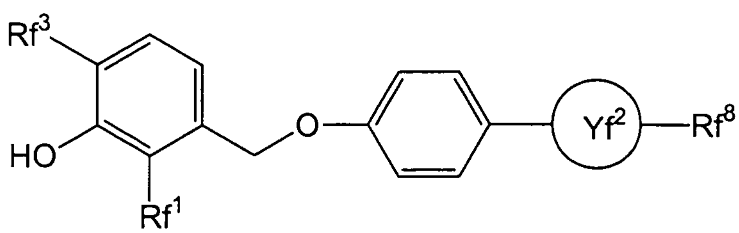

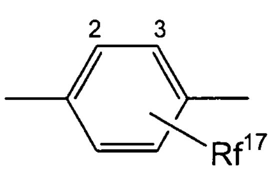

(Yf$^{2a}$)

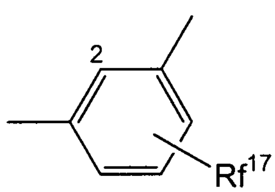

(Yf$^{2b}$)

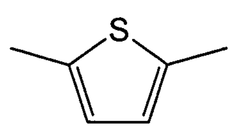

(Yf$^{2c}$)

| Reference Example No. | $Rf^1$ | $Rf^3$ | $Yf^2$ | $Rf^8$ | $Rf^{17}$ |
|---|---|---|---|---|---|
| Reference Example 13 | COO (t-Bu) | $CF_3$ | $Yf^{2a}$ | $CH_2COOH$ | H |

(continued)

| Reference Example No. | $Rf^1$ | $Rf^3$ | $Yf^2$ | $Rf^8$ | $Rf^{17}$ |
|---|---|---|---|---|---|
| Reference Example 14 | COO(t-Bu) | $CF_3$ | $Yf^{2a}$ | $CH_2COOH$ | 3-Cl |
| Reference Example 15 | COO(t-Bu) | $CF_3$ | $Yf^{2a}$ | $CH_2COOH$ | 2-Et |
| Reference Example 16 | COO(t-Bu) | $CF_3$ | $Yf^{2b}$ | $CH_2COOH$ | 2-Me |
| Reference Example 17 | COO(t-Bu) | $CF_3$ | $Yf^{2a}$ | cbx-cPr | 3-F |
| Reference Example 18 | COO(t-Bu) | iPr | $Yf^{2a}$ | $CH_2COOH$ | H |
| Reference Example 19 | COO(t-Bu) | Cl | $Yf^{2a}$ | $CH_2COOH$ | H |
| Reference Example 20 | COO(t-Bu) | F | $Yf^{2a}$ | $CH_2COOH$ | H |
| Reference Example 21 | COO(t-Bu) | $CF_3$ | $Yf^{2c}$ | $CH_2COOH$ | |
| Reference Example 22 | COO(t-Bu) | $CF_3$ | $Yf^{2a}$ | $CH_2COOH$ | $2\text{-}NO_2$ |
| Reference Example 23 | COO(t-Bu) | $CF_3$ | $Yf^{2a}$ | $CH_2COOH$ | 3-F |
| Reference Example 24 | COO(t-Bu) | $CF_3$ | $Yf^{2a}$ | $CH_2COOH$ | 2-Me |
| Reference Example 25 | COO(t-Bu) | $CF_3$ | $Yf^{2a}$ | $CH_2COOH$ | 2-MeO |
| Reference Example 26 | COO(t-Bu) | $CF_3$ | $Yf^{2a}$ | $CH_2COOH$ | 2-Cl |
| Reference Example 27 | COO(t-Bu) | $CF_3$ | $Yf^{2a}$ | $CH_2COOH$ | $2\text{-}CF_3$ |
| Reference Example 28 | COO(t-Bu) | $CF_3$ | $Yf^{2a}$ | cbx-cPr | H |
| Reference Example 29 | COO(t-Bu) | $CF_3$ | $Yf^{2b}$ | $SO_2Me$ | H |
| Reference Example 30 | COO(t-Bu) | $CF_3$ | $Yf^{2a}$ | $CH_2COOH$ | $2\text{-}NH_2$ |
| Reference Example 31 | COO(t-Bu) | $CF_3$ | $Yf^{2a}$ | $CH_2COOH$ | $2\text{-}NMe_2$ |
| Reference Example 32 | COO(t-Bu) | $CF_3$ | $Yf^{2a}$ | $CH(CH_2OH)COOH$ | H |
| Reference Example 33 | COO(t-Bu) | $CF_3$ | $Yf^{2a}$ | $CH_2COOH$ | 2-iPr |
| Reference Example 34 | COO(t-Bu) | $CF_3$ | $Yf^{2a}$ | COOH | H |
| Reference Example 35 | COO(t-Bu) | $CF_3$ | $Yf^{2a}$ | $CH_2COOH$ | 2-CHO |
| Reference Example 36 | COO(t-Bu) | $CF_3$ | $Yf^{2a}$ | $CH_2COOH$ | $2\text{-}CH_2OH$ |
| Reference Example 37 | COO(t-Bu) | $CF_3$ | $Yf^{2a}$ | $CH_2COOH$ | 2-CN |

(Example 1) Assay of mouse peritoneal macrophage tissue factor mRNA

3 ml of thioglycolate (Sigma Chemical) were administered into the abdominal cavity of male C57BL/6J mice (Charles River) followed 4 days later by intraperitoneal administration of 10 ml of phosphate-buffered saline (hereinafter PBS) containing heparin (Hishiyama Pharmaceutical) at a concentration of 5 U/ml and recovery of intraperitoneal macrophages using a syringe. After centrifuging the recovered macrophages at 1000 rpm and 4°C for 5 minutes, the supernatant was discarded followed by suspending in RPMI 1640 medium (Gibco Laboratories) containing normal fetal bovine serum (hereinafter FBS) at a concentration of 10%. The macrophages were adjusted to a concentration of 4 x $10^6$ cells/ml, disseminated in 1 ml aliquots in a 12-well plate, and cultured for 3 hours at 37°C using a $CO_2$ incubator. Subsequently, the cells were washed with PBS, and the medium was replaced with RPMI medium containing lipoprotein-deficient serum (hereinafter LPDS) (Sigma Chemical) at a concentration of 5%. Test compounds dissolved in dimethylsulfoxide (DMSO) at a concentration of 1 $\mu$M were added to make a final DMSO concentration of 0.1%, and after warming for 18 hours at 37°C, lipopolysaccharide (hereinafter LPS) (Sigma Chemical) was added to make a concentration of 100 ng/ml. The cells were recovered 6 hours later, RNA was extracted using the Rneasy Mini Kit (Qiagen), and after carrying out a reverse transcription reaction using the First-Strand cDNA Synthesis Kit (Amersham Biosciences), the expressed amounts of tissue factor mRNA and cyclophylin mRNA were measured by quantitative RT-PCR (TaqMan, Applied Biosystems 7700 Sequence Detector).

TF-1: 5'-GGCCACCATCTTTATCATCC-3'
TF-2: 5'-TGTTCTTCCCTTTCTGTCCC-3'
TF-P: 5'-FAM-CCATATCTCTGTGCAAGCGCAGAAAGAACC-TAMRA-3'
Cyl-1: 5'-CGATGACGAGCCCTTGG-3'
Cyl-2: 5'-TCTGCTGTCTTTGGAACTTTGTC-3'
Cyl-P: 5'-FAM-CGCGTCTCCTTTGAGCTGTTTGCA-TAMRA-3'

Quantitative RT-PCR for tissue factor was carried out using the aforementioned TF-1 and TF-2 as primers and TF-P as probes, while quantitative PCR for cyclophilin was carried out using the aforementioned Cyl-1 and Cyl-2 as primers and Cyl-P as probes. Both were carried out by warming for 2 minutes at 50°C and then for 10 minutes at 95°C,

followed by 40 warming cycles consisting of warming for 15 seconds at 95°C and then for 1 minute at 60°C. The expressed amount of tissue factor mRNA was calculated as a relative value of the expressed amount of cyclophilin mRNA. The expressed amounts of tissue factor mRNA resulting from the test compounds at a concentration of 1 μM or 10 μM based on a value of 100 for the expressed amount of tissue factor mRNA in a case of treatment with DMSO at a concentration of 1% are shown in Table 7.

[Table 7]

| Test Compound No. | Expressed Amount of Tissue Factor mRNA | Concentration (μM) |
|---|---|---|
| Compound A | 20.0 | 1 |
| Compound B | 47.3 | 1 |
| Compound C | 68.8 | 1 |
| Compound D | 39.0 | 1 |
| Compound E | 37.7 | 1 |
| Compound F | 76.9 | 1 |
| Compound G | 44.7 | 10 |
| Compound H | 56.0 | 10 |
| Compound I | 48.6 | 1 |
| Compound J | 39.3 | 10 |
| Reference Example 1 | 41.1 | 10 |
| Reference Example 2 | 45.2 | 10 |
| Reference Example 3 | 67.9 | 1 |
| Reference Example 4 | 57.6 | 1 |
| Reference Example 5 | 64.3 | 1 |
| Reference Example 6 | 62.8 | 1 |
| Reference Example 7 | 68.0 | 1 |
| Reference Example 8 | 51.8 | 1 |
| Reference Example 9 | 66.0 | 1 |
| Reference Example 10 | 68.9 | 1 |
| Reference Example 11 | 61.1 | 1 |
| Reference Example 12 | 54.3 | 1 |
| Reference Example 13 | 65.4 | 1 |
| Reference Example 14 | 61.2 | 1 |
| Reference Example 15 | 55.5 | 1 |
| Reference Example 16 | 65.4 | 1 |
| Reference Example 17 | 57.2 | 1 |
| Reference Example 18 | 56.9 | 1 |
| Reference Example 19 | 51.6 | 10 |
| Reference Example 20 | 58.1 | 10 |
| Reference Example 21 | 68.9 | 1 |
| Reference Example 22 | 54.0 | 1 |
| Reference Example 23 | 51.6 | 1 |
| Reference Example 24 | 61.8 | 1 |
| Reference Example 25 | 64.0 | 1 |
| Reference Example 26 | 42.3 | 1 |
| Reference Example 27 | 63.5 | 1 |
| Reference Example 28 | 41.7 | 10 |
| Reference Example 29 | 88.4 | 10 |
| Reference Example 30 | 65.6 | 1 |
| Reference Example 31 | 33.7 | 10 |
| Reference Example 32 | 48.5 | 10 |
| Reference Example 33 | 37.2 | 10 |
| Reference Example 34 | 39.0 | 10 |

From the above results, an LXR ligand (and particularly an LXR agonist) was demonstrated to have a superior inhibitory activity for production of tissue factor, and be useful as a medicament for the treatment and/or prophylaxis of vascular restenosis following angioplasty, endarterectomy, percutaneous transluminal coronary angioplasty or stent implantation, or treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor and cancer metastasis.

(Example 2) Assay of tissue factor mRNA using LPS-dosed mouse

Test compounds were dissolved in a solution comprising a 4:1 mixture of propylene glycol (Wako Pure Chemical Industries) and Tween 80 (Kao) (hereinafter PG/Tween) followed by oral administration by gavage for 7 days at 10 mg/kg once a day in the evening to male C57BL/6J mice (Charles River). LPS was administered intraperitoneally at 4 mg/kg at 9:00 AM on the day following the 7th day of administration of PG/Tween, after which the animals were laparotomized under ether anesthesia 6 hours later to excise the kidneys. RNA was extracted from the kidneys using Trizol reagent (Invitrogen). After carrying out a reverse transcription reaction on the resulting RNA using the First-Strand cDNA Synthesis Kit, the expressed amounts of tissue factor mRNA and cyclophilin mRNA were measured in the same manner as the aforementioned Test Example 1. The expressed amounts of tissue factor mRNA for the test compounds when administered at a concentration of 10 mg/kg based on a value of 100 for the expressed amount of tissue factor mRNA in a case of administration of PG/Tween only are shown in Table 8.

[Table 8]

| Test Compound | Expressed Amount of Tissue Factor mRNA |
|---|---|
| Compound C | 54.1 |
| Compound D | 63.5 |

From the above results, an LXR ligand (and particularly an LXR agonist) was demonstrated to have a superior inhibitory activity for production of tissue factor, and be useful as a medicament for the treatment and/or prophylaxis of vascular restenosis following angioplasty, endarterectomy, percutaneous transluminal coronary angioplasty or stent implantation, or treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor and cancer metastasis.

(Test Example 1) Co-transfection assay [Method for testing LXR binding activity]

An effect of activating or inhibiting LXR transcription activity of a test compound can be measured by a co-transfection which is a cell-based assay. LXR is known to function by forming a heterodimer with RXR. In a co-transfection assay, LXR and RXR expression plasmids and a luciferase reporter expression plasmid containing three copies of an LXR-RXR heterodimer-responding DNA sequence are first inserted into mammalian cells by transient transfection. Next, when the transfected cells are treated with a test compound having LXR agonist activity, the transcription activating effect of LXR is enhanced, and the LXR agonist activity of a test compound can be measured as an increase in luciferase activity. Similarly, the LXR antagonist activity of a test compound can be measured by determining the strength by which a test compound competitively inhibits an activation of transcription by an LXR agonist.

[1] Substances used

(1) CV-1 African green monkey kidney cells (ATCC CCL-70)
(2) Co-transfection expression plasmid, pCDNA-hLXRα or pCDNA-hLXRβ, reporter (LXREx3-pTAL-Luc Vector)
(3) Lipofect AMINE, Plus Reagent (Invitrogen) transfection reagent
(4) Cell lysis buffer [Passive lysis buffer; 5 x (Promega Corporation) is diluted with distilled water]
(5) Luciferase assay reagent (Promega Corporation)
(6) Medium (Dulbecco's Modified Eagle Medium (Gibco) 500 ml, Gentamicin Reagent Solution (Gibco) 2.5 ml, 2 mM L-Gluta Max I Supplement (Gibco) 5.0 ml, MEM Sodium pyruvate solution (Gibco) 5.0 ml, penicillin-streptomycin (Gibco) 5.0 ml, charcoal/dextran-treated FBS (HyClone) 50 ml)
(7) OPTI-MEM I Reduced-Serum Medium (Gibco)

[2] Adjustment of screening reagents
The aforementioned CV-1 cells were disseminated into a 96-well assay plate (Costar 3610) to a concentration of 2 x $10^4$ cells/100 μM/well followed by incubating overnight at 37°C.
DNA transfection was carried out according to the protocol provided with the transfection reagent. 10 μl of OPTI-MEM I Reduced-Serum Medium (Gibco) and 0.5 μl of Lipofect AMINE (Invitrogen) were added to two 50 ml tubes followed by shaking the mixed solutions to obtain Solution A. The substances of (1) below were respectively added to each tube followed by shaking the mixed solutions and allowing to stand undisturbed for 15 minutes to obtain Solution B. In addition, Solution C was obtained by carrying out the same procedure using the substances of (2) below.

(1) 10 μl of OPTI-MEM I Reduced-Serum Medium, 1 μl of Plus Reagent (Invitrogen) and 0.1 μg of DNA [PCMX-LXRα (33 ng) and LXRE (66 ng ) ] ;
(2) 10 μl of OPTI-MEM I Reduced-Serum Medium, 1 μl of Plus Reagent (Invitrogen) and 0.1 μg of DNA [PCMX-LXRβ (33 ng) and LXRE (66 ng)].

The entire amount of the aforementioned Solution A was respectively added to the aforementioned Solution B followed by shaking and allowing to stand undisturbed for 15 minutes to obtain LXRα solution. In addition, LXRβ solution was obtained by carrying out the same procedure using the aforementioned Solutions C and A.
After removing the medium from the 96-well assay plate used to incubate the CV-1 cells as described above by decanting, and completely removing any moisture, 50 μl/well of OPTI-MEM I Reduced-Serum Medium were added to each well followed by the addition of the aforementioned LXRα solution or LXRβ solution to each well at 20 μl/well and incubating for 3 hours at 37°C.
Three hours later, 20% charcoal FBS-DMEM was added to each well at 70 μl/well. FBS-DMEM used for the medium was prepared by mixing charcoal and dextran-treated FBS at a ratio of 9:1. Next, test compounds adjusted to concentrations of 1 mM, 0.3 mM, 0.1 mM, 30 μM, 10 μM, 3 μM, 1 μM or 0 μM with DMSO were added to each well at 1.4 μl/well. The actual concentrations of test compounds in the wells at this time were 1/100 of the concentrations indicated above. The CV-1 cells contained in each well prepared in the manner described above were incubated overnight at 37°C.
[3] Measurement Procedure

The CV-1 cells were observed microscopically following the aforementioned incubation. After removing the medium by decantation and removing sufficiently any moisture, a white seal was affixed to the bottom of each well. Passive lysis buffer (5x) (Promega corporation) diluted 5-fold with distilled water was added to each well at 20 μl/well, and the CV-1 cells were lysed over the course of 15 minutes using a plate shaker. Luciferase assay reagent (Promega Corporation) was added to each well at 100 μl/well followed by measurement of luciferase activity using the Wallac ARVO HTS 1429 Multilabel Counter (registered trademark: Perkin Elmer) or the Analyst HT (registered trademark: BioSystems).
$EC_{50}$ values, which indicate the strength of an activity of test compounds, and efficacy, which represents the % activation ability of test compounds, were able to be determined by LXR/LXRE co-transfection assay. Efficacy is represented with the relative activation ability based on a control compound having LXR agonist activity or a control (DMSO/solvent) not having LXR agonist activity. In this assay, N-(2,2,2-trifluoroethyl)-N-[4-[2,2,2-trifluoro-1-hydroxy-1-(trifluoromethyl)ethyl]phenyl] benzene sulfonamide (Compound 12 described on page 55 of International Patent Publication WO2000/054759; aforementioned Compound A) was used as a control compound having LXR agonist activity.
A concentration-response curve was prepared from the measured values at a total 8 points of dilution series concentrations in (1/2) Log units. The measured value at each concentration was calculated as the mean of the values of 4 wells in the 96-well plate for a single concentration. The data of this assay was fit to the following equations to calculate $EC_{50}$ values.

$$Y = Bottom + (Top-Bottom)/(I+10^{Z})$$

$$Z = (logEC_{50}-X)*HillSlope$$

The $EC_{50}$ value is defined as the concentration at which a test compound provides the intermediate value

between the maximum response (top) and baseline (bottom) (see "Fitting to Sigmoidal dose-response (variable slope)" (Graph Pad PRISM Version 3.02)). The value for relative efficacy or % control based on the control compound as an LXR agonist was determined by a comparison with the maximum response value indicated by Compound A used for the control compound.

In a case of testing with this assay, the compounds of Reference Examples 1 to 37 demonstrate superior binding activity or transcription activating effect on LXRα and LXRβ.

(Reference Example 1)

6-Chloro-7-methoxy-3-{2-methyl-5-[2,2,2-trifluoro-1-hydroxy-1-(trifluoromethyl)ethyl]phenyl}-2-(3-thienylmethyl)-4 (3H)-quinazolinone

The procedures were carried out similarly to the method described in the literature (Example 1 on page 271 of International Patent Publication WO2003/106435) using 5-chloro-4-methoxyanthranylic acid (201 mg, 1.0 mmol) synthesized by a method described in the literature (Reference Example I, ii of US4287341), phenylacetic acid (142 mg, 1.0 mmol), triphenylphosphite (0.29 ml, 1.1 mmol) and 2-(3-amino-4-methylphenyl)-1,1,1,3,3,3-hexafluoro-2-propanol (273 mg, 1.0 mmol) synthesized by a method described in the literature [Example 147 (1) on page 260 of International Patent Publication WO2005/023782] to obtain the title desired compound as a colorless solid (344 mg, yield: 61%).
$^1$H-NMR (500MHz, DMSO-$d_6$) : δ 8.89 (1H, br) , 8.06 (1H, s), 7.78 (1H, s), 7.70 (1H, d, J = 8.0 Hz), 7.42 (1H, d, J = 8.0 Hz), 7.34-7.41 (2H, m), 6.70 (1H, s), 6.59 (1H, d, J = 5.0 Hz), 4.05 (3H, s), 3.81 (1H, d, J = 15.0 Hz), 3.76 (1H, d, J = 15.0 Hz), 1.63 (3H, s).
ESI (ES+) (m/z) : 563 ($[M+H]^+$), ESI (ES-) (m/z) : 561 ( $[M-H]^+$) .

(Reference Example 2)

2-(3-Fluorobenzyl)-6,7-dimethoxy-3-{2-methyl-5-[2,2,2-trifluoro-1-hydroxy-1-(trifluoromethyl)ethyl]phenyl}-4(3H)-quinazolinone

The procedures were carried out similarly to a method described in the literature (Example 1 on page 271 of International Patent Publication WO2003/106435) using 4,5-dimethoxyanthranylic acid (208 mg, 1.05 mmol), phenylacetic acid (165 mg, 1.07 mmol), triphenylphosphite (390mg, 1.26 mmol) and 2-(3-amino-4-methylphenyl)-1,1,1,3,3,3-hexafluoro-2-propanol (290 mg, 1.06 mmol) to obtain the title desired compound as a colorless solid (191 mg, yield: 32%).
$^1$H-NMR (400MHz, DMSO-$d_6$): δ 8.85 (1H, s), 7.68-7.66 (2H, m), 7.41 (1H, s), 7.37 (1H, d, J = 8.6 Hz), 7.23-7.17 (2H, m), 7.02-6.97 (1H, m), 6.64 (1H, d, J = 7.8 Hz), 6.46-6.42 (1H, m), 3.94 (3H, s), 3.86 (3H, s), 3.85-3.71 (2H, m), 1.60 (3H, s).
FABMS (m/z) : 571 ($[M+H]^+$).

(Reference Example 3)

tert-Butyl 2-({4-[acetyl(methyl)amino]phenoxy}methyl)-6-fluoro-1H-indole-1-carboxylate

(3-1)

A solution of 4-fluoro-1-methyl-2-nitrobenzene (10.6 g, 68.3 mmol) in tetrahydrofuran (28 ml) was added to a suspension of sodium hydride (55% oily, 5.96 g, 38.7 mmol) in tetrahydrofuran (28 ml) under ice-cooling, and the mixture was stirred at room temperature for 30 minutes. Diethyl oxalate (74.0 ml, 546 mmol) was added thereto and the mixture was stirred at 40°C for one day. Water was added to the reaction mixture under ice-cooling, and the mixture was extracted with ethyl acetate. The organic layer was washed with saturated aqueous NaCl solution and was dried over anhydrous sodium sulfate. The residue obtained by evaporating the solvent and excess diethyl oxalate under reduced pressure was purified by silica gel column chromatography (elution solvent: n-hexane/ethyl acetate=8/1-6/1) to obtain ethyl 3-(4-fluoro-2-nitrophenyl)-2-oxopropanoate as a yellow oil (5.51 g, yield: 32%).
$^1$H-NMR (400MHz, $CDCl_3$): δ 7.92 (1H, dd, J = 8.3, 2.4 Hz), 7.39-7.31 (2H, m), 4.52 (2H, s), 4.35 (2H, q, J = 7.3 Hz), 1.41 (3H, t, J = 7.3 Hz).

(3-2)

Ethyl 3-(4-fluoro-2-nitrophenyl)-2-oxopropanoate (5.51 g, 21.6 mmol) obtained in Reference Example (3-1) was dissolved in a mixed solvent of ethanol-acetic acid (1:1, 84 ml), and iron powder (10.9 g, 144 mmol) was added

thereto, followed by heating under reflux for 3.5 hours. After the reaction mixture was diluted with tetrahydrofuran, the insolubles were filtered through Celite, and the residue obtained by concentrating the filtrate under reduced pressure was purified by silica gel column chromatography (elution solvent: methylene chloride/acetone=15/1) to obtain ethyl 6-fluoro-1H-indole-2-carboxylate (3.62 g, yield: 81%).
$^1$H-NMR (400MHz, $CDCl_3$): δ 8.89 (1H, br. s), 7.61 (1H, dd, J = 8.8, 5.5 Hz), 7.20 (1H, m), 7.09 (1H, dd, J = 9.4, 2.0 Hz), 6.94 (1H, ddd, J = 9.4, 8.8, 2.0 Hz), 4.41 (2H, q, J = 7.0 Hz), 1.42 (3H, t, J = 7.0 Hz).

(3-3)

Ethyl 6-fluoro-1H-indole-2-carboxylate (1.70 g, 8.19 mmol) obtained in Reference Example (3-2) was dissolved in methylene chloride (82 ml) and triethylamine (4.55 ml, 32.8 mmol), di-tert-butyl dicarboxylate (3.57 g, 16.4 mmol) and N,N-dimethylaminopyridine (100 mg, 0.819 mmol) were added thereto at room temperature, followed by stirring overnight. Water and saturated aqueous NaCl solution were added to the reaction mixture and, after the mixture was extracted with methylene chloride, the organic layer was dried over anhydrous sodium sulfate. The residue obtained by evaporating the solvent under reduced pressure was purified by silica gel column chromatography (elution solvent: n-hexane/ethyl acetate=9/1) to obtain 1-tert-butyl 2-ethyl 6-fluoro-1H-indole-1,2-dicarboxylate as a yellow oil (1.94 g, yield: 95%).
$^1$H-NMR (400MHz, $CDCl_3$) : δ 7.77 (1H, dd, J = 10.2, 2.4 Hz) , 7.51(1H, dd, J = 8.6, 5.8 Hz), 7.05 (1H, s), 7.00 (1H, app. td, J = 9.0, 2.4 Hz), 4.36 (2H, q, J = 7.0 Hz), 1.63 (9H, s), 1.30 (3H, t, J = 7.0 Hz).

(3-4)

1-tert-Butyl 2-ethyl 6-fluoro-1H-indole-1,2-dicarboxylate (19.0 g, 57.9 mmol) obtained in Reference Example (3-3) was dissolved in toluene (290 ml) and a 1M-toluene solution of diisobutyl aluminum hydride (174 ml, 174 mmol) was added thereto at -78˚C, followed by stirring of the mixture while raising the temperature from -78˚C to -20˚C over 2.5 hours. Sodium sulfate decahydrate (143 g) was added to the reaction mixture and, after the mixture was stirred at room temperature for 10 minutes, it was diluted with toluene. Anhydrous magnesium sulfate (50 g) and Celite (30 g) were added thereto, and the mixture was stirred for 10 minutes. The insolubles were filtered through Celite and the residue obtained by concentrating the filtrate under reduced pressure was purified by silica gel column chromatography (elution solvent: n-hexane/ethyl acetate=3/2) to obtain tert-butyl 6-fluoro-2-(hydroxymethyl)-1H-indole-1-carboxylate as a pale yellow oil (9.20 g, yield: 60%).
$^1$H-NMR (400MHz, $CDCl_3$): δ 7.70 (1H, dd, J = 10.6, 2.4 Hz), 7.42 (1H, dd, J = 8.6, 5.9 Hz), 6.99 (1H, ddd, J = 9.4, 8.6, 2.4 Hz), 6.55 (1H, s), 4.79 (2H, d, J = 7.4 Hz), 3.64 (1H, t, J = 7.4 Hz), 1.73 (9H, s).

(3-5)

tert-Butyl 6-fluoro-2-(hydroxymethyl)-1H-indole-1-carboxylate (172 mg, 0.524 mmol) obtained in Reference Example (3-4) and triphenylphosphine (206 mg, 0.786 mmol) were dissolved in tetrahydrofuran (5 ml) and carbon tetrabromide (261 mg, 0.786 mmol) was added thereto, followed by stirring for 20 minutes. After the insolubles were filtered through Celite, the solvent of the filtrate was evaporated under reduced pressure, and the obtained reaction mixture and N-(4-hydroxyphenyl)-N-methylacetamide (87 mg, 0.524 mmol) were dissolved in N,N-dimethylformamide (2 ml). Cesium carbonate (222 mg, 0.681 mol) was added thereto and the mixture was stirred at room temperature overnight. The reaction mixture was diluted with ethyl acetate and the mixture was washed with water and saturated aqueous NaCl solution. The organic layer was dried over anhydrous sodium sulfate, and the residue obtained by evaporating the solvent under reduced pressure was purified by silica gel preparative thin layer chromatography (development solvent: methylene chloride/acetone=3/1) to obtain the title compound as pale brown crystals (85 mg, yield: 39%).
$^1$H-NMR (400MHz, $CDCl_3$): δ 7.88 (1H, dd, J = 11.0, 2.4 Hz), 7.44 (1H, dd, J = 8.8, 5.6 Hz), 7.12 (2H, d, J = 8.8 Hz), 7.00 (1H, m), 7.01 (2H, d, J = 8.8 Hz), 6.70 (1H, s), 5.39 (2H, s), 3.24 (3H, s), 1.86 (3H, s), 1.66 (9H, s).
MS (FAB) (m/z) : 413 ($[M+H]^+$).

(Reference Example 4)

tert-Butyl 2-({4-[(cyclopropylcarbonothioyl)(methyl)amino]phenoxy}methyl)-4,6-difluoro-1H-indole-1-carboxylate

(4-1)

(3,5-Difluorophenyl)hydrazine hydrochloride (1.13 g, 6.28 mmol) was suspended in benzene (14 ml), and triethylamine (0.917 ml, 6.59 mmol) and ethyl pyruvate (0.733 ml, 6.59 mmol) were added thereto under ice-cooling. The mixture was stirred for one hour, further stirred at room temperature for one hour, and thereafter heated under reflux

for 4 hours. Water was added to the reaction mixture and, after the organic layer extracted with ethyl acetate was washed with saturated aqueous NaCl solution, it was dried over anhydrous sodium sulfate, and the solvent was evaporated under reduced pressure. The thus obtained yellow solid was dissolved in toluene (13 ml) and the mixture was added to polyphosphoric acid (7.54 g), followed by heating under reflux overnight. After water was added thereto, the insolubles were filtered through Celite, and the filtrate was extracted with ethyl acetate. The organic layer was washed with saturated aqueous NaCl solution, and dried over anhydrous sodium sulfate. The residue obtained by evaporating the solvent under reduced pressure was purified by silica gel column chromatography (elution solvent: n-hexane/ethyl acetate=6/1) to obtain ethyl 4,6-difluoro-1H-indole-2-carboxylate as a yellow powder (873 mg, yield: 62%).
$^1$H-NMR (400MHz, $CDCl_3$) δ 8. 97 (1H, br. s), 7.26 (1H, s), 6.91 (1H, br. d, J = 8.8 Hz), 6.65 (1H, ddd, J = 10.3, 8.1, 2.2 Hz), 7.18 (1H, t, J = 1.5 Hz), 4.41 (2H, q, J = 7.3 Hz), 1.42 (3H, t, J = 7.3 Hz) .

(4-2)

Ethyl 4,6-difluoro-1H-indole-2-carboxylate (9.25 g, 41.1 mmol) obtained in Reference Example (4-1) was dissolved in ethyl acetate (150 ml) and triethylamine (10.3 mg, 73.9 mmol), di-tert-butyl dicarbonate (9.87 g, 45.2 mmol) and N,N-dimethylaminopyridine (251 mg, 2.05 mmol) were added thereto at room temperature, followed by stirring for 3 hours. Water and saturated aqueous NaCl solution were added to the reaction mixture, and the mixture was extracted with ethyl acetate, and the organic layer was successively washed with O.1N-hydrochloric acid, water and saturated aqueous NaCl solution, and dried over anhydrous sodium sulfate. The residue obtained by evaporating the solvent under reduced pressure was dissolved in toluene (300 ml) and diisobutyl aluminum hydride-1.0 M toluene solution (100 ml, 100 mmol) was added thereto at -78˚C. The mixture was stirred while raising the temperature from -78˚C to 0˚C over 2.5 hours. Sodium sulfate decahydrate (50 g) was added to the reaction mixture and, after the mixture was stirred at room temperature for 20 minutes, it was diluted with toluene. Anhydrous magnesium sulfate (40g) and Celite (40 g) were added thereto, and the mixture was further stirred for 15 minutes. The insolubles were filtered through Celite and the filtrate was concentrated under reduced pressure to obtain a pale yellow solid. Recrystallization was carried out using n-hexane-ethyl acetate to obtain tert-butyl 4,6-difluoro-2-(hydroxymethyl)-1H-indole-1-carboxylate (4.52 g, yield: 39%) as colorless crystals.
$^1$H-NMR (400MHz, $CDCl_3$): δ 7.53 (1H, dd, J = 9.8, 2.4 Hz), 6.74 (1H, td, J = 9.8, 2.4 Hz), 6.65 (1H, s), 4.79 (2H, d, J = 7.4 Hz), 3.49 (1H, t, J = 7.4 Hz), 1.73 (9H, s).

(4-3)

tert-Butyl 4,6-difluoro-2-(hydroxymethyl)-1H-indole-1-carboxylate (590 mg, 2.08 mmol) obtained in Reference Example (4-2) and triphenylphosphine (819 mg, 3.12 mmol) were dissolved in tetrahydrofuran (10 ml,) and carbon tetrabromide (1.04 g, 3.12 mmol) was added thereto, followed by stirring for 30 minutes. After the insolubles were filtered through Celite, the solvent of the filtrate was evaporated under reduced pressure. The thus obtained reaction mixture and allyl 4-hydroxyphenyl(methyl)carbamate (431 mg, 2.08 mmol) were dissolved in N,N-dimethylformamide (10 ml), and cesium carbonate (1.02 g, 3.12 mmol) was added thereto, followed by stirring of the mixture at room temperature overnight. The reaction mixture was diluted with ethyl acetate and the mixture was washed with water and saturated aqueous NaCl solution. The organic layer was dried over anhydrous sodium sulfate, and the residue obtained by evaporating the solvent under reduced pressure was purified by silica gel preparative thin layer chromatography (development solvent: methylene chloride/acetone=9/1-6/1) to obtain tert-butyl 2-[(4-{[(allyloxy)carbonyl](methyl)amino}phenoxy)methyl]-4,6-difluoro-1H-indole-1-carboxylate as a yellow oil (759 mg, yield: 77%).
$^1$H-NMR (400MHz, $CDCl_3$) δ 7.71 (1H, dd, J = 10.2, 2.0 Hz), 7.18 (2H, m), 6.96 (2H, d, J = 8.6 Hz), 6.79 (1H, s), 6.74 (1H, td, J = 9.0, 2.0 Hz), 5.87 (1H, m), 5.34 (2H, s), 5.17 (2H, m), 4.60 (2H, m), 3.29 (3H, s), 1.65 (9H, s).

(4-4)

tert-Butyl 2-[(4-{[(allyloxy)carbonyl](methyl)amino}phenoxy)methyl]-4,6-difluoro-1H-indole-1-carboxylate (729 mg, 1.54 mmol) obtained in Reference Example (4-3) was dissolved in 1,4-dioxane (15 ml) and water (0.75 ml), tetrakis (triphenylphosphine)palladium (0) (18 mg, 15 μmol) and pyrrolidine (154 μl, 1.85 mmol) were added thereto at room temperature, followed by stirring of the mixture for 10 minutes. 1N-Hydrochloric acid was added to the reaction mixture and, after the mixture was extracted with ethyl acetate, the organic layer was washed with saturated aqueous NaCl solution, and dried over anhydrous sodium sulfate. n-Hexane and ethyl acetate were added to the residue obtained by evaporating the solvent under reduced pressure, and the precipitated powder was collected by filtration to obtain tert-butyl 4,6-difluoro-2-{[4-(methylamino)phenoxy]methyl}-1H-indole-1-carboxylate as a yellow solid (439 mg, yield: 73%).
$^1$H-NMR (400MHz, $CDCl_3$) : δ 7.71 (1H, dd, J = 10.2, 2.0 Hz), 6.89 (2H, d, J = 9.0 Hz), 6.77 (1H, s), 6.75-6.70 (4H, m), 5.27 (2H, s), 2.84 (3H, s), 1.65 (9H, s).

(4-5)

tert-Butyl 4,6-difluoro-2-{[4-(methylamino)phenoxy]methyl}-1H-indole-1-carboxylate (50 mg, 0.129 mmol) obtained in Reference Example (4-4) was dissolved in methylene chloride (1.5 ml) and triethylamine (27 μl, 0.193 mmol) and cyclopropanecarbonyl chloride (16 μl, 0.180 mmol) were added thereto at room temperature, followed by stirring for 3 hours. The reaction mixture was purified by silica gel preparative thin layer chromatography (development solvent: n-hexane/ethyl acetate=3/2) to obtain tert-butyl 2-({4-[(cyclopropylcarbonyl)(methyl)amino]phenoxy}methyl)-4,6-difluoro-1H-indole-1-carboxylate as a colorless powder (34 mg, yield: 58%).
$^1$H-NMR (400MHz, $CDCl_3$): δ 7.70 (1H, dd, J = 10.2, 2.0 Hz) , 7.22 (2H, d, J = 8.8 Hz), 7.02 (2H, d, J = 8.8 Hz), 6.80 (1H, s), 6.75 (1H, td, J = 9.4, 2.0 Hz), 5.37 (2H, s), 3.27 (3H, s), 1.66 (9H, s), 1.39 (1H, m), 1.00 (2H, m), 0.61 (2H, m).

(4-6)

tert-Butyl 2-({4-[(cyclopropylcarbonyl)(methyl)amino]phenoxy}methyl)-4,6-difluoro-1H-indole-1-carboxylate (1.00 g, 2.19 mmol) obtained in Reference Example (4-5) was dissolved in tetrahydrofuran (22 ml) and 2,4-bis(4-methoxyphenyl)-1,3-dithia-2,4-diphosphethane-2,4-disulfide (1.33 g, 3.29 mmol) was added thereto, followed by heating under reflux for 4 hours. The residue obtained by concentrating the reaction mixture was purified by silica gel preparative thin layer chromatography (development solvent: n-hexane/ethyl acetate=9/1) to obtain the title compound as a colorless powder (966 mg, yield: 93%).
$^1$H-NMR (400MHz, $CDCl_3$) : δ 7.69 (1H, dd, J = 10.2, 2.0 Hz), 7.19 (2H, d, J = 8.6 Hz), 7.05 (2H, d, J = 8.6 Hz), 6.80 (1H, s), 6.75 (1H, td, J = 9.8, 2.0 Hz), 5.39 (2H, s), 3.75 (3H, s), 1.74 (1H, m), 1.67 (9H, s), 1.32 (2H, m), 0.78 (2H, m).
MS (FAB) (m/z) : 473 ($[M+H]^+$).

(Reference Example 5)

tert-Butyl 6-({4-[(cyclopropylcarbonyl)(methyl)amino]phenoxy}methyl)-2-hydroxy-3-(trifluoromethyl)benzoate

(5-1)

n-Butyl lithium-1.58M tetrahydrofuran solution (65.9 ml, 104 mmol) was added dropwise to a solution of 2-[2-(trifluoromethyl)phenoxy]tetrahydro-2H-pyrane (21.38 g, 86.8 mmol) synthesized according to a method described in the literature (Miller, J. A. et al., J. Org. Chem., 1993, vol. 58, pp 2637-2639) and N,N,N',N'-tetramethylethlenediamine (15.7 ml, 104 mmol) in diethyl ether (230 ml) at -20˚C over 10 minutes.. After the reaction mixture was stirred at -20˚C for 30 minutes, the mixture was further stirred at room temperature for 40 minutes. The reaction mixture was cooled to -30˚C and, after N,N-dimethylformamide (13.5 ml, 174 mmol) was added thereto, the mixture was further stirred at room temperature for 1 hour. The reaction mixture was carefully poured into cooled water and, after the mixture was extracted (three times) with ethyl acetate, the organic layer was successively washed with 1N-hydrochloric acid, 5% aqueous sodium hydrogencarbonate solution, water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=20/1-10/1). The thus obtained 2-(tetrahydro-2H-pyran-2-yloxy)-3-(trifluoromethyl)benzaldehyde as a pale yellow oil was left to stand at room temperature overnight to obtain 2-hydroxy-3-(trifluoromethyl)benzaldehyde as a pale yellow solid (31.73 g, yield: 96%).
$^1$H-NMR (400MHz, $CDCl_3$): δ 11.70 (1H, s), 9.93 (1H, s), 7.80 (1H, d, J = 7.8 Hz), 7.75 (1H, d, J = 7.8 Hz), 7.10 (1H, t, J = 7.8 Hz).

In the following, $^1$H-NMR spectrum of the intermediate, i.e., 2-(tetrahydro-2H-pyran-2-yloxy)-3-(trifluoromethyl) benzaldehyde is shown.
$^1$H-NMR (400MHz, $CDCl_3$) : δ 10.33 (1H, s), 8.02 (1H, dd, J = 7.8, 1.5 Hz), 7.83 (1H, dd, J = 7.8, 1.5 Hz), 7.33 (1H, t, J = 7.8 Hz), 4.80 (1H, dd, J = 7.4, 2.7 Hz), 3.99 (1H, m), 3.43 (1H, m), 2.07 (1H, m), 1.96 (1H, m), 1.86 (1H, m), 1.67-1.50 (3H, m).

(5-2)

Trimethyl ortho-formate (130 ml, 1.19 mol) and camphorsulfonic acid (1.55 g, 6.67 mmol) were added to a solution of 2-hydroxy-3-(trifluoromethyl)benzaldehyde (31.7 g, 167 mmol) obtained in Reference Example (5-1) in methanol (50 ml), and the mixture was stirred at 50˚C for 6 hours. The reaction mixture was poured into 1% aqueous sodium hydrogencarbonate solution and, after the mixture was extracted (three times) with ethyl acetate, the organic layer was successively washed with water (twice) and saturated aqueous NaCl solution, and dried over anhydrous sodium sulfate. The residue obtained by concentrating the organic layer was dissolved in methylene chloride (400 ml), and diisopropylethylamine (50.9 ml, 292 mmol) and chloromethyl methyl ether (15.4 ml, 203 mmol) were added thereto under ice-cooling, followed by stirring of the mixture overnight. The reaction mixture was poured into water and, after the mixture was extracted (twice) with ethyl acetate, the organic layer was successively washed with 0.5N-hydrochloric acid, 5% aqueous sodium hydrogencarbonate solution, water and saturated aqueous NaCl solution, and dried over anhydrous sodium sulfate. The residue obtained by evaporating the solvent under reduced pressure was purified by silica gel column chromatography (elution solvent: n-hexane/ethyl acetate=14/1-10/1) to obtain 1-(dimethoxymethyl)-2-(methoxymethoxy)-3-(trifluoromethyl)benzene as a pale yellow oil (42.2 g, yield: 93%).
$^1$H-NMR (400MHz, $CDCl_3$): δ 7.77 (1H, dd, J = 7.8, 1.6 Hz), 7.59 (1H, dd, J = 7.8, 1.6 Hz), 7.24 (1H, t, J = 7.8 Hz), 5.67 (1H, s), 5.07 (2H, s), 3.65 (3H, s), 3.38 (6H, s).

(5-3)

n-Butyl lithium-1.59M tetrahydrofuran solution (196 ml, 312 mmol) was added dropwise to a solution of 1-(dimethoxymethyl)-2-(methoxymethoxy)-3-(trifluoromethyl)benzene (39.3 g, 140 mmol) obtained in Reference Example (5-2) and N,N,N',N'-tetramethylethylenediamine (46.9 ml, 311 mmol) in diethyl ether (410 ml) at -25˚C over 20, minutes. After the reaction mixture was stirred at 0˚C for 30 minutes, the mixture was further stirred at room temperature for 1.5 hours. The reaction mixture was cooled to -30˚C and, after N,N-dimethylformamide (41.9 ml, 541 mmol) was added thereto, the mixture was further stirred at room temperature for 1 hour. The reaction mixture was carefully poured into cold 0.1N-hydrochloric acid and, after the mixture was extracted (four times) with ethyl acetate, the organic layer was successively washed with 0.1N-hydrochloric acid, water (three times) and saturated aqueous NaCl solution, and dried over anhydrous sodium sulfate. The solvent was evaporated under reduced pressure to obtain crude 2-(dimethoxymethyl)-3-(methoxymethoxy)-4-(trifluoromethyl)benzaldehyde. The present compound was used in Reference Example (5-4) without further purification.
$^1$H-NMR (400MHz, $CDCl_3$) : δ 10.71 (1H, s), 7.81 (1H, d, J = 8.2 Hz), 7.70 (1H, d, J = 8.2 Hz), 5.79 (1H, s), 5.07 (2H, s), 3.67 (3H, s), 3.50 (6H, s).
MS (FAB) (+0.1N KIaq. ) (m/z): 347 ($[M+K]^+$).

(5-4)

Sodium borohydride (5.11 g, 135 mmol) was added to a mixed solution of the crude 2-(dimethoxymethyl)-3-(methoxymethoxy)-4-(trifluoromethyl)benzaldehyde obtained in Reference Example (5-3) in tetrahydrofuran-methanol (5:1, 100 ml) under ice-cooling, and the mixture was stirred overnight. The reaction mixture was poured into water and, after the mixture was extracted (four times) with ethyl acetate, the organic layer was successively washed with water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=5/1-2/1) to obtain [2-(dimethoxymethyl)-3-(methoxymethoxy)-4-(trifluoromethyl)phenyl]methanol as an orange-colored oil (22.6 g, yield: 52%).
$^1$H-NMR (400MHz, $CDCl_3$) δ 7.59 (1H, d, J = 8.2 Hz), 7.31 (1H, d, J = 8.2 Hz), 5.81 (1H, s), 5.01 (2H, s), 4.85 (2H, d, J = 7.0 Hz), 3.65 (3H, s), 3.50 (6H, s), 3.36 (1H, t, J = 7.0 Hz) .
MS (FAB) (m/z): 309 ($[M-H]^+$).

(5-5)

Allyl chloroformate (6.90 ml, 65.3 mmol) was added to a solution of 4-methylaminophenol sulfate (5.11 g, 29.7 mmol) and triethylamine (12.3 ml, 89.1 mmol) in methylene chloride (100 ml), and the mixture was stirred at room temperature for 2 hours. Saturated aqueous sodium hydrogencarbonate solution was poured into the reaction mixture and, after the mixture was extracted with ethyl acetate, the organic layer was successively washed with 1N-hydrochloric acid and saturated aqueous NaCl solution, and dried over anhydrous sodium sulfate. The solvent was evaporated under reduced pressure and, after the thus obtained residue was dissolved in methanol (50 ml), potassium carbonate (5.00 g, 36.2 mmol) was added thereto, and the mixture was stirred at room temperature for 5 hours. Water was poured into the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=3/1-1/1) to obtain allyl 4-hydroxyphenyl(methyl)carbamate (4.58 g, yield: 74%).
$^1$H-NMR (400MHz, $CDCl_3$) δ 7.04 (2H, d, J = 8.6 Hz), 6.73 (2H, br. s), 5.90 (1H, br. s), 5.18 (2H, br. s), 4.61 (2H, br. s), 3.26 (3H, s).

(5-6)

Diethyl azodicarboxylate (1.32 ml, 5.50 mmol) was added to a solution of [2-(dimethoxymethyl)-3-(methoxymethoxy)-4-(trifluoromethyl)phenyl]methanol (1.42 g, 4.58 mmol) obtained in Reference Example (5-4), allyl 4-hydroxyphenyl (methyl)carbamate (1.04 g, 5.04 mmol) obtained in Reference Example (5-5) and triphenylphosphine (1.44 g, 5.50 mmol) in tetrahydrofuran (20 ml), and the mixture was stirred at room temperature for 2 hours. Water was added to the reaction mixture and, after the mixture was extracted with ethyl acetate, the organic layer was successively washed with 1N aqueous sodium hydroxide solution, water and saturated aqueous NaCl solution, and dried over anhydrous sodium sulfate. The residue obtained by evaporating the solvent under reduced pressure was purified by silica gel column chromatography (elution solvent: n-hexane/ethyl acetate=10/1-1/1) to obtain allyl 4-{[2-(dimethoxymethyl)-3-(methoxymethoxy)-4-(trifluoromethyl)benzyl]oxy}phenyl(methyl)carbamate (1.45 g, yield: 63%).
$^1$H-NMR (400MHz, $CDCl_3$) : δ 7.63-7.53 (2H, m), 7.12 (2H, d, J = 8.8 Hz), 6.99-6.94 (2H, d, J = 8.8 Hz), 5.88 (1H, br. s), 5.75 (1H, s), 5.48 (2H, s), 5.18 (2H, br. s), 5.03 (2H, br. s), 4.59 (2H, br. s), 3.66 (3H, s), 3.47 (6H, s), 3.26 (3H, s).

(5-7)

Pyrrolidine (1.90 ml, 22.8 mmol) and tetrakis(triphenylphosphine)palladium (0) (439 mg, 0.38 mmol) were added to a mixed solution of allyl 4-{ [2-(dimethoxymethyl)-3-(methoxymethoxy)-4-(trifluoromethyl)benzyl]oxy}phenyl (methyl)carbamate (3.86 g, 7.61 mmol) obtained in Reference Example (5-6) in 1,4-dioxane (80 ml) and water (4 ml), and the mixture was stirred at room temperature for 3 hours. Water was poured into the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=4/1-1/1) to obtain N-(4-{[2-(dimethoxymethyl)-3-(methoxymethoxy)-4-(trifluoromethyl)benzyl]oxy}phenyl)-N-methylamine (3.01 g, yield: 95%).
$^1$H-NMR (400MHz, $CDCl_3$) : δ 7.59-7.54 (2H, m), 6.85 (2H, d, J = 9.0 Hz), 6.56 (2H, d, J = 9.0 Hz), 5.73 (1H, s), 5.41 (2H, s), 5.03 (2H, s), 3.66 (3H, s), 3.45 (6H, s), 2.79 (3H, s).

(5-8)

Triethylamine (0.420 ml, 3.01 mmol) and cyclopropanecarbonyl chloride (0.162 ml, 1.80 mmol) were successively added to a solution of N-(4-{[2-(dimethoxymethyl)-3-(methoxymethoxy)-4-(trifluoromethyl)benzyl]oxy}phenyl)-N-methylamine (500 mg, 1.20 mmol) obtained in Reference Example (5-7) in methylene chloride (8 ml) under ice-cooling, and the mixture was stirred at room temperature overnight. The reaction mixture was poured into 5% aqueous sodium hydrogencarbonate solution and, after the mixture was extracted (twice) with ethyl acetate, the organic layer was successively washed with water and saturated aqueous NaCl solution, and dried over anhydrous sodium sulfate. The solvent was evaporated under reduced pressure to obtain the residue. 4N-Hydrochloric acid (2.5 ml) was added to a solution of the thus obtained residue in tetrahydrofuran (13 ml), and the mixture was stirred at 50˚C for 5 hours. The reaction mixture was poured into water and, after the mixture was extracted (twice) with ethyl acetate, the organic layer was successively washed with water and saturated aqueous NaCl solution and dried over anhydrous sodium sulfate. The solvent was evaporated under reduced pressure to obtain the residue. A solution of sodium chlorite (650 mg, 7.19 mmol) and sodium dihydrogenphosphate monohydrate (650 mg, 4.71 mmol) in water (5.0 ml) was added dropwise to a mixed solution of the obtained residue in tert-butyl alcohol (12.5 ml), 1,4-dioxane (3.0 ml) and 2-methyl-2-butene (3.5 ml), and the mixture was stirred at room temperature for 30 minutes. After 5% aqueous sodium thiosulfate solution was added to the reaction mixture, the mixture was poured into 0.5N-hydrochloric acid, and extracted (twice) with ethyl acetate. The organic layer was successively washed with water and saturated aqueous NaCl solution and dried over anhydrous sodium sulfate. The solvent was evaporated under reduced pressure to obtain the residue. After the obtained residue was dissolved in toluene (9.0 ml) and 1,4-dioxane (6.0 ml), N,N-dimethylformamide di-tert-butyl acetal (1.15 ml, 4.80 mmol) was added thereto, and the mixture was heated under reflux for 1.5 hours. The reaction mixture was poured into O.1N-hydrochloric acid and, after the mixture was extracted (three times) with ethyl acetate, the organic layer was successively washed with water (two times) and saturated aqueous NaCl solution, and dried over anhydrous sodium sulfate. The residue obtained by evaporating the solvent under reduced pressure was purified by silica gel thin layer chromatography (development solvent: n-hexane/ethyl acetate=2/1) to obtain the title compound as a colorless powder (245 mg, yield: 44%).
$^1$H-NMR (400MHz, $CDCl_3$): δ 12.24 (1H, s), 7.71 (1H, d, J = 8.2 Hz), 7.26 (1H, d, J = 8.2 Hz), 7.22 (2H, d, J = 8.6 Hz), 6.95 (2H, d, J = 8.6 Hz), 5.35 (2H, s), 3.26 (3H, s), 1.65 (9H, s), 1.38 (1H, m), 1.00 (2H, m), 0.61 (2H, m). HRMS (FAB) (m/z) : calcd. for $C_{24}H_{27}O_5NF_3$ ( [M+H] $^+$) : 466.1841; found: 466.1839.

(Reference Example 6)

tert-Butyl 2-hydroxy-6-({4-[methyl(methylsulfonyl)amino]phenoxy}methyl)-3-(trifluoromethyl)benzoate

(6-1)

4N-Hydrochloric acid (5 ml) was added to a solution of allyl 4-{[2-(dimethoxymethyl)-3-(methoxymethoxy)-4-(trifluoromethyl)benzyl]oxy}phenyl(methyl)carbamate (4.00 g, 8.01 mmol) obtained in Reference Example (5-6) in tetrahydrofuran (20 ml), and the mixture was stirred at 55˚C for 4 hours. Water was added to the reaction mixture and, after the mixture was extracted with ethyl acetate, the organic layer was washed with saturated aqueous NaCl solution and dried over anhydrous sodium sulfate. Allyl bromide (831 μl, 9.61 mmol) and potassium carbonate (1.33 g, 9.61 mmol) were added to a solution of the residue obtained by evaporating the solvent under reduced pressure in N,N-dimethylformamide (10 ml), and the mixture was stirred at 50˚C for 3 hours. Water was poured into the reaction mixture and, after the mixture was extracted with ethyl acetate, the organic layer was successively washed with water and saturated aqueous NaCl solution, and dried over anhydrous sodium sulfate. 2-Methyl-2-butene (20 ml) was added to a mixed solution of the residue obtained by evaporating the solvent under reduced pressure in 1,4-dioxane (20 ml) and tert-butanol (75 ml). A solution of sodium chlorite (4.33 g, 48.1 mmol) and sodium dihydrogenphosphate monohydrate (4.33 g, 31.4 mmol) in water (30 ml) was added dropwise to the reaction mixture, and the mixture was stirred at room temperature for 2 hours. 10% Aqueous sodium thiosulfate solution was poured into the reaction mixture and, after the mixture was stirred, it was acidified with 1N-hydrochloric acid. After the mixture was further extracted with ethyl acetate, the organic layer was successively washed with water and saturated aqueous NaCl solution, and dried over anhydrous sodium sulfate. N,N-Dimethylformamide di-tert-butyl acetal (7.67 ml, 32.0 mmol) was added to a solution of the residue obtained by evaporating the solvent under reduced pressure in toluene (50 ml), and the mixture was stirred at 110˚C for 6 hours. Water was added to the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=4/1-7/3) to obtain tert-butyl 2-(allyloxy)-6-[(4-{[(allyloxy)carbonyl](methyl)amino}phenoxy)methyl]-3-(trifluoromethyl)benzoate (2.80 g, yield: 67%).
$^1$H-NMR (400MHz, $CDCl_3$) : δ 7.64 (1H, d, J = 7.8 Hz), 7.37 (1H, d, J = 7.8 Hz), 7.16 (2H, d, J = 9.0 Hz), 6.90 (2H, d, J = 9.0 Hz), 6.11-6.02 (1H, m), 5.89 (1H, br. s), 5.42 (1H, m), 5.27 (1H, m), 5.17 (2H, br. s), 5.11 (2H, br. s), 4.65-4.55 (4H, m), 3.27 (3H, s), 1.57 (9H, s).

(6-2)

Pyrrolidine (1.34 ml, 16.1 mmol) and tetrakis(triphenylphosphine)palladium (0) (186 mg, 0.16 mmol) were added to a mixed solution of tert-butyl 2-(allyloxy)-6-[(4-{[(allyloxy)carbonyl](methyl)amino}phenoxy)methyl]-3-(trifluoromethyl)benzoate (2.80 g, 5.37 mmol) obtained in Reference Example (6-1) in 1,4-dioxane (20 ml) and water (1.0 ml), and the mixture was stirred at room temperature for 3 hours. Water was poured into the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=5/1-2/1) to obtain tert-butyl 2-hydroxy-6-{[4-(methylamino)phenoxy]methyl}-3-(trifluoromethyl)benzoate (1.64 g, yield: 77%).
$^1$H-NMR (500MHz, $CDCl_3$): δ 12.23 (1H, br. s), 7.67 (1H, d, J = 8.3 Hz), 7.28 (1H, d, J = 8.3 Hz), 6.79 (2H, d, J = 8.8 Hz), 6.58 (2H, d, J = 8.8 Hz), 5.26 (2H, s), 2.80 (3H, s) , 1.62 (9H, s).

(6-3)

Triethylamine (3.44 ml, 24.8 mmol) was added to a solution of tert-butyl 2-hydroxy-6-{[4-(methylamino)phenoxy]methyl}-3-(trifluoromethyl)benzoate (2.46 g, 6.20 mmol) obtained in Reference Example (6-2) in N,N-dimethylformamide (20 ml), and tert-butylchlorodimethylsilane (2.79 g, 18.6 mmol) was added thereto, followed by stirring of the mixture at room temperature for 12 hours. Water was poured into the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=4/1-1/1) to obtain tert-butyl 2-{[tert-butyl(dimethyl)silyl]oxy}-6-{[4-(methylamino)phenoxy]methyl}-3-(trifluoromethyl)benzoate (2.38 g, yield: 97%).
$^1$H-NMR (400MHz, $CDCl_3$) δ 7.56 (1H, d, J = 8.2 Hz), 7.27 (1H, d, J = 8.2 Hz), 6.79 (2H, d, J = 9.0 Hz), 6.55 (2H, d, J = 9.0 Hz), 5.06 (2H, s), 2.79 (3H, s), 1.58 (9H, s), 1.01 (9H, s), 0.20 (6H, s).

(6-4)

Triethylamine (53 μl, 0.50 mmol) was added to a solution of tert-butyl 2-{[tert-butyl(dimethyl)silyl]oxy}-6-{[4-(methylamino)phenoxy]methyl}-3-(trifluoromethyl)benzoate (98.0 mg, 0.192 mmol) obtained in Reference Example (6-3) in methylene chloride (4 ml) and methanesulfonyl chloride (22 μl, 0.29 mmol) was added thereto, followed by stirring of the mixture at room temperature overnight. Further, a small amount of methanol and tetra-n-butyl ammonium fluoride-1.0M tetrahydrofuran solution (1.9 ml, 1.9 mmol) were added to the reaction mixture, and the mixture was stirred at room temperature for 6 hours. Water was poured into the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel thin layer chromatography (development solvent: n-hexane/ethyl acetate=1/1) to obtain the title compound as a colorless amorphous solid (85.7 mg, yield: 94%).
$^1$H-NMR (400MHz, $CDCl_3$) : δ 12.25 (1H, br. s), 7.68 (1H, d, J = 8.2 Hz), 7.31 (2H, d, J = 9.0 Hz), 7.24 (1H, d, J = 8.2 Hz), 6.91 (2H, d, J = 9.0 Hz), 5.34 (2H, s), 3.29 (3H, s), 2.84 (3H, s), 1.66 (9H, s).
MS (FAB) (m/z): 475 ($[M]^+$).

(Reference Example 7)

tert-Butyl 6-({4-[acetyl(methyl)amino]phenoxy}methyl)-3-ethyl-2-hydroxybenzoate

(7-1)

tert-Butyl{[4-iodo-3-(methoxymethoxy)benzyl]oxy}dimethylsilane was obtained by a reaction described in the literature (Corey, E. J., et al., J. Am. Chem. Soc., 1972, vol. 94, pp. 6190-6191) using [4-iodo-3-(methoxymethoxy)phenyl]methanol synthesized by a literature method (Winkle, M. R., et al., J. Org. Chem., 1982, vol. 47, p. 2101-2108).

Diisopropylamine (10 ml), cuprous iodide (I) (200 mg, 1.1 mmol), dichlorobis(triphenylphosphine)palladium (II) (720 mg, 1.03 mmol) and trimethylsilyl acetylene (10.8 ml, 76.4 mmol) were successively added to a solution of the obtained tert-butyl{[4-iodo-3-(methoxymethoxy)benzyl]oxy}dimethylsilane (20.8 g, 50.9 mmol) in N,N-dimethylformamide (50 ml), and the mixture was heated under reflux for 1.5 hours. The temperature of the reaction mixture was returned to room temperature, and the mixture was poured into water, followed by extraction of the mixture with ethyl acetate. The organic layer was successively washed with water and saturated aqueous NaCl solution, and dried over anhydrous sodium sulfate. The solvent was evaporated under reduced pressure to obtain the residue. Potassium carbonate (7.03 g, 50.9 mmol) was added to a solution of the obtained residue in methanol (1.0 ml), and the mixture was stirred at room temperature for 1.5 hours. The insolubles were separated by filtration through Celite, and the filtrate was concentrated under reduced pressure. The obtained residue was subjected to silica gel column chromatography (elution solvent: n-hexane/ethyl acetate=70/1-30/1) to obtain tert-butyl{[4-ethynyl-3-(methoxymethoxy)benzyl]oxy}dimethylsilane (10.9 g).

5% Rhodium-alumina (700 mg) was added to a solution of the obtained tert-butyl{[4-ethynyl-3-(methoxymethoxy)benzyl]oxy}dimethylsilane in methanol (250 ml), and the mixture was stirred at room temperature under a hydrogen atmosphere for 8 hours. The catalyst was removed by filtration through Celite and the filtrate was concentrated under reduced pressure. The thus obtained residue was purified by silica gel chromatography (elution solvent: n-hexane/ethyl acetate=40/0-40/1) to obtain tert-butyl{[4-ethyl-3-(methoxymethoxy)benzyl]oxy}dimethylsilane (9.30 g).
$^1$H-NMR (400MHz, $CDCl_3$): δ 7.11 (1H, d, J = 7.8 Hz), 7.05 (1H, s), 6.90 (1H, d, J = 7.8 Hz), 5.19 (2H, s), 4.70 (2H, s), 3.48 (3H, s), 2.64 (2H, q, J = 7.8 Hz), 1.19 (3H, t, J = 7.8 Hz), 0.94 (9H, s), 0.10 (6H, s).

(7-2)

n-Butyl lithium-1.58M hexane solution (2.7 ml, 4.3 mmol) was added to a solution of tert-butyl{[4-ethyl-3-(methoxymethoxy)benzyl]oxy}dimethylsilane (1.0 g, 3.3 mmol) obtained in Reference Example (7-1) and N,N,N',N'-tetramethylethylenediamine (0.50 ml, 4.2 mmol) in diethyl ether (10 ml) at -78˚C, and the mixture was stirred under ice-cooling for 1 hour. Further, after the mixture was cooled to -40˚C, di-tert-butyl dicarbonate (930 mg, 4.2 mmol) was added thereto, and the temperature of the mixture was gradually raised to room temperature. Water was poured into the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=100/1) to obtain tert-butyl 6-({[tert-butyl(dimethyl)silyl]oxy}methyl)-3-ethyl-2-(methoxymethoxy)benzoate (443 mg, 33%).
$^1$H-NMR (400MHz, $CDCl_3$) : δ 7.26 (1H, d, J = 8.2 Hz), 7.24 (1H, d, J = 8.2 Hz), 5.01 (2H, s), 4.73 (2H, s), 3.57 (3H, s),

2.71 (2H, q, J = 7.8 Hz), 1.59 (9H, s), 1.22 (3H, t, J = 7.8 Hz), 0.93 (9H, s), 0.07 (6H, s).

(7-3)

Acetic acid (63 μl, 1.1 mmol) and tetra-n-butylammonium fluoride-1.0M tetrahydrofuran solution (1.1 ml, 1.1 mmol) were added to a solution of tert-butyl 6-({[tert-butyl(dimethyl)silyl]oxy}methyl)-3-ethyl-2-(methoxymethoxy)benzoate obtained in Reference Example (7-2) (443 mg, 1.08 mmol) in tetrahydrofuran (10 ml), and the mixture was stirred at room temperature for 3 hours. Water was poured into the reaction mixture and, after the mixture was extracted with ethyl acetate, the organic layer was successively washed with water and saturated aqueous NaCl solution, and dried over anhydrous sodium sulfate. The solvent was evaporated under reduced pressure to obtain tert-butyl 3-ethyl-6-(hydroxymethyl)-2-(methoxymethoxy)benzoate (320 mg, 99%).
$^1$H-NMR (500MHz, $CDCl_3$) : δ 7.26 (1H, d, J = 7.8 Hz), 7.14 (1H, d, J = 7.8 Hz), 5.02 (2H, s), 4.56 (2H, s), 3.58 (3H, s), 2.74 (2H, q, J = 7.8 Hz), 1.62 (9H, s), 1.23 (3H, t, J = 7.8 Hz) .

(7-4)

N,N,N',N'-Tetra-methylazodicarboxamide (220 mg, 1.27 mmol) and tri-n-butylphosphine (320 mg, 1.28 mmol) were added to a solution of tert-butyl 3-ethyl-6-(hydroxymethyl)-2-(methoxymethoxy)benzoate (320 mg, 1.08 mmol) obtained in Reference Example (7-3) and N-(4-hydroxyphenyl)-N-methylacetamide (165 mg, 1.00 mmol) synthesized according to the literature (Harvison, P. J. et al., J. Med. Chem., 1986, vol. 29, pp. 1737-1743) in tetrahydrofuran (10 ml), and the mixture was stirred at room temperature overnight. Water was poured into the reaction mixture and, after the mixture was extracted with ethyl acetate, the organic layer was successively washed with water and saturated aqueous NaCl solution, and dried over anhydrous sodium sulfate. The solvent was evaporated under reduced pressure, and the residue was purified by silica gel thin layer chromatography (development solvent: n-hexane/ethyl acetate=2/1) to obtain tert-butyl 6-({4-[acetyl(methyl)amino]phenoxy}methyl)-3-ethyl-2-(methoxymethoxy)benzoate (205 mg, 43%).
$^1$H-NMR (400MHz, $CDCl_3$) : δ 7.27 (1H, d, J = 7.8 Hz), 7.20 (1H, d, J = 7.8 Hz), 7.08 (2H, d, J = 8.6 Hz), 6.95 (2H, d, J = 8.6 Hz), 5.07 (2H, s), 5.05 (2H, s), 3.60 (3H, s), 3.22 (3H, s), 2.75 (2H, q, J = 7.8 Hz), 1.84 (3H, s), 1.54 (9H, s), 1.24 (3H, t, J = 7.8 Hz).

(7-5)

Trimethylchlorosilane (63 μl, 0.50 mmol) and tetra-n-butylammonium bromide (160 mg, 0.50 mmol) were added to a solution of tert-butyl 6-({4-[acetyl (methyl) amino]phenoxy}methyl)-3-ethyl-2-(methoxymethoxy)benzoate (147 mg, 0.331 mmol) obtained in Reference Example (7-4) in methylene chloride (3 ml), and the mixture was heated under reflux for 5 hours. Water was poured into the reaction mixture and, after the mixture was extracted with ethyl acetate, the organic layer was successively washed with water and saturated aqueous NaCl solution, and dried over anhydrous sodium sulfate. The solvent was evaporated under reduced pressure, and the residue was purified by silica gel thin layer chromatography (development solvent: n-hexane/ethyl acetate=2/1) to obtain the title compound as a colorless powder (107 mg, 81%).
$^1$H-NMR (400MHz, $CDCl_3$) δ 11.70 (1H, s), 7.29 (1H, d, J = 7.8 Hz), 7.10 (2H, d, J = 8.8 Hz), 7.02 (1H, d, J = 7.8 Hz), 6.94 (2H, d, J = 8.8 Hz), 5.28 (2H, S), 3.23 (3H, s), 2.69 (2H, q, J = 7.4 Hz), 1.85 (3H, s), 1.57 (9H, s), 1.23 (3H, t, J = 7.4 Hz).
MS (FAB) (m/z) : 400 ( $[M+H]^+$) .

(Reference Example 8)

tert-Butyl 6-({4-[(cyclopropylacetyl)(methyl)amino]phenoxy}methyl)-2-hydroxy-3-(trifluoromethyl)benzoate

N,N-Dimethylaminopyridine (20 mg) and 1-ethyl-3-(3-dimethylaminopropyl)carbodiimide hydrochloride (362 mg, 1.90 mmol) were added to a solution of tert-butyl 2-{ [tert-butyl(dimethyl)silyl]oxy}-6-{[4-(methylamino)phenoxy] methyl}-3-(trifluoromethyl)benzoate (486 mg, 0.950 mmol) obtained in Reference Example (6-3) and cyclopropylacetic acid (142 mg, 1.42 mmol) in methylene chloride (4 ml), and the mixture was stirred at room temperature overnight. Further, a small amount of water and tetra-n-butylammonium fluoride-1.0M tetrahydrofuran solution (9.5 ml, 9.5 mmol) were added to the reaction solution, and the mixture was stirred at room temperature for 6 hours. Water was poured into the reaction mixture and, after the mixture was extracted with ethyl acetate, the organic layer was successively washed with water and saturated aqueous NaCl solution, and dried over anhydrous sodium sulfate. The solvent was evaporated under reduced pressure, and the obtained residue was purified by silica gel thin layer chromatography (development solvent: n-hexane/ethyl acetate=2/1) to obtain the title compound as a colorless oil (299 mg, yield: 66%).
$^1$H-NMR (400MHz, $CDCl_3$) δ 12.19 (1H, s), 7.69 (1H, d, J = 8.2 Hz), 7.23 (1H, d, J = 8.2 Hz), 7.08 (2H, d, J = 8.8 Hz),

6.91 (2H, d, J = 8.8 Hz) , 5.32 (2H, s), 3.24 (3H, s), 1.99 (2H, d, J = 7.0 Hz), 1.63 (9H, s), 1.06-0.95 (1H, m), 0.51-0.41 (2H, m), 0.00 - -0.05 (2H, m).
MS (FAB) (m/z) : 480 ($[M+H]^+$).

(Reference Example 9)

(4'-{[1-(tert-Butoxycarbonyl)-6-fluoro-1H-indol-2-yl]methoxy}-1,1'-biphenyl-4-yl)acetic acid

(9-1)

Concentrated sulfuric acid (30 ml) was added to a solution of (4-bromophenyl)acetic acid (101 g, 468 mmol) in methanol (1000 ml) under ice-cooling, and the mixture was stirred at room temperature for 2 hours. The reaction mixture was concentrated and, after ethyl acetate was added to the residue, the mixture was successively washed with water, saturated aqueous sodium hydrogencarbonate solution and saturated aqueous NaCl solution, and dried over anhydrous sodium sulfate. The residue obtained by evaporating the solvent under reduced pressure was purified by silica gel column chromatography (elution solvent: n-hexane/ethyl acetate=5/1) to obtain methyl (4-bromophenyl)acetate (107 g, yield: 100%).
$^1$H-NMR (400MHz, $CDCl_3$) : δ 7.43 (2H, d, J = 8.6 Hz), 7.14 (2H, d, J = 8.6 Hz), 3.69 (3H, s), 3.57 (2H, s).

(9-2)

An aqueous 1M-sodium carbonate solution (558 ml) and tetrakis(triphenylphosphine)palladium (0) (2.34 g, 2.03 mmol) were added to a mixed solution of methyl (4-bromophenyl)acetate (116 g, 506 mmol) obtained in Reference Example (9-1) and 4-methoxyphenylboric acid (77.0 g, 507 mmol) in toluene-ethanol (6:1, 1167 ml), and the mixture was heated under reflux with stirring for 8 hours. After the reaction mixture was returned to room temperature, it was poured into water, and the mixture was extracted three times with ethyl acetate. The organic layer was successively washed with water (twice) and saturated aqueous NaCl solution, and dried over anhydrous sodium sulfate. The solvent was evaporated under reduced pressure to obtain methyl (4'-methoxy-1,1'-biphenyl-4-yl) acetate as a pale yellow powder (125 g, yield: 96%).
$^1$H-NMR (400MHz, $CDCl_3$): δ 7.52 (4H, d, J = 8.4 Hz), 7.33 (2H, d, J = 8.4 Hz), 6.97 (2H, d, J = 8.4 Hz), 3.85 (3H, s) , 3.71 (3H, s) , 3.66 (2H, s).

(9-3)

Acetic acid (20 ml) and hydrobromic acid (47%) (20 ml) were added to methyl (4'-methoxy-1,1'-biphenyl-4-yl) acetate (2.71 g, 488 mmol) obtained in Reference Example (9-2), and the mixture was heated under reflux for 8 hours. After the temperature of the reaction mixture was returned to room temperature, the mixture was poured into ice-water and stirred for 10 minutes. The produced precipitate was collected by filtration and, after it was washed with water, it was dried under reduced pressure to obtain (4'-hydroxy-1,1'-biphenyl-4-yl)acetic acid as a pale yellow powder (1.98 g, yield: 82%).
$^1$H-NMR (400MHz, DMSO-$d_6$): δ 9.60 (1H, br s), 8.24 (1H, s), 7.49 (2H, d, J = 8.6 Hz), 7.45 (2H, d, J = 8.6 Hz), 7.27 (2H, d, J = 8.6 Hz) , 6.83 (2H, d, J = 8.6 Hz). (2H peak was not detected due to overlapping with solvent peak.)
MS (EI) (m/z) : 228 ( $[M]^+$) .

(9-4)

Methanol (240 ml) and concentrated sulfuric acid (2.4 ml) were added to (4'-hydroxy-1,1'-biphenyl-4-yl)acetic acid (40.0 g, 175 mmol) obtained in Reference Example (9-3), and the mixture was heated under reflux for 48 hours. The temperature of the reaction mixture was returned to room temperature and, after the insolubles were removed by filtration, the filtrate was concentrated under reduced pressure to obtain a pale brown solid. The thus obtained solid was recrystallized from n-hexane-ethyl acetate to obtain methyl (4'-hydroxy-1,1'-biphenyl-4-yl)acetate as pale yellow crystals (32.3 g, yield: 76%).
$^1$H-NMR (400MHz, $CDCl_3$) δ 7.45 (2H, d, J = 8.2 Hz), 7.40 (2H, d, J = 8.6 Hz), 7.29 (2H, d, J = 8.2 Hz), 6.83 (2H, d, J = 8.6 Hz), 5.25 (1H, s), 3.71 (3H, s), 3.66 (2H, s).

(9-5)

A solution of 4-fluoro-1-methyl-2-nitrobenzene (10.6 g, 68.3 mmol) in tetrahydrofuran (28 ml) was added to a

suspension of sodium hydride (55% oily, 5.96 g, 38.7 mmol) in tetrahydrofuran (28 ml) under ice-cooling, and the mixture was stirred at room temperature for 30 minutes. Diethyl oxalate (74.0 ml, 546 mmol) was added thereto and the mixture was stirred at 40˚C for 24 hours. Water was added to the reaction mixture under ice-cooling and, after the mixture was extracted with ethyl acetate, the organic layer was washed with saturated aqueous NaCl solution and dried over anhydrous sodium sulfate. The solvent and diethyl oxalate were evaporated under reduced pressure. The thus obtained residue was purified by silica gel column chromatography (elution solvent: n-hexane/ethyl acetate=8/1-6/1) to obtain ethyl 3-(4-fluoro-2-nitrophenyl)-2-oxopropanoate as a yellow oil (5.51 g, yield: 32%).
$^1$H-NMR (400MHz, $CDCl_3$) δ 7.92 (1H, dd, J = 8.3, 2.4 Hz) 7.39-7.31 (2H, m), 4.52 (2H, s), 4.35 (2H, q, J = 7.3 Hz), 1.41 (3H, t, J = 7.3 Hz).

(9-6)

Ethyl 3-(4-fluoro-2-nitrophenyl)-2-oxopropanoate (5.51 g, 21.6 mmol) obtained in Reference Example (9-5) was dissolved in a mixed solvent of ethanol-acetic acid (1:1, 84 ml), and iron powder (10.9 g, 144 mmol) was added thereto, followed by heating under reflux of the mixture for 3.5 hours. The temperature of the reaction mixture was returned to room temperature, and the mixture was diluted with tetrahydrofuran. The insolubles were removed by filtration using Celite, and the filtrate was concentrated under reduced pressure. The thus obtained residue was purified by silica gel column chromatography (elution solvent: methylene chloride/acetone=15/1) to obtain ethyl 6-fluoro-1H-indole-2-carboxylate as yellow crystals (3.62 g, yield: 81%).
$^1$H-NMR (400MHz, $CDCl_3$) : δ 8.89 (1H, br. s), 7.61 (1H, dd, J = 8.8, 5.5 Hz), 7.20 (1H, m), 7.09 (1H, dd, J = 9.4, 2.0 Hz), 6.94 (1H, ddd, J = 9.4, 8.8, 2.0 Hz), 4.41 (2H, q, J = 7.0 Hz), 1.42 (3H, t, J = 7.0 Hz).

(9-7)

Triethylamine (4.55 ml, 32.8 mmol), di-tert-butyl dicarbonate (3.57 g, 16.4 mmol) and N,N-dimethylaminopyridine (100 mg, 0.819 mmol) were added to a solution of ethyl 6-fluoro-1H-indole-2-carboxylate (1.70 g, 8.19 mmol) obtained in Reference Example (9-6) in methylene chloride (82 ml) at room temperature, and the mixture was stirred overnight. Water and saturated aqueous NaCl solution were added to the reaction mixture and, after the mixture was extracted with methylene chloride, the organic layer was dried over anhydrous sodium sulfate. The residue obtained by evaporating the solvent under reduced pressure was purified by silica gel column chromatography (elution solvent: n-hexane/ethyl acetate=9/1) to obtain 1-tert-butyl 2-ethyl 6-fluoro-1H-indole-1,2-dicarboxylate as a yellow oil (1.94 g, yield: 95%).
$^1$H-NMR (400MHz, $CDCl_3$): δ 7.77 (1H, dd, J = 10.2, 2.4 Hz) , 7.51 (1H, dd, J = 8.6, 5.8 Hz), 7.05 (1H, s), 7.00 (1H, app td, J = 9.0, 2.4 Hz), 4.36 (2H, q, J = 7.0 Hz), 1.63 (9H, s), 1.30 (3H, t, J = 7.0 Hz).

(9-8)

Diisobutyl aluminum hydride-1.0M-toluene solution (174 ml, 174 mmol) was added to a solution of 1-tert-butyl 2-ethyl 6-fluoro-1H-indole-1,2-dicarboxylate (19.0 g, 57.9 mmol) obtained in Reference Example (9-7) in toluene (290 ml) at -78˚C, and the temperature was raised from -78˚C to -20˚C under stirring over 2.5 hours. Sodium sulfate decahydrate (143 g) was added to the reaction mixture, and the mixture was stirred at room temperature for 10 minutes. Toluene (300 ml), anhydrous magnesium sulfate (50 g) and Celite (30 g) were added thereto, and the mixture was further stirred for 10 minutes. The insolubles were removed by filtration using Celite, and the filtrate was concentrated under reduced pressure. The thus obtained residue was purified by silica gel chromatography (elution solvent: n-hexane/ethyl acetate=3/2) to obtain tert-butyl 6-fluoro-2-(hydroxymethyl)-1H-indole-1-carboxylate as a pale yellow oil (9.20 g, yield: 60%).
$^1$H-NMR (400MHz, $CDCl_3$): δ 7.70 (1H, dd, J = 10.6, 2.4 Hz), 7.42 (1H, dd, J = 8.6, 5.9 Hz), 6.99 (1H, ddd, J = 9.4, 8.6, 2.4 Hz), 6.55 (1H, s), 4.79 (2H, d, J = 7.4 Hz), 3.64 (1H, t, J = 7.4 Hz), 1.73 (9H, s).

(9-9)

N,N,N',N'-Tetramethylazodicarboxamide (Tokyo Chemical Industries, Inc., product No.: A1458) (4.39 g, 25.5 mmol) and tri-n-butylphosphine (5.92 ml, 25.5 mmol) were successively added to a solution of tert-butyl 6-fluoro-2-(hydroxymethyl)-1H-indole-1-carboxylate (4.50 g, 17.0 mmol) obtained in Reference Example (9-8) and methyl (4'-hydroxy-1,1'-biphenyl-4-yl)acetate (4.11 g, 17.0 mmol) obtained in Reference Example (9-4) in tetrahydrofuran (57 ml), and the mixture was stirred at room temperature for 4 hours. After the reaction mixture was filtered through Celite, the obtained filtrate was poured into water, and the mixture was extracted three times with ethyl acetate. The organic layer was successively washed with water and saturated aqueous NaCl solution, and dried over anhydrous sodium sulfate. The residue obtained by evaporating the solvent under reduced pressure was purified by silica gel preparative thin layer

chromatography (development solvent: n-hexane/ethyl acetate=5/1) to obtain tert-butyl 6-fluoro-2-[{4'-[(methoxycarbonyl)methyl]-1,1'-biphenyl-4-yl}oxy)methyl]-1H-indole-1-carboxylate as a white powder (5.50 g, yield: 66%).
$^1$H-NMR (400MHz, $CDCl_3$): δ 7.89 (1H, dd, J = 10.7, 2.0 Hz), 7.54-7.51 (4H, m), 7.43 (1H, dd, J = 8.3, 5.4 Hz), 7.33 (2H, d, J = 8.3 Hz), 7.05 (2H, d, J = 8.8 Hz), 6.99 (1H, ddd, J = 8.8, 8.3, 2.0 Hz), 6.71 (1H, s), 5.41 (2H, s), 3.71 (3H, s), 3.67 (2H, s), 1.66 (9H, s).

(9-10)

1N-Aqueous sodium hydroxide solution (33.7 ml, 33.7 mmol) was added to a solution of tert-butyl 6-fluoro-2-[({4'-[(methoxycarbonyl)methyl]-1,1'-biphenyl-4-yl}oxy)methyl]-1H-indole-1-carboxylate (5.50 g, 11.2 mmol) obtained in Reference Example (9-9) in tetrahydrofuran (112 ml), and the mixture was stirred at room temperature overnight. The reaction solution was poured into 1N-hydrochloric acid and, after the mixture was extracted three times with ethyl acetate, the organic layer was successively washed with water and saturated aqueous NaCl solution, and dried over anhydrous sodium sulfate. The residue obtained by evaporating the solvent under reduced pressure was recrystallized from ethyl acetate to obtain the title compound as a white powder (4.39 g, yield: 82%).
$^1$H-NMR (400MHz, $CDCl_3$): δ 7.90 (1H, dd, J = 11.0, 2.0 Hz), 7.53 (4H, d, J = 8.6 Hz), 7.43 (2H, dd, J = 8.6, 5.5 Hz), 7.35 (2H, d, J = 8.6 Hz), 7.05 (2H, d, J = 8.6 Hz), 6.99 (1H, ddd, J = 9.0, 8.6, 2.0 Hz), 6.71 (1H, s), 5.41 (2H, s), 3.71 (2H, s), 1.66 (9H, s).

(Reference Example 10)

(4'-{[1-(tert-Butoxycarbonyl)-6-fluoro-1H-indol-2-yl]methoxy}-3-chloro-1,1'-biphenyl-4-yl)acetic acid

(10-1)

Potassium carbonate (1.38 g, 10 mmol) and methyl iodide (0.623 ml, 10 mmol) were added to a solution of 4-bromo-2-chlorobenzoic acid (2.8 g, 8.5 mmol) in N,N-dimethylformamide (8 ml) under ice-cooling, and the mixture was stirred at room temperature for 3 hours. The reaction mixture was poured into water and, after the mixture was extracted with ethyl acetate, the organic layer was successively washed with water and saturated aqueous NaCl solution, and dried over anhydrous sodium sulfate. Diisobutyl aluminum hydride-1.0M toluene solution (24 ml, 24 mmol) was added dropwise to a solution of the oily residue obtained by evaporating the solvent under reduced pressure in toluene (30 ml) at -78˚C, and the mixture was raised to room temperature over 3 hours. Sodium sulfate decahydrate (12 g) was added to the reaction mixture, and the mixture was stirred at room temperature for 30 minutes. Celite and anhydrous magnesium sulfate were added thereto, and the mixture was stirred at room temperature for 30 minutes, followed by removal by filtration of the insolubles. The solvent was evaporated from the obtained filtrate under reduced pressure to obtain crude (4-bromo-2-chlorophenyl)methanol as a solid.
Carbon tetrabromide (3.2 g, 9.5 mmol) and triphenylphosphine (2.5 g, 9.5 mmol) were added to a solution of the obtained crude (4-bromo-2-chlorophenyl)methanol (1.84 g, 8.4 mmol) in tetrahydrofuran (12 ml) under ice-cooling, and the mixture was stirred at room temperature for 1 hour. n-Hexane was added to the reaction mixture, and the insolubles were removed by filtration. The thus obtained filtrate was poured into water and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=15/1-12/1) to obtain the crude compound.
Potassium cyanide (640 mg, 9.8 mmol) was added to a mixed solution of the obtained crude compound in ethanol-water (3:1, 20 ml) and the mixture was stirred at 60˚C for 1.5 hours. The reaction mixture was poured into water and, after the mixture was extracted with ethyl acetate, the organic layer was washed with saturated aqueous NaCl solution, and dried over anhydrous sodium sulfate. The residue obtained by evaporating the solvent under reduced pressure was purified by silica gel column chromatography (elution solvent: n-hexane/ethyl acetate=10/1-4/1) to obtain (4-bromo-2-chlorophenyl)acetonitrile as a pale yellow solid (1.4 g, yield: 71%).
$^1$H-NMR (400MHz, $CDCl_3$) : δ 7.61 (1H, d, J = 1.6 Hz), 7.47 (1H, br d, J = 8.0 Hz), 7.40 (1H, d, J = 8.0 Hz), 3.79 (2H, s) .

(10-2)

The procedures were carried out similarly to the methods of Reference Example (23-2) and Reference Example (23-3) using (4-bromo-2-chlorophenyl)acetonitrile (1.37 g, 5.9 mmol) obtained in Reference Example (10-1) and 4-(4,4,5,5-tetramethyl-1,3,2-dioxabororan-2-yl)phenol (1.3 g, 5.9 mmol) as the starting materials to obtain allyl (3-chloro-4'-hydroxy-1,1'-biphenyl-4-yl)acetate as a pale yellow solid (698 mg, two steps total yield: 39%).

In the present step, the reaction corresponding to Reference Example (23-2) was carried out at a reaction temperature of 85˚C.
$^1$H-NMR (400MHz, $CDCl_3$): δ 7.56 (1H, d, J = 1.6 Hz), 7.44 (2H, d, J = 8.8 Hz), 7.39 (1H, app d, J = 8.0 Hz), 7.33 (1H, d, J = 8.0 Hz), 6.89 (2H, d, J = 8.8 Hz), 5.98-5.90 (1H, m), 5.32 (1H, app d, J = 16.4 Hz), 5.24 (1H, app d, J = 10.8 Hz), 4.88 (1H, br s), 4.65 (2H, app d, J = 6.0 Hz), 3.83 (2H, s).

(10-3)

The procedures were carried out similarly to Reference Example (9-9) and Reference Example (17-4) using tert-butyl 6-fluoro-2-(hydroxymethyl)-1H-indole-l-carboxylate (109 mg, 0.41 mmol) obtained in Reference Example (9-8) and allyl (3-chloro-4'-hydroxy-1,1'-biphenyl-4-yl)acetate (123 mg, 0.41 mmol) obtained in Reference Example (10-2) to obtain the title compound as a colorless solid (104 mg, two steps total yield: 50%).
$^1$H-NMR (500MHz, $CDCl_3$): δ 7.89 (1H, dd, J = 10.5, 2.0 Hz), 7.60 (1H, d, J = 2.0,Hz), 7.51 (2H, d, J = 8.5 Hz), 7.45-7.42 (2H, m), 7.35 (1H, d, J = 8.0 Hz), 7.06 (2H, d, J = 8.5 Hz), 6.99 (1H, app td, J = 8.5, 2.0 Hz), 6.71 (1H, s), 5.41 (2H s), 3.86 (2H s), 1.66 (9H s).
MS (FAB) (m/z): 509 ($[M]^+$).

(Reference Example 11)

[5-(4-{[1-(tert-Butoxycarbonyl)-6-fluoro-1H-indol-2-yl]methoxy}phenyl)-2-thienyl]acetic acid

(11-1)

2N-Aqueous sodium carbonate solution (10 ml), tri-o-tolylphosphine (120 mg, 0.13 mmol) and tris(dibenzylideneacetone)dipalladium (0) (80 mg, 0.26 mmol) were added to a mixed solution of 4-(4,4,5,5-tetramethyl-1,3,2-dioxaborolan-2-yl)phenol (1.40 g, 6.4 mmol) and methyl (5-bromo-2-thienyl)acetate (2.00 g, 8.5 mmol) synthesized from methyl 2-thienylacetate according to a method described in the literature (Jackson, P. M. et al., J. Chem. Soc. Perkin Trans. L., 1990, vol. 11, pp. 2909-2918) in toluene-ethanol (5:1, 30 ml), and the mixture was heated under reflux with stirring for 3 hours. The reaction mixture was poured into water, and the mixture was extracted three times with ethyl acetate. The organic layer was successively washed with water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=1/6-1/3) to obtain methyl [5-(4-hydroxyphenyl)-2-thienyl]acetate (405 mg, yield: 26%).
$^1$H-NMR (400MHz, $CDCl_3$) : δ 7.43 (2H, d, J = 8.6 Hz), 7.01 (1H, d, J = 3.9 Hz), 6.86 (1H, d, J = 3.9 Hz) , 6.82 (2H, d, J = 8.6 Hz), 3.83 (2H, s), 3.75 (3H, s).

(11-2)

The procedures were carried out similarly to Reference Example (9-9) using tert-butyl 6-fluoro-2-(hydroxymethyl)-1H-indole-1-carboxylate (220 mg, 0.83 mmol) obtained in Reference Example (9-8) and methyl [5-(4-hydroxyphenyl)-2-thienyl]acetate (205 mg, 0.83 mmol) obtained in Reference Example (11-1) to obtain tert-butyl 6-fluoro-2-[(4-{5-[(methoxycarbonyl)methyl]-2-thienyl}phenoxy)methyl]-1H-indole-1-carboxylate as pale yellow crystals (83 mg, yield: 20%, recrystallization from n-hexane-ethyl acetate).
$^1$H-NMR (400MHz, $CDCl_3$) : δ 7.89 (1H, dd, J = 11.3, 2.0 Hz) , 7.51 (2H, d, J = 8.6 Hz), 7.42 (1H, dd, J = 8.6z), 7.05 (1H, d, J = 3.9 Hz), 7.01-6.95 (1H, m), 6.98 (2H, d, J = 8.6 Hz), 6.88 (1H, d, J = 3.9 Hz), 6.69 (1H, s), 5.38 (2H, s), 3.83 (2H, s), 3.75 (3H, s), 1.65 (9H, s).

(11-3)

The procedures were carried out similarly to Reference Example (9-10) using tert-butyl 6-fluoro-2-[(4-{5-[(methoxycarbonyl)methyl]-2-thienyl}phenoxy)methyl]-1H-indole-1-carboxylate (83 mg, 0.17 mmol) obtained in Reference Example (11-2) to obtain the title compound as colorless needle-like crystals (48 mg, yield: 59%, recrystallization from n-hexane-ethyl acetate).
$^1$H-NMR (400MHz, $CDCl_3$) : δ 7.89 (1H, dd, J = 10.9, 2.3 Hz) , 7.50 (2H, d, J = 8.6 Hz), 7.42 (1H, dd, J = 8.6, 5.5 Hz), 7.06 (1H, d, J = 3.9 Hz), 7.01-6.95 (3H, m), 6.91 (1H, br d, J = 3.9 Hz), 6.69 (1H, s), 5.38 (2H, s), 3.88 (2H, s), 1.65 (9H, s).

(Reference Example 12)

(4'-{[1-(tert-Butoxycarbonyl)-6-fluoro-1H-indol-2-yl]methoxy}-2-chloro-1,1'-biphenyl-4-yl)acetic acid

(12-1)

Sulfuric acid (1 ml) was added dropwise to a solution of 3-chloro-4-hydroxyphenylacetic acid (3.7 g, 20 mmol) in methanol at 0˚C. The temperature of the reaction solution was raised to room temperature, and the reaction solution was stirred for 4 hours. After the solvent was evaporated under reduced pressure, the mixture was poured into water, and the mixture was extracted with ethyl acetate. The organic layer was successively washed with water, saturated aqueous sodium hydrogencarbonate solution and saturated aqueous NaCl solution, and dried over anhydrous sodium sulfate. The residue obtained by evaporating the solvent under reduced pressure was purified by silica gel column chromatography (elution solvent: ethyl acetate) to obtain crude methyl (3-chloro-4-hydroxyphenyl) acetate. Pyridine (8.0 ml, 99 mmol) and trifluoromethanesulfonic acid anhydride (3.4 ml, 20 mmol) were added dropwise to a solution of the obtained crude methyl (3-chloro-4-hydroxyphenyl)acetate in methylene chloride (30 ml) under ice-cooling, and the mixture was stirred for 1 hour. The reaction mixture was poured into 1N-aqueous sodium hydroxide solution, and the mixture was extracted with methylene chloride. The organic layer was successively washed with water, 1N-hydrochloric acid and saturated aqueous NaCl solution, and dried over anhydrous sodium sulfate. The residue obtained by evaporating the solvent under reduced pressure was purified by silica gel column chromatography (elution solvent: n-hexane/ethyl acetate=3/1) to obtain methyl (3-chloro-4-{[(trifluoromethyl)sulfonyl]oxy}phenyl)acetate as a colorless solid (6.3 g, yield: 95%).
$^1$H-NMR (500MHz, $CDCl_3$): δ 7.48 (1H, d, J = 2.0 Hz), 7.31 (1H, d, J = 8.8 Hz), 7.27 (1H, dd, J = 8.8, 2.0 Hz), 3.73 (3H, s), 3.64 (2H, s).

(12-2)

2M-Aqueous sodium carbonate solution (1.5 ml), tris(dibenzylideneacetone)dipalladium (0) (23 mg, 0.025 mmol) and bis(2-diphenylphosphinophenyl)ether (DPEphos) (28 mg, 0.052 mmol) were added to a mixed solution of methyl (3-chloro-4-{[(trifluoromethyl)sulfonyl]oxy}phenyl)acetate (317 mg, 1.0 mmol) obtained in Reference Example (12-1) and 4-methoxyphenylboric acid (152 mg, 1.0 mmol) in toluene-ethanol (5:1, 9 ml), and the mixture was stirred at 100˚C for 5 hours. After the temperature of the reaction mixture was returned to room temperature, the reaction mixture was poured into water, and the mixture was extracted three times with ethyl acetate. The organic layer was successively washed with water (twice) and saturated aqueous NaCl solution, and dried over anhydrous sodium sulfate. The solvent was evaporated under reduced pressure to obtain methyl (2-chloro-4'-methoxy-1,1'-biphenyl-4-yl)acetate as a solid (192 mg, yield: 66%).
$^1$H-NMR (400MHz, $CDCl_3$) δ 7.39-7.36 (3H, m), 7.30-7.28 (1H, m), 7.21 (1H, dd, J = 7.6, 1.6 Hz), 6.96 (2H, d, J = 8.4 Hz), 3.86 (3H, s), 3.73 (3H, s), 3.64 (2H, s).

(12-3)

Boron trichloride-1.0N-methylene chloride solution (1.8 ml, 1.8 mmol) was added to a solution of methyl (2-chloro-4'-methoxy-1,1'-biphenyl-4-yl)acetate (228 mg, 0.73 mmol) obtained in Reference Example (12-2) and tetra-n-butylammonium iodide (325 mg, 0.88 mmol) in methylene chloride (4 ml) at -78˚C, and the mixture was stirred at room temperature for 2 hours. Ice was added to the reaction mixture and, after the mixture was extracted with ethyl acetate, the organic layer was successively washed with water and saturated aqueous NaCl solution, and dried over anhydrous, sodium sulfate. The residue obtained by evaporating the solvent under reduced pressure was purified by silica gel column chromatography (elution solvent: n-hexane/ethyl acetate=10/1-2/1) to obtain methyl (2-chloro-4'-hydroxy-1,1'-biphenyl-4-yl)acetate as a colorless solid (144 mg, yield: 71%).
$^1$H-NMR (400MHz, $CDCl_3$): δ 7.39 (1H, s), 7.32 (2H, d, J = 8.0 Hz), 7.29-7.26 (1H, m), 7.21 (1H, d, J = 7.2 Hz), 6.89 (2H, d, J = 8.0 Hz), 4.90 (1H, br s), 3.74 (3H, s), 3.64 (2H, s).

(12-4)

The procedures were carried out similarly to Reference Example (9-9) using tert-butyl 6-fluoro-2-(hydroxymethyl)-1H-indole-1-carboxylate (191 mg, 0.72 mmol) obtained in Reference Example (9-8) and methyl (2-chloro-4'-hydroxy-1,1'-biphenyl-4-yl)acetate (200 mg, 0.72 mmol) obtained in Reference Example (12-3) to obtain 2-[({2'-chloro-4'-[(methoxycarbonyl)methyl]-1,1'-biphenyl-4-yl}oxy)methyl]-6-fluoro-1H-indole-1-carboxylate as a colorless solid (349 mg, yield: 92%).

$^1$H-NMR (500MHz, $CDCl_3$) : δ 7.90 (1H, dd, J = 11.0, 2.5 Hz), 7.43 (1H, dd, J = 9.0, 5.5 Hz), 7.40-7.38 (3H, m), 7.30 (1H, d, J = 7.5 Hz), 7.22 (1H, dd, J = 7.5, 2.0 Hz), 7.05 (2H, d, J = 8.5 Hz), 6.99 (1H, app td, J = 9.0, 2.5 Hz), 6.72 (1H, s), 5.42 (2H, s), 3.74 (3H, s), 3.64 (2H, s), 1.66 (9H, s).

(12-5)

The procedures were carried out similarly to Reference Example (9-10) using 2-[({2'-chloro-4'-[(methoxycarbonyl)methyl]-1,1'-biphenyl-4-yl}oxy)methyl]-6-fluoro-1H-indole-1-carboxylate (349 mg, 0.64 mmol) obtained in Reference Example (12-4) to obtain the title compound as a colorless solid (189 mg, yield: 58%).
$^1$H-NMR (400Hz, $CDCl_3$): δ 7.89 (1H, dd, J = 11.2, 2.4 Hz), 7.46-7.38 (4H, m), 7.31 (1H, d, J = 8.0 Hz), 7.23 (1H, dd, J = 8.0, 2.0 Hz), 7.05 (2H, d, J 8.4 Hz), 6.99 (1H, app td, J = 8.8, 2.4 Hz), 6.72 (1H, s), 5.42 (2H, s), 3.69 (2H, s), 1.66 (9H, s).
MS (FAB) (m/z): 509 ($[M]^+$).

(Reference Example 13)

(4'-{[2-(tert-Butoxycarbonyl)-3-hydroxy-4-(trifluoromethyl)benzyl]oxy}-1,1'-biphenyl-4-yl)acetic acid

(13-1)

1,1$^1$-(Azodicarbonyl)dipiperidine (24.5 g, 97.2 mmol) and tri-n-butylphosphine (22.9 ml, 97.2 mmol) were successively added to a solution of [2-(dimethoxymethyl)-3-(methoxymethoxy)-4-(trifluoromethyl)phenyl]methanol (27.6 g, 89.1 mmol) obtained in Reference Example (5-4) and methyl (4'-hydroxy-1,1'-biphenyl-4-yl)acetate (19.6 g, 81.0 mmol) obtained in Reference Example (9-4) in tetrahydrofuran (300 ml), and the mixture was stirred at room temperature for 2 hours. After the produced white precipitate was removed by filtration, the precipitate was washed with ethyl acetate. The filtrate was poured into water and, after the mixture was extracted (three times) with ethyl acetate, the organic layer was successively washed with 3N-aqueous sodium hydroxide solution, water and saturated aqueous NaCl solution, and dried over anhydrous sodium sulfate. The solvent was evaporated under reduced pressure and the residue was purified by silica gel column chromatography (elution solvent: n-hexane/ethyl acetate=4/1) to obtain methyl (4'-{[2-(dimethoxymethyl)-3-(methoxymethoxy)-4-(trifluoromethyl)benzyl]oxy}-1,1'-biphenyl-4-yl)acetate (36.0 g, 83%).
$^1$H-NMR (400MHz, $CDCl_3$) : δ 7.56-7.54 (2H, br s), 7.47 (2H, d, J = 7.8 Hz), 7.46 (2H, d, J = 9.0 Hz), 7.29 (2H, d, J = 7.8 Hz), 7.01 (2H, d, J = 9.0 Hz), 5.75 (1H, s), 5.51 (2H, s), 5.03 (2H, s), 3.70 (3H, s), 3.66 (3H, s), 3.64 (2H, s), 3.48 (6H, s)

(13-2)

p-Toluenesulfonic acid monohydrate (15.4 g, 80.9 mmol) was added to a solution of methyl (4'-{[2-(dimethoxymethyl)-3-(methoxymethoxy)-4-(trifluoromethyl)benzyl]oxy}-1,1'-biphenyl-4-yl)acetate (36.0 g, 67.4 mmol) obtained in Reference Example (13-1) in acetone (200 ml), and the mixture was stirred at room temperature overnight. The reaction mixture was poured into water and, after the mixture was extracted with ethyl acetate, the organic layer was successively washed with water and saturated aqueous NaCl solution, and dried over anhydrous sodium sulfate. The residue obtained by evaporating the solvent under reduced pressure was dissolved in N,N-dimethylformamide (50 ml). Potassium carbonate (11.2 g, 80.9 mmol) and allyl bromide (7.00 ml, 80.9 mmol) were successively added thereto, and the reaction mixture was stirred at 50˚C for 2 hours. The reaction mixture was poured into water and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=9/1-1/1) to obtain methyl (4'-{[3-(allyloxy)-2-formyl-4-(trifluoromethyl)benzyl]oxy}-1,1'-biphenyl-4-yl)acetate (31.2 g, yield: 96%).
$^1$H-NMR (400MHz, $CDCl_3$) : δ 10.52 (1H, br s) , 7.86 (1H, d, J = 8.2 Hz), 7.76 (1H, d, J = 8.2 Hz), 7.51 (2H, d, J = 8.4 Hz) , 7.49 (2H, d, J = 8.4 Hz) , 7.31 (2H, d, J = 8.4 Hz), 7.04 (2H, d, J = 8.4 Hz), 6.16-6.04 (1H, m), 5.52-5.44 (3H, m), 5.39-5.34 (1H, m), 4.58 (2H, m), 3.70 (3H, s), 3.66 (2H, s).

(13-3)

A solution of sodium chlorite (23.2 g, 258 mmol) and sodium dihydrogenphosphate monohydrate (23.2 g, 169 mmol) in water (100 ml) were added dropwise to a mixed solution of methyl (4'-{[3-(allyloxy)-2-formyl-4-(trifluoromethyl) benzyl]oxy}-1,1'-biphenyl-4-yl)acetate (31.2 g, 64.4 mmol) obtained in Reference Example (13-2) in tert-butyl alcohol (200 ml), 1,4-dioxane (45 ml) and 2-methyl-2-butene (60 ml), and the mixture was stirred at room temperature for 4 hours. After 5% aqueous sodium thiosulfate solution was added to the reaction mixture, the mixture was poured into 5N-hydrochloric acid, and the mixture was extracted (twice) with ethyl acetate. The organic layer was successively

washed with water and saturated aqueous NaCl solution, and dried over anhydrous sodium sulfate. The solvent was evaporated under reduced pressure to obtain the residue. After the thus obtained residue was dissolved in toluene (200 ml), N,N-dimethylformamide di-tert-butyl acetal (61.7 ml, 258 mmol) was added thereto, and the mixture was heated under reflux for 6 hours. The reaction mixture was poured into water and, after the mixture was extracted (three times) with ethyl acetate, the organic layer was successively washed with water (twice) and saturated aqueous NaCl solution, and dried over anhydrous sodium sulfate. Pyrrolidine (8.07 ml, 96.6 mmol) and tetrakis(triphenylphosphine)palladium (743 mg, 0.64 mmol) were added to a mixed solution of the residue obtained by evaporating the solvent under reduced pressure in 1,4-dioxane (150 ml) and water (5 ml), and the mixture was stirred at room temperature for 3 hours. Water was poured into the reaction mixture and, after the mixture was extracted with ethyl acetate, the organic layer was successively washed with water and saturated aqueous NaCl solution, and dried over anhydrous sodium sulfate. The mixture was purified by silica gel column chromatography (elution solvent: n-hexane/ethyl acetate=20/1-1/1) to obtain tert-butyl 2-hydroxy-6-[({4'-[(methoxycarbonyl)methyl]-1,1'-biphenyl-4-yl}oxy)methyl]-3-(trifluoromethyl)benzoate as a white powder (18.6 g, yield: 56%).
$^1$H-NMR (400MHz, $CDCl_3$): δ 12.23 (1H, s), 7.68 (1H, d, J = 8.2 Hz), 7.50 (2H, d, J = 9.0 Hz), 7.48 (2H, d, J = 8.2 Hz), 7.30 (2H, d, J = 8.2 Hz), 7.25 (1H, d, J = 8.2 Hz), 6.95 (2H, d, J = 9.0 Hz), 5.35 (2H, s), 3.70 (3H, s), 3.65 (2H, s), 1.63 (9H, s).

(13-4)

3N-Aqueous sodium hydroxide solution (28.8 ml, 86.4 mmol) was added to a solution of tert-butyl 2-hydroxy-6-[({4'-[(methoxycarbonyl)methyl]-1,1'-biphenyl-4-yl}oxy)methyl]-3-(trifluoromethyl)benzoate (18.6 g, 36.0 mmol) obtained in Reference Example (13-3) in tetrahydrofuran (200 ml), and the mixture was stirred at room temperature for 2 hours. The reaction mixture was poured into 1N-hydrochloric acid and, after the mixture was extracted (three times) with ethyl acetate, the organic layer was successively washed with water and saturated aqueous NaCl solution, and dried over anhydrous sodium sulfate. The precipitate obtained by evaporating the solvent under reduced pressure was collected by filtration to obtain the title compound as a white powder (12.9 g, yield: 71%).
$^1$H-NMR (400MHz, acetone-$d_6$) : δ 12.26 (1H, br), 7.82 (1H, d, J = 8.2 Hz), 7.61 (2H, d, J = 8.6 Hz), 7.56 (2H, d, J = 8.2 Hz) , 7.41-7.33 (3H, m), 7.09 (2H, d, J = 8.6 Hz), 5.52 (2H, s), 3.66 (2H, s), 1.71 (9H, s).
Anal. calcd. for $C_{27}H_{25}F_3O_6$ : C, 64.54; H, 5.01; F, 11.34; found: C, 64.58; H, 5.04; F, 11.40.

(Reference Example 14)

(4'-{[2-(tert-Butoxycarbonyl)-3-hydroxy-4-(trifluoromethyl)benzyl]oxy}-3-chloro-1,1'-biphenyl-4-yl)acetic acid

(14-1)

The procedures were carried out similarly to the methods of Reference Example (13-1), Reference Example (13-2), Reference Example (21-3) and Reference Example (21-4) using allyl (3-chloro-4'-hydroxy-1,1'-biphenyl-4-yl) acetate (560 mg, 1.85 mmol) obtained in Reference Example (10-2) and [2-(dimethoxymethyl)-3-(methoxymethoxy)-4-(trifluoromethyl)phenyl]methanol (807 mg, 2.6 mmol) obtained in Reference Example (5-4) as the starting materials to obtain tert-butyl 2-(allyloxy)-6-[({4'-[(allyloxycarbonyl)methyl]-3'-chloro-1,1'-biphenyl-4-yl}oxy)methyl]-3-(trifluoromethyl)benzoate as a colorless solid (622 mg, four steps total yield: 54%).
$^1$H-NMR (400MHz, $CDCl_3$) : δ 7.65 (1H, d, J = 8.4 Hz), 7.57 (1H, d, J = 1.6 Hz), 7.49 (2H, d, J = 8.4 Hz), 7.42-7.38 (2H, m), 7.33 (1H, d, J = 8.4 Hz), 7.00 (2H, d, J = 8.4 Hz), 6.12-6.02 (1H, m), 5.98-5.88 (1H, m), 5.43 (1H, dd, J = 17.2, 1.6 Hz), 5.34-5.27 (2H, m), 5.24 (1H, dd, J = 11.2, 1.2 Hz), 5.16 (2H, s), 4.64 (2H, app d, J = 6.0 Hz), 4.58 (2H, app d, J = 5.6 Hz), 3.83 (2H, s), 1.58 (9H, s).

(14-2)

The procedures were carried out similarly to Reference Example (6-2) using tert-butyl 2-(allyloxy)-6-[({4'-[(allyloxycarbonyl)methyl]-3'-chloro-1,1'-biphenyl-4-yl}oxy)methyl]-3-(trifluoromethyl)benzoate (622 mg, 1.01 mmol) obtained in Reference Example (14-1) to obtain the title compound as a colorless powder (337 mg, yield: 62%).
$^1$H-NMR (500MHz, $CDCl_3$): δ 12.26, (1H, s), 7.71 (1H, d, J = 8.0 Hz), 7.60 (1H, d, 2.0 Hz), 7.51 (2H, d, J = 8.5 Hz), 7.43 (1H, dd, J = 8.0, 2.0 Hz), 7.35 (1H, d, J = 8.0 Hz), 7.28-7.26 (1H, m), 6.99 (2H, d, J = 8.5 Hz), 5.38 (2H, s), 3.86 (2H, s), 1.65 (9H, s).
MS (FAB) (m/z): 536 ($[M]^+$).
Anal. calcd. for $C_{27}H_{24}ClF_3O_6$ :C, 60.40; H, 4.51; F, 10.62; Cl, 6.60; found: C, 60.20; H, 4.39; F, 10.72; Cl, 6.69.

(Reference Example 15)

(4'-{[2-(tert-Butoxycarbonyl)-3-hydroxy-4-(trifluoromethyl)benzyl]oxy}-2-ethyl-1,1'-biphenyl-4-yl)acetic acid

(15-1)

N,N-Dimethylformamide (75 ml) was added to 3-bromo-4-methoxybenzyl cyanide (9.0 g, 40 mmol), [1,1'-bis (diphenylphosphino)ferrocene]dichloropalladium (II)-dichloromethane adduct (400 mg, 0.49 mmol) and potassium carbonate (24.0 g, 174 mmol) at room temperature. Further, triethylborane (1M-n-hexane solution; 50 ml, 50 mmol) was added dropwise thereto, and the reaction mixture was stirred at 70˚C for 5 hours. After the temperature of the reaction mixture was returned to room temperature, the mixture was poured into water, and the mixture was extracted with ethyl acetate. The organic later was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=8/1-5/1) to obtain oily (3-ethyl-4-methoxyphenyl)acetonitrile (2.6 g, yield: 38%) .
$^1$H-NMR (400MHz, $CDCl_3$) : δ 7.11 (1H, d, J = 8.4 Hz), 7.08 (1H, s), 6.81 (1H, d, J = 8.4 Hz), 3.83 (3H, s), 3.67 (2H, s), 2.63 (2H, q, J = 7.6 Hz), 1.19 (3H, t, J = 7.6 Hz).

(15-2)

The procedures were carried out similarly to Reference Example (12-3) and Reference Example (25-5) using (3-ethyl-4-methoxyphenyl)acetonitrile (6.10 g, 34.8 mmol) obtained in Reference Example (15-1) to obtain oily 4-(cyanomethyl)-2-ethylphenyl trifluoromethanesulfonate (8.1 g, two steps total yield: 78%).
$^1$H-NMR (500MHz, $CDCl_3$) : δ 7.32 (1H, d, J = 2.0 Hz), 7.28-7.23 (2H, m), 3.77 (2H, s), 2.76 (2H, q, J = 7.5 Hz), 1.28 (3H, t, J = 7.5 Hz) .

(15-3)

Toluene (150 ml), ethanol (30 ml) and distilled water (30 ml) were added to 4-(cyanomethyl)-2-ethylphenyl trifluoromethanesulfonate (9.7 g, 33 mmol) obtained in Reference Example (15-2), 4-methoxyphenylboric acid (5.3 g, 35 mmol), [1,1'-bis(diphenylphosphino)ferrocene]dichloropalladium (II)-dichloromethane adduct (2.7 g, 3.3 mmol) and sodium carbonate (10.0 g, 94 mmol), and the mixture was stirred at 80˚C for 4 hours. After the reaction mixture was cooled to room temperature, the insolubles were removed by filtration. The thus obtained filtrate was poured into water, and the mixture was extracted with ethyl acetate. The organic layer was successively washed with water and saturated aqueous NaCl solution and dried over anhydrous sodium sulfate. The residue obtained by evaporating the solvent under reduced pressure was purified by silica gel column chromatography (elution solvent: n-hexane/ethyl acetate=7/1-6/1) to obtain oily (2-ethyl-4'-methoxy-1,1'-biphenyl-4-yl)acetonitrile (5.5 g, yield: 66%).
$^1$H-NMR (400MHz, $CDCl_3$): δ 7.25-7.17 (5H, m), 6.95 (2H, d, J = 8.8 Hz), 3.86 (3H, s), 3.77 (2H, s), 2.61 (2H, q, J = 7.6 Hz), 1.10 (3H, t, J = 7.6 Hz).

(15-4)

Acetic acid (55 ml) and hydrobromic acid (55 ml) were added to (2-ethyl-4'-methoxy-1,1'-biphenyl-4-yl)acetonitrile (5.5 g, 22 mmol) obtained in Reference Example (15-3), and the mixture was stirred at 100˚C for 10 hours. After the reaction mixture was cooled to room temperature, ethyl acetate was added thereto, and the mixture was successively washed with water and saturated aqueous NaCl solution, and dried over anhydrous sodium sulfate. After methanol (150 ml) was added to the residue obtained by evaporating the solvent under reduced pressure, sulfuric acid (3 ml) was added thereto under ice-cooling. After the mixture was stirred at room temperature for 1 hour, the solvent was evaporated under reduced pressure. After ethyl acetate was added to the obtained residue, the mixture was washed with saturated aqueous NaCl solution, and dried over anhydrous sodium sulfate. The residue obtained by evaporating the solvent under reduced pressure was purified by silica gel column chromatography (elution solvent: n-hexane/ethyl acetate=5/1-2/1) to obtain methyl (2-ethyl-4'-hydroxy-1,1'-biphenyl-4-yl)acetate as a colorless solid (5.0 g, yield: 85%).
$^1$H-NMR (400MHz, $CDCl_3$) : δ 7.20-7.13 (5H, m) , 6.86 (2H, d, J = 8.0 Hz), 4.76 (1H, br s), 3.73 (3H, s), 3.65 (2H, s), 2.58 (2H, q, J = 7.2 Hz), 1.09 (3H, t, J = 7.2 Hz).

(15-5)

Triethylamine (2.74 ml, 19.7 mmol) was added to a solution of [2-(dimethoxymethyl)-3-(methoxymethoxy)-4-(trifluoromethyl)phenyl]methanol (5.09 g, 16.4 mmol) obtained in Reference Example (5-4) in ethyl acetate (50 ml). After methanesulfonyl chloride (1.33 ml, 17.2 mmol) was added dropwise thereto under ice-cooling, the reaction mixture was stirred at the same temperature for 30 minutes. The reaction mixture was filtered through Celite. The filtrate was successively washed with saturated aqueous sodium hydrogencarbonate solution, water and saturated aqueous NaCl solution, and dried over anhydrous sodium sulfate. n-Hexane was added to the residue obtained by evaporating the solvent under reduced pressure. The precipitated crystals were collected by filtration to obtain pale yellow 2-(dimethoxymethyl)-3-(methoxymethoxy)-4-(trifluoromethyl)benzyl methanesulfonate (5.37 g, yield: 84%).
$^1$H-NMR (400MHz, $CDCl_3$) δ 7.64 (1H, d, J = 8.2 Hz) , 7.49 (1H, d, J = 8.2 Hz), 5.72 (1H, s), 5.65 (2H, s), 5.01 (2H, s), 3.65 (3H, s), 3.45 (6H, s), 3.06 (3H, s).

(15-6)

The procedures were carried out similarly to Reference Example (16-4) and Reference Example (21-2) using methyl (2-ethyl-4'-hydroxy-1,1'-biphenyl-4-yl)acetate (4.90 g, 18.1 mmol) obtained in Reference Example (15-4) and 2-(dimethoxymethyl)-3-(methoxymethoxy)-4-(trifluoromethyl)benzyl methanesulfonate (8.35 g, 21.5 mmol) obtained in Reference Example (15-5) to obtain methyl (2-ethyl-4'-{[2-formyl-3-hydroxy-4-(trifluoromethyl)benzyl]oxy}-1,1'-biphenyl-4-yl)acetate as a colorless solid (6.77 g, two steps total yield: 79%).
$^1$H-NMR (400MHz, $CDCl_3$): δ 12.66 (1H, s), 10.40 (1H, s), 7.83 (1H, d, J = 8.0 Hz), 7.26 (2H, d, J = 8.0 Hz), 7.21 (1H, s), 7.14 (2H, br s), 7.11 (1H, d, J = 8.0 Hz), 7.00 (2H, d, J = 8.0 Hz), 3.73 (3H, s), 3.66 (2H, s), 2.58 (2H, q, J = 7.6 Hz), 1.10 (3H, t, J = 7.6 Hz).

(15-7)

The procedures were carried out similarly to Reference Example (21-3), Reference Example (21-4) and Reference Example (16-5) using methyl (2-ethyl-4'-{[2-formyl-3-hydroxy-4-(trifluoromethyl)benzyl]oxy}-1,1'-biphenyl-4-yl) acetate (6.77 g, 14.3 mmol) obtained in Reference Example (15-6) to obtain tert-butyl 2-hydroxy-6-[({2'-ethyl-4'-[(methoxycarbonyl)methyl]-1,1'-biphenyl-4-yl}oxy)methyl]-3-(trifluoromethyl)benzoate as a colorless solid (5.87 g, three steps total yield: 76%).
$^1$H-NMR (400MHz, $CDCl_3$) : δ 12.22 (1H, s), 7.69 (1H, d, J = 8.4 Hz), 7.28 (1H, d, J = 8.4 Hz), 7.21 (2H, d, J = 8.4 Hz), 7.18 (1H, s), 7.12 (2H, br s), 6.92 (2H, d, J = 8.4 Hz), 5.36 (2H, s), 3.72 (3H, s), 3.64 (2H, s), 2.58 (2H, q, J = 7.6 Hz), 1.64 (9H, s), 1.09 (3H, t, J = 7.6 Hz).
ESI (ES-) (m/z) : 543 ($[M-H]^+$).

(15-8)

The procedures were carried out similarly to Reference Example (9-10) using tert-butyl 2-hydroxy-6-[({2'-ethyl-4'-[(methoxycarbonyl)methyl]-1,1'-biphenyl-4-yl}oxy)methyl]-3-(trifluoromethyl)benzoate (5.0 g, 9.2 mmol) obtained in Reference Example (15-7) to obtain the title compound as a colorless solid (4.79 g, yield: 97%). $^1$H-NMR (400MHz, $CDCl_3$): δ 12.27 (1H, s), 7.72 (1H, d, J = 8.0 Hz), 7.30 (1H, d, J = 8.0 Hz), 7.26-7.22 (3H, m), 7.16 (2H, br s), 6.95 (2H, d, J = 8.0 Hz), 5.38 (2H, s), 3.70 (2H, s), 2.60 (2H, q, J = 7.6 Hz), 1.65 (9H, s), 1.10 (3H, t, J = 7.6 Hz).
ESI (ES-) (m/z) : 529 ($[M-H]^+$) .
Anal. calcd. for $C_{29}H_{29}F_3O_6$ :C, 65.65; H, 5.51; F, 10.74; found: C, 65.63; H, 5.53; F, 10.78.

(Reference Example 16)

(4'-{[2-(tert-Butoxycarbonyl)-3-hydroxy-4-(trifluoromethyl)benzyl]oxy}-2-methyl-1,1'-biphenyl-3-yl)acetic acid

(16-1)

A solution of sodium nitrite (1.92 g, 11.6 mmol) in water (2 ml) was added dropwise to a solution of (3-amino-2-methylphenyl)acetic acid (1.20 g, 17.2 mmol) synthesized according to a method described in the literature (Askam, V. et al., J. Chem. Soc. C; 1969, pp. 1935-1936) in 10% sulfuric acid (72 ml) under ice-cooling. After the mixture was stirred at room temperature for 1 hour, the reaction mixture was added dropwise to a solution of potassium iodide (3.66 g, 22.0 mmol) in water (11 ml). The temperature of the reaction mixture was raised to 90˚C, and the mixture was stirred for 2.5 hours. The reaction mixture was extracted with ethyl acetate, and the organic layer was successively washed with 10% aqueous sodium sulfite solution and saturated aqueous NaCl solution, and dried over anhydrous sodium sulfate. The procedure was carried out similarly to Reference Example (9-1) using the reaction mixture obtained by evaporating the solvent under reduced pressure to obtain methyl (3-iodo-2-methylphenyl)acetate (2.23 g, yield: 66%).

$^1H$-NMR (400MHz, $CDCl_3$): δ 7.02 (1H, t, J = 7.8 Hz), 6.80 (1H, d, J = 7.8 Hz), 6.70 (1H, d, J = 7.8 Hz), 3.70 (2H, s), 3.65 (3H, s), 2.19 (3H, s).

(16-2)

Palladium acetate (II) (37 mg, 0.16 mmol), tri-o-tolylphosphine (100 mg, 0.327 mmol) and 2N-aqueous sodium carbonate solution (2.5 ml) were added to a solution of methyl (3-iodo-2-methylphenyl)acetate (950 mg, 3.27 mmol) obtained in Reference Example (16-1) and 4-methoxyphenylboric acid (498 mg, 3.27 mmol) in N,N-dimethylformamide (8 ml), and the mixture was stirred at 80˚C for 5 hours. After the reaction mixture was diluted with ethyl acetate and water, the insolubles were removed by filtration through Celite. After the obtained filtrate was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=9/1) to obtain methyl (4'-methoxy-2-methyl-1,1'-biphenyl-3-yl)acetate as a yellow powder (59 mg, yield: 7%).
$^1H$-NMR (400MHz, $CDCl_3$) : δ 7.23-7.12 (5H, m), 6.92 (2H, d, J = 8.6 Hz), 3.84 (3H, s), 3.71 (2H, s), 3.70 (3H, s), 2.18 (3H, s).

(16-3)

The procedures were carried out similarly to Reference Example (12-3) using methyl (4'-methoxy-2-methyl-1,1'-biphenyl-3-yl)acetate (59 mg, 0.22 mmol) obtained in Reference Example (16-2) to obtain methyl (4'-hydroxy-2-methyl-1,1[1]-biphenyl-3-yl)acetate (28 mg, yield: 50%).
$^1H$-NMR (400MHz, $CDCl_3$): δ 7.45-7.39 (1H, m), 7.18-7.12 (4H, m), 6.84 (2H, d, J = 8.6 Hz), 4.90 (1H, s), 3.71 (5H, s), 2.18 (3H, s).

(16-4)

Cesium carbonate (43 mg, 0.13 mmol) was added to a solution of methyl (4'-hydroxy-2-methyl-1,1'-biphenyl-3-yl)acetate (28 mg, 0.11 mmol) obtained in Reference Example (16-3) and tert-butyl 6-(bromomethyl)-2-[(tert-butoxycarbonyl)oxy]-3-(trifluoromethyl)benzoate (50 mg, 0.11 mmol) obtained in Reference Example (21-5) in N,N-dimethylformamide (2 ml), and the mixture was stirred at room temperature overnight. The reaction mixture was poured into water and the mixture was extracted with ethyl acetate. The organic layer was successively washed with water (three times) and saturated aqueous NaCl solution, and dried over anhydrous sodium sulfate, followed by concentration under reduced pressure. The thus obtained residue was purified by silica gel preparative thin layer chromatography (development solvent: hexane/ethyl acetate=3/1) to obtain tert-butyl 2-[(tert-butoxycarbonyl)oxy]-6-[({3'-[(methoxycarbonyl)methyl]-2'-methyl-1,1'-biphenyl-4-yl}oxy)methyl-3-(trifluoromethyl)benzoate as a colorless oil (19 mg, yield: 28%).
$^1H$-NMR (400MHz, $CDCl_3$): δ 7.73 (1H, d, J = 8.6 Hz), 7.62 (1H, d, J = 8.6 Hz), 7.22 (2H, d, J = 8.6 Hz), 7.24-7.12 (5H, m), 6.97 (2H, d, J = 8.6 Hz), 5.27 (2H, s), 3.72 (2H, s), 3.71 (3H, s), 2.17 (3H, s), 1.58 (9H, s), 1.54 (9H, s).

(16-5)

Pyrrolidine (3 μl, 36 μmol) was added to a solution of tert-butyl 2-[(tert-butoxycarbonyl) oxy]-6-[({3'-[(methoxycarbonyl)methyl]-2'-methyl-1,1'-biphenyl-4-yl}oxy)methyl-3-(trifluoromethyl)benzoate (19 mg, 30 μmol) obtained in Reference Example (16-4) in tetrahydrofuran (1 ml), and the mixture was stirred at 40˚C for 1 hour. The residue obtained by evaporating the solvent under reduced pressure was purified by silica gel preparative thin layer chromatography (development solvent: n-hexane/ethyl acetate=3/1) to obtain tert-butyl 2-hydroxy-6-[({3'-[(methoxycarbonyl)methyl]-2'-methyl-1,1'-biphenyl-4-yl}oxy)methyl]-3-(trifluoromethyl)benzoate as a colorless oil (16 mg, yield: 100%).
$^1H$-NMR (400MHz, $CDCl_3$) : δ 12.27 (1H, s), 7.72 (1H, d, J = 8.2 Hz), 7.31 (1H, d, J = 8.2 Hz), 7.24 (2H, d, J = 8.6 Hz), 7.25-7.13 (5H, m), 6.95 (2H, d, J = 8.6 Hz), 5.38 (2H, s), 3.72 (5H, s) , 2.19 (3H, s) , 1.65 (9H, s).

(16-6)

The procedures were carried out similarly to Reference Example (13-4) using tert-butyl 2-hydroxy-6-[({3'-[(methoxycarbonyl)methyl]-2'-methyl-1,1'-biphenyl-4-yl}oxy)methyl]-3-(trifluoromethyl)benzoate (16 mg, 30 μmol) obtained in Reference Example (16-5) to obtain the title compound as a brown powder (12 mg, yield: 77%).
$^1H$-NMR (500MHz, $CDCl_3$): δ 12.27 (1H, s), 7.72 (1H, d, J = 8.3 Hz), 7.31 (1H, d, J = 8.3 Hz), 7.24 (2H, d, J = 8.3 Hz), 7.25-7.15 (5H, m), 6.96 (2H, d, J = 8.3 Hz), 5.38 (2H, s), 3.76 (2H, s), 2.21 (3H, s), 1.65 (9H, s).
MS (FAB) (m/z): 516 ($[M]^+$).

(Reference Example 17)

1-(4'-{[2-(tert-Butoxycarbonyl)-3-hydroxy-4-(trifluoromethyl)benzyl]oxy}-3-fluoro-1,1'-biphenyl-4-yl)cyclopropanecarboxylic acid

(17-1)

The procedures were carried out similarly to Reference Example (23-1) and Reference Example (23-3) using 4-bromo-2-fluorobenzyl bromide (6.0 g, 22 mmol) to obtain methyl (4-bromo-2-fluorophenyl)acetate (3.41 g, three steps total yield: 62%).

In the present step, methanol was used instead of allyl alcohol in the esterification reaction included in the step corresponding to Reference Example (23-3), and the reaction was carried out at a reaction temperature of 50˚C.
$^1$H-NMR (400MHz, $CDCl_3$): δ 7.29-7.23 (2H, m), 7.15 (1H, app t, J = 8.2 Hz), 3.72 (3H, s), 3.64 (2H, s).

(17-2)

The procedures were carried out similarly to Reference Example (28-1), Reference Example (23-2), Reference Example (9-10) and Reference Example (9-1) using methyl (4-bromo-2-fluorophenyl)acetate (1.24 g, 5.02 mmol) to obtain allyl 1-(3-fluoro-4'-hydroxy-1,1'-biphenyl-4-yl)cyclopropanecarboxylate as a white powder (1.00 g, yield: 64%).

In the above step, the hydrolysis step corresponding to Reference Example (9-10) was carried out at a reaction temperature of 60˚C. Further, in the esterification step corresponding to Reference Example (9-1), allyl alcohol was used instead of methanol.
$^1$H-NMR (400MHz, $CDCl_3$) : δ 7.39 (2H, d, J = 8.6 Hz), 7.29-7.15 (3H, m), 6.84 (2H, d, J = 8.6 Hz), 5.88-5.76 (1H, m), 5.28 (1H, br s), 5.21-5.11 (2H, m), 4.58-4.55 (2H, m), 1.73-1.69 (2H, m), 1.26-1.22 (2H, m).

(17-3)

The procedures were carried out similarly to the methods of Reference Example (13-1), Reference Example (13-2), Reference Example (21-3) and Reference Example (21-4) using allyl 1-(3-fluoro-4'-hydroxy-1,1'-biphenyl-4-yl) cyclopropanecarboxylate (303 mg, 0.97 mmol) obtained in Reference Example (17-2) and [2-(dimethoxymethyl)-3-(methoxymethoxy)-4-(trifluoromethyl)phenyl]methanol (421 mg, 1.36 mmol) obtained in Reference Example (5-4) as the starting material to obtain tert-butyl 2-(allyloxy)-6-{[(4'-{1-[(allyloxy)carbonyl]cyclopropyl}-3'-fluoro-1,1'-biphenyl-4-yl)oxy]methyl}-3-(trifluoromethyl)benzoate (219 mg, five steps total yield: 36%).
$^1$H-NMR (400MHz, $CDCl_3$) : δ 7.62 (1H, d, J = 8.2 Hz), 7.48 (2H, d, J = 8.6 Hz), 7.37 (1H, d, J = 8.2 Hz), 7.30-7.18 (3H, m), 6.98 (2H, d, J = 8.6 Hz) , 6.10-6.00 (1H, m), 5.86-5.76 (1H, m), 5.45-5.38 (1H, m), 5.29-5.24 (1H, m), 5.19-5.10 (4H, m), 4.58-4.54 (4H, m), 1.72-1.68 (2H, m), 1.57 (9H, s), 1.26-1.21 (2H, m).

(17-4)

Morpholine (65 μl, 0.73 mmol) and tetrakis(triphenylphosphine)palladium (0) (12 mg, 10.5 μmol) were successively added to a solution of tert-butyl 2-(allyloxy)-6-{[(4'-{1-[(allyloxy)carbonyl]cyclopropyl}-3'-fluoro-1,1'-biphenyl-4-yl) oxy]methyl}-3-(trifluoromethyl)benzoate (219 mg, 0.35 mmol) obtained in Reference Example (17-3) in tetrahydrofuran (2 ml), and the mixture was stirred at room temperature for 2 hours. Water was poured into the reaction mixture and, after the mixture was extracted with ethyl acetate, the organic layer was successively washed with 1N-hydrochloric acid, water and saturated aqueous NaCl solution, and dried over anhydrous sodium sulfate. After the residue obtained by evaporating the solvent under reduced pressure was purified by silica gel column chromatography (elution mixed solvent: n-hexane/ethyl acetate=1/2), it was crystallized from a mixed solvent of n-hexane-ethyl acetate to obtain the title compound as a white amorphous solid (amorphous) (121 mg, yield: 63%).
$^1$H-NMR (400MHz, $CDCl_3$): δ 12.22 (1H, s), 7.69 (1H, d, J = 8.2 Hz), 7.49 (2H, d, J = 8.6 Hz), 7.32-7.18 (4H, m), 6.96 (2H, d, J = 8.6 Hz), 5.36 (2H, s), 1.77-1.72 (2H, m), 1.64 (9H, s), 1.31-1.26 (2H, m).
MS (ESI) (m/z) : 545 ( $[M-H]^+$) .

(Reference Example 18)

(4'-{[2-(tert-Butoxycarbonyl)-3-hydroxy-4-isopropylbenzyl]oxy}-1,1'-biphenyl-4-yl)acetic acid

(18-1)

Trimethyl ortho-formate (2.35 ml, 21.4 mmol) and ammonium chloride (52 mg, 0.98 mmol) were added to a solution of 3-isopropyl-2-(methoxymethoxy)benzaldehyde (4.06 g, 19.5 mmol) synthesized according to a method described in the literature (James. R. et al., J. Med. Chem., 1980, vol. 23, pp. 1350-1357) in methanol (65 ml), and the mixture was stirred with heating under reflux for 1 hour. The solvent was evaporated under reduced pressure, and saturated aqueous sodium hydrogencarbonate solution was added to the residue, followed by extraction with ethyl acetate. The organic layer was washed with saturated aqueous NaCl solution, and dried over anhydrous sodium sulfate. The residue obtained by evaporating the solvent under reduced pressure was purified by silica gel column chromatography (elution solvent: n-hexane/ethyl acetate=12/0-12/1) to obtain 1-(dimethoxymethyl)-3-isopropyl-2-(methoxymethoxy)benzene as a colorless oil (4.49 g, yield: 91%).
$^1$H-NMR (400MHz, $CDCl_3$) : δ 7.39 (1H, dd, J = 7.8, 1.5 Hz), 7.28 (1H, dd, J = 7.8, 1.5 Hz), 7.15 (1H, t, J = 7.8 Hz), 5.64 (1H, s), 4.99 (2H, s), 3.63 (3H, s), 3.40 (1H, sp, J = 6.8 Hz), 3.37 (6H, s), 1.23 (6H, d, J = 6.8 Hz).

(18-2)

The procedures were carried out similarly to Reference Example (5-3), Reference Example (5-4), Reference Example (13-1), Reference Example (13-2) and Reference Example (13-3) using 1-(dimethoxymethyl)-3-isopropyl-2-(methoxymethoxy)benzene (4.49 g, 17.7 mmol) obtained in Reference Example (18-1) to obtain tert-butyl 2-hydroxy-3-isopropyl-6-[({4'-[(methoxycarbonyl)methyl]-1,1'-biphenyl-4-yl}oxy)methyl]benzoate as a yellow oil (432 mg, five steps total yield: 5%).
$^1$H-NMR (400MHz, $CDCl_3$) : δ 11.78 (1H, s), 7.52 (4H, d, J = 8.2 Hz), 7.34 (1H, d, J = 8.2 Hz), 7.33 (2H, d, J = 8.2 Hz), 7.08 (1H, d, J = 8.2 Hz), 6.99 (2H, d, J = 8.2 Hz), 5.31 (2H, s), 3.71 (2H, s), 3.55 (3H, s), 3.38 (1H, sp, J = 7.0 Hz), 1.58 (9H, s), 1.24 (6H, d, J = 7.0 Hz).

(18-3)

The procedures were carried out similarly to Reference Example (13-4) using tert-butyl 2-hydroxy-3-isopropyl-6-[({4'-[(methoxycarbonyl)methyl]-1,1'-biphenyl-4-yl}oxy)methyl]benzoate (432 mg, 0.880 mmol) obtained in Reference Example (18-2) to obtain the title compound as a white powder (176 mg, yield: 42%).
$^1$H-NMR (400MHz, $CDCl_3$) : δ 11.80 (1H, s), 7.54 (2H, d, J = 8.2 Hz), 7.52 (2H, d, J = 8 .2 Hz), 7.36 (2H, d, J = 8.2 Hz) , 7.34 (1H, d, J = 8.2 Hz) , 7.08 (1H, d, J = 8.2 Hz), 6.99 (2H, d, J = 8.2 Hz), 5.31 (2H, s), 3.71 (2H, s), 3.38 (1H, sp, J = 6.7 Hz), 1.58 (9H, s), 1.24 (6H, d, J = 6.7 Hz).
MS (FAB) (m/z) : 476 ([M]$^+$).

(Reference Example 19)

(4'-{[2-(tert-Butoxycarbonyl)-4-fluoro-3-hydroxybenzyl]oxy}-1,1'-biphenyl-4-yl)acetic acid

(19-1)

Paraformaldehyde (3.86 g, 133 mmol), magnesium chloride (6.32 g, 66.5 mmol) and triethylamine (11.6 ml, 83.3 mmol) were added to a solution of 2-fluoro-5-methylphenol (4.19 g, 33.3 mmol) in acetonitrile (100 ml), and the mixture was vigorously stirred at 90°C for 10 days. The reaction solution was poured into 1N-hydrochloric acid and the mixture was extracted with ethyl acetate. The organic layer was successively washed with 1N-hydrochloric acid, water and saturated aqueous NaCl solution, and dried over anhydrous sodium sulfate, followed by concentration under reduced pressure. The thus obtained residue was subjected to silica gel column chromatography (elution solvent: hexane/ethyl acetate=4/1) to obtain crude 3-fluoro-2-hydroxy-6-methylbenzaldehyde. The procedures were carried out similarly to Reference Example (21-3) and Reference Example (21-4) using the obtained crude product to obtain tert-butyl 2-[(tert-butoxycarbonyl)oxy]-3-fluoro-6-methylbenzoate (624 mg, three steps total yield: 6%). $^1$H-NMR (500MHz, $CDCl_3$): δ 7.11-6.96 (2H, m), 2.33 (3H, s), 1.58 (9H, s) , 1.54 (9H, s).

(19-2)

The procedures were carried out similarly to Reference Example (21-5), Reference Example (16-4), Reference Example (16-5) and Reference Example (13-4) using tert-butyl 2-[(tert-butoxycarbonyl)oxy]-3-fluoro-6-methylbenzoate (624 mg, 1.91 mmol) obtained in Reference Example (19-1) to obtain the title compound as a colorless powder (93 mg, four steps total yield: 34%).

In the above step corresponding to Reference Example (16-4), methyl (4'-hydroxy-1,1'-biphenyl-4-yl)acetate obtained in Reference Example (9-4) was used as the phenol derivative.

$^1$H-NMR (400MHz, $CDCl_3$) : δ 7.59-7.46 (4H, m), 7.33 (2H, d, J = 7.8 Hz), 7.24-7.19 (1H, app t J = 9.2 Hz), 7.08 (1H, dd, J = 8.4, 4.5 Hz), 6.98 (2H, d, J = 8.2 Hz), 5.28 (2H, s), 3.68 (2H, s), 1.60 (9H, s).

MS (ESI) (m/z) : 451 ( $[M-H]^+$) .

(Reference Example 20)

(4'-{[2-(tert-Butoxycarbonyl)-4-chloro-3-hydroxybenzyl]oxy}-1,1'-biphenyl-4-yl)acetic acid

(20-1)

Paraformaldehyde (4.73 g, 163 mmol), magnesium chloride (7.76 g, 81.7 mmol) and triethylamine (14.2 ml, 102 mmol) were added to a solution of 2-chloro-5-methylphenol (5.80 g, 40.8 mmol) in acetonitrile (100 ml), and the mixture was vigorously stirred at 90˚C for 10 hours. The reaction mixture was poured into 1N-hydrochloric acid, and the mixture was extracted with ethyl acetate. The organic layer was successively washed with 1N-hydrochloric acid, water and saturated aqueous NaCl solution, and dried over anhydrous sodium sulfate, followed by concentration under reduced pressure. The thus obtained residue was subjected to silica gel column chromatography (elution solvent: hexane/ethyl acetate=3/1) to obtain crude 3-chloro-2-hydroxy-6-methylbenzaldehyde. The procedures were carried out similarly to Reference Example (21-3) and Reference Example (21-4) using the obtained crude product to obtain tert-butyl 2-[(tert-butoxycarbonyl)oxy]-3-chloro-6-methylbenzoate (1.82 g, three steps total yield: 13%). $^1$H-NMR (400MHz, $CDCl_3$): δ 7.34-7.31 (1H, m), 7.04-7.01 (1H, m), 2.35 (3H, s), 1.59 (9H, s), 1.56 (9H, s).

(20-2)

The procedures were carried out similarly to Reference Example (21-5), Reference Example (16-4), Reference Example (16-5) and Reference Example (13-4) using tert-butyl 2-[(tert-butoxycarbonyl)oxy]-3-chloro-6-methylbenzoate (1.82 g, 5.32 mmol) obtained in Reference Example (20-1) to obtain the title compound as a white powder (30 mg, four steps total yield: 1%).

In the step corresponding to Reference Example (16-4), methyl (4'-hydroxy-1,1'-biphenyl-4-yl)acetate obtained in Reference Example (9-4) was used as the phenol derivative.

$^1$H-NMR (400MHz, $CDCl_3$) : δ 7.52-7.46 (5H, m) , 7.33 (2H, d, J = 8.6 Hz), 7.10 (1H, d, J = 8.6 Hz), 6.95 (2H, d, J = 8.6 Hz), 5.30 (2H, s), 3.68 (2H, s), 1.61 (9H, s).

MS (ESI) (m/z) : 467 ( $[M-H]^+$) .

(Reference Example 21)

[5-(4-{[2-(tert-Butoxycarbonyl)-3-hydroxy-4-(trifluoromethyl)benzyl]oxy}phenyl)-2-thienyl]acetic acid

(21-1)

n-Butyllithium-1.59M tetrahydrofuran solution (40.0 ml, 64.4 mmol) was added dropwise to a solution of 1-(dimethoxymethyl)-2-(methoxymethoxy)-3-(trifluoromethyl)benzene (12.0 g, 42.9 mmol) obtained in Reference Example (5-2) and N,N,N',N'-tetramethylethylenediamine (9.70 ml, 64.4 mmol) in tetrahydrofuran (100 ml) at -40˚C over 5 minutes. The reaction mixture was stirred at 0˚C for 15 minutes. After the reaction mixture was cooled to -40˚C, methyl iodide (5.3 ml, 85.85 mmol) was added thereto, and the mixture was further stirred at room temperature for 30 minutes. Saturated aqueous ammonium chloride solution was poured into the reaction mixture, and the mixture was extracted with ethyl acetate. The organic layer was successively washed with water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=5/1) to obtain oily 2-(dimethoxymethyl)-3-(methoxymethoxy)-1-methyl-4-(trifluoromethyl)benzene (7.19 g, yield: 57%).

$^1$H-NMR (400MHz, $CDCl_3$) : δ 7.44 (1H, d, J = 8.2 Hz), 7.04 (1H, d, J = 8.2 Hz), 5.70 (1H, s), 4.99 (2H, s), 3.64 (3H, s),

3.43 (6H, s), 2.55 (3H, s).

(21-2)

p-Toluenesulfonic acid monohydrate (5.10 g, 26.9 mmol) was added to a solution of 2-(dimethoxymethyl)-3-(methoxymethoxy)-1-methyl-4-(trifluoromethyl)benzene (7.19 g, 24.4 mmol) obtained in Reference Example (21-1) in acetone (100 ml), and the mixture was stirred at 50˚C for 1 hour. The reaction mixture was poured into water and, after the mixture was extracted (twice) with ethyl acetate, the organic layer was successively washed with water and saturated aqueous NaCl solution, and dried over anhydrous sodium sulfate. The solvent was evaporated under reduced pressure to obtain the residue. The thus obtained residue was purified by silica gel column chromatography (elution solvent: n-hexane/ethyl acetate=5/1) to obtain 2-hydroxy-6-methyl-3-(trifluoromethyl)benzaldehyde (4.65 g, yield: 93%).
$^1$H-NMR (400MHz, $CDCl_3$): δ 12.58 (1H, s), 10.32 (1H, s), 7.65 (1H, d, J = 7.8 Hz), 6.79 (1H, d, J = 7.8 Hz), 2.67 (3H, s).

(21-3)

A solution of sodium chlorite (6.0 g, 66.3 mmol) and sodium dihydrogenphosphate monohydrate (6.0 g, 43.5 mmol) in water (40 ml) was added dropwise to a mixed solution of 2-hydroxy-6-methyl-3-(trifluoromethyl)benzaldehyde (4.65 g, 22.8 mmol) obtained in Reference Example (21-2) in tert-butyl alcohol (90 ml), 1,4-dioxane (30 ml) and 2-methyl-2-butene (30 ml), and the mixture was stirred at room temperature for 1 hour. The reaction mixture was cooled with ice and, after 5% aqueous sodium thiosulfate solution was added thereto, the mixture was poured into 0.5N-hydrochloric acid, and the mixture was extracted with ethyl acetate (twice). The organic layer was successively washed with water and saturated aqueous NaCl solution, and dried over anhydrous sodium sulfate. The residue obtained by evaporating the solvent under reduced pressure was crystallized using ethyl acetate and n-hexane to obtain colorless 2-hydroxy-6-methyl-3-(trifluoromethyl)benzoic acid (4.21 g, yield: 84%).
$^1$H-NMR (400MHz, $CDCl_3$): δ 11.73 (1H, s), 7.63 (1H, d, J = 7.8 Hz), 6.84 (1H, d, J = 7.8 Hz), 2.67 (3H, s).

(21-4)

N,N-Dimethylaminopyridine (0.7 g, 5.7 mmol) and di-tert-butyl dicarbonate [$(tBuOCO)_2O$] (16.7 g, 76.5 mmol) were added to a mixed solution of 2-hydroxy-6-methyl-3-(trifluoromethyl)benzoic acid (4.21 g, 19.1 mmol) obtained in Reference Example (21-3) in tert-butyl alcohol-tetrahydrofuran (2:1, 60 ml), and the mixture was stirred at 60˚C for 3 hours. The reaction mixture was concentrated under reduced pressure, and the residue was purified by silica gel column chromatography (elution solvent: n-hexane/ethyl acetate=9/1) to obtain tert-butyl 2-[(tert-butoxycarbonyl)oxy]-6-methyl-3-(trifluoromethyl)benzoate (6.27 g, yield: 87%).
$^1$H-NMR (400MHz, $CDCl_3$): δ 7.53 (1H, d, J = 7.8 Hz), 7.17 (1H, d, J = 7.8 Hz), 2.43 (3H, s), 1.59 (9H, s), 1.53 (9H, s) .

(21-5)

N-Bromosuccinimide (9.70 g, 54.5 mmol) and benzoyl peroxide (0.7 g) were added to a solution of tert-butyl 2-[(tert-butoxycarbonyl)oxy]-6-methyl-3-(trifluoromethyl)benzoate (18.6 g, 49.6 mmol) obtained in Reference Example (21-4) in carbon tetrachloride (400 ml), and the mixture was heated under reflux for 5 hours. The temperature of the reaction mixture was returned to room temperature, and the solvent was evaporated under reduced pressure. After n-hexane was added to the thus obtained residue and the mixture was filtered, the filtrate was concentrated under reduced pressure. The obtained residue was purified by silica gel column chromatography (elution solvent: toluene) to obtain tert-butyl 6-(bromomethyl)-2-[(tert-butoxycarbonyl)oxy]-3-(trifluoromethyl)benzoate (11.66 g, yield: 52%).
$^1$H-NMR (400MHz, $CDCl_3$): δ 7.64 (1H, d, J = 8.2 Hz), 7.40 (1H, d, J = 8.2 Hz), 4.60 (2H, s), 1.63 (9H, s), 1.52 (9H, s).

(21-6)

Tris(dibenzylideneacetone)dipalladium (0) (110 mg, 0.12 mmol), tri-o-tolylphosphine (61 mg, 0.2 mmol) and 2N-aqueous sodium carbonate solution (4 ml) were added to a mixed solution of 4-(4,4,5,5-tetramethyl-1,3,2-dioxaborolan-2-yl)phenol (0.8 g, 3.65 mmol) and ethyl (5-bromo-2-thienyl)acetate (1.00g, 4.01 mmol) synthesized according to a method described in the literature (Jackson, P. M. et al., J. Chem. Soc. Perkin Trans. 1., 1990, vol. 11, pp. 2909-2918) in toluene-ethanol (5:1, 24 ml), and the mixture was stirred at 80˚C for 3 hours. Water was poured into the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=3/1) to obtain ethyl [5-(4-hydroxyphenyl)-2-thienyl]acetate (0.73 g, yield: 77%).

$^1$H-NMR (400MHz, $CDCl_3$): δ 7.41 (2H, d, J = 8.6 Hz), 7.00 (1H, d, J = 3.5 Hz), 6.85 (1H, d, J = 3.5 Hz), 6.80 (2H, d, J = 8.6 Hz), 4.89 (1H, s), 4.19 (2H, q, J = 7.0 Hz), 3.80 (2H, s), 1.29 (3H, t, J = 7.0 Hz).

(21-7)

The procedures were carried out similarly to Reference Example (16-4), Reference Example (16-5) and Reference Example (9-10) using tert-butyl 6-(bromomethyl)-2-[(tert-butoxycarbonyl)oxy]-3-(trifluoromethyl)benzoate (366 mg, 0.8 mmol) obtained in Reference Example (21-5) and ethyl [5-(4-hydroxyphenyl)-2-thienyl]acetate (211 mg, 0.8 mmol) obtained in Reference Example (21-6) to obtain the title compound as a colorless powder (56 mg, three steps total yield: 14%).

In the above step corresponding to Reference Example (16-4), potassium carbonate was used instead of cesium carbonate as the base.

$^1$H-NMR (400MHz, DMSO-$d_6$) : δ 12.58 (1H, s) , 11.44 (1H, s), 7.82 (1H, d, J = 7.8 Hz) , 7.56 (2H, d, J = 7.8 Hz) , 7.29 (1H, d, J = 7.8 Hz), 7.23 (1H, d, J = 3.5 Hz), 7.02 (2H, d, J = 7.8 Hz), 6.91 (1H, d, J = 3.5 Hz), 5.35 (2H, s), 3.81 (2H, s), 1.56 (9H, s).

MS (ESI) (m/z): 507 ($[M-H]^+$).

(Reference Example 22)

(4'-{[2-(tert-Butoxycarbonyl)-3-hydroxy-4-(trifluoromethyl)benzyl]oxy}-2-nitro-1,1'-biphenyl-4-yl)acetic acid

(22-1)

The procedures were carried out similarly to Reference Example (9-2), Reference Example (21-5), Reference Example (23-1) and Reference Example (15-4) using 4-methoxyphenylboric acid (17.0 g, 112 mmol) and 4-bromo-2-nitrotoluene (22.1 g, 102 mmol) to obtain methyl (4'-hydroxy-2-nitro-1,1'-biphenyl-4-yl)acetate (3.53 g, four steps total yield: 12%).

$^1$H-NMR (500MHz, $CDCl_3$) δ 7.73-7.69 (1H, m), 7.52-7.47 (1H, m), 7.41-7.36 (1H, m), 7.18-7.10 (2H, m), 6.94-6.86 (2H, m), 3.80-3.67 (5H, m).

(22-2)

The procedures were carried out similarly to Reference Example (16-4), Reference Example (16-5) and Reference Example (9-10) using methyl (4'-hydroxy-2-nitro-1,1'-biphenyl-4-yl)acetate (1.71 g, 3.76 mmol) obtained in Reference Example (22-1) and tert-butyl 6-(bromomethyl)-2-[(tert-butoxycarbonyl)oxy]-3-(trifluoromethyl)benzoate (1.08 g, 3.76 mmol) obtained in Reference Example (21-5) to obtain the yellow oily title compound (138 mg, three steps total yield: 7%).

$^1$H-NMR (400MHz, $CDCl_3$): δ 7.75 (1H, d, J = 1.2 Hz), 7.69 (1H, d, J = 8.0 Hz), 7.50 (1H, dd, J = 8.0, 1.2 Hz), 7.38 (1H, d, J = 8.0 Hz), 7.26-7.19 (3H, m), 6.94 (2H, d, J = 8.0 Hz), 5.35 (2H, s), 3.76 (2H, s), 1.63 (9H, s).

MS (FAB) (m/z) : 547 ( $[M]^+$).

(Reference Example 23)

(4'-{[2-(tert-Butoxycarbonyl)-3-hydroxy-4-(trifluoromethyl) benzyl] oxy}-3-fluoro-1,1'-biphenyl-4-yl)acetic acid

(23-1)

Potassium cyanide (1.3 g, 20 mmol) was added to a mixed solution of 4-bromo-2-fluorobenzyl bromide (5.00 g, 18.7 mmol) in ethanol-water (3:1, 40 ml), and the mixture was stirred at 60˚C for 2 hours. The reaction mixture was poured into water and, after the mixture was extracted with ethyl acetate, the organic layer was washed with saturated aqueous NaCl solution, and dried over anhydrous sodium sulfate. The residue obtained by evaporating the solvent under reduced pressure was purified by silica gel column chromatography (elution solvent: n-hexane/ethyl acetate) to obtain to obtain (4-bromo-2-fluorophenyl)acetonitrile as a colorless solid (3.75 g, yield: 94%).

$^1$H-NMR (400MHz, $CDCl_3$) δ 7.35-7.26 (3H, m) , 3.72 (2H, s).

(23-2)

Tetrakis(triphenylphosphine)palladium (0) (317 mg, 0.274 mmol) and potassium carbonate (4.5 g, 32.6 mmol)

were added to a mixed solution of (4-bromo-2-fluorophenyl)acetonitrile (3.0 g, 14 mmol) obtained in Reference Example (23-1) and 4-(4,4,5,5-tetramethyl-1,3,2-dioxaborolan-2-yl)phenol (3.45 g, 14 mmol) in N,N-dimethylacetamide-water (20:1, 31.5 ml), and the mixture was stirred at 100˚C for 2 hours. The reaction mixture was poured into 0.2N hydrochloric acid and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=2/1) to obtain (3-fluoro-4'-hydroxy-1,1'-biphenyl-4-yl)acetonitrile as a colorless solid (2.9 g, yield: 91%).
$^1$H-NMR (400MHz, MeOH-$d_4$): δ 7.50-7.34 (5H, m), 6.87 (2H, d, J = 8.8 Hz), 3.92 (2H, s).

(23-3)

Acetic acid (10 ml) and concentrated hydrochloric acid (10 ml) were added to (3-fluoro-4'-hydroxy-1,1'-biphenyl-4-yl)acetonitrile (2.4 g, 11 mmol) obtained in Reference Example (23-2), and the mixture was stirred at 110˚C for 1 hour. The reaction mixture was poured into water and, after the mixture was extracted with ethyl acetate, the organic layer was successively washed with water and saturated aqueous NaCl solution, and dried over anhydrous sodium sulfate. Allyl alcohol (20 ml) and concentrated sulfuric acid (1.5 ml) were successively added to the residue obtained by evaporating the solvent from the above organic layer under reduced pressure, and the mixture was stirred at room temperature for 1 hour. The reaction mixture was poured into water and, after the mixture was extracted (three times) with ethyl acetate, the organic layer was successively washed with water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=5/1-2/1) to obtain allyl (3-fluoro-4'-hydroxy-1,1'-biphenyl-4-yl)acetate as a colorless solid (2.0 g, two steps total yield: 66%).
$^1$H-NMR (400MHz, $CDCl_3$) : δ 7.42 (2H, d, J = 8.4 Hz), 7.31-7.21 (3H, m), 6.86 (2H, d, J = 8.4 Hz), 5.97-5.88 (1H, m), 5.32 (1H, app d, J = 16.4 Hz) , 5.24 (1H, app d, J = 10.4 Hz), 4.96 (1H, br s), 4.64 (2H, app d, J = 6.0 Hz), 3.73 (2H, s).

(23-4)

The procedures were carried out similarly to Reference Example (13-1) using allyl (3-fluoro-4'-hydroxy-1,1'-biphenyl-4-yl)acetate (3.10 g, 10.9 mmol) obtained in Reference Example (23-3) and [2-(dimethoxymethyl)-3-(methoxymethoxy)-4-(trifluoromethyl)phenyl]methanol (3.70 g, 12.0 mmol) obtained in Reference Example (5-4) to obtain pale yellow oily allyl (4'-{[2-(dimethoxymethyl)-3-(methoxymethoxy)-4-(trifluoromethyl)benzyl]oxy}-3-fluoro-1,1'-biphenyl-4-yl)acetate (3.89 g, yield: 61%).
$^1$H-NMR (400MHz, $CDCl_3$) : δ 7.57-7.56 (2H, m) , 7.47 (2H, d, J = 8.4 Hz), 7.29-7.22 (3H, m), 7.03 (2H, d, J = 8.4 Hz) , 5.97-5.87 (1H, m), 5.77 (1H, s), 5.53 (2H, s), 5.30 (1H, app d, J = 17.2 Hz), 5.23 (1H, app d, J = 10.4 Hz), 5.04 (2H, s), 4.63 (2H, app d, J = 5.6 Hz), 3.72 (2H, s), 3.67 (3H, s), 3.49 (6H, s).

(23-5)

The procedures were carried out similarly to Reference Example (13-2) using allyl (4'-{[2-(dimethoxymethyl)-3-(methoxymethoxy)-4-(trifluoromethyl)benzyl]oxy}-3-fluoro-1,1'-biphenyl-4-yl)acetate (3.89 g, 6.7 mmol) obtained in Reference Example (23-4) to obtain allyl (4'-{[3-(allyloxy)-2-formyl-4-(trifluoromethyl)benzyl]oxy}-3-fluoro-1,1'-biphenyl-4-yl)acetate as a pale yellow solid (2.52 g, yield: 71%).
$^1$H-NMR (500MHz, $CDCl_3$) : δ 10.55 (1H, s), 7.88 (1H, d, J = 8.5 Hz), 7.78 (1H, d, J = 8.5 Hz), 7.52 (2H, d, J = 8.5 Hz), 7.33-7.25 (3H, m), 7.06 (2H, d, J = 8.5 Hz), 6.16-6.08 (1H, m) , 5.96-5.89 (1H, m), 5.52 (2H, s), 5.49 (1H, dd, J = 17.5, 1.5 Hz), 5.38 (1H, dd, J = 10.0, 1.0 Hz), 5.31 (1H, dd, J = 17.0, 1.5 Hz), 5.24 (1H, dd, J = 10.0, 1.0 Hz), 4.64 (2H, app d, J = 5.5 Hz), 4.60 (2H, app d, J =5.5 Hz), 3.73 (2H, s).

(23-6)

A solution of sodium chlorite (2.6 g, 29 mmol) and sodium dihydrogenphosphate monohydrate (2.6 g, 19 mmol) in water (22 ml) were added dropwise to a mixed solution of allyl (4'-{[3-(allyloxy)-2-formyl-4-(trifluoromethyl)benzyl]oxy}-3-fluoro-1,1'-biphenyl-4-yl)acetate (2.52 g, 4.8 mmol) obtained in Reference Example (23-5) in tert-butyl alcohol (51 ml), 1,4-dioxane (17 ml) and 2-methyl-2-butene (17 ml), and the mixture was stirred at room temperature for 90 minutes. After 5% aqueous sodium thiosulfate solution was added to the reaction mixture, the mixture was poured into 1N-hydrochloric acid, and the mixture was extracted (twice) with ethyl acetate. The organic layer was successively washed with water and saturated aqueous NaCl solution, and dried over anhydrous sodium sulfate. The solvent was evaporated under reduced pressure to obtain the residue. Methylene chloride (50 ml), 2-methyl-1-propene (150 ml) and sulfuric acid (1 ml) were successively added to the obtained residue, and the mixture was stirred at room temperature

overnight. The reaction mixture was poured into 5% aqueous sodium hydrogencarbonate solution and, after the mixture was extracted (twice) with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=10/1-3/1) to obtain tert-butyl 2-(allyloxy)-6-[({4'-[(allyloxycarbonyl)methyl]-3'-fluoro-1,1'-biphenyl-4-yl}oxy)methyl]-3-(trifluoromethyl)benzoate as a colorless powder (2.38 g, yield: 83%).
$^1$H-NMR (500MHz, $CDCl_3$): δ 7.64 (1H, d, J =8.5 Hz), 7.50 (2H, d, J = 8.5 Hz), 7.39 (1H, d, J = 8.5 Hz), 7.31-7.23 (3H, m), 7.00 (2H, d, J = 8.5 Hz), 6.11-6.03 (1H, m), 5.96-5.88 (1H, m), 5.43 (1H, dd, J = 17.5, 1.5 Hz), 5.31 (1H, app d, J = 17.0 Hz), 5.28 (1H, app d, J = 10.0 Hz), 5.24 (1H, app d, J = 10.0 Hz), 5.16 (2H, s), 4.64 (2H, app d, J = 6.0 Hz) , 4.58 (2H, app d, J =5.5 Hz), 3.73 (2H, s), 1.58 (9H, s).

(23-7)

Morpholine (0.27 ml, 3.3 mmol) and tetrakis(triphenylphosphine)palladium (0) (57 mg, 0.049 mmol) were successively added to a solution of tert-butyl 2-(allyloxy)-6-[({4'-[(allyloxycarbonyl)methyl]-3'-fluoro-1,1'-biphenyl-4-yl}oxy) methyl]-3-(trifluoromethyl)benzoate (790 mg, 1.32 mmol) obtained in Reference Example (23-6) in tetrahydrofuran (8 ml), and the mixture was stirred at room temperature for 1 hour. Water was poured into the reaction mixture and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=3/1-0/1) and crystallized from a mixed solvent of methylene chloride-ethyl acetate to obtain the title compound as a colorless powder (323 mg, yield: 47%).
$^1$H-NMR (500MHz, $CDCl_3$) : δ 12.26 (1H, s) , 7.71 (1H, d, J = 8.5 Hz) , 7.52 (2H, d, J = 8.5 Hz), 7.32-7.26 (4H, m) , 6.98 (2H, d, J = 8.5 Hz), 5.38 (2H, s), 3.76 (2H, s), 1.65 (9H, s) .
MS (ESI) (m/z): 519 ($[M-H]^+$).

(Reference Example 24)

(4'-{[2-(tert-Butoxycarbonyl)-3-hydroxy-4-(trifluoromethyl)benzyl]oxy}-2-methyl-1,1'-biphenyl-4-yl)acetic acid

(24-1)

The procedures were carried out similarly to the methods of Reference Example (23-1) and Reference Example (23-2) using 1-bromo-4-(bromomethyl)-2-methylbenzene synthesized according to a method described in the literature (Hanessian, S. et al., J. Org. Chem., 2003, vol. 68, pp. 7204-7218) as the starting material to obtain (4'-hydroxy-2-methyl-1,1'-biphenyl-4-yl)acetonitrile as a pale yellow solid (969 mg, yield: 76%).
$^1$H-NMR (400MHz, $CDCl_3$) : δ 7.23-7.16 (5H, m), 6.89 (2H, d, J = 8.4 Hz), 4.86 (1H, br s), 3.75 (2H, s), 2.28 (3H, s).

(24-2)

The procedures were carried out similarly to Reference Example (23-3) using (4'-hydroxy-2-methyl-1,1'-biphenyl-4-yl)acetonitrile (969 mg, 4.35 mmol) obtained in Reference Example (24-1) to obtain pale yellow oily allyl (4'-hydroxy-2-methyl-1,1'-biphenyl-4-yl)acetate (1.19 g, yield: 97%).
$^1$H-NMR (400MHz, $CDCl_3$): δ 7.20-7.13 (5H, m), 6.86 (2H, d, J = 8.8 Hz), 5.98-5.89 (1H, m), 5.32 (1H, app dd, J = 17.2, 1.6 Hz), 5.24 (1H, app dd, J = 10.4, 1.6 Hz), 4.84 (1H, br s), 4.63 (2H, app d, J = 5.6 Hz), 3.65 (2H, s), 2.25 (3H, s).

(24-3)

The procedures were carried out similarly to the methods of Reference Example (13-1), Reference Example (13-2), Reference Example (21-3) and Reference Example (21-4) using allyl (4'-hydroxy-2-methyl-1,1'-biphenyl-4-yl) acetate (1.20 g, 4.3 mmol) obtained in Reference Example (24-2) and [2-(dimethoxymethyl)-3-(methoxymethoxy)-4-(trifluoromethyl)phenyl]methanol (1.84 g, 6.0 mmol) obtained in Reference Example (5-4) as the starting materials to obtain colorless oily tert-butyl 2-(allyloxy)-6-[({4'-[(allyloxycarbonyl)methyl]-2'-methyl-1,1'-biphenyl-4-yl}oxy)methyl] -3- (trifluoromethyl)benzoate (1.26 g, four steps total yield: 46%).
$^1$H-NMR (400MHz, $CDCl_3$): δ 7.66 (1H, d, J = 8.4 Hz), 7.42 (1H, d, J = 8.4 Hz), 7.23 (2H, d, J = 8.4 Hz), 7.18-7.13 (3H, m), 6.97 (2H, d, J = 8.4 Hz), 6.12-6.02 (1H, m), 5.98-5.89 (1H, m), 5.43 (1H, dd, J = 16.8, 1.2 Hz), 5.34-5.23 (3H, m), 5.16 (2H, s), 4.63 (2H, app d, J = 5.6 Hz), 4.58 (2H, app d, J = 5.6 Hz), 3.65 (2H, s), 2.25 (3H, s), 1.58 (9H, s).

(24-4)

The procedures were carried out similarly to Reference Example (6-2) using tert-butyl 2-(allyloxy)-6-[({4'-[(allyloxycarbonyl)methyl]-2'-methyl-1,1'-biphenyl-4-yl}oxy)methyl]-3-(trifluoromethyl)benzoate(1.26 g, 2.1 mmol) obtained in Reference Example (24-3) to obtain the title compound as a colorless powder (652 mg, yield: 60%). $^1$H-NMR (400MHz, $CDCl_3$): δ 12.27, (1H, s), 7.72 (1H, d, J = 8.0 Hz), 7.30 (1H, d, J = 8.0 Hz), 7.26-7.23 (2H, m), 7.20-7.15 (3H, m), 6.95 (2H, d, J = 8.8 Hz), 5.38 (2H, s), 3.68 (2H, s), 2.27 (3H, s), 1.65 (9H, s).
MS (FAB) (m/z): 516 ($[M]^+$).

(Reference Example 25)

(4'-{[2-(tert-Butoxycarbonyl)-3-hydroxy-4-(trifluoromethyl)benzyl]oxy}-2-methoxy-1,1'-biphenyl-4-yl)acetic acid

(25-1)

The procedures were carried out similarly to Reference Example (13-1) using [2-(dimethoxymethyl)-3-(methoxymethoxy)-4-(trifluoromethyl)phenyl]methanol (3.00 g, 9.66 mmol) obtained in Reference Example (5-4) and 4-bromophenol (2.00 g, 11.6 mmol) to obtain 1-[(4-bromophenoxy)methyl]-2-(dimethoxymethyl)-3-(methoxymethoxy)-4-(trifluoromethyl)benzene (3.46 g, yield: 77%).
$^1$H-NMR (400MHz, $CDCl_3$): δ 7.56 (1H, d, J = 8.6 Hz), 7.49 (1H, d, J = 8.6 Hz), 7.35 (2H, d, J = 9.0 Hz), 6.86 (2H, d, J = 9.0 Hz), 5.75 (1H, s), 5.46 (2H, s), 5.03 (2H, s), 3.66 (3H, s), 3.47 (6H, s).

(25-2)

The procedures were carried out similarly to Reference Example (21-2) and Reference Example (21-3) using 1-[(4-bromophenoxy)methyl]-2-(dimethoxymethyl)-3-(methoxymethoxy)-4-(trifluoromethyl)benzene (17.6 g, 56.0 mmol) obtained in Reference Example (25-1) to obtain 6-[(4-bromophenoxy)methyl]-2-hydroxyl-3-(trifluoromethyl)benzoic acid (12.0 g, two steps total yield: 55%).
$^1$H-NMR (400MHz, $CDCl_3$): δ 12.24 (1H, S) , 7.77 (1H, d, J = 8.0 Hz), 7.40 (2H, d, J = 9.0 Hz), 7.29 (1H, d, J = 8.0 Hz), 6.80 (2H, d, J = 9.0 Hz), 5.38 (2H, s).

(25-3)

The procedures were carried out similarly to Reference Example (21-4) and Reference Example (16-5) using 6-[(4-bromophenoxy)methyl]-2-hydroxyl-3-(trifluoromethyl)benzoic acid (3.22 g, 8.23 mmol) obtained in Reference Example (25-2) to obtain tert-butyl 6-[(4-bromophenoxy)methyl]-2-hydroxy-3-(trifluoromethyl)benzoate (2.26 g, two steps total yield: 61%).
$^1$H-NMR (400MHz, $CDCl_3$) δ 12.24 (1H, s), 7.69 (1H, d, J = 8.2 Hz), 7.40 (2H, d, J = 9.0 Hz), 7.21 (1H, d, J = 8.2 Hz), 6.80 (2H, d, J = 9.0 Hz), 5.30 (2H, s), 1.62 (9H, s).

(25-4)

4,4,4',4',5,5,5',5'-Octamethyl-2,2'-bi-1,3,2-dioxaborolane [bis(pinacolate)diboron; 7.36 g, 29.0 mmol], [1,1'-bis (diphenylphosphino)ferrocene]dichloropalladium (II)-dichloromethane adduct (1.00 g, 1.36 mmol) and potassium acetate (7.41 g, 75.6 mmol) were added to a solution of tert-butyl 6-[(4-bromophenoxy)methyl]-2-hydroxy-3-(trifluoromethyl) benzoate (10.2 g, 22.8 mmol) obtained in Reference Example (25-3) in dimethyl sulfoxide (200 ml), and the mixture was stirred at 70˚C for 3.5 hours. The reaction mixture was poured into water and, after the mixture was extracted with ethyl acetate, the organic layer was successively washed with water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=5/1-1/1) to obtain tert-butyl 2-hydroxy-{[4-(4,4,5,5-tetramethyl-1,3,2-dioxaborolan-2-yl)phenoxy]methyl}-3-(trifluoromethyl)benzoate (11.3 g, yield: 99%).
$^1$H-NMR (400MHz, $CDCl_3$): δ 12.27 (1H, s), 7.77 (2H, d, J = 8.8 Hz), 7.69 (1H, d, J = 8.4 Hz), 7.25 (1H, d, J = 8.4 Hz), 6.92 (2H, d, J = 8.8 Hz), 5.34 (2H, s), 1.33 (9H, s), 1.26 (12H, s).

(25-5)

Pyridine (2.10 ml, 25.9 mmol), trifluoromethanesulfonic acid anhydride (1.61 ml, 9.53 mmol) and 4-dimethylaminopyridine (30 mg, 0.25 mmol) were added to a solution of methyl (4-hydroxy-3-methoxyphenyl)acetate (1.70 g,

8.66 mmol) in methylene chloride (20 ml), and the mixture was stirred under ice-cooling for 10 minutes and at room temperature for 20 minutes. The reaction mixture was poured into water and, after the mixture was extracted with ethyl acetate, the organic layer was successively washed with water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=2/1) to obtain methyl (3-methoxy-4-{[(trifluoromethyl)sulfonyl]oxy}phenyl)acetate (2.84 g, yield: 99%).
$^1$H-NMR (400MHz, $CDCl_3$): δ 7.14 (1H, d, J = 8.2 Hz), 6.96 (1H, s), 6.86 (1H, d, J = 8.2 Hz), 3.90 (3H, s), 3.71 (3H, s), 3.62 (2H, s).

(25-6)

The procedures were carried out similarly to Reference Example (12-2) and Reference Example (9-10) using tert-butyl 2-hydroxy-{[4-(4,4,5,5-tetramethyl-1,3,2-dioxaborolan-2-yl) phenoxy] methyl}-3-(trifluoromethyl)benzoate (100 mg, 0.20 mmol) obtained in Reference Example (25-4) and methyl (3-methoxy-4-{[(trifluoromethyl)sulfonyl]oxy}phenyl) acetate (79 mg, 0.24 mmol) obtained in Reference Example (25-5) to obtain the title compound as a pale yellow powder (43 mg, yield: 40%). $^1$H-NMR (400MHz, DMSO-$d_6$) : δ 8.78 (1H, s), 7.79 (1H, d, J = 8.4 Hz), 7.39 (2H, d, J = 7.2 Hz), 7.27 (1H, d, J = 8.4 Hz), 7.17 (1H, d, J = 8.0 Hz) , 6.99-6.96 (3H, m), 6.86 (1H, dd, J = 8.0, 1.2 Hz), 5.34 (2H, s), 3.72 (3H, s), 3.57 (2H, s), 1.55 (9H, s).
MS (ESI) (m/z) : 531 ( $[M-H]^+$) .

(Reference Example 26)

(4'-{[2-(tert-Butoxycarbonyl)-3-hydroxy-4-(trifluoromethyl)benzyl]oxy}-2-chloro-1,1'-biphenyl-4-yl)acetic acid

The procedures were carried out similarly to Reference Example (16-4), Reference Example (16-5) and Reference Example (9-10) using methyl (2-chloro-4'-hydroxy-1,1'-biphenyl-4-yl)acetate (150 mg, 0.54 mmol) obtained in Reference Example (12-3) and tert-butyl 6-(bromomethyl)-2-[(tert-butoxycarbonyl)oxy]-3-(trifluoromethyl)benzoate (455 mg, 1.0 mmol) obtained in Reference Example (21-5) to obtain the colorless title compound (46 mg, three steps total yield: 15%).
$^1$H-NMR (400MHz, $CDCl_3$) : δ 12.28 (1H, s), 7.72 (1H, d, J = 8.0 Hz), 7.42 (1H, d, J = 2.0 Hz), 7.39 (2H, d, J = 8.4 Hz), 7.32-7.28 (2H, m), 7.24 (1H, dd, J = 8.0, 2.0 Hz), 6.97 (2H, d, J = 8.4 Hz), 5.39 (2H, s), 3.69 (2H, s), 1.65 (9H, s).
MS (FAB) (m/z): 536 ($[M]^+$).

(Reference Example 27)

(4'-{[2-(tert-Butoxycarbonyl)-3-hydroxy-4-(trifluoromethyl)benzyl]oxy}-2-trifluoromethyl-1,1'-biphenyl-4-yl)acetic acid

(27-1)

Carbon tetrabromide (3.48 g, 10.5 mmol) and triphenylphosphine (2.75 g, 10.5 mmol) were added to a solution of [4-chloro-3-(trifluoromethyl)phenyl]methanol (2.00 g, 9.59 mmol) in tetrahydrofuran (12 ml) under ice-cooling, and the mixture was stirred at room temperature for 1 hour. Hexane was added to the reaction mixture, and the insolubles were removed by filtration. The obtained filtrate was poured into water and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=4/1-2/1) to obtain 4-(bromomethyl)-1-chloro-2-(trifluoromethyl)benzene. Potassium cyanide (687 mg, 10.5 mmol) was added to a mixed solution of the obtained 4-(bromomethyl)-1-chloro-2-(trifluoromethyl)benzene in ethanol-water (3:1, 20 ml), and the mixture was stirred at 60˚C for 2 hours. The reaction mixture was poured into water and, after the mixture was extracted with ethyl acetate, the organic layer was washed with saturated aqueous NaCl solution and dried over anhydrous sodium sulfate. The residue obtained by evaporating the solvent under reduced pressure was subjected to silica gel column chromatography (elution solvent: n-hexane/ethyl acetate=4/1-2/1) to obtain crude [4-chloro-3-(trifluoromethyl)phenyl]acetonitrile. Acetic acid (6 ml) and concentrated hydrochloric acid (6 ml) were added to the obtained crude product, and the mixture was stirred at 100˚C for 2 hours. After the temperature of the reaction mixture was returned to room temperature, the mixture was poured into water, and the mixture was extracted with ethyl acetate. The organic layer was successively washed with water and saturated aqueous NaCl solution, and dried over anhydrous sodium sulfate. The solvent was evaporated under reduced pressure to obtain crude [4-chloro-3-(trifluoromethyl)phenyl]acetic acid. Methanol (12 ml) and concentrated sulfuric acid (1.0 ml) were added to the obtained crude product, and the mixture was stirred at 50˚C for 1 hour. The temperature of

the reaction mixture was returned to room temperature, and the solvent was evaporated under reduced pressure. After ethyl acetate was added thereto, the organic layer was successively washed with water, saturated aqueous sodium hydrogencarbonate solution and saturated aqueous NaCl solution, and dried over anhydrous sodium sulfate. The residue obtained by evaporating the solvent under reduced pressure was purified by silica gel column chromatography (elution solvent: n-hexane/ethyl acetate=10/1) to obtain methyl [4-chloro-3-(trifluoromethyl)phenyl]acetate (1.08 g, two steps total yield: 45%).
$^1$H-NMR (400MHz, $CDCl_3$) : δ 7.58 (1H, d, J = 2.0 Hz), 7.45 (1H, d, J = 8.0 Hz), 7.37 (1H, dd, J = 8.0, 2.0 Hz), 3.70 (3H, s), 3.64 (2H, s).

(27-2)

Tri-potassium phosphate (127 mg, 0.60 mmol), palladium acetate (8.00 mg, 40 μmmol) and 2-(dicyclohexylphosphino)-2',6'-dimethoxy-1,1'-biphenyl (S-PHOS) (16 mg, 40 μmol) were added to a solution of methyl [4-chloro-3-(trifluoromethyl)phenyl]acetate (51 mg, 0.20 mmol) obtained in Reference Example (27-1) and tert-butyl 2-hydroxy-{[4-(4,4,5,5-tetramethyl-1,3,2-dioxaborolan-2-yl)phenoxy]methyl}-3-(trifluoromethyl)benzoate (100 mg, 0.20 mmol) obtained in Reference Example (25-4) in toluene (2.00 ml), and the mixture was stirred at 60°C for 6 hours. After the temperature of the reaction mixture was returned to room temperature, the mixture was poured into water, and the mixture was extracted with ethyl acetate. The organic layer was successively washed with water and saturated aqueous NaCl solution, and dried over anhydrous sodium sulfate. The residue obtained by evaporating the solvent under reduced pressure was subjected to silica gel column chromatography (elution solvent: n-hexane/ethyl acetate=4/1-2/1) to obtain crude(tert-butyl2-hydroxy-6-[({4'-[(methoxycarbonyl)methyl]-2'-trifluoromethyl-1,1'-biphenyl-4-yl}oxy)methyl]-3-(trifluoromethyl)benzoate (85 mg).
The procedures were carried out similarly to Reference Example (9-10) using the obtained crude product (85 mg) to obtain the title compound as a pale yellow powder (24 mg, two steps total yield: 44%).
$^1$H-NMR (400MHz, $CDCl_3$) : δ 12.27 (1H, s), 7.71 (1H, d, J = 8.0 Hz), 7.65 (1H, s), 7.48 (1H, d, J = 7.6 Hz), 7.31-7.24 (4H, m), 6.94 (2H, d, J = 8.4 Hz) , 5.36 (2H, s), 3.75 (2H, s), 1.62 (9H, s).
MS (ESI) (m/z): 569 ($[M-H]^+$).

(Reference Example 28)

1-(4'-{[2-(tert-Butoxycarbonyl)-3-hydroxy-4-(trifluoromethyl)benzyl]oxy}-1,1'-biphenyl-4-yl)cyclopropanecarboxylic acid

(28-1)

Sodium hydride (55% oily) (2.40 g, 55.0 mmol) was added to a solution of methyl (4-bromophenyl)acetate (5.73 g, 25.0 mmol) obtained in Reference Example (9-1) in N,N-dimethylformamide (50 ml) at 0°C, and the mixture was stirred at room temperature for 10 minutes. The reaction mixture was cooled to 0°C and, after 1,2-dibromoethane (2.37 ml, 27.5 mmol) was added thereto, the mixture was further stirred at room temperature for 15 hours. Saturated aqueous ammonium choride solution was poured into the reaction mixture and, after the mixture was extracted with ethyl acetate, the organic layer was successively washed with water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=9/1) to obtain oily methyl 1-(4-bromophenyl)cyclopropanecarboxylate (2.97 g, yield: 47%).
$^1$H-NMR (400MHz, $CDCl_3$) : δ 7.43 (2H, d, J = 8.2 Hz) , 7.22 (2H, d, J = 8.2 Hz) , 3.63 (3H, s), 1.63-1.59 (2H, m) , 1.18-1.14 (2H, m).

(28-2)

The procedures were carried out similarly to Reference Example (23-2) using methyl (1-(4-bromophenyl)cyclopropanecarboxylate (2.96 g, 11.6 mmol) obtained in Reference Example (28-1) and 4-(4,4,5,5-tetramethyl-1,3,2-dioxaborolan-2-yl)phenol (2.55 g, 11.6 mmol) to obtain methyl 1-(4'-hydroxy-1,1'-biphenyl-4-yl)cyclopropanecarboxylate as a white powder (2.49 g, yield: 80%).
$^1$H-NMR (400MHz, $CDCl_3$): δ 7.46 (2H, d, J = 8.2 Hz), 7.44 (2H, d, J = 8.6 Hz), 7.36 (2H, d, J = 8.2 Hz), 6.86 (2H, d, J = 8.6 Hz), 4.95 (1H, s), 3.64 (3H, s), 1.65-1.61 (2H, m), 1.24-1.20 (2H, m) .

(28-3)

The procedures were carried out similarly to Reference Example (9-10) and Reference Example (34-1) using

methyl 1-(4'-hydroxy-1,1'-biphenyl-4-yl)cyclopropanecarboxylate (2.49 g, 9.28 mmol) obtained in Reference Example (28-2) to obtain allyl 1-(4'-hydroxy-1,1'-biphenyl-4-yl)cyclopropanecarboxylate as a white powder (2.1 g, two steps total yield: 77%).

In this step, the hydrolysis step corresponding to Reference Example (9-10) was carried out at a reaction temperature of 60˚C.

$^1$H-NMR (400MHz, $CDCl_3$): δ 7.47-7.41 (4H, m), 7.37 (2H, d, J = 8.2 Hz), 6.85 (2H, d, J = 8.6 Hz), 5.88-5.77 (1H, m), 5.20-5.12 (2H, m), 5.09 (1H, s), 4.57-4.54 (2H, m), 1.67-1.63 (2H, m), 1.27-1.22 (2H, m).

(28-4)

The procedures were carried out similarly to the methods of Reference Example (13-1), Reference Example (13-2) and Reference Example (21-3) using allyl 1-(4'-hydroxy-1,1'-biphenyl-4-yl)cyclopropanecarboxylate (479 mg, 1.63 mmol) obtained in Reference Example (28-3) and [2-(dimethoxymethyl)-3-(methoxymethoxy)-4-(trifluoromethyl) phenyl]methanol (505 mg, 1.63 mmol) obtained in Reference Example (5-4) as the the starting materials to obtain 2-(allyloxy)-6-{[(4'-{1-[(allyloxy)carbonyl]cyclopropyl}-1,1'-biphenyl-4-yl)oxy]methyl}-3-(trifluoromethyl)benzoic acid (175 mg, four steps total yield: 19%).
$^1$H-NMR (400MHz, $CDCl_3$): δ 7.72 (1H, d, J = 8.2 Hz), 7.51-7.45 (3H, m), 7.43 (2H, d, J = 8.2 Hz), 7.36 (2H, d, J = 8.2 Hz), 6.98 (2H, d, J = 8.6 Hz), 6.11-6.00 (1H, m), 5.87-5.76 (1H, m), 5.45-5.38 (1H, m), 5.30-5.23 (3H, m), 5.19-5.10 (2H, m), 4.61-4.58 (2H, m), 4.56-4.53 (2H, m), 1.66-1.62 (2H, m), 1.25-1.21 (2H, m).

(28-5)

2-(Allyloxy)-6-{[(4'-{1-[(allyloxy)carbonyl]cyclopropyl}-1,1'-biphenyl-4-yl)oxy]methyl}-3-(trifluoromethyl)benzoic acid (175 mg, 0.317 mmol) obtained in Reference Example (28-4) and N,N-dimethylformamide di-tert-butyl acetal (0.300 ml, 1.27 mmol) were dissolved in toluene (2 ml), and the mixture was heated under reflux for 2 hours. The reaction mixture was concentrated under reduced pressure, and the obtained residue was purified by silica gel column chromatography (elution solvent: n-hexane/ethyl acetate=3/1) to obtain tert-butyl 2-(allyloxy)-6-{[(4'-{1-[(allyloxy)carbonyl]cyclopropyl}-1,1'-biphenyl-4-yl)oxy]methyl}-3-(trifluoromethyl)benzoate (122 mg, yield: 63%).
$^1$H-NMR (400MHz, $CDCl_3$) : δ 7.62 (1H, d, J = 8.2 Hz), 7.50 (2H, d, J = 8.6 Hz), 7.47 (2H, d, J = 8.6 Hz), 7.40-7.36 (3H, m), 6.97 (2H, d, J = 8.6 Hz), 6.11-6.00 (1H, m), 5.88-5.76 (1H, m), 5.45-5.38 (1H, m), 5.29-5.24 (1H, m), 5.19-5.11 (4H, m), 4.58-4.53 (4H, m), 1.67-1.63 (2H, m), 1.58 (9H, s), 1.26-1.21 (2H, m).

(28-6)

The procedures were carried out similarly to Reference Example (17-4) using tert-butyl 2-(allyloxy)-6-{[(4'-{1-[(allyloxy)carbonyl]cyclopropyl}-1,1'-biphenyl-4-yl)oxy]methyl}-3-(trifluoromethyl)benzoate (122mg, 0.2 mmol) obtained in Reference Example (28-5) to obtain the title compound as a pale yellow powder (63 mg, yield: 59%). $^1$H-NMR (400MHz, $CDCl_3$) : δ 12.23 (1H, s), 7.68 (1H, d, J = 8.2 Hz), 7.50 (2H, d, J = 8.6 Hz), 7.48 (2H, d, J = 8.2 Hz), 7.39 (2H, d, J = 8.2 Hz), 7.25 (1H, d, J = 8.2 Hz) , 6.95 (2H, d, J = 8.6 Hz), 5.36 (2H, s), 1.72-1.68 (2H, m), 1.64 (9H, s), 1.33-1.28 (2H, m).
MS (ESI) (m/z): 527 ($[M-H]^+$).

(Reference Example 29)

tert-Butyl 2-hydroxy-6-({[3'-(methylsulfonyl)-1,1'-biphenyl-4-yl]oxy}methyl)-3-(trifluoromethyl)benzoate

2M-Aqueous sodium carbonate solution (1.0 ml) and [1,1'-bis(diphenylphosphino)ferrocene]dichloropalladium (II)-dichloromethane adduct (81 mg, 0.1 mmol) were added to a solution of 3-bromophenylmethylsulfone (235 mg, 1.0 mmol) and tert-butyl 2-hydroxy-{[4-(4,4,5,5-tetramethyl-1,3,2-dioxaborolan-2-yl)phenoxy]methyl}-3-(trifluoromethyl) benzoate (494 mg, 1.0 mmol) obtained in Reference Example (25-4) in dioxane (5.0 ml), and the mixture was stirred at 50˚C for 2 hours. After the temperature of the reaction mixture was returned to room temperature, the mixture was poured into water, and the mixture was extracted (three times) with ethyl acetate. The organic layer was successively washed with water (twice) and saturated aqueous NaCl solution, and dried over anhydrous sodium sulfate. The solvent was evaporated under reduced pressure to obtain the title compound as a solid (80 mg, yield: 15%).
$^1$H-NMR (400MHz, DMSO-$d_6$): δ 11.44 (1H, s), 8.12 (1H, s), 8.00 (1H, d, J = 8.0 Hz), 7.86 (1H, d, J = 8.0 Hz), 7.82 (1H, d, J = 8.0 Hz), 7.76 (2H, d, J = 8.0 Hz), 7.71 (1H, t, J = 8.0 Hz), 7.31 (1H, d, J = 8.8 Hz), 7.13 (2H, d, J = 8.0 Hz), 5.39 (2H, s), 3.29 (3H, s), 1.56 (9H, s).
MS (ESI) (m/z): 521 ($[M-H]^+$).

(Reference Example 30)

(2-Amino-4'-{[2-(tert-butoxycarbonyl)-3-hydroxy-4-(trifluoromethyl)benzyl]oxy}-1,1'-biphenyl-4-yl)acetic acid

Rhodium-alumina (Rh: 5%) (100 mg) was added to a solution of (4'-{[2-(tert-butoxycarbonyl)-3-hydroxy-4-(trifluoromethyl)benzyl]oxy}-2-nitro-1,1'-biphenyl-4-yl)acetic acid (130 mg, 0.237 mmol) obtained in Reference Example (22-2) in methanol (4 ml), and the mixture was stirred at room temperature under a hydrogen atmosphere for 2 days. The insolubles were removed by filtration through Celite, and the filtrate was concentrated. The thus obtained residue was purified by silica gel preparative thin layer chromatography (development solvent: methylene chloride/methanol=20/1) to obtain the title compound as a yellow amorphous solid (63 mg, yield: 51%).
$^1$H-NMR (400MHz, $CDCl_3$): δ 7.69 (1H, d, J = 8.0 Hz), 7.35 (2H, d, J = 8.6 Hz), 7.26 (1H, d, J = 8.0 Hz), 7.04 (1H, d, J = 8.0 Hz), 6.96 (2H, d, J = 8.6 Hz), 6.71 (1H, d, J = 8.0 Hz), 6.69 (1H, br s), 5.36 (2H, s), 3.58 (2H, s), 1.65 (9H, s).
MS (ESI) (m/z): 516 ($[M-H]^+$).

(Reference Example 31)

[4'-{[2-(tert-Butoxycarbonyl)-3-hydroxy-4-(trifluoromethyl)benzyl]oxy}-2-(dimethylamino)-1,1'-biphenyl-4-yl]acetic acid

36% aqueous formaline solution (0.5 ml), acetic acid (100 μl) and sodium cyanoborohydride (36 mg, 0.59 mmol) were successively added to a solution of (2-amino-4'-([2-(tert-butoxycarbonyl)-3-hydroxy-4-(trifluoromethyl)benzyl]oxy}-1,1'-biphenyl-4-yl)acetic acid (50 mg, 0.096 mmol) obtained in Reference Example (30) in acetonitrile (4 ml), and the mixture was stirred at room temperature overnight. The reaction mixture was poured into water and, after the mixture was extracted with ethyl acetate, the organic layer was successively washed with water and saturated aqueous NaCl solution, and dried over anhydrous sodium sulfate. The residue obtained by evaporating the solvent under reduced pressure was purified by silica gel preparative thin layer chromatography (development solvent: methylene chloride/methanol=20/1) to obtain the yellow oily title compound (48 mg, yield: 92%). $^1$H-NMR (400MHz, $CDCl_3$) : δ 7.68 (1H, d, J = 8.2 Hz), 7.48 (2H, d, J = 8.6 Hz), 7.27 (1H, d, J = 8.2 Hz), 7.12 (1H, d, J = 8.2 Hz), 6.93-6.87 (4H, m), 5.34 (2H, s), 3.63 (2H, s), 2.53 (6H, s), 1.63 (9H, s).
MS (ESI) (m/z): 544 ($[M-H]^+$).

(Reference Example 32)

2-(4'-{[2-(tert-Butoxycarbonyl)-3-hydroxy-4-(trifluoromethyl)benzyl]oxy}-1,1'-biphenyl-4-yl)-3-hydroxypropanoic acid

(32-1)

Dimethyl sulfoxide (3 ml), paraformaldehyde (purity: 90%, 300 mg) and sodium hydrogencarbonate (300 mg, 3.57 mmol) were added to tert-butyl 2-hydroxy-6-[({4'-[(methoxycarbonyl)methyl]-1,1'-biphenyl-4-yl}oxy)methyl]-3-(trifluoromethyl)benzoate (400 mg, 0.77 mmol) obtained in Reference Example (13-3), and the mixture was stirred at 60˚C for 3 hours. After the reaction mixture was cooled to room temperature, it was diluted with ethyl acetate. The organic later was successively washed with water and saturated aqueous NaCl solution, and dried over anhydrous sodium sulfate. The residue obtained by evaporating the solvent under reduced pressure was purified by silica gel column chromatography (elution solvent: n-hexane/ethyl acetate=4/1-1/3) to obtain tert-butyl 2-hydroxy-6-[({4'-[2-hydroxy-1-(methoxycarbonyl)ethyl]-1,1'-biphenyl-4-yl}oxy)methyl]-3-(trifluoromethyl)benzoate as a colorless solid (246 mg, yield: 58%).
$^1$H-NMR (500MHz, $CDCl_3$): δ 12.26 (1H, s), 7.71 (1H , d, J = 8.0 Hz), 7.53 (2H, d, J = 8.0 Hz), 7.52 (2H, d, J = 9.0 Hz), 7.32 (2H, d, J = 8.0 Hz), 7.28-7.26 (1H, m), 6.98 (2H, d, J = 9.0 Hz), 5.38 (2H, s), 4.18-4.14 (1H, m), 3.91-3.84 (2H, m), 3.74 (3H, s), 2.26-2.23 (1H, m), 1.65 (9H, s).

(32-2)

The procedures were carried out similarly to Reference Example (9-10) using tert-butyl 2-hydroxy-6-[({4'-[2-hydroxy-1-(methoxycarbonyl)ethyl]-1,1'-biphenyl-4-yl}oxy)methyl]-3-(trifluoromethyl)benzoate obtained in Reference Example (32-1) to obtain 2-(4'-{[2-(tert-butoxycarbonyl)-3-hydroxy-4-(trifluoromethyl)benzyl]oxy}-1,1'-biphenyl-4-yl)-3-hydroxypropanoic acid as a colorless solid (65 mg).
In the present step, 1,4-dioxane was used instead of tetrahydrofuran as the reaction solvent.
$^1$H-NMR (400MHz, $CDCl_3$): δ 12.26 (1H, s), 7.71 (1H, d, J = 8.0 Hz), 7.55 (2H, d, J = 8.0 Hz), 7.52 (2H ,d, J = 8.8 Hz), 7.36 (2H, d, J = 8.0 Hz), 7.29-7.26 (1H, m), 6.98 (2H , d, J = 8.8 Hz), 5.38 (2H, s), 4.21-4.17 (1H, m), 3.97-3.91 (2H, m),

1.65 (9H, s).
ESI (ES-) (m/z): 531 ([M-H]$^+$).

(Reference Example 33)

(4'-{[2-(tert-Butoxycarbonyl)-3-hydroxy-4-(trifluoromethyl)benzyl]oxy}-2-isopropyl-1,1'-biphenyl-4-yl)acetic acid

(33-1)

Isopropyl magnesium bromide-0.63M tetrahydrofuran solution (3.2 ml, 2.0 mmol) was added dropwise to a solution of zinc bromide (473 mg, 2.1 mmol) in tetrahydrofuran (2 ml) under ice-cooling. After the mixture was stirred for 15 minutes, the reaction mixture was cooled to -78°C. After 3-bromo-4-methoxybenzyl cyanide (226 mg, 1.0 mmol) and [1,1'-bis(diphenylphosphino)ferocene]dichloropalladium (II)-dichloromethane adduct (32 mg, 0.04 mmol) were added to the reaction mixture at -78°C, the temperature of the reaction mixture was raised to room temperature, and the mixture was further stirred for 5 hours. 1N-Hydrochloric acid was added to the reaction mixture, and the mixture was extracted with ethyl acetate. The organic layer was successively washed with water and saturated aqueous NaCl solution, and dried over anhydrous sodium sulfate. The residue obtained by evaporating the solvent under reduced pressure was subjected to silica gel column chromatography (elution solvent: n-hexane/ethyl acetate=8/1-6/1) to obtain crude (3-isopropyl-4-methoxyphenyl)acetonitrile (158 mg). $^1$H-NMR (400MHz, $CDCl_3$) : δ 7.12-7.11 (2H, m), 6.83 (1H , d, J = 9.2 Hz), 3.83 (3H, s), 3.69 (2H, s), 3.34-3.27 (1H, m), 1.21 (3H, d, J = 6.8 Hz), 1.20 (3H, d, J = 6.8 Hz).

(33-2)

The procedures were carried out similarly to Reference Example (15-4) using the crudely purified product of (3-isopropyl-4-methoxyphenyl)acetonitrile (158 mg) obtained in Reference Example (33-1) to obtain crude methyl (4-hydroxy-3-isopropylphenyl)acetate (163 mg).
$^1$H-NMR (400MHz, $CDCl_3$) : δ 7.08 (1H, s), 6.98 (1H, d, J = 8.0 Hz), 6.70 (1H, d, J = 8.0 Hz), 4.64 (1H, br s), 3.69 (3H, s), 3.55 (2H, s), 3.21-3.15 (1H, m), 1.25 (6H, d, J = 6.8 Hz).

(33-3)

The procedures were carried out similarly to Reference Example (25-5), Reference Example (9-2) and Reference Example (15-4) using the crude methyl (4-hydroxy-3-isopropylphenyl)acetate (163 mg) obtained in Reference Example (33-2) to obtain crude methyl (4'-hydroxy-2-isopropyl-1,1'-biphenyl-4-yl)acetate (147 mg).
$^1$H-NMR (400MHz, $CDCl_3$) : δ 7.26-7.12 (5H, m), 6.87-6.82 (2H, m), 3.74 (3H, s), 3.67(2H, s), 3.09-3.02 (1H, m), 1.15 (6H, d, J = 6.8 Hz).

(33-4)

The procedures were carried out similarly to Reference Example (16-4) and Reference Example (21-2) using the crudely purified product of methyl (4'-hydroxy-2-isopropyl-1,1'-biphenyl-4-yl)acetate (147 mg) obtained in Reference Example (33-3) and 2-(dimethoxymethyl)-3-(methoxymethoxy)-4-(trifluoromethyl)benzyl methanesulfonate (252 mg, 0.65 mmol) obtained in Reference Example (15-5) to obtain crude methyl (4'-[[2-formyl-3-hydroxy-4-(trifluoromethyl) benzyl]oxy]-2-isopropyl-1,1'-biphenyl-4-yl)acetate (169 mg).
$^1$H-NMR (500MHz, $CDCl_3$) δ 12.66 (1H, s), 10.39 (1H, s), 7.83 (1H, d, J = 8.0 Hz), 7.26-7.23 (3H, m), 7.12-7.10 (3H, m), 7.00 (2H, d, J = 8.5 Hz), 5.37 (2H, s), 3.73 (3H, s), 3.67 (2H, s), 3.05-3.02 (1H, m), 1.16 (6H, d, J = 6.5 Hz).

(33-5)

The procedures were carried out similarly to Reference Example (21-3), Reference Example (21-4) and Reference Example (16-5) using the crudely purified product of methyl (4'-[[2-formyl-3-hydroxy-4-(trifluoromethyl)benzyl] oxy]-2-isopropyl-1,1'-biphenyl-4-yl)acetate (169 mg) obtained in Reference Example (33-4) to obtain tert-butyl 2-hydroxy-6-[({2'-isopropyl-4'-[(methoxycarbonyl)methyl]-1,1'-biphenyl-4-yl}oxy)methyl]-3-(trifluoromethyl)benzoate (91 mg, 0.16 mmol).
In the above step similar to Reference Example (16-5), purification of the compound was carried out by using high speed liquid chromatography (column: G. L. Science, Innert Sil ODS-3; eluent: acetonitrile:water=93/7-98/2) followed by silica gel column chromatography.
$^1$H-NMR (500MHz, $CDCl_3$): δ 12.26 (1H, s), 7.72 (1H, d, J = 8.0 Hz), 7.32-7.26 (2H, m), 7.21 (2H, d, J = 8.5 Hz), 7.12

(2H, br s), 6.94 (2H, d, J = 8.5 Hz), 5.38 (2H, s), 3.73 (3H, s), 3.67 (2H, s), 3.09-3.04 (1H, m), 1.65 (9H, s), 1.15 (6H, d, J = 6.5 Hz).

(33-6)

The procedures were carried out similarly to Reference Example (9-10) using tert-butyl 2-hydroxy-6-[({2'-isopropyl-4'-[(methoxycarbonyl)methyl]-1,1'-biphenyl-4-yl}oxy)methyl]-3-(trifluoromethyl)benzoate (91 mg, 0.16 mmol) obtained in Reference Example (33-5) to obtain the title compound as a colorless solid (84 g, yield: 94%).

In the present step, 1,4-dioxane was used instead of tetrahydrofuran as the reaction solvent.

$^1$H-NMR (400MHz, $CDCl_3$): δ 12.23 (1H, s), 7.70 (1H, d, J = 8.4 Hz), 7.28 (1H, d, J = 8.4 Hz), 7.25-7.24 (1H, m), 7.19 (2H, d, J = 8.8 Hz), 7.12 (2H, br s), 6.92 (2H, d, J = 8.8 Hz), 5.36 (2H, s), 3.69 (2H, s), 3.07-3.04 (1H, m), 1.65 (9H, s), 1.15 (6H, d, J = 6.8 Hz).

ESI (ES-) (m/z) : 543 ($[M-H]^+$) .

(Reference Example 34)

4'-{[2-(tert-Butoxycarbonyl)-3-hydroxy-4-(trifluoromethyl)benzyl]oxy}-1,1'-biphenyl-4-carboxylic acid

(34-1)

Allyl alcohol (10 ml) and concentrated sulfuric acid (5 ml) were successively added to a solution of 4'-hydroxy-1,1'-biphenyl-4-carboxylic acid (820 mg, 3.82 mmol) in benzene (5 ml), and the mixture was stirred at 70°C for 5 hours. The reaction mixture was poured into water, and the mixture was extracted with ethyl acetate. The organic layer was successively washed with water (twice) and saturated aqueous NaCl solution, and dried over anhydrous sodium sulfate. The residue obtained by evaporating the solvent under reduced pressure was purified by silica gel column chromatography (elution solvent: n-hexane/ethyl acetate=5/1-3/1) to obtain allyl 4'-hydroxy-1,1'-biphenyl-4-carboxylate as a grayish white solid (582 mg, yield: 60%).

$^1$H-NMR (400MHz, $CDCl_3$): δ 8.08 (2H, d, J = 8.6 Hz), 7.59 (2H, d, J = 8.6 Hz), 7.50 (2H, d, J = 8.6 Hz), 6.91 (2H, d, J = 8.6 Hz), 6.10-5.99 (1H, m), 5.44-5.20 (1H, m), 5.31-5.26 (1H, m), 5.01 (1H, s), 4.83 (2H, d, J = 5.5 Hz).

(34-2)

The procedures were carried out similarly to Reference Example (16-4), Reference Example (16-5) and Reference Example (6-2) using allyl 4'-hydroxy-1,1'-biphenyl-4-carboxylate (190 mg, 0.75 mmol) obtained in Reference Example (34-1) and tert-butyl 6-(bromomethyl)-2-[(tert-butoxycarbonyl)oxy]-3-(trifluoromethyl)benzoate (400 mg, 0.88 mmol) obtained in Reference Example (21-5) to obtain the title compound as a colorless powder (293 mg, three steps total yield: 80%).

$^1$H-NMR (500MHz, DMSO-$d_6$): δ 12.9 (1H, br s), 11.4 (1H, br s), 7.98 (2H, d, J = 8.8 Hz), 7.82 (1H, d, J = 8.8 Hz), 7.76 (2H, d, J = 8.8 Hz), 7.72 (2H, d, J = 8.8 Hz), 7.30 (1H, d, J = 8.8 Hz), 7.11 (2H, d, J = 8.8 Hz), 5.39 (2H, s), 1.57 (9H, s).

MS (FAB+) (m/z) : 489 ($[M+H]^+$).

(Reference Example 35)

(4'-{[2-(tert-Butoxycarbonyl)-3-hydroxy-4-(trifluoromethyl)benzyl]oxy}-2-formyl-1,1'-biphenyl-4-yl)acetic acid

(35-1)

The procedures were carried out similarly to Reference Example (19-1), Reference Example (25-5) and Reference Example (15-3) using methyl 4-hydroxyphenylacetate (15.6 g, 110 mmol) to obtain methyl (2-formyl-4'-hydroxy-1,1'-biphenyl-4-yl)acetate (6.32 g, three steps total yield: 21%).

In the present step, in the reaction corresponding to Reference Example (19-1), the reaction time was 12 hours. Further, in the reaction corresponding to Reference Example (15-3), 4-(4,4,5,5-tetramethyl-1,3,2-dioxaborolan-2-yl) phenol was used as the boric acid ester reagent.

$^1$H-NMR (500MHz, $CDCl_3$): δ 9.96 (1H, s), 7.88 (1H, s), 7.55 (1H, app d, J = 7.8 Hz), 7.40 (1H, d, J = 7.8 Hz), 7.20 (2H, d, J = 8.3 Hz), 6.92 (2H, d, J = 8.3 Hz), 3.75-3.71 (5H, m) .

(35-2)

Potassium carbonate (1.61 g, 11.7 mmol) was added to a solution of tert-butyl 6-(bromomethyl)-2-[(tert-butoxycarbonyl)oxy]-3-(trifluoromethyl)benzoate (3.60 g, 8.56 mmol) obtained in Reference Example (21-5) and methyl (2-formyl-4'-hydroxy-1,1'-biphenyl-4-yl)acetate (2.10 g, 7.78 mmol) obtained in Reference Example (35-1) in acetone (50 ml) under ice-cooling, and the mixture was stirred at room temperature for 2 hours. The reaction mixture was poured into water, and the mixture was extracted with ethyl acetate. The organic layer was successively washed with water (three times) and saturated aqueous NaCl solution, and dried over anhydrous sodium sulfate, followed by concentration under reduced pressure. The thus obtained residue was purified by silica gel column chromatography (elution solvent: n-hexane/ethyl aceate=95/5-70/30) to obtain colorless oily tert-butyl 2-[(tert-butoxycarbonyl)oxy]-6-[({2'-formyl-4'-[(methoxycarbonyl)methyl]-1,1'-biphenyl-4-yl}oxy)methyl]-3-(trifluoromethyl)benzoate (3.06 g, yield: 61%).
$^1$H-NMR (400MHz, $CDCl_3$): δ 9.93 (1H, s), 7.87 (1H, s), 7.71 (1H, d, J = 8.2 Hz), 7.58 (1H, d, J = 8.2 Hz), 7.54 (1H, d, J = 7.8 Hz), 7.38 (1H, d, J = 7.8 Hz), 7.28 (2H, d, J = 8.2 Hz), 7.01 (2H, d, J = 8.2 Hz), 5.27 (2H, s), 3.75-3.70 (5H, m), 1.58 (9H, s), 1.54 (9H, s).

(35-3)

The procedures were carried out similarly to Reference Example (16-5) and Reference Example (9-10) using tert-butyl 2-[(tert-butoxycarbonyl)oxy]-6-[({2'-formyl-4'-[(methoxycarbonyl)methyl]-1,1'-biphenyl-4-yl}oxy)methyl]-3-(trifluoromethyl)benzoate (88 mg, 0.14 mmol) obtained in Reference Example (35-2) to obtain the title compound as a white powder (28 mg, two steps total yield: 38%).
$^1$H-NMR (400MHz, $CDCl_3$) : δ 12.23 (1H, s), 9.94 (1H, s), 7.89 (1H, d, J = 2.0 Hz), 7.70 (1H, d, J = 8.2 Hz), 7.55 (1H, dd, J = 7.8, 2.0 Hz), 7.40 (1H, d, J = 7.8 Hz), 7.29 (2H, d, J = 8.6 Hz), 7.26 (1H, d, J = 8.2 Hz), 7.00 (2H, d, J = 8.6 Hz), 5.38 (2H, s), 3.76 (2H, s), 1.65 (9H, s).
ESI (ES-) (m/z) : 529 ($[M-H]^+$).

(Reference Example 36)

(4'-{[2-(tert-Butoxycarbonyl)-3-hydroxy-4-(trifluoromethyl)benzyl]oxy}-2-(hydroxymethyl)-1,1'-biphenyl-4-yl)acetic acid

The procedures were carried out similarly to Reference Example (5-4), Reference Example (16-5) and Reference Example (9-10) using tert-butyl 2-[(tert-butoxycarbonyl)oxy]-6-[({2'-formyl-4'-[(methoxycarbonyl)methyl]-1,1'-biphenyl-4-yl}oxy)methyl]-3-(trifluoromethyl)benzoate (200 mg, 0.371 mmol) obtained in Reference Example (35-2) to obtain the title compound as a white powder (62 mg, three steps total yield: 31%).
$^1$H-NMR (400MHz, $CDCl_3$) : δ 12.22 (1H, s), 7.68 (1H, d, J = 8.2 Hz), 7.44 (1H, s), 7.30-7.18 (5H, m), 6.93 (2H, d, J = 8.2 Hz), 5.35 (2H, s), 4.59 (2H, s), 3.70 (2H, s), 1.64 (9H, s),
ESI (ES-) (m/z) : 531 ( $[M-H]^+$) .

(Reference Example 37)

(4'-{[2-(tert-Butoxycarbonyl)-3-hydroxy-4-(trifluoromethyl)benzyl]oxy}-2-cyano-1,1'-biphenyl-4-yl)acetic acid

(37-1)

Pyridine (49 μl, 0.62 mmol) and hydroxylamine hydrochloride (42 mg, 0.62 mmol) were added to a solution of tert-butyl 2-[(tert-butoxycarbonyl)oxy]-6-[({2'-formyl-4'-[(methoxycarbonyl)methyl]-1,1'-biphenyl-4-yl}oxy)methyl]-3-(trifluoromethyl)benzoate (198 mg, 0.307 mmol) obtained in Reference Example (35-2) in ethanol (4 ml), and the mixture was stirred at room temperature for 4 hours. The reaction mixture was diluted with ethyl acetate, and the mixture was successively washed with water and saturated aqueous NaCl solution, and dried over anhydrous sodium sulfate. The residue obtained by evaporating the solvent under reduced pressure was subjected to silica gel preparative thin layer chromatography (development solvent: n-hexane/ethyl acetate=2/1) to obtain crude tert-butyl 2-[(tert-butoxycarbonyl)oxy]-6-[({2'-[(hydroxyimino)methyl]-4'-[(methoxycarbonyl)methyl]-1,1'-biphenyl-4-yl}oxy)methyl]-3-(trifluoromethyl)benzoate (170 mg).

Triethylamine (71 μl, 0.51 mmol) and methanesulfonyl chloride (23 μl, 0.31 mmol) were added to a solution of the obtained crude tert-butyl 2-[(tert-butoxycarbonyl)oxy]-6-[({2'-[(hydroxyimino)methyl]-4'-[(methoxycarbonyl)methyl]-1,1'-biphenyl-4-yl}oxy)methyl]-3-(trifluoromethyl)benzoate (170 mg) in dichloromethane (4 ml), and the mixture was stirred at room temperature for 1 hour. The reaction mixture was diluted with ethyl acetate, and the mixture was successively washed with satuated aqueous sodium hydrogencarbonate solution, water and saturated aqueous NaCl solution, and dried over anhydrous sodium sulfate. The solvent was evaporated under reduced pressure. Triethylamine (71 μl, 0.51 mmol) was added to a solution of the obtained residue in ethanol (4 ml), and the mixture was heated under reflux for 14 hours. The solvent was evaporated under reduced pressure, and the thus obtained residue was subjected to silica gel preparative thin layer chromatography (development solvent: n-hexane/ethyl acetate=2/1) to obtain crude tert-butyl 2-[(tert-butoxycarbonyl)oxy]-6-[({2'-cyano-4'-[(methoxycarbonyl)methyl]-1,1'-biphenyl-4-yl}oxy)methyl]-3-(trifluoromethyl)benzoate (125 mg).

Pyrrolidine (33 μl, 0.39 mmol) was added to a solution of the obtained crude tert-butyl 2-[(tert-butoxycarbonyl) oxy]-6-[({2'-cyano-4'-[(methoxycarbonyl)methyl]-1,1'-biphenyl-4-yl}oxy)methyl]-3-(trifluoromethyl)benzoate(125 mg) in 1,4-dioxane (4 ml), and the mixture was stirred at 50°C for 2 hours. The residue obtained by evaporating the solvent under reduced pressure was purified by silica gel preparative thin layer chromatography (development solvent: n-hexane/ ethyl acetate=5/1) to obtain tert-butyl 6-[({2'-cyano-4'-[(methoxycarbonyl)methyl]-1,1'-biphenyl-4-yl}oxy)methyl]-2-hydroxy-3-(trifluoromethyl)benzoate (100 mg, 60%).
$^1$H-NMR (400MHz, $CDCl_3$): δ 12.30 (1H, s), 7.71 (1H, d, J = 8.6 Hz), 7.67 (1H, d, J = 1.8 Hz), 7.55 (1H, dd, J = 8.6, 1.8 Hz), 7.51 (2H, d, J = 8.6 Hz), 7.46 (1H, d, J = 8.6 Hz), 7.27 (1H, d, J = 8.6 Hz), 7.03 (2H, d, J = 8.6 Hz), 5.40 (2H, s), 3.74 (3H, s), 3.70 (2H, s) , 1.65 (9H, s).

(37-2)

The procedures were carried out similarly to Reference Example (9-10) using tert-butyl 6-[({2'-cyano-4'-[(methoxycarbonyl)methyl]-1,1'-biphenyl-4-yl}oxy)methyl]-3-(trifluoromethyl)benzoate (100 mg, 0.184 mmol) obtained in Reference Example (37-1) to obtain the title compound as a white amorphous solid (80 mg, 82%).
$^1$H-NMR (400MHz, $CDCl_3$) : δ 12.29 (1H, s), 7.71 (1H, d, J = 8.0 Hz), 7.68 (1H, d, J = 1.6 Hz), 7.55 (1H, dd, J = 8.0, 1.6 Hz), 7.51 (2H, d, J = 8.6 Hz), 7.47 (1H, d, J = 8.0 Hz), 7.27 (1H, d, J = 8.0 Hz), 7.02 (2H, d, J = 8.6 Hz), 5.40 (2H, s), 3.74 (2H, s), 1.65 (9H, s).
MS (FAB) (m/z) : 528 ($[M+H]^+$).

(Formulation Example 1) Hard capsules

The compound of Reference Example 1 in the form of a powder (100 mg), lactose (150 mg), cellulose (50 mg) and magnesium stearate (6 mg) are filled into standard two-part hard gelatin capsules to produce hard capsules, followed by washing and drying.

(Formulation Example 2) Soft capsules

A mixture of a digestive oil such as soybean oil or olive oil and the compound of Reference Example 2 is injected into gelatin to produce soft capsules so as to contain 100 mg of active ingredient, followed by washing and drying.

(Formulation Example 3) Tablets

Tablets are prepared according to usually used methods using the compound of Reference Example 3 (100 mg), colloidal silicon dioxide (0.2 mg), magnesium stearate (5 mg), microcrystalline cellulose (275 mg), starch (11 mg) and lactose (98.8 mg). The resulting tablets can be coated as necessary.

(Formulation Example 4) Suspension

A suspension is prepared so as to contain a finely powdered compound of Reference Example 4 (100 mg), sodium carboxymethyl cellulose (100 mg), sodium benzoate (5 mg), sorbitol solution (Japanese Pharmacopoeia, 1.0 g) and vanillin (0.025 ml) in 5 ml of suspension.

(Formulation Example 5) Cream

A cream is produced by mixing a finely powdered compound of Reference Example 5 (100 mg) into 5 g of a cream consisting of white petrolatum (40 wt%), microcrystalline wax (3 wt%), lanolin (10 wt%), sorbitan monolaurate (5 wt%), 0.3% polyoxyethylene (20) sorbitan monolaurate (0.3 wt%) and water (41.7 wt%).

[Industrial applicability]

A medicament of the present invention has an activity of inhibiting production of tissue factor, and has an

avtivity of decreasing thrombus formation in the living bodies of warm-blooded animals. Thus, a medicament of the present invention is useful for the treatment and/or prophylaxis of vascular restenosis following angioplasty, endarterectomy, percutaneous transluminal coronary angioplasty (PTCA) or stent implantation, or treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor or cancer metastasis.

## Claims

**1.** A medicament having an activity of inhibiting production of tissue factor, wherein the medicament comprises an LXR ligand as an active ingredient.

**2.** A medicament according to claim 1 which has an activity of decreasing thrombus formation.

**3.** A medicament for the treatment and/or prophylaxis of a disease selected from the group consisting of vascular restenosis following angioplasty, endarterectomy, percutaneous transluminal coronary angioplasty (PTCA) and stent implantation, wherein the medicament comprises an LXR ligand as an active ingredient.

**4.** A medicament for the treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor, or cancer

metastasis, wherein the medicament comprises an LXR ligand as an active ingredient.

**5.** A medicament for the treatment and/or prophylaxis of blood coagulation diseases, wherein the medicament comprises an LXR ligand as an active ingredient.

**6.** A medicament according to any one of claims 1 to 5, wherein the LXR ligand is an LXR agonist or an LXR antagonist.

**7.** A medicament according to any one of claims 1 to 5, wherein the LXR ligand is an LXR agonist.

**8.** A medicament according to any one of claims 1 to 7, wherein the LXR ligand is a compound represented by the general formula (Ia)

[Chemical formula 1]

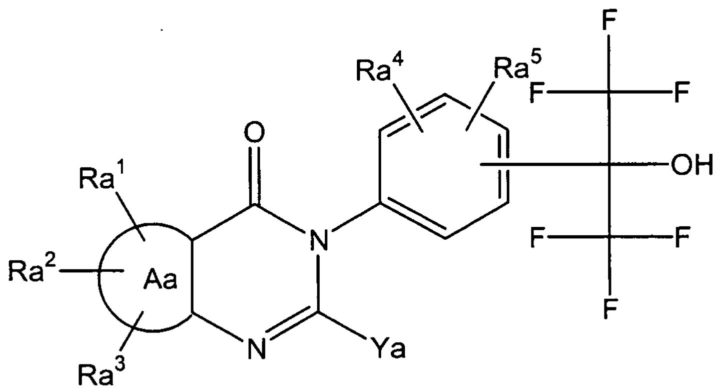

(Ia)

or a pharmacologically acceptable salt or ester thereof;
wherein $Ra^1$, $Ra^2$ and $Ra^3$ may be the same or different, and each represents a hydrogen atom, a hydroxyl group, a fluorine atom, a chlorine atom, a methyl group, an ethyl group, a trifluoromethyl group, a methoxy group, an ethoxy group or an acetylamino group, or $Ra^1$ and $Ra^2$ together represent a methylenedioxy group;
$Ra^4$ and $Ra^5$ may be the same or different, and each represents a hydrogen atom, a chlorine atom, a methyl group or a methoxy group;
Ya represents a benzyl group, a substituted benzyl group (said substituent is one group selected from the group consisting of a $C_1$-$C_6$ alkyl group, a $C_1$-$C_6$ alkoxy group and a halogeno group), a thienylmethyl group, a substituted thienylmethyl group (said substituent is one group selected from the group consisting of a $C_1$-$C_6$ alkyl group, a $C_1$-$C_6$ alkoxy group and a halogeno group), a pyridylmethyl group or a substituted pyridylmethyl group (said substituent is one group selected from the group consisting of a $C_1$-$C_6$ alkyl group, a $C_1$-$C_6$ alkoxy group and a halogeno group); and
Aa represents a phenyl group.

**9.** A medicament according to any one of claims 1 to 7, wherein the LXR ligand is a compound represented by the general formula (Ib)

[Chemical formula 2]

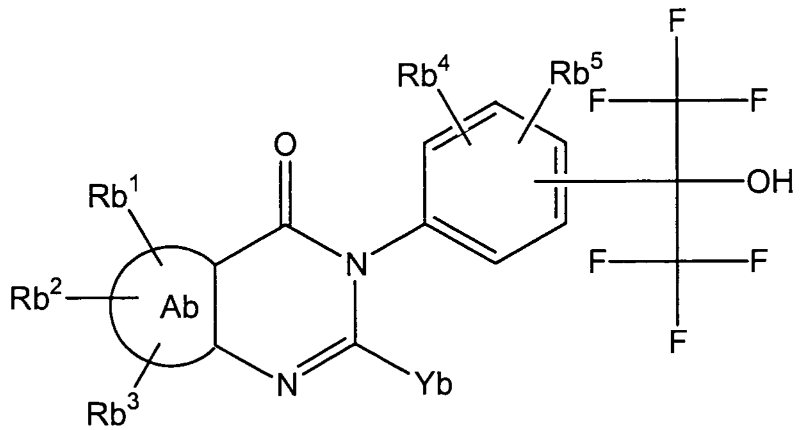

(Ib)

or a pharmacologically acceptable salt or ester thereof;
wherein Ab represents a phenyl group;
$Rb^1$ represents a 5- to 7-membered heterocyclyl group or a group represented by the formula: $-O-Rb^{1a}$ [wherein $Rb^{1a}$ represents a substituted $C_1$-$C_6$ alkyl group (said substituent(s) are the same or different and are 1 or 2 group (s) selected from the group consisting of a hydroxyl group, a hydroxymethoxy group, a hydroxyethoxy group, an amino group, a methylamino group and an ethylamino group)];
$Rb^2$ represents a hydrogen atom, a methyl group, a hydroxyl group, a methoxy group, an amino group, a fluoro group or a chloro group;
$Rb^3$ represents a hydrogen atom;
$Rb^4$ and $Rb^5$ are the same or different and each represents a hydrogen atom, a methyl group, an ethyl group, a methoxy group, a fluoro group or a chloro group; and
Yb represents a benzyl group, a substituted benzyl group (said substituent is one group selected from the group consisting of $C_1$-$C_6$ alkyl group, $C_1$-$C_6$ alkoxy group and a halogeno group), a thienylmethyl group, a substituted thienylmethyl group (said substituent is one group selected from the group consisting of $C_1$-$C_6$ alkyl group, $C_1$-$C_6$ alkoxy group and a halogeno group), a pyridylmethyl group or a substituted pyridylmethyl group (said substituent is one group selected from the group consisting of $C_1$-$C_6$ alkyl group, $C_1$-$C_6$ alkoxy group and a halogeno group) .

**10.** A medicament according to any one of claims 1 to 7, wherein the LXR ligand is a compound represented by the general formula (Ic)

[Chemical formula 3]

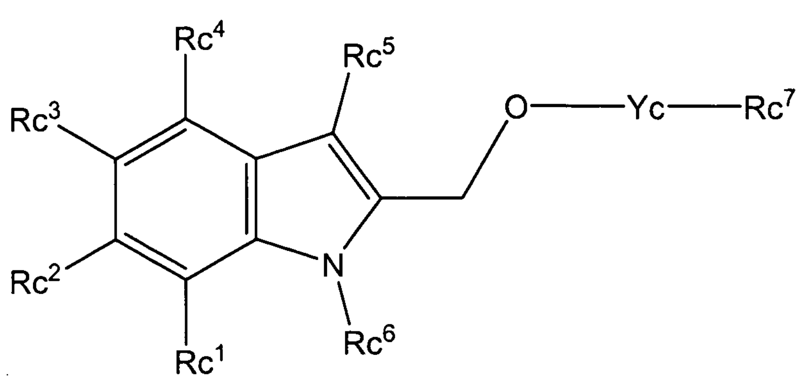

(Ic)

or a pharmacologically acceptable salt or ester thereof;
wherein $Rc^1$, $Rc^2$, $Rc^3$ and $Rc^4$ are the same or different and each represents a hydrogen atom, a $C_1$-$C_3$ alkyl group, a fluoromethyl group, a chloromethyl group, a difluoromethyl group, a trifluoromethyl group, a pentafluoroethyl group, a methoxy group, an ethoxy group, a fluoromethoxy group, a chloromethoxy group, a difluoromethoxy group, a trifluoromethoxy group, a pentafluoroethoxy group, a methanesulfonyl group, an ethanesulfonyl group, a fluoro group, a chloro group or a bromo group;
$Rc^5$ represents a hydrogen atom;
$Rc^6$ represents a group having the formula: $-CORc^8$ [wherein $Rc^a$ represents a $C_3$-$C_6$ alkoxy group or a halogeno $C_3$-$C_5$ alkoxy group (said halogeno $C_3$-$C_5$ alkoxy group represents a $C_3$-$C_5$ alkoxy group substituted with 1 to 5 fluoro or chloro groups)];
$Rc^7$ represents a group having the formula: - $N(Rc^{10})ZcRc^{11}$ [wherein $Rc^{10}$ represents a methyl group, an ethyl group or a cyclopropyl group, $Rc^{11}$ represents a $C_1$-$C_4$ alkyl group, a substituted $C_1$-$C_4$ alkyl group (said substituent is one group selected from Substituent group αc), a cyclopropyl-($C_1$-$C_2$ alkyl) group, a $C_3$-$C_4$ cycloalkyl group or a $C_2$-$C_4$ alkenyl group, and Zc represents a group having the formula: -CO-, -CS- or $-SO_2-$];
Yc represents a phenyl group, a substituted phenyl group (said substituent is one group selected from Substituent group βc), a pyridyl group or a substituted pyridyl group (said substituent is one group selected from Substituent group βc) ;
Substituent group αc represents the group consisting of a hydroxyl group, a methoxy group, an ethoxy group, a fluoromethoxy group, a chloromethoxy group, a difluoromethoxy group, a trifluoromethoxy group, a pentafluoroethoxy group, a benzyloxy group, a phenyloxy group, an amino group, a methylamino group, an ethylamino group, a dimethylamino group, a diethylamino group, a dimethylaminocarbonyl group, a diethylaminocarbonyl group, a fluoro group and a chloro group; and
Substituent group βc represents the group consisting of a $C_1$-$C_4$ alkyl group, a halogeno $C_1$-$C_4$ alkyl group (said halogeno $C_1$-$C_4$ alkyl group represents a $C_1$-$C_4$ alkyl group substituted with 1 to 5 fluoro, chloro or bromo groups), a $C_1$-$C_4$ alkoxy group, a ($C_1$-$C_4$ alkoxy)carbonyl group, a cyano group, a nitro group, a fluoro group, a chloro group and a bromo group.

**11.** A medicament according to any one of claims 1 to 7, wherein the LXR ligand is a compound represented by the general formula (Id)

[Chemical formula 4]

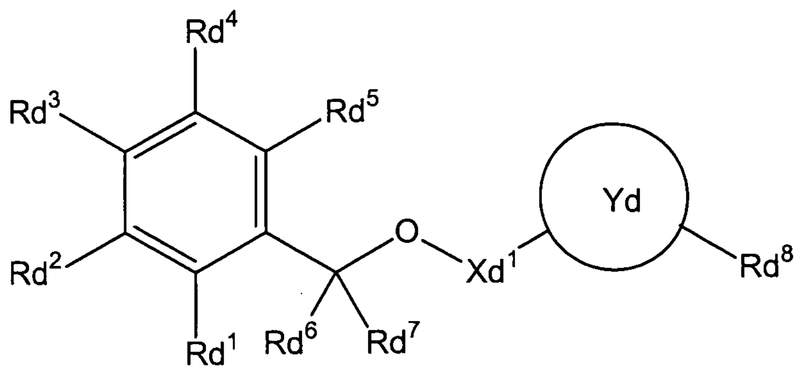

(Id)

or a pharmacologically acceptable salt or ester thereof;
wherein $Rd^1$ represents a group having the formula: - $CORd^9$ [wherein $Rd^9$ represents a $C_1$-$C_6$ alkoxy group or a halogeno $C_1$-$C_4$ alkoxy group (said halogeno $C_1$-$C_4$ alkoxy group represents a $C_1$-$C_4$ alkoxy group substituted with 1 to 5 halogeno groups)];
$Rd^2$ represents a hydrogen atom, a trifluoromethyl group, a 2,2,2-trifluoroethyl group, a pentafluoroethyl group, a hydroxyl group, a fluoro group or a chloro group;
$Rd^3$ represents a $C_1$-$C_4$ alkyl group, a halogeno $C_1$-$C_4$ alkyl group (said halogeno $C_1$-$C_4$ alkyl group represents a $C_1$-$C_4$ alkyl group substituted with 1 to 5 halogeno groups), a $C_3$-$C_5$ cycloalkyl group, a $C_2$-$C_4$ alkenyl group, a $C_1$-$C_4$ alkoxy group, a fluoro group or a chloro group;

$Rd^4$ and $Rd^5$ represent a hydrogen atom;
$Rd^6$ and $Rd^7$ represent a hydrogen atom;
$Rd^8$ represents a group having the formula: - $N(Rd^{10})ZdRd^{11}$ [wherein $Rd^{10}$ represents a methyl group, an ethyl group, a 1-propyl group or a 2-propyl group, $Rd^{11}$ represents a $C_1$-$C_4$ alkyl group, a substituted $C_1$-$C_4$ alkyl group (said substituent is one group selected from Substituent group αd), a ($C_3$-$C_4$ cycloalkyl)methyl group, a $C_3$-$C_4$ cycloalkyl group or a vinyl group, and Zd represents a group having the formula: -CO-, -CS- or -$SO_2$-];
$Xd^1$ represents a single bond;
Yd represents a phenyl group, a substituted phenyl group (said substituent is one group selected from Substituent group βd) or a pyridyl group;
Substituent group αd represents the group consisting of a methoxy group, a methylthio group, a methylamino group and a dimethylamino group; and
Substituent group βd represents the group consisting of a methoxy group, a methylamino group, a dimethylamino group, a fluoro group and a chloro group.

**12.** A medicament according to any one of claims 1 to 7, wherein the LXR ligand is a compound represented by general formula (Ie)

[Chemical formula 5]

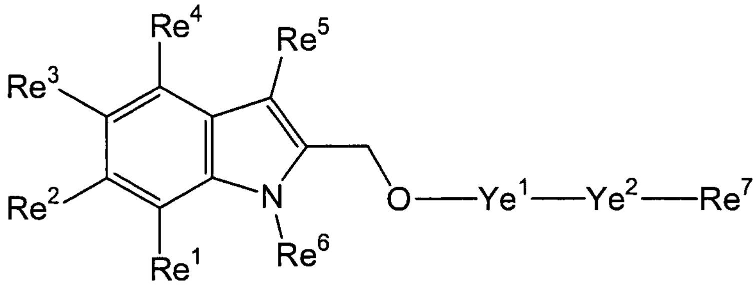

(Ie)

or a pharmacologically acceptable salt or ester thereof;
wherein $Re^1$, $Re^2$, $Re^3$ and $Re^4$ are the same or different and each represents a hydrogen atom, a $C_1$-$C_3$ alkyl group, a trifluoromethyl group, a 2,2,2-trifluoroethyl group, a pentafluoroethyl group, a hydroxyl group, a methoxy group, an ethoxy group, a fluoro group, a chloro group or a bromo group;
$Re^5$ represents a hydrogen atom;
$Re^6$ represents a group having the formula: -$CORe^8$ [wherein $Re^8$ represents a $C_1$-$C_6$ alkoxy group or a halogeno $C_1$-$C_4$ alkoxy group (said halogeno $C_1$-$C_4$ alkoxy group represents a $C_1$-$C_4$ alkoxy group substituted with 1 to 5 fluoro or chloro groups)];
$Re^7$ represents a group having the formula: -$Xe^2Re^{10}$ [wherein $Re^{10}$ represents a group having the formula: -$CORe^{11}$ (wherein $Re^{11}$ represents a hydroxyl group, a methoxy group or an ethoxy group) or a group having the formula: -$SO_2Re^{12}$ (wherein $Re^{12}$ represents a methyl group or an ethyl group), and $Xe^2$ represents a single bond, a methylene group or a substituted methylene group (said substituents are two fluoro groups, or two substituents may together form an ethylene group)];
$Ye^1$ represents a phenyl group;
$Ye^2$ represents a phenyl group, a substituted phenyl group (said substituent(s) are the same or different and are one or two group(s) selected from Substituent group αe), a thienyl group, a thiazolyl group, a pyridyl group or a substituted thienyl group, a substituted thiazolyl group or a substituted pyridyl group (said substituent(s) are the same or different and are one or two group(s) selected from Substituent group αe); and
Substituent group αe represents the group consisting of a $C_1$-$C_4$ alkyl group, a trifluoromethyl group, a 2,2,2-trifluoroethyl group, a pentafluoroethyl group, a $C_2$-$C_4$ alkenyl group, a $C_2$-$C_4$ alkynyl group, a $C_3$-$C_4$ cycloalkyl group, a hydroxyl group, a methoxy group, an ethoxy group, a methanesulfonyl group, an ethanesulfonyl group, an amino group, a methylamino group, an ethylamino group, a dimethylamino group, a diethylamino group, a formyl group, a methylcarbonyl group, an ethylcarbonyl group, a nitro group, a fluoro group and a chloro group.

**13.** A medicament according to any one of claims 1 to 7, wherein the LXR ligand is a compound represented by the general formula (If)

[Chemical formula 6]

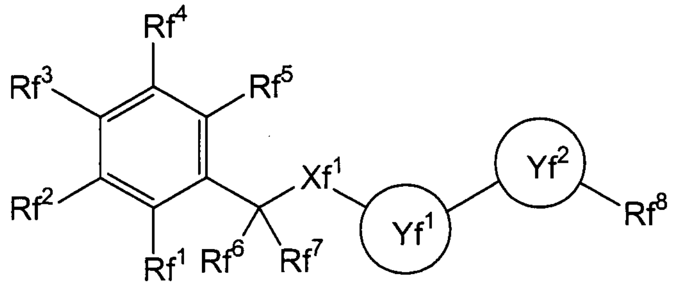

(If)

or a pharmacologically acceptable salt or ester thereof;

wherein $Rf^1$ represents a group having the formula - $CORf^9$ [wherein $Rf^9$ represents a $C_1$-$C_{10}$ alkyl group, a $C_1$-$C_{10}$ alkoxy group, a halogeno $C_1$-$C_{10}$ alkoxy group (wherein said halogeno $C_1$-$C_{10}$ alkoxy group represents a $C_1$-$C_{10}$ alkoxy group substituted with 1 to 7 halogeno groups), a phenyl-($C_1$-$C_{10}$ alkoxy) group, a $C_1$-$C_{10}$ alkylamino group or a di($C_1$-$C_{10}$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom)];

$Rf^2$ represents a hydrogen atom, a halogeno $C_1$-$C_4$ alkyl group (wherein said halogeno $C_1$-$C_4$ alkyl group represents a $C_1$-$C_4$ alkyl group substituted with 1 to 5 halogeno groups), a hydroxyl group, a $C_1$-$C_4$ alkoxy group, an amino group, a $C_1$-$C_4$ alkylamino group, a di ($C_1$-$C_4$ alkyl) amino group (wherein said alkyl groups may be the same or different) or a halogeno group;

$Rf^3$ represents a hydrogen atom, a $C_1$-$C_6$ alkyl group, a halogeno $C_1$-$C_6$ alkyl group (wherein said halogeno $C_1$-$C_6$ alkyl group represents a $C_1$-$C_6$ alkyl group substituted with 1 to 7 halogeno groups), a ($C_1$-$C_4$ alkoxy)-($C_1$-$C_4$ alkyl) group, a ($C_1$-$C_4$ alkylthio)-($C_1$-$C_4$ alkyl) group, a ($C_1$-$C_4$ alkylsulfinyl)-($C_1$-$C_4$ alkyl) group, a ($C_1$-$C_4$ alkylsulfonyl)-($C_1$-$C_4$ alkyl) group, a ($C_1$-$C_4$ alkylamino)-($C_1$-$C_4$ alkyl) group, a [di($C_1$-$C_4$ alkyl)amino]-($C_1$-$C_4$ alkyl) group (wherein said alkyl groups may be the same or different), a $C_3$-$C_6$ cycloalkyl group, a $C_2$-$C_6$ alkenyl group, a $C_2$-$C_6$ alkynyl group, a hydroxyl group, a $C_1$-$C_6$ alkoxy group, a halogeno $C_1$-$C_6$ alkoxy group (wherein said halogeno $C_1$-$C_6$ alkoxy group represents a $C_1$-$C_6$ alkoxy group substituted with 1 to 7 halogeno groups), a $C_1$-$C_6$ alkylthio group, a $C_1$-$C_6$ alkylsulfinyl group, a $C_1$-$C_6$ alkylsulfonyl group, an amino group, a $C_1$-$C_6$ alkylamino group, a di ($C_1$-$C_6$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a ($C_1$-$C_6$ alkoxy)carbonyl group, a cyano group, a nitro group or a halogeno group;

$Rf^4$ and $Rf^5$ may be the same or different and each represents a hydrogen atom, a $C_1$-$C_4$ alkyl group, a halogeno $C_1$-$C_4$ alkyl group (wherein said halogeno $C_1$-$C_4$ alkyl group represents a $C_1$-$C_4$ alkyl group substituted with 1 to 5 halogeno groups), a $C_3$-$C_6$ cycloalkyl group, a hydroxyl group, a $C_1$-$C_4$ alkoxy group, a halogeno $C_1$-$C_4$ alkoxy group (wherein said halogeno $C_1$-$C_4$ alkoxy group represents a $C_1$-$C_4$ alkoxy group substituted with 1 to 5 halogeno groups) or a halogeno group;

$Rf^6$ and $Rf^7$ may be the same or different and each represents a hydrogen atom or a $C_1$-$C_3$ alkyl group;

$Rf^8$ represents a group having the formula -$Xf^2Rf^{10}$ [wherein $Rf^{10}$ represents a group having the formula -$CORf^{11}$ [wherein $Rf^{11}$ represents a hydroxyl group, a $C_1$-$C_6$ alkoxy group, a ($C_3$-$C_8$ cycloalkyl)-($C_1$-$C_6$ alkyl)oxy group, a $C_3$-$C_8$ cycloalkyloxy group, an amino group, a $C_1$-$C_6$ alkylamino group, a [($C_3$-$C_8$ cycloalkyl)-($C_1$-$C_6$ alkyl)] amino group, a $C_3$-$C_8$ cycloalkylamino group, a di ($C_1$-$C_6$ alkyl) amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom) , a di [($C_3$-$C_8$ cycloalkyl) - ($C_1$-$C_6$ alkyl)]amino group, a di ($C_3$-$C_8$ cycloalkyl) amino group, a N-[($C_3$-$C_8$ cycloalkyl) - ($C_1$-$C_6$ alkyl) ] -N- ($C_1$-$C_6$ alkyl) amino group, a N- ($C_3$-$C_8$ cycloalkyl)-N-($C_1$-$C_6$ alkyl) amino group, a N- [($C_3$-$C_8$ cycloalkyl) - ($C_1$-$C_6$ alkyl) -N-($C_3$-$C_8$ cycloalkyl)amino group, a hydroxylamino group or a hydroxy ($C_1$-$C_6$ alkyl) amino group] ,

a group having the formula -$SO_2Rf^{12}$ [wherein $Rf^{12}$ represents a $C_1$-$C_6$ alkyl group, a ($C_3$-$C_8$ cycloalkyl) - ($C_1$-$C_6$ alkyl) group, a $C_3$-$C_8$ cycloalkyl group, an amino group, a $C_1$-$C_6$ alkylamino group, a [($C_3$-$C_8$ cycloalkyl) - ($C_1$-$C_6$ alkyl) ] amino group, a $C_3$-$C_8$ cycloalkylamino group, a di ($C_1$-$C_6$ alkyl)amino group (wherein said alkyl groups may be the same or different, and two of said alkyl groups may, together with the nitrogen atom of said amino group,

form a 5- to 7-membered saturated heterocyclyl group containing 1 to 3 atoms selected from the group consisting of a nitrogen atom, an oxygen atom and a sulfur atom), a di [($C_3$-$C_8$ cycloalkyl) - ($C_1$-$C_6$ alkyl)] amino group, a di ($C_3$-$C_8$ cycloalkyl) amino group, a N-[($C_3$-$C_8$ cycloalkyl) - ($C_1$-$C_6$ alkyl)]-N-($C_1$-$C_6$ alkyl) amino group, a N- ($C_3$-$C_8$ cycloalkyl)- N- ($C_1$-$C_6$ alkyl) amino group or a N- [($C_3$-$C_8$ cycloalkyl) - ($C_1$-$C_6$ alkyl)]-N-($C_3$-$C_8$ cycloalkyl) amino group], a group having the formula -N($Rf^{13}$)CO$Rf^{14}$ [wherein $Rf^{13}$ represents a hydrogen atom, a $C_1$-$C_6$ alkyl group, a ($C_3$-$C_8$ cycloalkyl) - ($C_1$-$C_6$ alkyl) group or a $C_3$-$C_8$ cycloalkyl group, and $Rf^{14}$ represents a hydrogen atom, a $C_1$-$C_6$ alkyl group, a ($C_3$-$C_8$ cycloalkyl) - ($C_1$-$C_6$ alkyl) group or a $C_3$-$C_8$ cycloalkyl group],

a group having the formula -N($Rf^{13}$)$SO_2Rf^{15}$ [wherein $Rf^{13}$ has the same meaning as defined above, and $Rf^{15}$ represents a $C_1$-$C_6$ alkyl group, a ($C_3$-$C_8$ cycloalkyl) - ($C_1$-$C_6$ alkyl) group or a $C_3$-$C_8$ cycloalkyl group], or a tetrazol-5-yl group, and

$Xf^2$ represents a single bond, a $C_1$-$C_4$ alkylene group or a substituted $C_1$-$C_4$ alkylene group (wherein said substituent (s) may be the same or different, and are 1 or 2 group(s) selected from Substituent group γf, or two of said substituents may together form an ethylene group or a trimethylene group)];

$Xf^1$ is a group having the formula -NH-, -N$Rf^{16}$-(wherein $Rf^{16}$ represents a $C_1$-$C_4$ alkyl group), -O-, -S-, - SO- or -$SO_2$-;

$Yf^1$ is a phenyl group, a substituted phenyl group (wherein said substituent(s) may be the same or different, and are 1 to 3 group(s) selected from Substituent group αf), a 5- to 6-membered aromatic heterocyclyl group or a substituted 5- to 6-membered aromatic heterocyclyl group (wherein said substituent(s) may be the same or different, and are 1 to 3 group(s) selected from Substituent group αf);

$Yf^2$ represents a 6- to 10-membered aryl group, a substituted 6- to 10-membered aryl group (wherein said substituent (s) may be the same or different, and are 1 to 3 group(s) selected from Substituent group βf), a 9- to 10-membered unsaturated cyclic hydrocarbon group (provided that $Yf^1$ is bound to a benzene ring moiety in said unsaturated cyclic hydrocarbon group), a substituted 9- to 10-membered unsaturated cyclic hydrocarbon group (provided that $Yf^1$ is bound to a benzene ring moiety in said unsaturated cyclic hydrocarbon group, and said substituent(s) may be the same or different, and are 1 to 3 group(s) selected from Substituent group βf), a 5- to 10-membered aromatic heterocyclyl group, a substituted 5- to 10-membered aromatic heterocyclyl group (wherein said substituent(s) may be the same or different, and are 1 to 3 group(s) selected from Substituent group βf), a 9- to 10-membered unsaturated heterocyclyl group (provided that $Yf^1$ is bound to an aromatic ring moiety in said unsaturated heterocyclyl group) or a substituted 9- to 10-membered unsaturated heterocyclyl group (provided that $Yf^1$ is bound to an aromatic ring moiety in said unsaturated heterocyclyl group, and said substituent(s) may be the same or different and are 1 to 3 group(s) selected from Substituent group (βf);

Substituent group αf represents the group consisting of a $C_1$-$C_4$ alkyl group, a halogeno $C_1$-$C_4$ alkyl group (wherein said halogeno $C_1$-$C_4$ alkyl group represents a $C_1$-$C_4$ alkyl group substituted with 1 to 5 halogeno groups), a hydroxyl group, a $C_1$-$C_4$ alkoxy group and a halogeno group;

Substituent group βf represents the group consisting of a $C_1$-$C_6$ alkyl group, a hydroxy($C_1$-$C_6$ alkyl) group, a carboxy ($C_1$-$C_6$ alkyl) group, a ($C_1$-$C_6$ alkoxy) carbonyl-($C_1$-$C_6$ alkyl) group, a halogeno $C_1$-$C_6$ alkyl group (wherein said halogeno $C_1$-$C_6$ alkyl group represents a $C_1$-$C_6$ alkyl group substituted with 1 to 7 halogeno groups), a ($C_3$-$C_8$ cycloalkyl)-($C_1$-$C_6$ alkyl) group, a $C_2$-$C_7$ alkenyl group, a $C_2$-$C_7$ alkynyl group, a $C_3$-$C_8$ cycloalkyl group, a hydroxyl group, a $C_1$-$C_6$ alkoxy group, a halogeno $C_1$-$C_6$ alkoxy group (wherein said halogeno $C_1$-$C_6$ alkoxy group represents a $C_1$-$C_6$ alkoxy group substituted with 1 to 7 halogeno groups), a $C_1$-$C_6$ alkylthio group, a $C_1$-$C_6$ alkylsulfinyl group, a $C_1$-$C_6$ alkylsulfonyl group, an amino group, a $C_1$-$C_6$ alkylamino group, a $C_3$-$C_8$ cycloalkylamino group, a di($C_1$-$C_6$ alkyl)amino group (wherein said alkyl groups may be the same or different and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a di($C_3$-$C_8$ cycloalkyl) amino group, a N-($C_3$-$C_8$ cycloalkyl)-N-($C_1$-$C_6$ alkyl)amino group, a formylamino group, a ($C_1$-$C_6$ alkyl) carbonylamino group, a ($C_3$-$C_8$ cycloalkyl)carbonylamino group, a N-[($C_1$-$C_6$ alkyl)carbonyl]-N-($C_1$-$C_6$ alkyl) amino group, a N-[($C_3$-$C_8$ cycloalkyl)carbonyl]-N-($C_1$-$C_6$ alkyl) amino group, a $C_1$-$C_6$ alkylsulfonylamino group, a N-($C_1$-$C_6$ alkylsulfonyl)-N-($C_1$-$C_6$ alkyl) amino group, a N-($C_1$-$C_6$ alkylsulfonyl)-N-($C_3$-$C_8$ cycloalkyl)amino group, a formyl group, a ($C_1$-$C_6$ alkyl)carbonyl group, a carboxyl group, a ($C_1$-$C_6$ alkoxy)carbonyl group, a carbamoyl group, a ($C_1$-$C_6$ alkylamino)carbonyl group, a ($C_3$-$C_8$ cycloalkylamino)carbonyl group, a di($C_1$-$C_6$ alkyl)aminocarbonyl group (wherein said alkyl groups may be the same or different and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a N-($C_3$-$C_8$ cycloalkyl)-N-($C_1$-$C_6$ alkyl)aminocarbonyl group, a cyano group, a nitro group and a halogeno group; and

Substituent group γf represents the group consisting of a $C_1$-$C_6$ alkyl group, a hydroxy($C_1$-$C_6$ alkyl) group, a ($C_1$-$C_6$ alkoxy) - ($C_1$-$C_6$ alkyl) group, a mercapto ($C_1$-$C_6$ alkyl) group, a ($C_1$-$C_6$ alkylthio) - ($C_1$-$C_6$ alkyl) group, a ($C_1$-$C_6$ alkylsulfinyl) - ($C_1$-$C_6$ alkyl) group, a ($C_1$-$C_6$ alkylsulfonyl)-($C_1$-$C_6$ alkyl) group, an amino ($C_1$-$C_6$ alkyl) group, a ($C_1$-$C_6$ alkylamino) - ($C_1$-$C_6$ alkyl) group, a ($C_3$-$C_8$ cycloalkylamino) - ($C_1$-$C_6$ alkyl) group, a di ($C_1$-$C_6$ alkyl) amino- ($C_1$-$C_6$ alkyl) group (wherein said alkyl groups may be the same or different, and two of said alkyl groups of the di($C_1$-$C_6$

alkyl)amino moiety may, together with the nitrogen atom of said amino group, form a 5- to 7-membered saturated heterocyclyl group containing 1 to 3 atoms selected from the group consisting of a nitrogen atom, an oxygen atom and a sulfur atom), a di ($C_3$-$C_8$ cycloalkyl) amino- ($C_1$-$C_6$ alkyl) group, a [N-($C_3$-$C_8$ cycloalkyl)-N-($C_1$-$C_6$ alkyl) amino]-($C_1$-$C_6$ alkyl) group, a hydroxyl group, a $C_1$-$C_6$ alkoxy group, a $C_3$-$C_8$ cycloalkyloxy group, a mercapto group, a $C_1$-$C_6$ alkylthio group, a $C_3$-$C_8$ cycloalkylthio group, a $C_1$-$C_6$ alkylsulfinyl group, a $C_3$-$C_8$ cycloalkylsulfinyl group, a $C_1$-$C_6$ alkylsulfonyl group, a $C_3$-$C_8$ cycloalkylsulfonyl group, an amino group, a $C_1$-$C_6$ alkylamino group, a $C_3$-$C_8$ cycloalkylamino group, a di($C_1$-$C_6$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a di($C_3$-$C_8$ cycloalkyl)amino group, a N-($C_3$-$C_8$ cycloalkyl)-N-($C_1$-$C_6$ alkyl) amino group, and a halogeno group).

**14.** A medicament according to claim 13, wherein in the general formula (If),

$Rf^1$ is a group having the formula $-CORf^{9a}$ [wherein $Rf^{9a}$ represents a $C_1$-$C_6$ alkyl group, a $C_1$-$C_8$ alkoxy group, a halogeno $C_1$-$C_6$ alkoxy group (wherein said halogeno $C_1$-$C_6$ alkoxy group represents a $C_1$-$C_6$ alkoxy group substituted with 1 to 7 halogeno groups), a $C_1$-$C_6$ alkylamino group or a di($C_1$-$C_6$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom)];

$Rf^2$ is a hydrogen atom, a trifluoromethyl group, a 2,2,2-trifluoroethyl group, a pentafluoroethyl group, a hydroxyl group, a fluoro group or a chloro group;

$Rf^3$ is a hydrogen atom, a $C_1$-$C_4$ alkyl group, a halogeno $C_1$-$C_4$ alkyl group (wherein said halogeno $C_1$-$C_4$ alkyl group represents a $C_1$-$C_4$ alkyl group substituted with 1 to 5 halogeno groups), a $C_3$-$C_5$ cycloalkyl group, a $C_2$-$C_4$ alkenyl group, a $C_2$-$C_4$ alkynyl group, a hydroxyl group, a $C_1$-$C_4$ alkoxy group, a halogeno $C_1$-$C_4$ alkoxy group (wherein said halogeno $C_1$-$C_4$ alkoxy group represents a $C_1$-$C_4$ alkoxy group substituted with 1 to 5 halogeno groups), a $C_1$-$C_4$ alkylthio group, a $C_1$-$C_4$ alkylsulfinyl group, a $C_1$-$C_4$ alkylsulfonyl group, an amino group, a $C_1$-$C_4$ alkylamino group, di($C_1$-$C_4$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a fluoro group, a chloro group or a bromo group;

$Rf^4$ and $Rf^5$ may be the same or different, and each is a hydrogen atom, a methyl group, an ethyl group, a trifluoromethyl group, a methoxy group, a fluoro group, a chloro group or a bromo group;

$Rf^6$ and $Rf^7$ may be the same or different, and each is a hydrogen atom or a methyl group;

$Rf^8$ represents a group having the formula $-Xf^{2a}Rf^{10a}$ [wherein $Rf^{10a}$ represents a group having the formula $-CORf^{11a}$ [wherein $Rf^{11a}$ represents a hydroxyl group, a $C_1$-$C_4$ alkoxy group, a ($C_3$-$C_6$ cycloakyl)-($C_1$-$C_4$ alkyl)oxy group, a $C_3$-$C_6$ cycloalkyloxy group, an amino group, a $C_1$-$C_4$ alkylamino group, a [($C_3$-$C_6$ cycloalkyl) - ($C_1$-$C_4$ alkyl)]amino group, a $C_3$-$C_6$ cycloalkylamino group, a di($C_1$-$C_4$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a hydroxylamino group or a hydroxyl ($C_1$-$C_4$ alkyl) amino group],

a group having the formula $-SO_2Rf^{12a}$ [wherein $Rf^{12a}$ represents a $C_1$-$C_4$ alkyl group, a ($C_3$-$C_6$ cycloalkyl)-($C_1$-$C_4$ alkyl) group, a $C_3$-$C_6$ cycloalkyl group, an amino group, a $C_1$-$C_4$ alkylamino group, a [ ($C_3$-$C_6$ cycloalkyl) - ($C_1$-$C_4$ alkyl)]amino group, a $C_3$-$C_6$ cycloalkylamino group or a di($C_1$-$C_4$ alkyl)amino group (wherein said alkyl groups may be the same or different, and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom)],

a group having the formula $-N(R^{13a})CORf^{14a}$ [wherein $Rf^{13a}$ represents a hydrogen atom, a $C_1$-$C_4$ alkyl group, a ($C_3$-$C_5$ cycloalkyl)-($C_1$-$C_2$ alkyl) group or a $C_3$-$C_5$ cycloalkyl group, and $Rf^{14a}$ represents a hydrogen atom, a $C_1$-$C_4$ alkyl group, a ($C_3$-$C_5$ cycloalkyl)-($C_1$-$C_2$ alkyl) group or a $C_3$-$C_5$ cycloalkyl group],

a group having the formula $-N(Rf^{13a})SO_2Rf^{15a}$ [wherein $Rf^{13a}$ has the same meaning as defined above, and $Rf^{15a}$ represents a $C_1$-$C_4$ alkyl group, a ($C_3$-$C_5$ cycloalkyl)-($C_1$-$C_2$ alkyl) group or a $C_3$-$C_5$ cycloalkyl group], or a tetrazol-5-yl group, and

$Xf^{2a}$ represents a single bond, a $C_1$-$C_2$ alkylene group or a substituted $C_1$-$C_2$ alkylene group (wherein said substituent (s) may be the same or different, and are 1 or 2 group(s) selected from Substituent group $\gamma$f1, or two of said substituents may together form an ethylene group or a trimethylene group)];

$Xf^1$ is a group having the formula -NH-, -O- or -S-;

$Yf^1$ is a phenyl group, a substituted phenyl group (wherein said substituent(s) may be the same or different, and are 1 or 2 group(s) selected from Substituent group $\alpha$f1), a 5- to 6-membered aromatic heterocyclyl group (wherein

said heterocyclyl group represents a pyrrolyl group, a furyl group, a thienyl group, an imidazolyl group, an oxazolyl group, a thiazolyl group, a pyridyl group or a pyridazinyl group), or a substituted 5- to 6-membered aromatic heterocyclyl group (wherein said heterocyclyl group is a pyrrolyl group, a furyl group, a thienyl group, an imidazolyl group, an oxazolyl group, a thiazolyl group, a pyridyl group or a pyridazinyl group, and said substituent(s) may be the same or different, and are 1 or 2 group(s) selected from Substituent group $\alpha$f1);

$Yf^2$ is a phenyl group, a substituted phenyl group (wherein said substituent(s) may be the same or different, and are 1 to 3 group(s) selected from Substituent group $\beta$f1), an indanyl group or a tetrahydronaphthyl group (provided that $Yf^1$ is bound to a benzene ring moiety in said indanyl group or said tetrahydronaphthyl group), a substituted indanyl group or a substituted tetrahydronaphthyl group (provided that $Yf^1$ is bound to a benzene ring moiety in said indanyl group or said tetrahydronaphthyl group, and said substituent(s) may be the same or different, and are 1 to 3 group(s) selected from Substituent group $\beta$f1), a 5- to 6-membered aromatic heterocyclyl group (wherein said heterocyclyl group represents a pyrrolyl group, a furyl group, a thienyl group, an imidazolyl group, an oxazolyl group, a thiazolyl group, a pyridyl group or a pyrimidinyl group), a substituted 5- to 6-membered aromatic heterocyclyl group (said heterocyclyl group represents a pyrrolyl group, a furyl group, a thienyl group, an imidazolyl group, an oxazolyl group, a thiazolyl group, a pyridyl group or a pyrimidinyl group, and said substituent(s) may be the same or different, and are 1 to 3 group(s) selected from Substituent group $\beta$f1), a 9- to 10-membered unsaturated heterocyclyl group (provided that $Yf^1$ is bound to an aromatic ring moiety in said unsaturated heterocyclyl group, and said unsaturated heterocyclyl group represents an indolinyl group, a dihydrobenzofuryl group, a dihydrobenzothienyl group, a tetrahydroquinolyl group or a cromanyl group) or a substituted 9- to 10-membered unsaturated heterocyclyl group (provided that $Yf^1$ is bound to an aromatic ring moiety in said unsaturated heterocyclyl group, said unsaturated heterocyclyl group represents an indolinyl group, a dihydrobenzofuryl group, a dihydrobenzothienyl group, a tetrahydroquinolyl group or a cromanyl group, and said substituent(s) may be the same or different and are 1 to 3 group(s) selected from Substituent group ($\beta$f1);

Substituent group $\alpha$f1 is the group consisting of a methyl group, an ethyl group, a trifluoromethyl group, a methoxy group, an ethoxy group, a fluoro group and a chloro group;

Substituent group $\beta$f1 is the group consisting of a $C_1$-$C_6$ alkyl group, a hydroxy($C_1$-$C_6$ alkyl) group, a carboxy($C_1$-$C_4$ alkyl) group, a ($C_1$-$C_4$ alkoxy)carbonyl-($C_1$-$C_4$ alkyl) group, a halogeno $C_1$-$C_4$ alkyl group (wherein said halogeno $C_1$-$C_4$ alkyl group represents a $C_1$-$C_4$ alkyl group substituted with 1 to 5 halogeno groups), a ($C_3$-$C_6$ cycloalkyl)-($C_1$-$C_4$ alkyl) group, a $C_2$-$C_5$ alkenyl group, a $C_2$-$C_5$ alkynyl group, a $C_3$-$C_6$ cycloalkyl group, a hydroxyl group, a $C_1$-$C_4$ alkoxy group, a halogeno $C_1$-$C_4$ alkoxy group (wherein said halogeno $C_1$-$C_4$ alkoxy group represents a $C_1$-$C_4$ alkoxy group substituted with 1 to 5 halogeno groups), a $C_1$-$C_4$ alkylthio group, a $C_1$-$C_4$ alkylsulfinyl group, a $C_1$-$C_4$ alkylsulfonyl group, an amino group, a $C_1$-$C_4$ alkylamino group, a $C_3$-$C_6$ cycloalkylamino group, a di($C_1$-$C_4$ alkyl)amino group (wherein said alkyl groups may be the same or different and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a formylamino group, a ($C_1$-$C_4$ alkyl) carbonylamino group, a ($C_3$-$C_6$ cycloalkyl)carbonylamino group, a N-[($C_1$-$C_4$ alkyl)carbonyl]-N-($C_1$-$C_4$ alkyl) amino group, a N-[($C_3$-$C_6$ cycloalkyl)carbonyl]-N-($C_1$-$C_4$ alkyl) amino group, a $C_1$-$C_4$ alkylsulfonylamino group, a N- ($C_1$-$C_4$ alkylsulfonyl)-N-($C_1$-$C_4$ alkyl)amino group, a formyl group, a ($C_1$-$C_4$ alkyl)carbonyl group, a carboxyl group, a ($C_1$-$C_4$ alkoxy)carbonyl group, a carbamoyl group, a ($C_1$-$C_4$ alkylamino)carbonyl group, a di($C_1$-$C_4$ alkyl) aminocarbonyl group (wherein said alkyl groups may be the same or different and two of said alkyl groups may, together with the nitrogen atom of said amino group, form a 5- to 7-membered saturated heterocyclyl group containing 1 to 3 atoms selected from the group consisting of a nitrogen atom, an oxygen atom and a sulfur atom), a cyano group, a nitro group, a fluoro group, a chloro group and a bromo group; and

Substituent group $\gamma$f1 is the group consisting of a methyl group, an ethyl group, a hydroxymethyl group, a hydroxyethyl group, a methoxymethyl group, a methoxyethyl group, a methylthiomethyl group, a methylthioethyl group, an aminomethyl group, an aminoethyl group, a methylaminomethyl group, an ethylaminomethyl group, a methylaminoethyl group, a cyclopropylaminomethyl group, a cyclopropylaminoethyl group, a dimethylaminomethyl group, a dimethylaminoethyl group, a (N-methyl-N-ethylamino)methyl group, a dicyclopropylaminomethyl group, a hydroxyl group, a methoxy group, an ethoxy group, a cyclopropyloxy group, a methylthio group, an ethylthio group, a cyclopropylthio group, an amino group, a methylamino group, an ethylamino group, a cyclopropylamino group, a cyclobutylamino group, a dimethylamino group, a diethylamino group, a dicyclopropylamino group, a N-cyclopropyl-N-methylamino group, a fluoro group and a chloro group.

**15.** A medicament according to claim 13, wherein in the general formula (If),

$Rf^1$ is a group having the formula -CO$Rf^{9b}$ [wherein $Rf^{9b}$ represents a $C_1$-$C_6$ alkoxy group or a halogeno $C_1$-$C_4$ alkoxy group (wherein said halogeno $C_1$-$C_4$ alkoxy group represents a $C_1$-$C_4$ alkoxy group substituted with 1 to 5 halogeno groups)];

$Rf^2$ is a hydrogen atom or a hydroxyl group;

$Rf^3$ is a hydrogen atom, a $C_1$-$C_4$ alkyl group, a halogeno $C_1$-$C_4$ alkyl group (wherein said halogeno $C_1$-$C_4$ alkyl group represents a $C_1$-$C_4$ alkyl group substituted with 1 to 5 halogeno groups), a $C_3$-$C_5$ cycloalkyl group, a $C_2$-$C_4$ alkenyl group, a $C_1$-$C_4$ alkoxy group, a fluoro group or a chloro group;
$Rf^4$ and $Rf^5$ may be the same or different, and each is a hydrogen atom, a methyl group, an ethyl group, a trifluoromethyl group, a methoxy group, a fluoro group, a chloro group or a bromo group;
$Rf^6$ and $Rf^7$ may be the same or different, and each is a hydrogen atom or a methyl group;
$Rf^8$ is a group having the formula $-Xf^{2b}Rf^{10b}$ [wherein $Rf^{10b}$ represents a group having the formula $-CORf^{11b}$ [wherein $Rf^{11b}$ represents a hydroxyl group, a $C_1$-$C_4$ alkoxy group, a ($C_3$-$C_5$ cycloalkyl)-($C_1$-$C_2$ alkyl)oxy group, a $C_3$-$C_5$ cycloalkyloxy group, an amino group, a methylamino group, an ethylamino group, a dimethylamino group, a diethylamino group, a methylethylamino group or a hydroxylamino group],
a group having the formula $-SO_2Rf^{12b}$ [wherein $Rf^{12b}$ represents a $C_1$-$C_4$ alkyl group, a ($C_3$-$C_5$ cycloalkyl) - ($C_1$-$C_2$ alkyl) group or a $C_3$-$C_5$ cycloalkyl group], or a tetrazol-5-yl group, and
$Xf^{2b}$ represents a single bond, a methylene group, an ethylene group or a substituted methylene group or a substituted ethylene group (wherein said substituent(s) may be the same or different, and are 1 or 2 group(s) selected from Substituent group γf2, or two of said substituents may together form an ethylene group or a trimethylene group)];
$Xf^1$ is a group having the formula -NH-, -O- or -S-;
$Yf^1$ is a phenyl group (wherein the substitution positions at which $Xf^1$,and $Yf^2$ bind to said phenyl group are the 1 and 3 positions or the 1 and 4 positions), a substituted phenyl group (wherein said substituent is one group selected from Substituent group αf2, and the substitution positions at which $Xf^1$ and $Yf^2$ bind to said phenyl group are the 1 and 3 positions or the 1 and 4 positions), a thienyl group (wherein the substitution positions at which $Xf^1$ and $Yf^2$ bind to said thienyl group are the 2 and 5 positions), a substituted thienyl group (wherein said substituent is one group selected from Substituent group αf2, and the substitution positions at which $Xf^1$ and $Yf^2$ bind to said thienyl group are the 2 and 5 positions), a pyridyl group (wherein the substitution positions at which $Xf^1$ and $Yf^2$ bind to said pyridyl group are the 2 and 5 positions or the 3 and 6 positions) or a substituted pyridyl group (wherein said substituent is one group selected from Substituent group αf2, and the substitution positions at which $Xf^1$ and $Yf^2$ bind to said pyridyl group are the 2 and 5 positions or the 3 and 6 positions) ;
$Yf^2$ is a phenyl group (wherein the substitution positions at which $Yf^1$ and $Rf^8$ bind to said phenyl group are the 1 and 3 positions or the 1 and 4 positions), a substituted phenyl group (wherein said substituent(s) may be the same or different, and are 1 or 2 group(s) selected from Substituent group βf2, and the substitution positions at which $Yf^1$ and $Rf^8$ bind to said phenyl group are the 1 and 3 positions or the 1 and 4 positions), a thienyl group (wherein the substitution positions at which $Yf^1$ and $Rf^8$ bind to said thienyl group are the 2 and 5 positions), a substituted thienyl group (wherein said substituent(s) may be the same or different, and are 1 or 2 group(s) selected from Substituent group βf2, and the substitution positions at which $Yf^1$ and $Rf^8$ bind to said thienyl group are the 2 and 5 positions), a thiazolyl group (wherein the substitution positions at which $Yf^1$ and $Rf^8$ bind to said thiazolyl group are the 2 and 5 positions), a substituted thiazolyl group (wherein said substituent(s) may be the same or different, and are 1 or 2 group(s) selected from Substituent group βf2, and the substitution positions at which $Yf^1$ and $Rf^8$ bind to said thiazolyl group are the 2 and 5 positions), a pyridyl group (wherein the substitution positions at which $Yf^1$ and $Rf^8$ bind to said pyridyl group are the 2 and 5 positions) or a substituted pyridyl group (wherein said substituent(s) may be the same or different, and are 1 or 2 group(s) selected from Substituent group βf2, and the substitution positions at which $Yf^1$ and $Rf^8$ bind to said pyridyl group are the 2 and 5 positions);
Substituent group αf2 is the group consisting of a methyl group, a fluoro group and a chloro group;
Substituent group βf2 is the group consisting of a $C_1$-$C_4$ alkyl group, a hydroxymethyl group, a 1-hydroxyethyl group, a trifluoromethyl group, a 2,2,2-trifluoroethyl group, a pentafluoroethyl group, a $C_2$-$C_4$ alkenyl group, a $C_2$-$C_4$ alkynyl group, a $C_3$-$C_4$ cycloalkyl group, a hydroxyl group, a methoxy group, an ethoxy group, a methanesulfonyl group, an ethanesulfonyl group, an amino group, a methylamino group, an ethylamino group, a dimethylamino group, a diethylamino group, a formyl group, a methylcarbonyl group, an ethylcarbonyl group, a cyano group, a nitro group, a fluoro group and a chloro group; and
Substituent group γf2 is the group consisting of a methyl group, an ethyl group, a hydroxymethyl group, a methoxymethyl group, an aminomethyl group, a methylaminomethyl group, a dimethylaminomethyl group, a (N-methyl-N-ethylamino)methyl group, a methoxy group, a methylamino group, a dimethylamino group, a fluoro group and a chloro group.

**16.** A medicament according to claim 13, wherein in the general formula (If),
$Rf^1$ is a group having the formula $-CORf^{9c}$ (wherein, $Rf^{9c}$ represents a $C_3$-$C_5$ alkoxy group);
$Rf^2$ is a hydroxyl group;
$Rf^3$ is a methyl group, an ethyl group, a 2-propyl group, a 2-methyl-2-propyl group, a trifluoromethyl group, a 2,2,2-trifluoroethyl group, a cyclopropyl group or a vinyl group;
$Rf^4$ and $Rf^5$ are a hydrogen atom;

$Rf^6$ and $Rf^7$ are a hydrogen atom;
$Rf^8$ is a group having the formula $-Xt^{2c}Rf^{10c}$ [wherein $Rf^{10c}$ represents a group having the formula $-CORf^{11c}$ (wherein $Rf^{11c}$ represents a hydroxyl group or a methoxy group), or
a group having the formula $-SO_2Rf^{12c}$ (wherein $Rf^{12c}$ represents a methyl group), and
$Xf^{2c}$ represents a single bond, a methylene group or a substituted methylene group (wherein said substituent represents a hydroxymethyl group, or two substituents may together form an ethylene group)];
$Xf^1$ is a group having the formula -O- ;
$Yf^1$ is a phenyl group (wherein the substitution positions at which $Xf^1$ and $Yf^2$ bind to said phenyl group are the 1 and 4 positions);
$Yf^2$ represents a phenyl group (wherein the substitution positions at which $Yf^1$ and $Rf^8$ bind to said phenyl group are the 1 and 4 positions), a substituted phenyl group (wherein said substituent is one group selected from Substituent group βf3, and the substitution positions at which $Yf^1$ and $Rf^8$ bind to said phenyl group are the 1 and 4 positions) or a substituted phenyl group (wherein said substituent is one group selected from Substituent group βf3, and the substitution positions at which $Yf^1$, $Rf^8$ and the group selected from Substituent group βf3 bind to said phenyl group are the 1, 3 and 2 positions, respectively); and
Substituent group βf3 is the group consisting of a methyl group, an ethyl group, a 2-propyl group, a hydroxymethyl group, a trifluoromethyl group, a cyclopropyl group, a methoxy group, a methanesulfonyl group, an amino group, a methylamino group, a dimethylamino group, a methylcarbonyl group, an ethylcarbonyl group, a cyano group, a nitro group, a fluoro group and a chloro group.

**17.** A medicament according to claim 13, wherein in the general formula (If),
$Rf^1$ is a group having the formula $-CORf^{9d}$ (wherein, $Rf^{9d}$ represents a 2-methyl-2-propoxy group);
$Rf^2$ is a hydroxyl group;
$Rf^3$ is a trifluoromethyl group;
$Rf^4$ and $Rf^5$ are a hydrogen atom;
$Rf^6$ and $Rf^7$ are a hydrogen atom;
$Rf^8$ is a group having the formula $-Xf^{2d}Rf^{10d}$ [wherein $Rf^{10d}$ represents a group having the formula $-CORf^{11d}$ (wherein $Rf^{11d}$ represents a hydroxyl group), and
$Xf^{2d}$ is a methylene group or a substituted methylene group (wherein two of said substituents together form an ethylene group)];
$Xf^1$ is a group having the formula -O-;
$Yf^1$ is a phenyl group (wherein the substitution positions at which $Xf^1$ and $Yf^2$ bind to said phenyl group are the 1 and 4 positions); and,
$Yf^2$ is a phenyl group (wherein the substitution positions at which $Yf^1$ and $Rf^8$ bind to said phenyl group are the 1 and 4 positions), a substituted phenyl group (wherein said substituent is one group selected from Substituent group βf3, and the substitution positions at which $Yf^1$ and $Rf^8$ bind to said phenyl group are the 1 and 4 positions) or a substituted phenyl group (wherein said substituent is one group selected from Substituent group βf3, and the substitution positions at which $Yf^1$, $Rf^8$ and the group selected from Substituent group βf3 bind to said phenyl group are the 1, 3 and 2 positions, respectively).

**18.** A medicament according to claim 13, wherein the LXR ligand is a compound selected from the group consisting of
(4'-{[2-(tert-Butoxycarbonyl)-3-hydroxy-4-(trifluoromethyl)benzyl]oxy}-1,1'-biphenyl-4-yl)acetic acid,
1- (4'-{[2- (tert-Butoxycarbonyl) -3-hydroxy-4-(trifluoromethyl)benzyl]oxy}-1,1'-biphenyl-4-yl)cyclopropanecarboxylic acid,
2-(4'-{[2-(tert-Butoxycarbonyl)-3-hydroxy-4-(trifluoromethyl)benzyl]oxy}-1,1'-biphenyl-4-yl)-3-hydroxypropanoic acid,
(4'-{[2-(tert-Butoxycarbonyl)-3-hydroxy-4-(trifluoromethyl)benzyl]oxy}-2-methyl-1,1'-biphenyl-3-yl)acetic acid,
(4'-{[2-(tert-Butoxycarbonyl)-3-hydroxy-4-(trifluoromethyl)benzyl]oxy}-2-methyl-1,1'-biphenyl-4-yl)acetic acid,
(4'-{[2-(tert-Butoxycarbonyl)-3-hydroxy-4-(trifluoromethyl)benzyl]oxy}-2-chloro-1,1'-biphenyl-4-yl)acetic acid,
(4'-{[2-(tert-Butoxycarbonyl)-3-hydroxy-4-(trifluoromethyl)benzyl]oxy}-3-fluoro-1,1'-biphenyl-4-yl)acetic acid,
(4'-{[2-(tert-Butoxycarbonyl)-3-hydroxy-4-(trifluoromethyl)benzyl]oxy}-3-chloro-1,1'-biphenyl-4-yl)acetic acid,
1-(4'-{[2-(tert-Butoxycarbonyl)-3-hydroxy-4-(trifluoromethyl)benzyl]oxy}-3-fluoro-1,1'-biphenyl-4-yl)cyclopropanecarboxylic acid,
(4'-{[2-(tert-Butoxycarbonyl)-3-hydroxy-4-(trifluoromethyl)benzyl]oxy}-3-methoxy-1,1'-biphenyl-4-yl)acetic acid,
(4'-{[2-(tert-Butoxycarbonyl)-3-hydroxy-4-(trifluoromethyl)benzyl]oxy}-2-trifluoromethyl-1,1'-biphenyl-4-yl)acetic acid,
tert-Butyl 6-[({2'-ethyl-4'-[(methoxycarbonyl)methyl]-1,1'-biphenyl-4-yl}oxy)methyl]-2-hydroxy-3-(trifluoromethyl)benzoate,

(4'-{[2-(tert-Butoxycarbonyl)-3-hydroxy-4-(trifluoromethyl)benzyl]oxy}-2-ethyl-1,1'-biphenyl-4-yl)acetic acid,
(4'-{[2-(tert-Butoxycarbonyl)-3-hydroxy-4-(trifluoromethyl)benzyl]oxy}-2-nitro-1,1'-biphenyl-4-yl)acetic acid,
(2-Amino-4'-{[2-(tert-butoxycarbonyl)-3-hydroxy-4-(trifluoromethyl)benzyl]oxy}-1,1'-biphenyl-4-yl)acetic acid,
(4'-{[2-(tert-Butoxycarbonyl)-3-hydroxy-4-(trifluoromethyl)benzyl]oxy}-2-formyl-1,1'-biphenyl-4-yl)acetic acid,
(4'-{[2-(tert-Butoxycarbonyl)-3-hydroxy-4-(trifluoromethyl)benzyl]oxy}-2-(hydroxymethyl)-1,1'-biphenyl-4-yl)acetic acid, and
(4'-{[2-(tert-Butoxycarbonyl)-3-hydroxy-4-(trifluoromethyl)benzyl]oxy}-2-cyano-1,1'-biphenyl-4-yl)acetic acid,
or a pharmacologically acceptable salt or ester thereof.

**19.** A medicament according to any one of claims 1 to 7, wherein the LXR ligand is
N-(2,2,2-Trifluoroethyl)-N-{4-[2,2,2-trifluoro-1-hydroxy-1-(trifluoromethyl)ethyl]phenyl}benzenesulfonamide,
3-Chloro-4-(3-(2-propyl-3-trifluoromethyl-6-benz-[4,5]-isoxazoloxy)propylthio)phenylacetic acid,
2-(3-{3-[[2-Chloro-3-(trifluoromethyl)benzyl](2,2-diphenylethyl)amino]propoxy}-phenyl)acetic acid,
2-Benzyl-6,7-dimethoxy-3-[4-[2,2,2-trifluoro-1-hydroxy-1-(trifluoromethyl)ethyl]phenyl]-4(3H)-quinazolinone,
2-Benzyl-6-(2-hydroxyethoxy)-3-[4-[2,2,2-trifluoro-1-hydroxy-1- (trifluoromethyl) ethyl] phenyl] -4 (3H) - quinazolinone,
2-Benzyl-6- (2-pyridyl) -3- [4- [2, 2,2-trifluoro-1-hydroxy-1- (trifluoromethyl) ethyl] phenyl] -4 (3H) - quinazolinone,
6-(1H-Imidazol-1-yl)-2-(4-methylbenzyl)-3-{2-methyl-4- [2,2,2-trifluoro-1-hydroxy-1-(trifluoromethyl)ethyl]phenyl}-4(3H)-quinazolinone,
2-Benzyl-6-fluoro-3-{3-methoxy-4-[2,2,2-trifluoro-1-hydroxy-1-(trifluoromethyl)ethyl]phenyl}-7-(4-morpholinyl)-4(3H)-quinazolinone,
3-{2-Methyl-4-[2,2,2-trifluoro-1-hydroxy-1-(trifluoromethyl)ethyl]phenyl}-2-(3-pyridylmethyl)-6-(1H-1,2,4-triazol-1-yl)-4(3H)-quinazolinone,
N,N-Dimethyl-3β-hydroxycholenamide,
6-Chloro-7-methoxy-3-{2-methyl-5-[2,2,2-trifluoro-1-hydroxy-1-(trifluoromethyl)ethyl]phenyl}-2-(3-thienylmethyl)-4(3H)-quinazolinone,
2-(3- Fluorobenzyl)- 6,7- dimethoxy- 3- {2- methyl- 5-[2,2,2- trifluoro- 1- hydroxy- 1-(trifluoromethyl) ethyl] phenyl}- 4(3H)-quinazolinone,
tert-Butyl 2-({4-[acetyl(methyl)amino]phenoxy}methyl)-6-fluoro-1H-indol-1-carboxylate,
tert-Butyl 2-({4-[(cyclopropylcarbonothioyl)(methyl)amino]phenoxy}methyl)-4,6-difluoro-1H-indol-1-carboxylate,
tert-Butyl 6-({4-[(cyclopropylcarbonyl)(methyl)amino]phenoxy}methyl)-2-hydroxy-3-(trifluoromethyl)benzoate,
tert-Butyl 2-hydroxy-6-({4-[methyl(methylsulfonyl)amino]phenoxy}methyl)-3-(trifluoromethyl)benzoate,
tert-Butyl 6-({4-[acetyl(methyl)amino]phenoxy}methyl)-3-ethyl-2-hydroxybenzoate,
tert-Butyl 6-({4-[(cyclopropylacetyl)(methyl)amino]phenoxy}methyl)-2-hydroxy-3-(trifluoromethyl)benzoate,
(4'-{[1-(tert-Butoxycarbonyl)-6-fluoro-1H-indol-2-yl]methoxy}-1,1'-biphenyl-4-yl)acetic acid,
(4'-{[1-(tert-Butoxycarbonyl)-6-fluoro-1H-indol-2-yl]methoxy}-3-chloro-1,1'-biphenyl-4-yl)acetic acid,
[5-(4-{[1-(tert-Butoxycarbonyl)-6-fluoro-1H-indol-2-yl]methoxy}phenyl)-2-thienyl]acetic acid,
(4'-{[1-(tert-Butoxycarbonyl)-6-fluoro-1H-indol-2-yl]methoxy}-2-chloro-1,1'-biphenyl-4-yl)acetic acid,
(4'-{[2-(tert-Butoxycarbonyl)-3-hydroxy-4-(trifluoromethyl)benzyl]oxy}-1,1'-biphenyl-4-yl)acetic acid,
(4'-{[2-(tert-Butoxycarbonyl)-3-hydroxy-4-(trifluoromethyl)benzyl]oxy}-3-chloro-1,1'-biphenyl-4-yl)acetic acid,
(4'-{[2-(tert-Butoxycarbonyl)-3-hydroxy-4-(trifluoromethyl)benzyl]oxy}-2-ethyl-1,1'-biphenyl-4-yl)acetic acid,
(4'-{[2-(tert-Butoxycarbonyl)-3-hydroxy-4-(trifluoromethyl)benzyl]oxy}-2-methyl-1,1'-biphenyl-3-yl)acetic acid,
1-(4'-{[2-(tert-Butoxycarbonyl)-3-hydroxy-4-(trifluoromethyl) benzyl]oxy}-3-fluoro-1,1'-biphenyl-4-yl)cyclopropanecarboxylic acid,
(4'-{[2-(tert-Butoxycarbonyl)-3-hydroxy-4-isopropylbenzyl]oxy}-1,1'-biphenyl-4-yl)acetic acid,
(4'-{[2-(tert-Butoxycarbonyl)-4-fluoro-3-hydroxybenzyl]oxy}-1,1'-biphenyl-4-yl)acetic acid,
(4'-{[2-(tert-Butoxycarbonyl)-4-chloro-3-hydroxybenzyl]oxy}-1,1'-biphenyl-4-yl)acetic acid,
[5-(4-{[2-(tert-Butoxycarbonyl)-3-hydroxy-4-(trifluoromethyl)benzyl]oxy}phenyl)-2-thienyl]acetic acid,
(4'-{[2-(tert-Butoxycarbonyl)-3-hydroxy-4-(trifluoromethyl)benzyl]oxy}-2-nitro-1,1'-biphenyl-4-yl)acetic acid,
(4'-{[2-(tert-Butoxycarbonyl)-3-hydroxy-4-(trifluoromethyl)benzyl]oxy}-3-fluoro-1,1'-biphenyl-4-yl)acetic acid,
(4'-{[2-(tert-Butoxycarbonyl)-3-hydroxy-4-(trifluoromethyl)benzyl]oxy}-2-methyl-1,1'-biphenyl-4-yl)acetic acid,
(4'-{[2-(tert-Butoxycarbonyl)-3-hydroxy-4-(trifluoromethyl)benzyl]oxy}-2-methoxy-1,1'-biphenyl-4-yl)acetic acid,
(4'-{[2-(tert-Butoxycarbonyl)-3-hydroxy-4-(trifluoromethyl)benzyl]oxy}-2-chloro-1,1'-biphenyl-4-yl)acetic acid,
(4'-{[2-(tert-Butoxycarbonyl)-3-hydroxy-4-(trifluoromethyl)benzyl]oxy}-2-trifluoromethyl-1,1'-biphenyl-4-yl)acetic acid,
1-(4'-{[2-(tert-Butoxycarbonyl)-3-hydroxy-4-(trifluoromethyl)benzyl]oxy}-1,1'-biphenyl-4-yl)cyclopropanecarboxylic acid,
tert-Butyl 2-hydroxy-6-({[3'-(methylsulfonyl)-1,1'-biphenyl-4-yl]oxy}methyl)-3-(trifluoromethyl)benzoate,

(2-Amino-4'-{[2-(tert-butoxycarbonyl)-3-hydroxy-4-(trifluoromethyl)benzyl]oxy}-1,1'-biphenyl-4-yl)acetic acid,
[4'-{[2-(tert-Butoxycarbonyl)-3-hydroxy-4-(trifluoromethyl)benzyl]oxy}-2-(dimethylamino)-1,1'-biphehyl-4-yl]acetic acid,
2-(4'-{[2-(tert-Butoxycarbonyl)-3-hydroxy-4-(trifluoromethyl)benzyl]oxy}-1,1'-biphenyl-4-yl)-3-hydroxypropanoic acid,
(4'-{[2-(tert-Butoxycarbonyl)-3-hydroxy-4-(trifluoromethyl)benzyl]oxy}-2-isopropyl-1,1'-biphenyl-4-yl)acetic acid,
4'-{[2-(tert-Butoxycarbonyl)-3-hydroxy-4-(trifluoromethyl)benzyl]oxy}-1,1'-biphenyl-4-carboxylic acid,
(4'-{[2-(tert-Butoxycarbonyl)-3-hydroxy-4-(trifluoromethyl)benzyl]oxy}-2-formyl-1,1'-biphenyl-4-yl)acetic acid,
(4'-{[2-(tert-Butoxycarbonyl)-3-hydroxy-4-(trifluoromethyl)benzyl]oxy}-2-(hydroxymethyl)-1,1'-biphenyl-4-yl)acetic acid, or
(4'-{[2-(tert-Butoxycarbonyl)-3-hydroxy-4-(trifluoromethyl)benzyl]oxy)-2-cyano-1,1'-biphenyl-4-yl)acetic acid.

**20.** A medicament according to any one of claims 1 to 7, wherein the LXR ligand is
2-Benzyl-6,7-dimethoxy-3-[4-[2,2,2-trifluoro-1-hydroxy-1-(trifluoromethyl) ethyl] phenyl] -4 (3H) - quinazolinone,
2-Benzyl-6-(2-hydroxyethoxy)-3-[4-[2,2,2-trifluoro-1-hydroxy-1-(trifluoromethyl)ethyl]phenyl]-4(3H)-quinazolinone,
3-{2-Methyl-4-[2,2,2-trifluoro-1-hydroxy-1-(trifluoromethyl)ethyl]phenyl}-2-(3-pyridylmethyl)-6-(1H-1,2,4-triazoly-1-yl)-4(3H)-quinazolinone,
tert-Butyl 2-({4-[(cyclopropylcarbonothioyl)(methyl)amino]phenoxy}methyl)-4,6-difluoro-1H-indol-1-carboxylate,
tert-Butyl 6-({4-[(cyclopropylcarbonyl)(methyl)amino]phenoxy}methyl)-2-hydroxy-3-(trifluoromethyl)benzoate,
tert-Butyl 2-hydroxy-6-({4-[methyl(methylsulfonyl)amino]phenoxy}methyl)-3-(trifluoromethyl)benzoate,
(4'-{[1-(tert-Butoxycarbonyl)-6-fluoro-1H-indol-2-yl]methoxy}-1,1'-biphenyl-4-yl)acetic acid,
(4'-f[2-(tert-Butoxycarbonyl)-3-hydroxy-4-(trifluoromethyl)benzyl]oxy}-1,1'-biphenyl-4-yl)acetic acid,
(4'-{[2-(tert-Butoxycarbonyl)-3-hydroxy-4-(trifluoromethyl) benzyl] oxy}-3-chloro-1,1'-biphenyl-4-yl) acetic acid,
(4'-{[2-(tert-Butoxycarbonyl)-3-hydroxy-4-(trifluoromethyl)benzyl]oxy}-2-ethyl-1,1'-biphenyl-4-yl)acetic acid,
(4'-{[2-(tert-Butoxycarbonyl)-3-hydroxy-4-(trifluoromethyl)benzyl]oxy}-2-methyl-1,1'-biphenyl-3-yl)acetic acid,
(4'-{[2-(tert-Butoxycarbonyl)-3-hydroxy-4-(trifluoromethyl)benzyl]oxy}-3-fluoro-1,1'-biphenyl-4-yl)acetic acid,
(4'-{[2-(tert-Butoxycarbonyl)-3-hydroxy-4-(trifluoromethyl)benzyl]oxy}-2-methoxy-1,1'-biphenyl-4-yl)acetic acid,
(4'-{[2-(tert-Butoxycarbonyl)-3-hydroxy-4-(trifluoromethyl) benzyl] oxy}-2-chloro-1,1'-biphenyl-4-yl)acetic acid,
tert-Butyl 2-hydroxy-6-({[3'-(methylsulfonyl)-1,1'-biphenyl-4-yl]oxy}methyl)-3-(trifluoromethyl)benzoate,
2-(4'-{[2-(tert-Butoxycarbonyl)-3-hydroxy-4-(trifluoromethyl)benzyl]oxy}-1,1'-biphenyl-4-yl)-3-hydroxypropanoic acid,
(4'-{[2-(tert-Butoxycarbonyl)-3-hydroxy-4-(trifluoromethyl)benzyl]oxy}-2-formyl-1,1'-biphenyl-4-yl)acetic acid,
(4'-{[2-(tert-Butoxycarbonyl)-3-hydroxy-4-(trifluoromethyl)benzyl]oxy}-2-(hydroxymethyl)-1,1'-biphenyl-4-yl)acetic acid, or
(4'-{[2-(tert-Butoxycarbonyl)-3-hydroxy-4-(trifluoromethyl)benzyl]oxy}-2-cyano-1,1'-biphenyl-4-yl)acetic acid.

**21.** A method for inhibiting production of tissue factor comprising the step of administering an effective amount of an LXR ligand to a warm-blooded animal.

**22.** A method according to claim 21 which has an activity of decreasing thrombus formation.

**23.** A method for treatment and/or prophylaxis of a disease selected from a group consisting of vascular restenosis following angioplasty, endarterectomy, percutaneous transluminal coronary angioplasty (PTCA) or stent implantation, comprising a step of administering an effective amount of an LXR ligand to a warm-blooded animal.

**24.** A method for treatment and/or prophylaxis of blood coagulation diseases, diseases induced by platelet aggregation including stable or unstable angina pectoris, cardiovascular and cerebrovascular diseases including thromboembolism formation diseases accompanying diabetes, rethrombosis following thrombolysis, cerebral ischemic attack, infarction, stroke, ischemia-derived dementia, peripheral artery disease, thromboembolism formation diseases during use of an aorta-coronary artery bypass, glomerulosclerosis, renal embolism, tumor or cancer metastasis, comprising a step of administering an effective amount of an LXR ligand to a warm-blooded animal.

**25.** A method for treatment and/or prophylaxis of blood coagulation diseases, comprising a step of administering an effective amount of an LXR ligand to a warm-blooded animal.

**26.** A method according to any one of claims 21 to 25, wherein the LXR ligand is an LXR agonist or LXR antagonist.

**27.** A method according to any one of claims 21 to 25, wherein the LXR ligand is an LXR agonist.

**28.** A method according to any one of claims 21 to 27, wherein the LXR ligand is a compound represented by the general formula (Ia) described in claim 8 or a pharmacologically acceptable salt or ester thereof.

**29.** A method according to any one of claims 21 to 27, wherein the LXR ligand is a compound represented by the general formula (Ib) described in claim 9 or a pharmacologically acceptable salt or ester thereof.

**30.** A method according to any one of claims 21 to 27, wherein the LXR ligand is a compound represented by the general formula (Ic) described in claim 10 or a pharmacologically acceptable salt or ester thereof.

**31.** A method according to any one of claims 21 to 27, wherein the LXR ligand is a compound represented by the general formula (Id) described in claim 11 or a pharmacologically acceptable salt or ester thereof.

**32.** A method according to any one of claims 21 to 27, wherein the LXR ligand is a compound represented by the general formula (Ie) described in claim 12 or a pharmacologically acceptable salt or ester thereof.

**33.** A method according to any one of claims 21 to 27, wherein the LXR ligand is a compound represented by the general formula (If) described in claim 13 or a pharmacologically acceptable salt or ester thereof.

**34.** A method according to any one of claims 21 to 27, wherein the LXR ligand is a compound represented by the general formula (If) described in claim 14 or a pharmacologically acceptable salt or ester thereof.

**35.** A method according to any one of claims 21 to 27, wherein the LXR ligand is a compound represented by the general formula (If) described in claim 15 or a pharmacologically acceptable salt or ester thereof.

**36.** A method according to any one of claims 21 to 27, wherein the LXR ligand is a compound represented by the general formula (If) described in claim 16 or a pharmacologically acceptable salt or ester thereof.

**37.** A method according to any one of claims 21 to 27, wherein the LXR ligand is a compound represented by the general formula (If) described in claim 17 or a pharmacologically acceptable salt or ester thereof.

**38.** A method according to any one of claims 21 to 27, wherein the LXR ligand is a compound described in claim 18, or a pharmacologically acceptable salt or ester thereof.

**39.** A method according to any one of claims 21 to 27, wherein the LXR ligand is a compound described in claim 19 or a pharmacologically acceptable salt or ester thereof.

**40.** A method according to any one of claims 21 to 27, wherein the LXR ligand is a compound described in claim 20 or a pharmacologically acceptable salt or ester thereof.

**41.** A method according to any one of claims 21 to 40, wherein the warm-blooded animal is a human.

INTERNATIONAL SEARCH REPORT

International application No.
PCT/JP2005/012185

A. CLASSIFICATION OF SUBJECT MATTER
Int.Cl[7] A61K45/00, 9/10, 31/18, 31/404, 31/423, 31/517, 31/5377, A61P7/02, 9/00, 13/12, 35/00, 35/04, 43/00, C07D239/70, 239/90, 239/91, 261/20, 401/04, 401/06, 401/12, 403/04, 403/06, 403/12,

According to International Patent Classification (IPC) or to both national classification and IPC

B. FIELDS SEARCHED

Minimum documentation searched (classification system followed by classification symbols)
Int.Cl[7] A61K45/00, 9/10, 31/18, 31/404, 31/423, 31/517, 31/5377, A61P7/02, 9/00, 13/12, 35/00, 35/04, 43/00, C07D239/70, 239/90, 239/91, 261/20, 401/04, 401/06, 401/12, 403/04, 403/06, 403/12,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
BIOSIS(STN), CAplus(STN), EMBASE(STN), MEDLINE(STN), JICST(JOIS)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2003/106435 A1 (SANKYO CO., LTD.), 24 December, 2003 (24.12.03). Particularly, Abstract; Claims & AU 2003238157 A1 | 1-9,19,20<br>10-18 |
| Y<br>A | ELISAF, M., Effects of fibrates on serum metabolic parameters, Curr.Med.Res.Opin., 2002, Vol.18, No.5, pp.269-76, particularly, Abstract | 1-9,19,20<br>10-18 |
| P,X | TERASAKA, N. et al., Liver X receptor agonists inhibit tissue factor expression in macrophages, FEBS J, 2005, Vol.272, No.6, pp.1546-56 | 1-20 |

☐ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 July, 2005 (25.07.05) | 09 August, 2005 (09.08.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.
PCT/JP2005/012185

Continuation of A. CLASSIFICATION OF SUBJECT MATTER
(International Patent Classification (IPC))

Int.Cl[7] 409/04, 417/04, C07J9/00

(According to International Patent Classification (IPC) or to both national classification and IPC)

Continuation of B. FIELDS SEARCHED
Minimum documentation searched (International Patent Classification (IPC))

Int.Cl[7] 409/04, 417/04, C07J9/00

Minimum documentation searched (classification system followed by classification symbols)

Form PCT/ISA/210 (extra sheet) (January 2004)

INTERNATIONAL SEARCH REPORT

International application No.
PCT/JP2005/012185

**Box No. II Observations where certain claims were found unsearchable (Continuation of item 2 of first sheet)**

This international search report has not been established in respect of certain claims under Article 17(2)(a) for the following reasons:

1. [x] Claims Nos.: 21-41
because they relate to subject matter not required to be searched by this Authority, namely:
Claims 21 to 41 pertain to [methods for treatment of the human body by therapy] and thus relate to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02062302 A
- WO 03039480 A
- WO 03090746 A
- WO 0246141 A
- WO 03103651 A
- WO 03084544 A
- WO 02046181 A
- WO 02046172 A
- WO 02024632 A
- WO 2004009091 A
- WO 03031408 A
- WO 03045382 A
- WO 03053352 A
- WO 2004011448 A
- WO 03099769 A
- WO 03099775 A
- WO 03059874 A
- WO 03082192 A
- WO 03082802 A
- WO 03082205 A
- WO 0160818 A
- WO 0054759 A
- WO 03063796 A
- WO 03063576 A
- WO 03059884 A
- WO 0141704 A
- WO 03090869 A
- WO 2004024161 A
- WO 2004024162 A
- WO 2004026816 A
- WO 03090732 A
- WO 2004043939 A
- WO 2004072041 A
- WO 2004072042 A
- WO 02004072046 A
- WO 2004076418 A
- WO 2004103376 A
- WO 2005005416 A
- WO 2005005417 A
- WO 2005016277 A
- WO 2005023188 A
- WO 2005023196 A
- WO 2005023247 A
- US 20040152681 A
- WO 03106435 A
- WO 2005023782 A
- WO 2003106435 A
- JP 2005009142 W
- JP 2005146390 A
- JP 2005011928 W
- JP 2005189264 A
- JP 2005110908 A
- JP 2004311821 A
- JP 2005187686 A
- WO 2004072046 A
- WO 2000054759 A
- WO 1997028137 A
- WO 200224632 A
- US 4287341 A


### Non-patent literature cited in the description


- **T. H. GREENE ; P. G. WUTS.** Protective Groups in Organic Synthesis. John Wiley & Sons. Inc, 1999
- **T. H. GREENE ; P. G. WUTS.** Protective Groups in Organic Synthesis. John Wiley & Sons, Inc, 1999
- **R. C. LAROCK.** Comprehensive Organic Transformations. John Wiley & Sons, Inc, 1999
- **T. H. GREENE ; P. G. WUTS.** Protective Groups in Organic Synthesis. John Wiley & Sons,. Inc, 1999
- **J. TSUJI.** Palladium Reagents and Catalysis: New perspectives for the 21st Century. John Wiley & Sons, Inc, 2004
- **JACKSON, P. M.** *J. Chem. Soc, Perkin Trans. 1,* 1990, vol. 11, 2909-2918
- **THEODORA W. GREENE ; PETER G. M. WUTS.** Protective Groups in Organic Synthesis. John Wiley & Sons, Inc, 1991
- *Endocrinology,* 2002, vol. 143, 2548-2558
- *J. Med. Chem.,* 2001, vol. 44, 886-897
- **MILLER, J. A. et al.** *J. Org. Chem.,* 1993, vol. 58, 2637-2639
- **COREY, E. J. et al.** *J. Am. Chem. Soc.,* 1972, vol. 94, 6190-6191
- **WINKLE, M. R. et al.** *J. Org. Chem.,* 1982, vol. 47, 2101-2108
- **HARVISON, P. J. et al.** *J. Med. Chem.,* 1986, vol. 29, 1737-1743

- **JACKSON, P. M. et al.** *J. Chem. Soc. Perkin Trans. L.,* 1990, vol. 11, 2909-2918
- **ASKAM, V. et al.** *J. Chem. Soc. C,* 1969, 1935-1936
- **JAMES. R. et al.** *J. Med. Chem.,* 1980, vol. 23, 1350-1357
- **JACKSON, P. M. et al.** *J. Chem. Soc. Perkin Trans. 1.,* 1990, vol. 11, 2909-2918
- **HANESSIAN, S. et al.** *J. Org. Chem.,* 2003, vol. 68, 7204-7218

SEQUENCE LISTING

<110> Sankyo Company Limited

<120> Suppressant of production of tissue factor

<130> EPP96205 (FP0511)

<140> EP 05755860.3
<141> 2005-07-01

<150> JP 2004-196468
<151> 2004-07-02

<160> 6

<170> PatentIn version 3.3

<210> 1
<211> 20
<212> DNA
<213> artificial

<220>
<223> Primer TF-1

<400> 1
ggccaccatc tttatcatcc
20

<210> 2
<211> 20
<212> DNA
<213> artificial

<220>
<223> Primer TF-2

<400> 2
tgttcttccc tttctgtccc
20

<210> 3
<211> 30
<212> DNA
<213> artificial

<220>
<223> Ntd Sequence of Probe TF-P

<400> 3
ccatatctct gtgcaagcgc agaaagaacc
30

```
<210>  4
<211>  17
<212>  DNA
<213>  artificial

<220>
<223>  Primer Cyl-1

<400>  4
cgatgacgag cccttgg
17


<210>  5
<211>  23
<212>  DNA
<213>  artificial

<220>
<223>  Primer Cyl-2

<400>  5
tctgctgtct ttggaacttt gtc
23


<210>  6
<211>  24
<212>  DNA
<213>  artificial

<220>
<223>  Ntd Sequence of Probe Cyl-P

<400>  6
cgcgtctcct ttgagctgtt tgca
24
```